(19) Europäisches Patentamt
European Patent Office
Office européen des brevets

(11) EP 0 687 251 B1

(12) **FASCICULE DE BREVET EUROPEEN**

(45) Date de publication et mention de la délivrance du brevet:
**27.02.2002 Bulletin 2002/09**

(21) Numéro de dépôt: **95905164.0**

(22) Date de dépôt: **23.12.1994**

(51) Int Cl.[7]: **C07D 209/40**, A61K 31/40, C07D 209/34, C07D 405/12

(86) Numéro de dépôt international:
**PCT/FR94/01528**

(87) Numéro de publication internationale:
**WO 95/18105 (06.07.1995 Gazette 1995/29)**

# (54) DERIVES DE 1,3-DIHYDROINDOL-2-ONE SUBSTITUES EN 3 PAR UN GROUPE AZOTE COMME AGONISTES ET/OU ANTAGONISTES DE LA VASOPRESSINE ET/OU DE L'OCYTOCINE

IN POSITION 3 DURCH EINE STICKSTOFFGRUPPE SUBSTITUIERTE 1,3-DIHYDROINDOL-2-ON DERIVATE ALS VASOPRESSIN UND/ODER OCYTOCIN AGONISTEN UND/ODER ANTAGONISTEN

1,3-DIHYDROINDOL-2-ONE DERIVATIVES SUBSTITUTED IN POSITION 3 BY A NITROGEN GROUP AS VASOPRESSIN AND/OR OCYTOCINE AGONISTS AND/OR ANTAGONISTS

(84) Etats contractants désignés:
**AT BE CH DE DK ES FR GB GR IE IT LI LU MC NL PT SE**

(30) Priorité: **24.12.1993 FR 9315638**

(43) Date de publication de la demande:
**20.12.1995 Bulletin 1995/51**

(73) Titulaire: **SANOFI-SYNTHELABO**
**75013 Paris (FR)**

(72) Inventeurs:
- **WAGNON, Jean**
  **F-34000 Montpellier (FR)**
- **TONNERRE, Bernard**
  **F-34570 Vailhauques (FR)**
- **DI MALTA, Alain**
  **F-34980 Saint-Clément-de-Rivière (FR)**
- **ROUX, Richard**
  **F-34570 Vailhauques (FR)**
- **AMIEL, Marie-Sophie**
  **F-34470 Perols (FR)**
- **SERRADEIL-LEGAL, Claudine**
  **F-31750 Escalquens (FR)**

(74) Mandataire: **Gillard, Marie-Louise et al**
**Cabinet Beau de Loménie 158, rue de l'Université**
**75340 Paris Cédex 07 (FR)**

(56) Documents cités:
**EP-A- 0 469 984**
**WO-A-93/15051**
**FR-A- 1 509 373**

- **CHEMICAL ABSTRACTS, vol. 100, no. 5, 30 Janvier 1984, Columbus, Ohio, US; abstract no. 34367y, BOLOTOV,V.V. ET AL. 'Synthesis and ...' & KHIM.-FARM. ZH. 1983.17(9),1060-3**
- **CHEMICAL ABSTRACTS, vol. 100, no. 3, 16 Janvier 1984, Columbus, Ohio, US; abstract no. 22860r, TEUBER, HANS JOACHIM ET AL. 'Rearrangement of ...' & LIEBIGS ANN. CHEM. 1983,(10),1744-59**
- **CHEMICAL ABSTRACTS, vol. 92, no. 13, 31 Mars 1980, Columbus, Ohio, US; abstract no. 110770h, BOLOTOV, V.V. ET AL. 'Synthesis and ...' & KHIM.-FARM. ZH. 1979,13(11),45-9**
- **CHEMICAL ABSTRACTS, vol. 85, no. 11, 13 Septembre 1976, Columbus, Ohio, US; abstract no. 77930r, WESTON,GEORGE O. 'Substituted N-...' & DE,A,25 53 662 (WYETH, JOHN, AND BROTHER LTD.)**
- **CHEMICAL ABSTRACTS, vol. 78, no. 13, 2 Avril 1973, Columbus, Ohio, US; abstract no. 84223w, WALSER,A. ET AL. 'Cyclization ...' & J. ORG. CHEM. 1973,38(3),449-56**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

- **CHEMICAL ABSTRACTS, vol. 69, no. 25, 16 Décembre 1968, Columbus, Ohio, US; abstract no. 106550u, '3-Aryl ....' & GB,A,1 125 671 (PARKE,DAVIS AND CO.) 28 Août 1968**

Remarques:

Le dossier contient des informations techniques présentées postérieurement au dépôt de la demande et ne figurant pas dans le présent fascicule.

## Description

La présente invention a pour objet des dérivés de 1,3-dihydroindol-2-one substitués en -3- par un groupe azoté, leur préparation et les compositions pharmaceutiques en contenant.

Les composés selon la présente invention ont une affinité pour les récepteurs de la vasopressine et/ou de l'ocytocine.

La vasopressine est une hormone connue pour son effet antidiurétique et son effet dans la régulation de la pression artérielle. Elle stimule plusieurs types de récepteurs : $V_1$ ($V_{1a}$, $V_{1b}$), $V_2$. Ces récepteurs sont localisés dans le foie, les vaisseaux (coronaires, rénaux, cérébraux), les plaquettes, le rein, l'utérus, les glandes surrénales, le système nerveux central, l'hypophyse. L'ocytocine a une structure peptidique proche de celle de la vasopressine. Les récepteurs de l'ocytocine se trouvent aussi sur le muscle lisse de l'utérus, ils se trouvent également sur des cellules myoépithéliales de la glande mammaire, dans le système nerveux central et dans le rein. La localisation des différents récepteurs est décrite dans : S. JARS et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants : Presse Médicale, 1987, 16 (10), 481-485, J. Lab. Clin. Med., 1989, 114 (6), 617-632 et Pharmacol. Rev., 1991, 43 (1), 73-108. La vasopressine exerce ainsi des effets cardiovasculaires, hépatiques, antidiurétiques, agrégants et des effets sur le système nerveux central et périphérique, et sur la sphère utérine. L'ocytocine intervient dans la parturition, la lactation et le comportement sexuel.

Les composés selon la présente invention permettent soit de mimer les effets de l'hormone (pour les agonistes), soit de les inhiber (pour les antagonistes), de façon sélective. Les antagonistes des récepteurs de la vasopressine peuvent intervenir sur la régulation de la circulation centrale et périphérique, notamment les circulations coronaire, rénale et gastrique, ainsi que sur la régulation hydrique et la libération de l'hormone adrénocorticotrophique (ACTH). Les agonistes de la vasopressine peuvent remplacer avantageusement la vasopressine ou ses analogues dans le traitement du diabète insipide ; ils peuvent également être utilisés dans le traitement de l'énurésie, et dans la régulation de l'hémostase : traitement de l'hémophilie, du syndrome de Von Willebrand, antidote des agrégants plaquettaires, F. A. LASZLO, , Pharmacol. Rev., 1991, 43, 73-108. Drug Investigation, 1990, 2 (Suppl. 5), 1-47. Les hormones elles-mêmes : la vasopressine et l'ocytocine ainsi que certains de leurs analogues peptidiques ou non peptidiques sont utilisés en thérapeutique et ont montré leur efficacité. On peut citer plusieurs revues et de nombreux articles de la littérature Vasopressin : P. GROSS et al. ed. John Libbey Eurotext, 1993, en particulier 243-257 et 549-562. F.A. LASZLO and F.A. LASZLO Jr., Clinical perspectives for vasopressin antagonists, Drug News Perspect., 1993, 6 (8) ; W.G. NORTH, J. Clin. Endocrinol., 1991, 73, 1316-1320. J.J. LEGROS et al., Prog. Neuro-Pharmcol. Biol. Psychiat., 1988, 12, 571-586 ; K.E. ANDERSSON et al., Drugs Today, 1988, 24 (7) 509-528 ; D.L. STUMP et al., Drugs, 1990, 39, 38-53 ; S. CALTABIANO et al., Drugs Future, 1988, 13, 25-30 ; Y. MURA et al., Clin. Nephrol. 1993, 40, 60-61 ; Faseb J., 1994, 8 (5), A 587 : 3398.

Ainsi les composés selon l'invention sont utiles notamment dans le traitement des affections du système nerveux central et périphérique, du système cardiovasculaire, de la sphère rénale, de la sphère gastrique et dans les troubles du comportement sexuel, chez l'homme et chez l'animal.

Le brevet ZA 83 09532 décrit notamment, un dérivé 1,3-dihydroindol-2-one de formule:

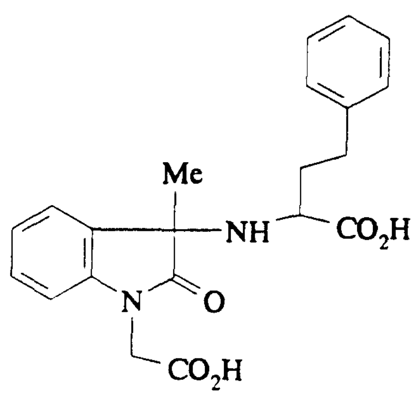

1

Ce composé est utile comme inhibiteur d'enzyme de conversion et comme agent antihypertenseur.

La demande de brevet GB 1 125 671 décrit des dérivés de 3-amino-3-arylindolone de formule :

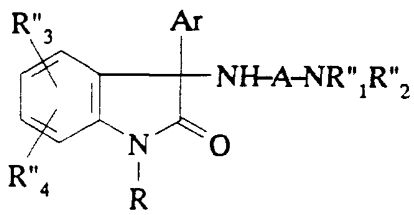

2

dans laquelle :

- R représente l'hydrogène, un groupement alkyle en $C_1$-$C_4$ ou dialkylamino en $C_1$-$C_4$;
- A représente un alkylène en $C_2$-$C_5$ ;
- R"$_1$ et R"$_2$ représentent chacun un alkyle ou ensemble avec l'atome d'azote auquel ils sont liés représentent un radical hétérocyclique comme le radical pipéridino, pyrrolidino ou morpholino par exemple ;
- R"$_3$ et R"$_4$ représentent chacun l'hydrogène, un alkyle en $C_1$-$C_4$, un alcoxy en $C_1$-$C_4$ ou un halogène ;
- Ar représente un benzyle ou un phényle éventuellement substitué.

Ces composés sont utiles comme agents diurétiques.

Récemment plusieurs demandes de brevet ont décrit des familles de composés à structure non peptidique ayant une activité sur les récepteurs de la vasopressine et/ou de l'ocytocine. On peut citer les demandes EP 382 185, EP 444 945, EP 514 667, EP 469 984 et EP 526 348, les demandes WO 91/05549 et WO 93/15051 et plus particulièrement la demande de brevet JP-03/127732. Cette dernière décrit des dérivés de l'acide indole-3-propanoïque de formule :

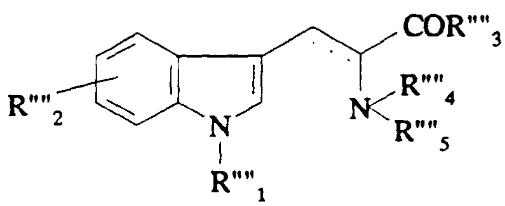

3

dans laquelle :

- R""$_1$ représente l'hydrogène, un alkyle, un alcényle, un phénylalkyle, un tétrahydrofuryle, un alcoxycarbonyle, un alcoxycarbonylalkyle, un carboxyalkyle

ou un alcanoyle ;

- R""$_2$ représente l'hydrogène, un hydroxyle, un alcoxy, un alkyle, un phénylalkyle, un phénylalcoxy ou un halogène ;
- R""$_3$ représente un hydrogène, un alcoxy, un groupe amino libre ou substitué ou un résidu d'aminoacide ;
- R""$_4$ représente l'hydrogène, un alkyle ou un phénylalkyle ;
- R""$_5$ représente un benzoyle, un phényle, un alkyle, un phénylalcénylcarbonyle, un thiénylcarbonyle, un phénylsulfonyle, un pyridylcarbonyle ou un imidazolylcarbonyle, les groupes phényle et alkyle du substituant R""$_5$ pouvant être substitués.

Ces composés sont des antagonistes de la vasopressine.

Le brevet US 4 803 217 revendique des hapalindolinones obtenues par fermentation qui sont des antagonistes de la vasopressine. Ces composés sont représentés par la formule suivante :

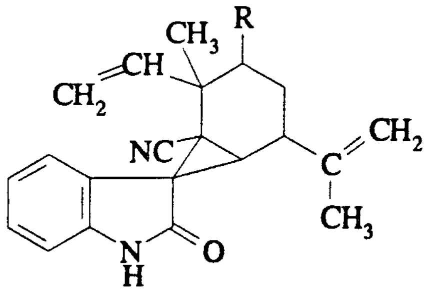

4

dans laquelle R représente H ou Cl.

Selon l'un de ses aspects la présente invention a pour objet des composés de formule :

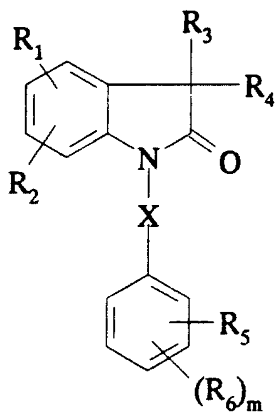

(I)

dans laquelle :

- $R_1$ et $R_2$ représentent chacun indépendamment un hydrogène ; un halogène ; un ($C_1$-$C_7$)alkyle ; un ($C_1$-$C_7$)alcoxy ; un trifluorométhyle ;
- $R_3$ représente un ($C_1$-$C_7$)alkyle ; un ($C_3$-$C_7$)cycloalkyle ; un cyclohexyle substitué par un ou deux ($C_1$-$C_4$)alkyles ; un cyclohexylméthyle ; un phényle non substitué ou substitué une ou plusieurs fois par un halogène, un ($C_1$-$C_7$) alkyle, un hydroxy, un ($C_1$-$C_7$)alcoxy, un benzyloxy, un acétoxy ; un benzyle non substitué ou substitué une ou plusieurs fois par un halogène, un ($C_1$-$C_7$)alkyle, un hydroxy, un ($C_1$-$C_7$)alcoxy, un benzyloxy, un acétoxy ;
- $R_4$ représente un azido ; un groupe 2,2-diméthylhydrazino; un ($C_1$-$C_7$)alkylsulfonamido ; un phénylsulfonamido dans lequel le phényle est non substitué ou substitué une ou plusieurs fois par un halogène, un ($C_1$-$C_7$)alkyle, un hydroxy, un ($C_1$-$C_7$)alcoxy, un benzyloxy, un acétoxy ; un diméthylaminosulfonamido ; un groupe $NR_7R_8$ ; un groupe $NR_9R_{10}$ ; un groupe $NR_9R_{11}$ ; un radical hétérocyclique $R_{12}$ ; un pipérazin-1-yle substitué en position 4 par un groupe ($C_2$-$C_{10}$)alkylène substitué par un groupe amino libre ou portant un groupe protecteur ; un groupe lactoylamino ; un groupe mandéloylamino ; un groupe N'-(1-phényléthyl)uréido ; un groupe N'-(1-napht-1-yléthyl) uréido ;
- $R_5$ représente l'hydrogène ou l'une des valeurs désignées pour $R_6$ ;
- $R_6$ représente un halogène ; un ($C_1$-$C_7$)alkyle; un trifluorométhyle ; un cyano ; un aminométhyle dans lequel l'amino est libre ou substitué par un ou deux ($C_1$-$C_7$)alkyles ; un nitro ; un groupe $NR_9R_{11}$ ; un radical hétérocyclique choisi parmi le pyrrol-1-yle, le Δ3-pyrrolin-1-yle, la pyrrolidin-1-yle, la morpholin-4-yle ; un groupe $OR_{13}$ ; un groupe $SR_{13}$ ; un guanidino non substitué ou substitué en 3 par un ou deux ($C_1$-$C_7$)alkyles, un phényle, un benzyle ; un formyle ; un ($C_1$-$C_7$)alkylcarbonyle ; un carbamoyle substitué par $R_{14}$ et $R_{15}$ ; un thiocarbamoyle libre ou substitué par un ou deux ($C_1$-$C_7$)alkyles ; un sulfamoyle substitué par $R_{16}$ et $R_{17}$ ; un carboxy ; un ($C_1$-$C_7$)alcoxycarbonyle ; un phénoxycarbonyle ; un benzyloxycarbonyle ; un ($C_1$-$C_7$)alkylsulfonamido ; un phénylsulfonamido dans lequel le phényle est non substitué ou substitué une ou plusieurs fois par un halogène, un ($C_1$-$C_7$)alkyle, un hydroxy, un ($C_1$-$C_7$)alcoxy, un benzyloxy, un acétoxy ; un diméthylaminosulfonamido ; un 2-(4-méthylphényl)benzamido ;
- ou $R_5$ et $R_6$ ensemble avec le phényle auquel ils sont liés constituent un groupe :

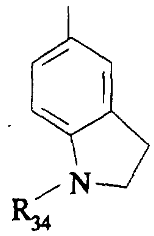

à la condition que X soit $-SO_2-$ ;

- $R_7$ représente un $(C_1-C_7)$alcoxycarbonyle ;
- $R_8$ représente un $(C_1-C_7)$alcoxycarbonylamino ; un N-méthyl-N-$(C_1-C_7)$alcoxycarbonylamino ;
- $R_9$ représente un hydrogène ; un $(C_1-C_7)$alkyle ;
- $R_{10}$ représente un groupe $CR_{18}R_{19}R_{20}$ ; un groupe $(CH_2)_pR_{35}$ ; un $(C_2-C_{10})$alkylène substitué par $R_{21}$ ; un groupe $-CH_2CN$ ; un groupe $C(CH_3)(CH_2OH)_2$ ou $C(CH_2OH)_3$ ; un radical carbocyclique non aromatique en $C_3-C_{15}$ ; un phényle non substitué ou substitué une ou plusieurs fois par un halogène, un $(C_1-C_7)$alkyle, un hydroxy, un $(C_1-C_7)$alcoxy, un benzyloxy, un acétoxy, un nitro, un amino libre ou substitué par un ou deux $(C_1-C_7)$alkyles ; un benzyle dans lequel le phényle est non substitué ou substitué une ou plusieurs fois par un halogène, un $(C_1-C_7)$alkyle, un hydroxy, un $(C_1-C_7)$alcoxy, un benzyloxy, un acétoxy, un nitro, un amino libre ou substitué par un ou deux $(C_1-C_7)$alkyles ; un phénéthyle dans lequel le phényle est non substitué ou substitué une ou plusieurs fois par un halogène, un $(C_1-C_7)$alkyle, un hydroxy, un $(C_1-C_7)$alcoxy, un benzyloxy, un acétoxy, un nitro, un amino libre ou substitué par un ou deux $(C_1-C_7)$alkyles ; un phénéthyle dans lequel le phényle est substitué par un 3,4-méthylènedioxy ou un 3,4-éthylènedioxy ;
- $R_{11}$ représente un hydrogène ; un $(C_1-C_{12})$alkyle ; un $(C_3-C_7)$cycloalkylméthyle ; un groupe $OR_{13}$ ; un formyle ; un $(C_1-C_7)$alkylcarbonyle ; un $(C_1-C_7)$alkylthiocarbonyle ; un $(C_3-C_7)$cycloalkylcarbonyle ; un $(C_3-C_7)$cycloalkylthiocarbonyle ; un benzoyle non substitué ou substitué une ou plusieurs fois par un halogène, un $(C_1-C_7)$alkyle, un hydroxy, un $(C_1-C_7)$alcoxy, un benzyloxy, un acétoxy ; un phénylacétyle dans lequel le noyau benzénique est non substitué ou substitué une ou plusieurs fois par un halogène, un $(C_1-C_7)$alkyle, un hydroxy, un $(C_1-C_7)$alcoxy, un benzyloxy, un acétoxy ; un pyridylcarbonyle ; un thiénylcarbonyle ; un furylcarbonyle ; un pipérid-4-ylcarbonyle non substitué

ou substitué en 1 par un $(C_1-C_7)$alkyle ou par un groupe protecteur ; un $(C_1-C_7)$alcoxycarbonyle ; un phénoxycarbonyle ; un phénoxythiocarbonyle ; un benzyloxycarbonyle ; un carbamoyle substitué par $R_{22}$ et $R_{23}$ ; un thiocarbamoyle substitué par $R_{22}$ et $R_{23}$ ; un groupe $CH_2R_{36}$ ; un ω-$R_{24}$ $(C_1-C_6)$alkylcarbonyle ; un ω-$R_{32}R_{33}N(C_1-C_4)$alkylcarbonyle ;

- R12 représente un morpholin-4-yle ; un thiomorpholin-4-yle ; un azétidin-1-yle non substitué ou substitué en 2 par un carboxy ou substitué en 3 par un amino libre ou portant un groupe protecteur ; une perhydroazépin-1-yle ; une pipérazin-1-yle non substituée ou substituée en 4 par $R_{25}$ ; un pipérid-1-yle non substitué ou substitué par $R_{26}$ ; une pyrrolidin-1-yle non substituée ou substituée par $R_{27}$ ; une thiazolidin-3-yle non substituée ou substituée par $R_{27}$;
- $R_{13}$ représente un hydrogène ; un $(C_1-C_7)$alkyle ; un benzyle ; un allyle ; un tétrahydropyran-2-yle ;
- $R_{14}$ et $R_{15}$ représentent chacun indépendamment l'hydrogène ; un $(C_1-C_7)$alkyle ; $R_{15}$ peut de plus représenter un $(C_1-C_7)$alkylène substitué par $R_{24}$, un cyano, un trifluorométhyle, un amino libre ou substitué par un ou deux $(C_1-C_7)$alkyles ; un $(C_3-C_7)$cycloalkyle ; un phényle non substitué ou substitué une ou plusieurs fois par un halogène, un $(C_1-C_7)$alkyle, un hydroxy, un $(C_1-C_7)$alcoxy, un benzyloxy ; un groupe $R_{28}$ ;
- ou $R_{14}$ et $R_{15}$ ensemble avec l'atome d'azote auquel ils sont liés constituent un radical hétérocyclique $R_{12}$ ;
- $R_{16}$ et $R_{17}$ représentent chacun indépendamment un hydrogène, un $(C_1-C_7)$alkyle ; $R_{17}$ peut de plus représenter un $(C_3-C_7)$cycloalkyle ou un groupe $R_{28}$ ;
- ou $R_{16}$ et $R_{17}$ ensemble avec l'atome d'azote auquel ils sont liés constituent un radical hétérocyclique $R_{12}$ ;
- $R_{18}$ représente un $(C_1-C_7)$alkyle ; un phényle ; un benzyle ; un cyclohexylméthyle ; un phénéthyle ; un imidazol-4-ylméthyle libre ou portant un groupe protecteur ; un indol-3-ylméthyle libre ou portant un groupe protecteur ; un hydroxyméthyle libre ou portant un groupe protecteur ; un 2-hydroxyéthyle libre ou portant un groupe protecteur ; un 1-hydroxyéthyle libre ou portant un groupe protecteur ; un 4-hydroxybenzyle libre ou portant un groupe protecteur ; un mercaptométhyle libre ou portant un groupe protecteur ; un 2-mercaptoéthyle libre ou portant un groupe protecteur ; un 2-méthylthioéthyle ; un 2-méthylsulfinyléthyle ; un 2-méthylsulfonyléthyle ; un 4-aminobutyle libre ou portant un groupe protecteur ; un 3-aminopropyle libre ou portant un groupe protecteur ; un carboxyméthyle libre ou portant un groupe protecteur ; un 2-carboxyéthyle libre ou portant un groupe protecteur ; un

carbamoylméthyle ; un 2-carbamoyléthyle ; un 3-guanidinopropyle libre ou portant un groupe protecteur ; un radical carbocyclique non aromatique en $C_3$-$C_{15}$ ;

- $R_{19}$ représente l'hydrogène ; $R_{19}$ peut de plus représenter un ($C_1$-$C_7$)alkyle lorsque $R_{18}$ représente un ($C_1$-$C_7$) alkyle ;
- ou $R_{18}$ et $R_{19}$ ensemble avec l'atome de carbone auquel ils sont liés constituent un radical carbocyclique non aromatique en $C_3$-$C_{15}$ ;
- $R_{20}$ représente $R_{24}$ ; un groupe $CH_2OR_{13}$ ; un aminométhyle dans lequel l'amino est libre ou substitué par un ou deux ($C_1$-$C_7$)alkyles ou par un groupe protecteur ;
- $R_{21}$ représente $R_{36}$ ; un groupe $OR_{37}$ ; un groupe $NR_{32}R_{33}$ ; un cyano ; un groupe -S($C_1$-$C_7$)alkyle ; un groupe -SO($C_1$-$C_7$)alkyle ; un groupe
- $SO_2$($C_1$-$C_7$)alkyle ;
- $R_{22}$ et $R_{23}$ représentent chacun indépendamment un hydrogène ; un ($C_1$-$C_7$)alkyle ; et $R_{23}$ peut de plus représenter un ($C_3$-$C_7$)cycloalkyle ; un groupe $CR_{18}R_{19}R_{20}$ ; un groupe $CH_2R_{24}$ ; un groupe $C(CH_3)(CH_2OH)_2$ ou $C(CH_2OH)_3$ ; un ($C_2$-$C_6$)alkylène substitué par $R_{29}$ ;
- ou $R_{22}$ et $R_{23}$ ensemble avec l'atome d'azote auquel ils sont liés constituent un radical hétérocyclique $R_{12}$ ou une cis-2,6-diméthylpipérid-1-yle ;
- $R_{24}$ représente un carboxy ; un ($C_1$-$C_7$)alcoxycarbonyle ; un benzyloxycarbonyle ; un carbamoyle libre ou substitué par un ou deux ($C_1$-$C_7$)alkyles ;
- $R_{25}$ représente un ($C_1$-$C_7$)alkyle ; un phényle ; un benzyle ; un formyle ; un ($C_1$-$C_7$)alkylcarbonyle ; un ($C_1$-$C_7$) alcoxycarbonyle ; un benzyloxycarbonyle ;
- $R_{26}$ représente $R_{24}$ ; un amino libre ou substitué par un ou deux ($C_1$-$C_7$)alkyles ou par un groupe protecteur ; un groupe $OR_{13}$ ; un groupe $CH_2OR_{13}$ ;
- $R_{27}$ représente $R_{24}$, un groupe $CH_2R_{24}$, un groupe $CH_2OR_{13}$, un aminométhyle dans lequel l'amino est libre ou substitué par un ou deux ($C_1$-$C_7$)alkyles ou par un groupe protecteur ;
- $R_{28}$ représente un groupe $CH(CH_2OH)_2$ ou $CH(CH_3)CH_2OH$ ou $C(CH_3)(CH_2OH)_2$ ou $C(CH_3)_2CH_2OH$ ou $C(CH_2OH)_3$ ou $CH_2CH_2OH$ ;
- $R_{29}$ représente un groupe $R_{24}$, un groupe $OR_{13}$, un groupe $NR_{30}R_{31}$ ;
- $R_{30}$ et $R_{31}$ représentent chacun indépendamment un hydrogène, un ($C_1$-$C_7$)alkyle;
- ou $R_{30}$ et $R_{31}$ ensemble avec l'atome d'azote auquel ils sont liés constituent un radical hététocyclique $R_{12}$;
- $R_{32}$ et $R_{33}$ représentent chacun indépendamment un hydrogène ; un ($C_1$-$C_7$)alkyle ; et $R_{33}$ peut de plus représenter un acétyle ; un phényle ; un benzyle ; un ($C_1$-$C_7$)alcoxycarbonyle ; un benzyloxycarbonyle ; un ($C_1$-$C_6$)alkylène substitué par $R_{24}$ ; un ($C_2$-$C_6$)alkylène substitué par un hydroxy ou un ($C_1$-$C_7$)alcoxy ; un ($C_2$-$C_6$)alkylène substitué par un amino libre ou substitué par un ou deux ($C_1$-$C_7$)alkyles ou par un groupe protecteur ;
- ou $R_{32}$ et $R_{33}$ ensemble avec l'atome d'azote auquel ils sont liés constituent un radical hétérocyclique $R_{12}$ ;
- $R_{34}$ représente un formyle ; un ($C_1$-$C_7$)alkylcarbonyle ; un ($C_1$-$C_7$)alcoxycarbonyle ; un phénoxycarbonyle ; un carbamoyle substitué par deux ($C_1$-$C_7$)alkyles ; un groupe $COR_{12}$ ;
- $R_{35}$ représente un pipérid-4-yle non substitué ou substitué en position 1 par un ($C_1$-$C_7$)alcoxycarbonyle ou par un ($C_1$-$C_7$)alkyle ; un pyrid-2-yle ;
- $R_{36}$ représente un carboxy ; un ($C_1$-$C_7$)alcoxycarbonyle ; un benzyloxycarbonyle ; un carbamoyle libre ou substitué par $R_{38}$ et $R_{39}$ ;
- $R_{37}$ représente $R_{13}$ ; un ($C_3$-$C_7$)cycloalkyle ; un ($C_1$-$C_6$)alkylène substitué par $R_{24}$ ; un ($C_2$-$C_6$)alkylène substitué par un hydroxy ou un ($C_1$-$C_7$)alcoxy ; un ($C_2$-$C_6$)alkylène substitué par un amino libre ou substitué par un ou deux ($C_1$-$C_7$)alkyles ou par un groupe protecteur;
- $R_{38}$ et $R_{39}$ représentent chacun indépendamment un hydrogène ; un ($C_1$-$C_7$)alkyle ; $R_{39}$ peut de plus représenter un ($C_1$-$C_6$)alkylène substitué par $R_{24}$ ; un ($C_2$-$C_6$)alkylène substitué par un hydroxy ou un ($C_1$-$C_7$)alcoxy ; un ($C_2$-$C_6$)alkylène substitué par un amino libre ou substitué par un ou deux ($C_1$-$C_7$)alkyles ou par un groupe protecteur.
- X représente $SO_2$ ; $CH_2$ ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi : halogène ; ($C_1$-$C_7$) alkyle ; ($C_1$-$C_7$)alcoxy ;
- p représente un nombre entier qui peut varier de 0 à 3;

ainsi que leurs sels éventuels.

Avantageusement, l'invention concerne lès composés de formule (I) dans laquelle:

- $R_1$ et $R_2$ représentent chacun indépendamment un hydrogène ; un halogène ; un ($C_1$-$C_7$)alkyle ; un ($C_1$-$C_7$)alcoxy ; un trifluorométhyle ;

- $R_3$ représente un ($C_1$-$C_7$)alkyle ; un ($C_3$-$C_7$)cycloalkyle ; un cyclohexyle substitué par un ou deux ($C_1$-$C_4$)alkyles ; un phényle non substitué ou substitué une ou plusieurs fois par un halogène, un ($C_1$-$C_7$)alkyle, un hydroxy, un ($C_1$-$C_7$)alcoxy, un benzyloxy, un acétoxy ;
- $R_4$ représente un azido ; un ($C_1$-$C_7$)alkylsulfonamido ; un phénylsulfonamido dans lequel le phényle est non substitué ou substitué une ou plusieurs fois par un halogène, un ($C_1$-$C_7$)alkyle, un hydroxy, un ($C_1$-$C_7$)alcoxy, un benzyloxy, un acétoxy ; un diméthylaminosulfonamido ; un groupe $NR_7R_8$ ; un groupe $NR_9R_{10}$ ; un groupe $NR_9R_{11}$ ; un radical hétérocyclique $R_{12}$ ; un groupe lactoylamino; un groupe mandéloylamino ; un groupe N'-(1-phényléthyl) uréido; un groupe N'-(1-napht-1-yléthyl)uréido ;
- $R_5$ représente l'hydrogène ou l'une des valeurs désignées pour $R_6$ ;
- $R_6$ représente un halogène ; un ($C_1$-$C_7$)alkyle ; un trifluorométhyle ; un cyano ; un aminométhyle dans lequel l'amino est libre ou substitué par un ou deux ($C_1$-$C_7$)alkyles ; un nitro ; un groupe $NR_9R_{11}$ ; un radical hétérocyclique choisi parmi le pyrrol-1-yle, le Δ3-pyrrolin-1-yle, la pyrrolidin-1-yle, la morpholin-4-yle ; un groupe $OR_{13}$ ; un groupe $SR_{13}$ ; un guanidino non substitué ou substitué en 3 par un ou deux ($C_1$-$C_7$)alkyles, un phényle, un benzyle ; un formyle ; un ($C_1$-$C_7$)alkylcarbonyle ; un carbamoyle substitué par $R_{14}$ et $R_{15}$ ; un thiocarbamoyle libre ou substitué par un ou deux ($C_1$-$C_7$)alkyles ; un sulfamoyle substitué par $R_{16}$ et $R_{17}$ ; un carboxy ; un ($C_1$-$C_7$)alcoxycarbonyle ; un phénoxycarbonyle ; un benzyloxycarbonyle ; un ($C_1$-$C_7$)alkylsulfonamido ; un phénylsulfonamido dans lequel le phényle est non substitué ou substitué une ou plusieurs fois par un halogène, un ($C_1$-$C_7$)alkyle, un hydroxy, un ($C_1$-$C_7$)alcoxy, un benzyloxy, un acétoxy ; un diméthylaminosulfonamido ;
- $R_7$ représente un ($C_1$-$C_7$)alcoxycarbonyle ;
- $R_8$ représente un ($C_1$-$C_7$)alcoxycarbonylamino ; un N-méthyl-N-($C_1$-$C_7$)alcoxycarbonylamino ;
- $R_9$ représente un hydrogène ; un ($C_1$-$C_7$)alkyle ;
- $R_{10}$ représente un groupe $CR_{18}R_{19}R_{20}$ ; un groupe $(CH_2)_tR_{21}$ ; un groupe $C(CH_3)(CH_2OH)_2$ ou $C(CH_2OH)_3$ ; un radical carbocyclique non aromatique en $C_3$-$C_{15}$ ; un phényle non substitué ou substitué une ou plusieurs fois par un halogène, un ($C_1$-$C_7$)alkyle, un hydroxy, un ($C_1$-$C_7$)alcoxy, un benzyloxy, un acétoxy ; un benzyle dans lequel le phényle est non substitué ou substitué une ou plusieurs fois par un halogène, un ($C_1$-$C_7$)alkyle, un hydroxy, un ($C_1$-$C_7$)alcoxy, un benzyloxy, un acétoxy ; un phénéthyle dans lequel le phényle est non substitué ou substitué une ou plusieurs fois par un halogène, ($C_1$-$C_7$)un alkyle, un hydroxy, un ($C_1$-$C_7$)alcoxy, un benzyloxy, un acétoxy ;
- $R_{11}$ représente un hydrogène ; un ($C_1$-$C_{12}$)alkyle ; un ($C_3$-$C_7$)cycloalkylméthyle ; un groupe $OR_{13}$ ; un formyle ; un ($C_1$-$C_7$)alkylcarbonyle ; un ($C_1$-$C_7$)alkylthiocarbonyle ; un ($C_3$-$C_7$)cycloalkylcarbonyle ; un ($C_3$-$C_7$) cycloalkylthiocarbonyle ; un benzoyle non substitué ou substitué une ou plusieurs fois par un halogène, un ($C_1$-$C_7$) alkyle, un hydroxy, un ($C_1$-$C_7$)alcoxy, un benzyloxy, un acétoxy ; un phénylacétyle dans lequel le noyau benzénique est non substitué ou substitué une ou plusieurs fois par un halogène, un ($C_1$-$C_7$)alkyle, un hydroxy, un ($C_1$-$C_7$) alcoxy, un benzyloxy, un acétoxy ; un pyridylcarbonyle ; un thiénylcarbonyle ; un furylcarbonyle ; un pipérid-4-ylcarbonyle non substitué ou substitué en 1 par un ($C_1$-$C_7$)alkyle ou par un groupe protecteur ; un ($C_1$-$C_7$) alcoxycarbonyle ; un phénoxycarbonyle ; un phénoxythiocarbonyle ; un benzyloxycarbonyle ; un carbamoyle substitué par $R_{22}$ et $R_{23}$ ; un thiocarbamoyle substitué par $R_{22}$ et $R_{23}$ ; un groupe $CH_2R_{24}$ ; un ω-$R_{24}$ ($C_1$-$C_6$) alkylcarbonyle ; un ω-amino($C_1$-$C_4$)alkylcarbonyle dans lequel l'amino est libre ou substitué par un ou deux alkyles en $C_1$-$C_7$ ou par un groupe protecteur ;
- $R_{12}$ représente un morpholin-4-yle ; un thiomorpholin-4-yle ; un azétidin-1-yle non substitué ou substitué en 2 par un carboxy ou substitué en 3 par un amino libre ou portant un groupe protecteur ; une perhydroazépin-1-yle ; une pipérazin-1-yle non substituée ou substituée en 4 par $R_{25}$ ; une pipérid-1-yle non substituée ou substituée par $R_{26}$ ; une pyrrolidin-1-yle non substituée ou substituée par $R_{27}$ ; une thiazolidin-3-yle non substituée ou substituée par $R_{27}$ ;
- $R_{13}$ représente un hydrogène ; un ($C_1$-$C_7$)alkyle ; un benzyle ; un allyle ; un tétrahydropyran-2-yle ;
- $R_{14}$ et $R_{15}$ représentent chacun indépendamment l'hydrogène ; un ($C_1$-$C_7$)alkyle ; $R_{15}$ peut de plus représenter un ($C_1$-$C_7$)alkyle substitué par un cyano, un trifluorométhyle, un amino libre ou substitué par un ou deux ($C_1$-$C_7$) alkyles ; un ($C_3$-$C_7$)cycloalkyle ; un phényle non substitué ou substitué une ou plusieurs fois par un halogène, un ($C_1$-$C_7$)alkyle, un hydroxy, un ($C_1$-$C_7$)alcoxy, un benzyloxy ; un groupe $R_{28}$ ;
- ou $R_{14}$ et $R_{15}$ ensemble avec l'atome d'azote auquel ils sont liés constituent un radical hétérocyclique $R_{12}$ ;
- $R_{16}$ et $R_{17}$ représentent chacun indépendamment un hydrogène, un ($C_1$-$C_7$)alkyle ; $R_{17}$ peut de plus représenter un ($C_3$-$C_7$)cycloalkyle ou un groupe $R_{28}$ ;
- ou $R_{16}$ et $R_{17}$ ensemble avec l'atome d'azote auquel ils sont liés constituent un radical hétérocyclique $R_{12}$ ;
- $R_{18}$ représente un ($C_1$-$C_7$)alkyle ; un phényle ; un benzyle ; un cyclohexylméthyle ; un phénéthyle ; un imidazol-4-ylméthyle libre ou portant un groupe protecteur ; un indol-3-ylméthyle libre ou portant un groupe protecteur ; un hydroxyméthyle libre ou portant un groupe protecteur ; un 2-hydroxyéthyle libre ou portant un groupe protecteur ; un 1-hydroxyéthyle libre ou portant un groupe protecteur ; un 4-hydroxybenzyle libre ou portant un groupe protecteur ; un mercaptométhyle libre ou portant un groupe protecteur ; un 2-mercaptoéthyle libre ou portant un groupe protecteur ; un 2-méthylthioéthyle ; un 2-méthylsulfinyléthyle ; un 2-méthylsulfonyléthyle ; un 4-aminobutyle libre ou portant un groupe protecteur ; un 3-aminopropyle libre ou portant un groupe protecteur ; un carboxyméthyle libre ou portant un groupe protecteur ; un 2-carboxyéthyle libre ou portant un groupe protecteur ; un carbamoylméthyle ; un 2-carbamoyléthyle ; un 3-guanidinopropyle libre ou portant un groupe protecteur ; un radical carbocyclique non aromatique en $C_3$-$C_{15}$ ;

- $R_{19}$ représente l'hydrogène ; $R_{19}$ peut de plus représenter un ($C_1$-$C_7$)alkyle lorsque $R_{18}$ représente un ($C_1$-$C_7$) alkyle ;
- ou $R_{18}$ et $R_{19}$ ensemble avec l'atome de carbone auquel ils sont liés constituent un carbocycle non aromatique en $C_3$-$C_{15}$ ;
- $R_{20}$ représente $R_{24}$ ; un groupe $CH_2OR_{13}$ ; un aminométhyle dans lequel l'amino est libre ou substitué par un ou deux ($C_1$-$C_7$)alkyles ou par un groupe protecteur ;
- $R_{21}$ représente $R_{24}$ ; un groupe $OR_{13}$ ; un groupe $NR_{32}R_{33}$ ; un cyano ;
- $R_{22}$ et $R_{23}$ représentent chacun indépendamment un hydrogène ; un ($C_1$-$C_7$)alkyle ; et $R_{23}$ peut de plus représenter un ($C_3$-$C_7$)cycloalkyle ; un ($C_2$-$C_3$)alkylène substitué par $R_{29}$ ; un groupe $CR_{18}R_{19}R_{20}$ ; un groupe $CH_2R_{24}$ ; un groupe $C(CH_3)(CH_2OH)_2$ ou $C(CH_2OH)_3$ ;
- ou $R_{22}$ et $R_{23}$ ensemble avec l'atome d'azote auquel ils sont liés constituent un radical hétérocyclique $R_{12}$ ; une cis-2,6-diméthylpipérid-1-yle ;
- $R_{24}$ représente un carboxy ; un ($C_1$-$C_7$)alcoxycarbonyle ; un benzyloxycarbonyle ; un carbamoyle libre ou substitué par un ou deux ($C_1$-$C_7$)alkyles ;
- $R_{25}$ représente un ($C_1$-$C_7$)alkyle ; un phényle ; un benzyle ; un formyle ; un ($C_1$-$C_6$)alkylcarbonyle ; un ($C_1$-$C_7$) alcoxycarbonyle ; un benzyloxycarbonyle ;
- $R_{26}$ représente $R_{24}$ ; un amino libre ou substitué par un ou deux ($C_1$-$C_7$)alkyles ou par un groupe protecteur ; un groupe $OR_{13}$ ; un groupe $CH_2OR_{13}$ ;
- $R_{27}$ représente $R_{24}$, un groupe $CH_2R_{24}$, un groupe $CH_2OR_{13}$, un aminométhyle dans lequel l'amino est libre ou substitué par un ou deux ($C_1$-$C_7$)alkyles ou par un groupe protecteur ;
- $R_{28}$ représente un groupe $CH(CH_2OH)_2$ ou $CH(CH_3)CH_2OH$ ou $C(CH_3)(CH_2OH)_2$ ou $C(CH_3)_2CH_2OH$ ou $C(CH_2OH)_3$ ou $CH_2CH_2OH$ ;
- $R_{29}$ représente un groupe $R_{24}$, un groupe $OR_{13}$, un groupe $NR_{30}R_{31}$ ;
- $R_{30}$ et $R_{31}$ représentent chacun indépendamment un hydrogène, un ($C_1$-$C_7$)alkyle ;
- ou $R_{30}$ et $R_{31}$ ensemble avec l'atome d'azote auquel ils sont liés constituent un radical hététocyclique $R_{12}$ ;
- $R_{32}$ et $R_{33}$ représentent chacun indépendamment un hydrogène ; un ($C_1$-$C_7$)alkyle ; et $R_{33}$ peut de plus représenter un acétyle ; un phényle ; un benzyle ; un alkylène en $C_2$-$C_3$ substitué par un groupe $OR_{13}$, un amino libre ou substitué par un ou deux ($C_1$-$C_7$)alkyles ou par un groupe protecteur ;
- ou $R_{32}$ et $R_{33}$ ensemble avec l'atome d'azote auquel ils sont liés constituent un radical hétérocyclique $R_{12}$ ;
- X représente $SO_2$ ; $CH_2$ ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi : halogène ; ($C_1$-$C_7$)alkyle ; ($C_1$-$C_7$)alcoxy ;
- t représente un nombre entier qui peut varier de 2 à 10 ; ainsi que leurs sels éventuels.

Lorsqu'un composé selon l'invention présente un ou plusieurs carbones asymétriques, l'invention comprend tous les isomères optiques de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels physiologiquement acceptables tels que le chlorhydrate, le bromhydrate, le sulfate, l'hydrogénosulfate, le dihydrogénophosphate, le maléate, le fumarate, le 2-naphtalènesulfonate, le paratoluènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Selon la présente invention, par halogène on entend un atome choisi parmi le fluor, le chlore, le brome ou l'iode, de préférence le fluor ou le chlore.

Par groupe protecteur des groupes amine, hydroxyle, thiol ou carboxy on entend les groupes protecteurs tels que ceux décrits dans Protective Groups in Organic Synthesis, T.W. Greene et P.G.M. Wutts, ed. John Wiley et Sons, 1991 et dans Protective Groups in Organic Chemistry, J.F.W. Mc Omie, Ed. Plenum Press, 1973.

Selon la présente invention, par alkyle en $C_1$-$C_7$ ou respectivement en $C_1$-$C_4$ ou en $C_1$-$C_6$ ou en $C_2$-$C_6$ ou en $C_1$-$C_{12}$ ou en $C_2$-$C_{10}$, on entend un alkyle droit ou ramifié en $C_1$-$C_7$ ou respectivement en $C_1$-$C_4$ ou en $C_1$-$C_6$, ou

en $C_2$-$C_6$ ou en $C_1$-$C_{12}$ ou en $C_2$-$C_{10}$.

Les radicaux carbocycliques non aromatiques en $C_3$-$C_{15}$ comprennent les radicaux mono ou polycycliques, condensés ou pontés, saturés ou insaturés, éventuellement terpéniques. Ces radicaux sont éventuellement mono ou poly substitués par un alkyle en $C_1$-$C_4$. Les radicaux monocycliques incluent les cycloalkyles par exemple cyclopropyle, cyclopentyle, cyclohexyle, cycloheptyle, cyclooctyle, cyclododécyle. Les radicaux polycycliques incluent par exemple le norbornane, l'adamantane.

Dans le groupe $-CR_{18}R_{19}R_{20}$ lorsque $R_{18}$ et $R_{19}$ ensemble avec l'atome de carbone auquel ils sont liés forment un carbocycle non aromatique en $C_3$-$C_{15}$, ledit carbocycle est tel que défini pour les radicaux correspondants ci-dessus.

Les composés de formule (I) dans lesquels $R_1$ est en position 5 de l'indol-2-one et $R_2$ est en position 6 ou représente l'hydrogène sont des composés préférés.

Les composés de formule (I) dans lesquels $R_1$ est un atome de chlore ou un groupe éthoxy en position 5 de l'indol-2-one et $R_2$ est l'hydrogène ou un atome de chlore ou un groupe méthyle en position 6 sont des composés préférés.

Les composés de formule (I) dans lesquels $R_3$ est un chlorophényle, un méthoxyphényle, un cyclohexyle sont des composés préférés.

Les composés de formule (I) dans lesquels $R_4$ est un groupe amino, un ($C_1$-$C_{12}$)alkylamino, un pipérazin-1-yle substitué en position 4 par un groupe ($C_2$-$C_{10}$)alkyle substitué par un amino, un groupe $-N(R_9)(CH_2)_pR_{35}$, un groupe $-N(R_9)(C_2$-$C_{10})alkyl$-$R_{21}$, un groupe $-N(R_9)CO(C_1$-$C_4)alkyl$-$NR_{32}R_{33}$ sont des composés préférés.

Les composés de formule (I) dans lesquels $R_5$ est soit l'hydrogène soit un groupe méthoxy en position 2 et $R_6$ en position 4 représente un benzamido non substitué ou substitué par un méthoxy, un carbamoyle substitué par $R_{14}$ et $R_{15}$, un dialkyluréido en $C_1$-$C_7$, ou $R_5$ et $R_6$ ensemble avec le phényle auquel ils sont liés constituent un groupe :

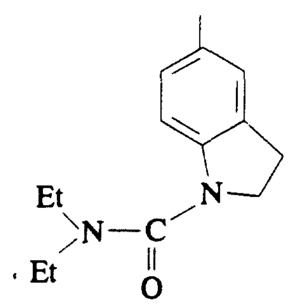

à la condition que X soit $-SO_2-$ sont des composés préférés.

De façon toute particulière, on préfère les composés de formule :

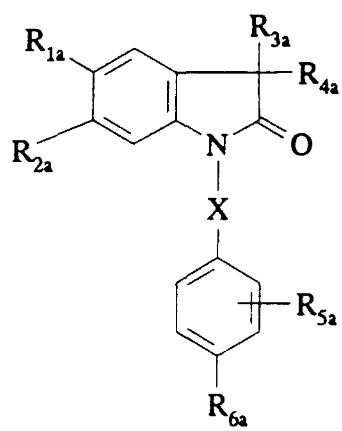

(Ia)

dans laquelle :

- $R_{1a}$ représente un éthoxy ou un chlore ;
- $R_{2a}$ représente l'hydrogène, un chlore ou un groupe méthyle ;

- $R_{3a}$ représente un chlorophényle, un méthoxyphényle ou un cyclohexyle ;
- $R_{4a}$ représente un amino ; un ($C_1$-$C_{12}$)alkylamino ; un radical pipérazin-1-yle substitué en position 4 par un groupe ($C_2$-$C_{10}$)alkylène substitué par un amino ; un groupe -N($R_9$)($CH_2$)$_p$$R_{35}$ ; un groupe -N($R_9$)($C_2$-$C_{10}$)alkyl-$R_{21}$ ; un groupe
- N($R_9$)CO($C_1$-$C_4$)alkyl-$NR_{32}R_{33}$ ;
- $R_{5a}$ représente l'hydrogène ou un 2-méthoxy ;
- $R_{6a}$ représente un benzamido dans lequel le phényle est non substitué ou substitué par un méthoxy ; un groupe -$CONR_{14}R_{15}$ ; un groupe -N',N'-di($C_1$-$C_7$)alkyluréido ;
- ou $R_{5a}$ et $R_{6a}$ ensemble avec le phényle auquel ils sont liés constituent un groupe :

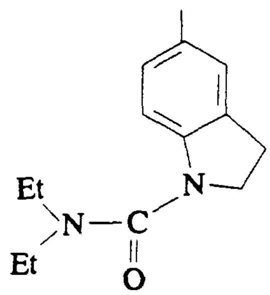

à la condition que X soit $SO_2$ ;
- les substituants X, $R_9$, $R_{14}$, $R_{15}$, $R_{21}$, $R_{32}$, $R_{33}$, $R_{35}$ étant tels que définis ci-dessus pour les composés de formule (I);

ainsi que leurs sels.

Dans la description et dans les exemples, les abréviations suivantes sont utilisées.

DCM : dichlorométhane
Ether : éther diéthylique
Ether iso : éther diisopropylique
$CCl_4$ : tétrachlorure de carbone
MeOH : méthanol
EtOH : éthanol
AcOEt : acétate d'éthyle
DMF : diméthylformamide
THF : tétrahydrofurane
DIPEA : diisopropyléthylamine
$NEt_3$ : triéthylamine
DBU : 1,8-diazabicyclo[5.4.0]undec-7-ène
AcOH : acide acétique
HCl : acide chlorhydrique
TFA : acide trifluoroacétique
NaCl : chlorure de sodium
Boc : *tert*-butoxycarbonyle
$(Boc)_2O$ : di-*tert*-butyl dicarbonate
Z : benzyloxycarbonyle
Bz : benzyle
Me, OMe : méthyle, méthoxy
Et, OEt : éthyle, éthoxy
Pr, iPr : n-propyle, isopropyle
Bu, iBu, tBu : butyle, isobutyle, *tert*-butyle
Ms : mésyle
BOP : hexafluorophosphate de benzotriazol-1-yloxy-tris(diméthylamino-phosphonium)
Réactif de Lawesson : 2,4-bis-(4-méthoxyphényl)-1,3-dithia-2,4-diphosphétane-2,4-disulfure
Silice H : gel de silice 60 H commercialisé par MERCK (DARMSTADT)
F : point de fusion
TA : température ambiante
RMN : résonnance magnétique nucléaire

s : singulet
se : singulet élargi
d : doublet
t : triplet
qd : quadruplet
m : massif
mt : multiplet
t de d : triplet de doublet
d de d : doublet de doublet
sep : septuplet

La présente invention a également pour objet un procédé de préparation des composés selon l'invention caractérisé en ce que :

1) on fait agir sur un composé de formule :

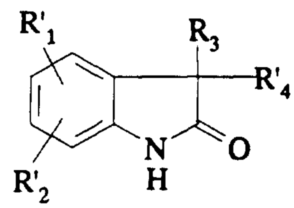

(II)

dans laquelle R'$_1$, R'$_2$ et R'$_4$ représentent, respectivement, soit $R_1$, $R_2$ et $R_4$ tels que définis pour (I), soit des groupes précurseurs de $R_1$, $R_2$ et $R_4$, et $R_3$ est tel que défini pour (I), un halogénure de formule :

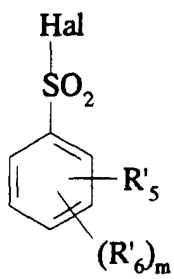

ou

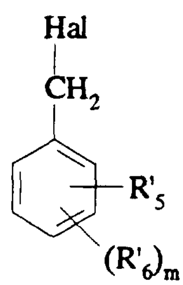

(III) (IV)

dans laquelle Hal représente un atome d'halogène, de préférence le chlore ou le brome et R'$_5$ et R'$_6$ représentent, respectivement, soit $R_5$ et $R_6$ tels que définis ci-dessus pour (I), soit des groupes précurseurs de $R_5$ et $R_6$ ; et,
2) soit, lorsque R'$_1$ = $R_1$, R'$_2$ = $R_2$, R'$_4$ = $R_4$, R'$_5$ = $R_5$ et R'$_6$ = $R_6$, on isole le composé de formule (I) ainsi obtenu ;
3) soit, lorsque l'un quelconque des groupes R'$_1$, R'$_2$, R'$_4$, R'$_5$ et/ou R'$_6$ représente respectivement un groupe précurseur de $R_1$, $R_2$, $R_4$, $R_5$ et/ou $R_6$, on soumet le composé obtenu à l'étape 1) à un traitement ultérieur pour préparer le composé de formule (I) par transformation de l'un quelconque des groupes R'$_1$, R'$_2$, R'$_4$, R'$_5$ et/ou R'$_6$ en, respectivement, $R_1$, $R_2$, $R_4$, $R_5$ et/ou $R_6$.
4) éventuellement, on transforme le composé obtenu à l'étape 2) ou à l'étape 3) en l'un de ses sels.

On appelle composé (I'), un composé portant un substituant R'$_1$, R'$_2$, R'$_4$, R'$_5$ et/ou R'$_6$ précurseurs de $R_1$, $R_2$, $R_4$, $R_5$ et/ou $R_6$.
La réaction de l'étape 1) est effectuée dans un solvant anhydre tel que le DMF ou le THF, en présence d'un hydrure métallique tel que l'hydrure de sodium par exemple, ou en présence d'un alcoolate comme le *tert*-butylate de potassium.
On peut préparer des dérivés de 1,3-dihydroindol-2-one de formule (II) selon les procédés décrits dans les brevets ZA 83 09532, 85 04765 ; brevet GB : 1 125 671.
On peut également préparer des dérivés de 1,3-dihydroindol-2-one substitués en 3 par des groupes azotés

par des méthodes bien connues de l'homme du métier, telles que celles décrites dans les publications suivantes :

Farm. Zh. (Kiev),1976, 5, 30-33.
Khim-Farm. Zh., 1979, 13 (11), 45-49.
J. Pharm. Sci., 1975, 64 (4), 639-642.
Chem. Pharm. Bull, 1978, 26 (9), 2866-2873.
J. Org. Chem., 1964, 29, 1206.

Plus particulièrement, on peut obtenir les 1,3-dihydroindol-2-ones (II) en faisant réagir un composé 3-halogéno-1,3-dihydroindol-2-one de formule:

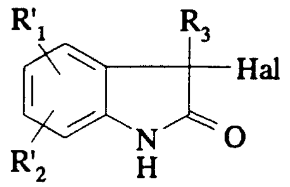

(V)

avec un composé de formule :

$$H\text{-}R'_4 \qquad (VI)$$

dans lesquelles $R'_1$, $R'_2$, $R_3$ et $R'_4$ sont tels que définis ci-dessus et Hal représente un halogène, de préférence le chlore ou le brome. La réaction peut être effectuée en présence d'une base telle que la DIPEA ou la triéthylamine, qui se comporte comme un accepteur d'acide ou en utilisant un excès du composé (VI).

Les 3-halogéno-1,3-dihydroindol-2-ones (V) sont obtenus par l'une quelconque de diverses méthodes décrites dans la littérature.

Par exemple, on transforme directement un composé de formule :

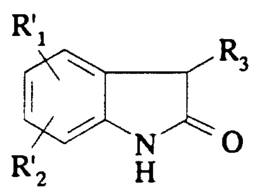

(VII)

dans laquelle $R'_1$, $R'_2$ et $R_3$ sont tels que définis précédemment, en un composé 3-halogéno-1,3-dihydroindol-2-one (V) au moyen d'un agent d'halogénation comme le brome (selon le procédé décrit dans Farm. Zh. (Kiev), 1976, 5, 30-33) ou comme le N-chlorosuccinimide dans un solvant comme le $CCl_4$.

Les composés de formule (VII) sont préparés selon J. Org. Chem., 1968, 33 (4), 1640-1643. De façon particulière, on peut préparer un composé de formule (VII) dans laquelle $R_3$ représente un cyclohexylméthyle ou un benzyle, non substitué ou substitué, d'après J. Med. Chem., 1965, 8, 626-637.

Selon un autre exemple de préparation des composés de formule (V), on fait réagir un dérivé de l'isatine de formule :

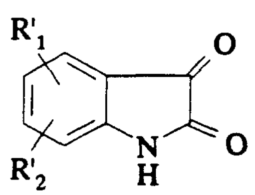

(VIII)

avec un composé de formule :

(IX) $R_3M$

ou

$$R_3MgBr \quad (X)$$

dans lesquelles $R'_1$, $R'_2$ et $R_3$ sont tels que définis précédemment et M représente un substituant métallique réactif comme le lithium, dans des conditions anhydres, et on hydrolyse le produit intermédiaire obtenu pour obtenir un composé 3-hydroxy-1,3-dihydroindol-2-one de formule :

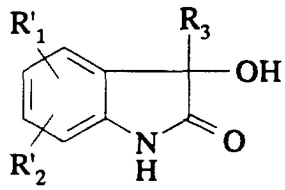

(XI)

puis ce dernier composé est transformé en composé 3-halogéno-1,3-dihydroindol-2-one (V) correspondant par réaction avec le chlorure de thionyle ou un agent d'halogénation similaire.

On peut également préparer un composé de formule (XI), par oxydation par l'air d'un composé de formule (VII) en présence d'une base telle que l'hydrure de sodium, et en présence de diméthyldisulfure.

Selon une autre méthode de préparation des composés de formule (II), on fait réagir un composé de formule (XI) avec le chlorure de méthanesulfonyle, en présence d'une base telle que la triéthylamine par exemple, dans un solvant anhydre tel que le THF; le mésylate ainsi obtenu est transformé en 1,3-dihydroindol-2-one de formule (II) par réaction avec un composé de formule (VI).

Préférentiellement, pour préparer un composé de formule (II) dans lequel $R'_4$ est azido, on fait réagir sur un composé (V), l'azidure de sodium ; la réaction s'effectue dans l'acétonitrile à reflux ou selon le procédé décrit par Y. Tamura et coll. dans Chem. Pharm. Bull., 1978, 26 (9), 2866-2873.

Pour préparer un composé de formule (II) dans lequel $R'_4$ est un amino, on peut faire réagir un composé de formule (II) dans lequel $R'_4$ est azido avec le propane-1,3-dithiol en présence d'une base telle que la triéthylamine d'après le procédé décrit par H. Bayley et Coll. dans Tetrahedron Letters, 1978, 39, 3633-3634.

Ce procédé est particulièrement préféré pour préparer un composé de formule (II) dans laquelle $R'_4$ est un amino et $R'_1$ en position 5 représente un $(C_1-C_7)$alcoxy.

Pour préparer un composé (II) dans lequel $R'_4$ est un groupe $NR_7R_8$, on fait agir sur un composé (VII), un composé de formule :

$$R_7\text{-}N = R_8 \quad (XII)$$

c'est-à-dire :

$$AlkOOC\text{-}N = N\text{-}COOAlk$$

dans laquelle Alk représente un $(C_1-C_7)$alkyle ; la réaction s'effectue dans un solvant anhydre tel que le THF, en présence d'une base telle que le diisopropylamidure de lithium.

Pour préparer un composé de formule (II) dans laquelle $R'_4$ est un résidu d'aminoacide ($R'_4$ = $-N(R_9)CR_{18}R_{19}R_{20}$) on fait agir sur un composé (V), un aminoacide, ou un de ses dérivés, éventuellement protégé par les groupements protecteurs habituels en synthèse peptidique, de formule :

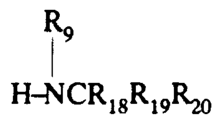 (XIII)

Les aminoacides non commerciaux sont préparés selon la synthèse de Strecker, Ann., 1850, 75, 27 ou selon la synthèse de H.T. Bucherer et al., J. Pract. Chem., 1934, 141, 5, suivie d'une hydrolyse pour conduire aux aminoacides ; par exemple, l'acide 2-aminoadamantane-2-carboxylique est préparé selon H.T. Nagasawa et al., J. Med. Chem., 1973, 16 (7), 823.

Les acides α-amino-1-adamantylacétique et α-amino-2-adamantylacétique sont préparés selon B. Gaspert et al., Croatica Chemica Acta, 1976, 48 (2), 169-178.

L'acide 2-aminonorbornane-2-carboxylique est préparé selon H.S. Tager et al., J. Am. Chem. Soc., 1972, 94, 968.

Les acides α-aminocycloalkylcarboxyliques sont préparés selon J.W. Tsang et al., J. Med. Chem., 1984, 27, 1663.

Les cyclopentylglycines R et S sont préparées selon la demande de brevet européen EP 477 049.

Les cyclohexylglycines R et S sont préparées selon Rudman et al., J. Am. Chem. Soc., 1952, 74, 551.

On peut également préparer les cyclohexylglycines R et S par hydrogénation catalytique des phénylglycines R et S.

On peut aussi préparer les acides a-aminocycloalkylcarboxyliques de configuration R ou S par hydrolyse enzymatique, stéréospécifique, des dérivés N-acétylés racémiques correspondants, selon J. Hill et al., J. Org. Chem., 1965, 1321.

Pour préparer un composé de formule (II) dans laquelle $R'_4$ représente un groupe $-N(R_9)(CH_2)_pR_{35}$, on fait agir sur un composé (V), un composé de formule :

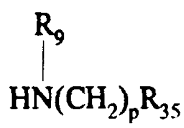 (XV)

Les composés de formule (XV) sont connus ou préparés par des méthodes connues. Par exemple pour préparer les composés de formule (XV) dans laquelle $R_{35}$ représente un pipérid-4-yle substitué en position 1 par un Boc, on procède selon Prugh J.D. et coll., Synthetic Communications, 1992, 22 16, 2357-2360 lorsque p = 1 ; et lorsque p = 2 ou 3, on applique le procédé décrit dans J. Med. Chem., 1989, 32, 391-396, ou le procédé décrit dans le Schéma 1.

Schéma 1

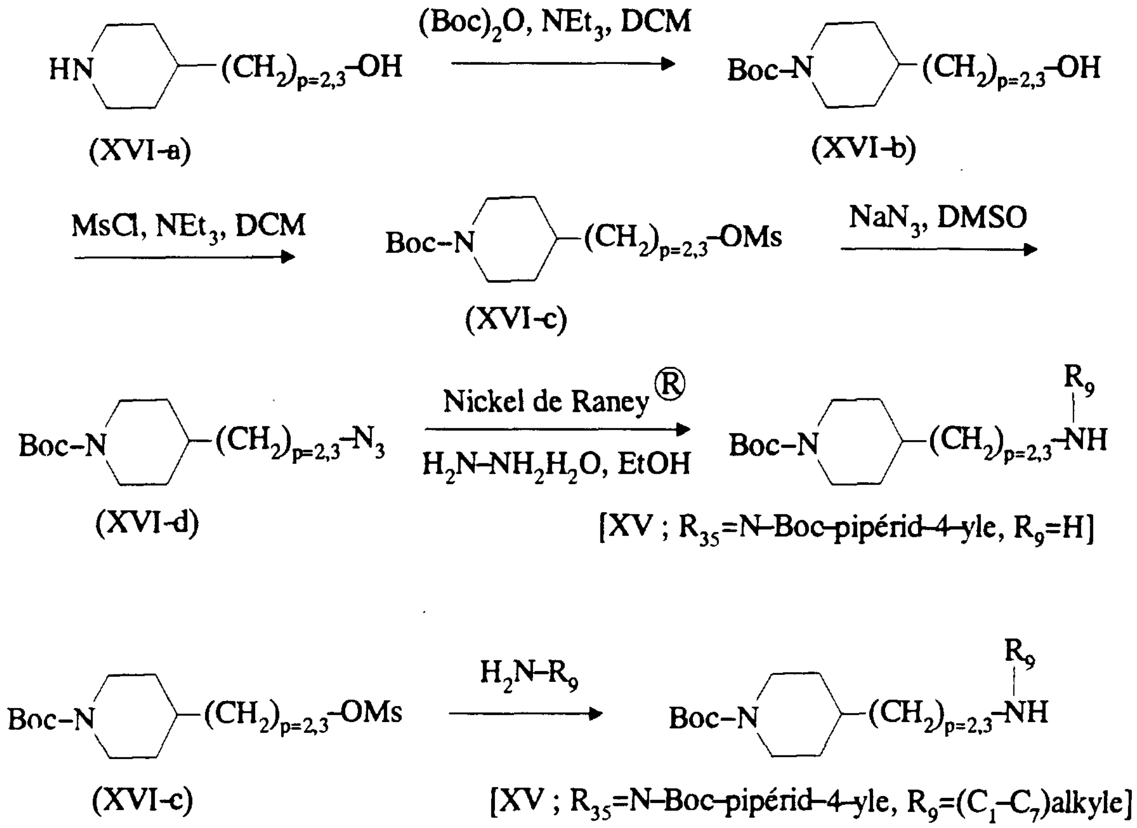


Pour préparer un composé de formule (II) dans laquelle $R'_4$ représente un groupe-$N(R_9)CH_2R_{36}$ dans lequel $R_{36}$ représente un ($C_1$-$C_7$)alcoxycarbonyle ou respectivement un benzyloxycarbonyle, on fait agir sur un composé (V), un composé de formule :

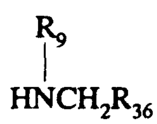 (XVII)

dans laquelle $R_{36}$ représente un ($C_1$-$C_7$)alcoxycarbonyle ou respectivement un benzyloxycarbonyle. A partir d'un tel composé on prépare par des méthodes connues un composé de formule (II) dans laquelle $R'_4$ représente un groupe -$N(R_9)CH_2COOH$ qui, par réaction avec un composé de formule $HNR_{38}R_{39}$ selon les techniques convenables du couplage amidique, permet d'obtenir un composé de formule (II) dans laquelle $R'_4$ représente un groupe -$N(R_9)$ $CH_2CONR_{38}R_{39}$.

Selon les mêmes procédés décrits ci-dessus, on prépare les composés de formule (II) dans laquelle $R'_4$ représente un groupe -$N(R_9)$-($C_2$-$C_{10}$)alkyl-$R_{36}$ dans lequel $R_{36}$ représente un ($C_1$-$C_7$)alcoxycarbonyle, un benzyloxycarbonyle ou un carbamoyle substitué par $R_{38}$ et $R_{39}$.

Pour préparer un composé de formule (II) dans laquelle $R'_4$ représente un groupe-$N(R_9)$($C_2$-$C_{10}$)alkyl-$OR_{37}$, on fait agir sur un composé (V), un composé de formule:

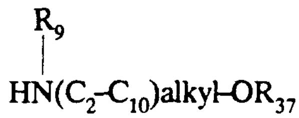

(XVIII)

Les composés de formule (XVIII) sont connus ou préparés par des méthodes connues.

Pour préparer un composé de formule (II) dans laquelle R'$_4$ représente un groupe -N($R_9$)($C_2$-$C_{10}$)alkyl-$NR_{32}R_{33}$ on fait agir sur un composé (V), un composé de formule :

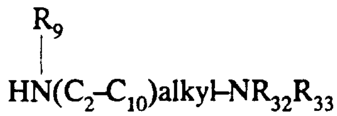

(XIX)

Les composés de formule (XIX) sont connus ou préparés par des méthodes connues. Par exemple les composés de formule (XIX) se préparent d'après les procédés décrits dans J. Med. Chem., 1989, 32, 391-396, dans Synthesis, 1982, 404-405, dans J. Am. Chem. Soc., 1946, 68, 10-14, ou dans le brevet EP-0429344. On peut également préparer les composés de formule (XIX) dans laquelle $R_{33}$ est autre qu'un groupe protecteur, en suivant les différentes étapes du procédé décrit dans le Schéma 2.

Schéma 2

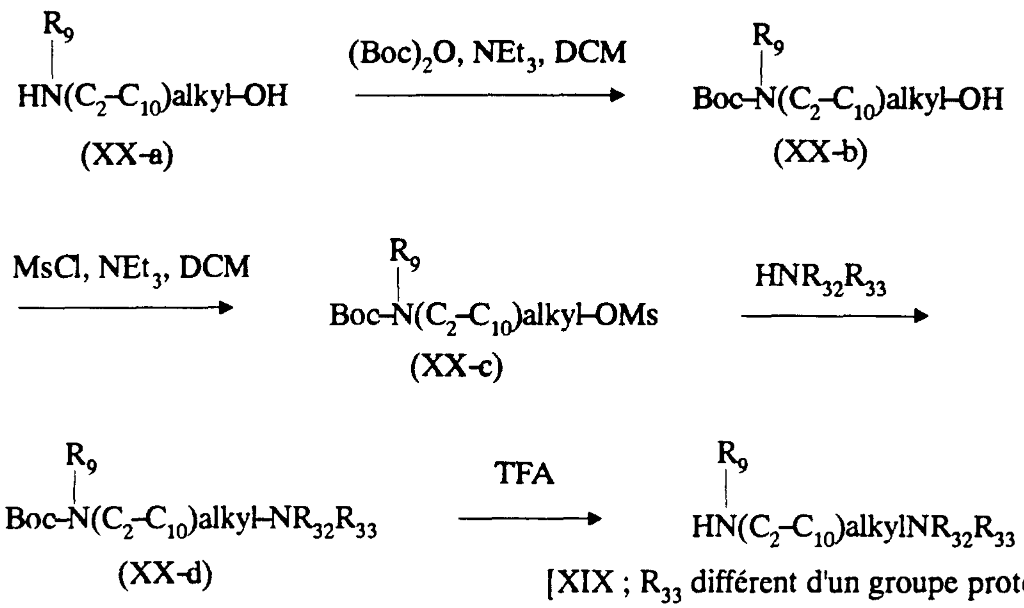


(XX-a) (XX-b) (XX-c) (XX-d)

[XIX ; $R_{33}$ différent d'un groupe protecteur]

Pour préparer un composé de formule (II) dans laquelle R'$_4$ représente un groupe -N($R_9$)($C_2$-$C_{10}$)alkyl-S($C_1$-$C_7$)alkyle, on fait réagir sur un composé (V), un composé de formule :

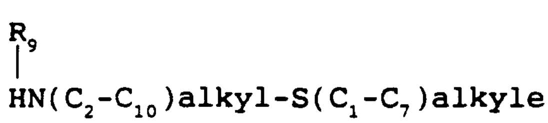

(XVIII)'

Les composés de formule (XVIII)' sont connus ou préparés par des méthodes connues. A partir des composés de formule (II) dans laquelle $R'_4$ représente un groupe $-N(R_9)(C_2-C_{10})$alkyl-$S(C_1-C_7)$alkyle, on prépare les composés de formule (II) dans laquelle $R'_4$ représente un groupe $-N(R_9)(C_2-C_{10})$alkyl-$SO(C_1-C_7)$alkyle ou un groupe $-N(R_9)(C_2-C_{10})$alkyl-$SO_2(C_1-C_7)$alkyle, selon des méthodes connues de l'homme de l'art.

On peut également préparer des composés de formule (II) dans laquelle $R'_4$ représente un $(C_1-C_7)$alkylamino ou un $(C_1-C_{12})$alkylamino par réaction d'un composé de formule (II) dans laquelle $R'_4$ représente un groupe amino avec un aldéhyde ou une cétone, en milieu acide, en présence d'un agent réducteur tel que le cyanoborohydrure de sodium ; par une réaction identique, on prépare les composés de formule (II) dans laquelle $R'_4$ représente un dialkylamino dans lequel les alkyles sont semblables ou différents, l'un des alkyles étant en $C_1-C_7$ et l'autre étant en $C_1-C_{12}$.

On peut également préparer les composés de formule (II) dans laquelle $R'_4$ représente un groupe amino substitué par un benzyle, éventuellement substitué, par action d'un chlorure de benzyle, éventuellement substitué, sur un composé de formule (II) dans laquelle $R'_4$ est un groupe amino ou alkylamino dans lequel l'alkyle est en $C_1-C_7$.

Pour préparer les composés de formule (II) dans laquelle $R'_4$ représente un groupe amino substitué par un cycloalkylméthyle dans lequel le cycloalkyle est en $C_3-C_7$ ou respectivement un phénéthyle éventuellement substitué, on peut également faire réagir un composé de formule (II) dans laquelle $R'_4$ représente un groupe amino avec un cycloalkylcarboxaldéhyde dans lequel le cycloalkyle est en $C_3-C_7$ ou respectivement un phénylacétaldéhyde éventuellement substitué, en milieu acide, en présence d'un agent réducteur tel que le cyanoborohydrure de sodium.

Pour préparer un composé de formule (II) dans laquelle $R'_4$ représente un groupe $\omega$-$R_{32}R_{33}N(C_1-C_4)$alkylCON$(R_9)$- on fait réagir un halogènure d'halogéno$(C_1-C_4)$alkylcarbonyle, tel que le bromure de bromoacétyle, le chlorure de 3-chloropropionyle, le chlorure de 4-chlorobutyryle ou le chlorure de 5-chlorovaléryle par exemple, avec un composé de formule (II) dans laquelle $R'_4$ représente un groupe $R_9NH$- ; puis par réaction du composé intermédiaire obtenu avec un composé $HNR_{32}R_{33}$, on obtient le composé de formule (II) désigné ci-dessus. De façon particulière, par réaction d'un composé de formule (II) dans laquelle $R'_4$ représente un groupe $R_9NH$- avec le chlorure d'acryloyle, en présence d'une base telle que la pyridine, puis par réaction du composé intermédiaire obtenu avec un composé $HNR_{32}R_{33}$, on obtient un composé de formule (II) dans laquelle $R'_4$ représente un groupe $-N(R_9)COCH_2CH_2NR_{32}R_{33}$.

Parmi les composés de formule (VII) et parmi les composés de formule (V) certains sont nouveaux et constituent un objet ultérieur de la présente invention.

Ainsi les composés de formule :

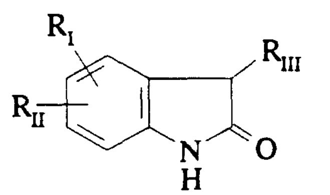

(VII')

et les composés de formule :

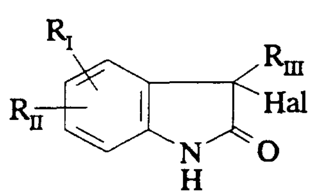

(V')

dans lesquelles :

- $R_I$ et $R_{II}$ représentent chacun indépendamment un hydrogène, un halogène, un $(C_1-C_7)$alkyle, un $(C_1-C_7)$alcoxy, un trifluorométhyle, un nitro, un amino, un hydroxy, un benzyloxy ;
- $R_{III}$ représente un phényle substitué une ou plusieurs fois par un halogène, un $(C_1-C_7)$alkyle, un hydroxy, un $(C_1-C_7)$alcoxy, un benzyloxy, un acétoxy ;
- Hal représente un halogène ;

sont nouveaux et font partie de l'invention.

De même parmi les composés de formule (II), certains sont nouveaux et constituent un objet de la présente invention.

Ainsi les composés de formule :

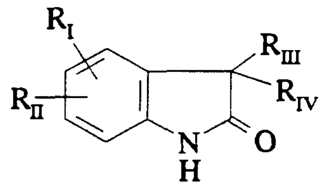

(II')

dans laquelle :

- $R_I$, $R_{II}$, $R_{III}$ sont tels que définis ci-dessus pour (V') et (VII') et
- $R_{IV}$ représente un azido, un amino, un groupe 2,2-diméthylhydrazino, un groupe $NR_7R_8$, un groupe $NR_9R_{10}$, $NR_9R_{XI}$, un radical hétérocyclique $R_{12}$, un radical pipérazin-1-yle substitué en position 4 par un groupe ($C_2$-$C_{10}$) alkylène substitué par un groupe amino libre ou portant un groupe protecteur ;
- $R_{XI}$ représente un ($C_1$-$C_{12}$)alkyle ; un ($C_3$-$C_7$)cycloalkylméthyle; un groupe $OR_{13}$ ;un groupe $CH_2R_{36}$ ; un groupe ω-$R_{32}R_{33}N$($C_1$-$C_4$)alkylcarbonyle ;

et $R_7$, $R_8$, $R_9$, $R_{10}$, $R_{12}$ $R_{13}$, $R_{32}$, $R_{33}$ et $R_{36}$ sont tels que définis ci-dessus pour (I); sont nouveaux et font partie de l'invention.

Les halogénures de benzènesulfonyle (III) sont préparés par des méthodes connues.

Ainsi par exemple, le chlorure de 4-diméthylaminobenzènesulfonyle est préparé selon C.N. Sukenik et al., J. Amer. Chem. Soc., 1977, 99, 851-858. Plus généralement, les halogénures de benzènesulfonyle (III) substitués par un groupe diméthylamino sont connus ou préparés par des méthodes connues ; le chlorure de *p*-benzyloxybenzènesulfonyle est préparé selon la demande de brevet européen EP 229 566.

Le chlorure d'alcoxybenzènesulfonyle est préparé à partir de l'alcoxybenzènesulfonate de sodium, lui-même préparé par action d'un halogénure d'alkyle sur l'hydroxybenzènesulfonate de sodium.

Le chlorure de 2,4-diméthoxybenzènesulfonyle est préparé selon J. Am. Chem. Soc, 1952, 74, 2008.

Les halogénures de benzènesulfonyle de formule :

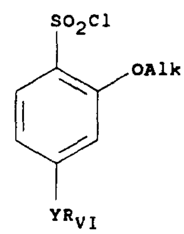

(III')

dans laquelle :

- Alk représente un ($C_1$-$C_7$)alkyle.
- Y représente O ou S ;
- $R_{VI}$ représente un ($C_1$-$C_7$)alkyle, un allyle, un benzyle, sont préparés d'après D. Hofmann et al. dans Liebigs Ann. Chem., 1982, 287-297.

On opère sur des composés benzèniques portant les substituants $YR_{VI}$ et OAlk en position -1,3 par action du chlorosulfonate de triméthylsilyle dans un solvant tel que le DCM à TA. On applique ensuite la méthode de R. Passerini et al. dans Gazz. Chim. Ital., 1960, 90, 1277-89 et on neutralise ensuite, par exemple par du carbonate alcalin, puis on fait agir un halogénure tel que $POCl_3$ pour obtenir l'halogénure de benzènesulfonyle souhaité.

Les halogénures de benzènesulfonyle (III) substitués par un ($C_1$-$C_7$)alcoxycarbonyle, un phénoxycarbonyle, un benzyloxycarbonyle, un($C_1$-$C_7$)alkylthio, un benzylthio sont préparés selon Col. Czechoslov. Chem. Commun., 1984, 49, 1184, à partir d'un dérivé de l'aniline substitué par le même groupement, ledit dérivé de l'aniline étant lui-même obtenu à partir du dérivé nitré correspondant.

Les dérivés de l'acide nitrobenzoïque sont connus ; par une réaction appropriée d'estérification de cet acide, on obtient les esters d'alkyle et de phényle correspondants.

Les dihalogénures de benzène di-sulfonyle (III, $R'_6$ = $SO_2Hal$) sont connus ou préparés par des méthodes

connues. Par exemple, le dichlorure de 2,4-diméthoxybenzène-1,5-disulfonyle est décrit par R.J.W. Cremlyn dans J. Chem. Soc. C, 1969, 1341-1345.

Les halogénures de benzène sulfonyle (III) dans lesquels $R'_6$ en position 4 représente un sulfamoyle substitué par $R_{16}$ et $R_{17}$ ($R'_6$ = $-SO_2NR_{16}R_{17}$) et $R'_5$ en position 2 représente un ($C_1$-$C_7$)alcoxy peuvent être préparés selon le procédé suivant : on prépare des halogénures de 3-alcoxy-4-nitrobenzènesulfonyle par action de l'acide chlorosulfonique sur des composés 2-alcoxynitrobenzène et on fait réagir le chlorure de sulfonyle ainsi obtenu avec des composés $HNR_{16}R_{17}$. On obtient les halogénures de benzènesulfonyle correspondants selon Col. Czechoslov. Chem. Commun., 1984, 49, 1184, à partir des dérivés de l'aniline substitués par le même groupement, lesdits dérivés de l'aniline étant eux-mêmes obtenus à partir des dérivés nitrés correspondants.

L'halogénure de benzène sulfonyle (III) dans lequel $R'_6$ en position 4 représente un groupe N'-N'-diéthyluréido [$R'_6$ = $-NHCON(Et)_2$] peut être préparé par action de l'acide chlorosulfonique sur la N',N'-diéthyl-N-phénylurée, elle-même obtenue par réaction de l'aniline avec le chlorure de diéthylcarbamoyle.

Le chlorure de 4-(morpholin-4-yl)benzènesulfonyle est préparé selon le procédé décrit dans Arzneim. -Forsch./Drug Res., 1994, 44 (I), N° 4, 501-509.

L'halogénure de benzènesulfonyle de formule (III) dans laquelle $R'_5$ et $R'_6$ ensemble avec le phényle auquel ils sont liés constituent un groupe :

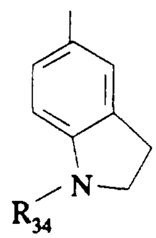

se prépare par action de l'acide chlorosulfonique sur un composé de formule :

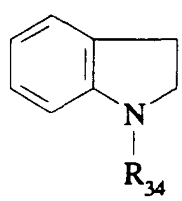

(XXI)

dans laquelle $R_{34}$ est tel que défini pour (I).

Les composés de formule (XXI) dans laquelle $R_{34}$ représente un formyle ou respectivement un ($C_1$-$C_7$)alkylcarbonyle, s'obtiennent par action de l'acide formique dans l'anhydride acétique ou respectivement par l'action d'un anhydride approprié ou d'un chlorure d'acide approprié, en présence d'une amine comme la triéthylamine, sur l'indoline. De même par action d'un chloroformiate d'alkyle en $C_1$-$C_7$ ou de phényle sur l'indoline, en présence d'une base telle que la pyridine, on obtient les composés de formule (XXI) dans laquelle $R_{34}$ représente un ($C_1$-$C_7$)alcoxycarbonyle ou un phénoxycarbonyle.

Par action d'un chlorure de carbamoyle substitué par deux ($C_1$-$C_7$)alkyles sur l'indoline, en présence d'une base telle que la pyridine, on obtient les composés de formule (XXI) dans laquelle $R_{34}$ représente un carbamoyle substitué par deux ($C_1$-$C_7$)alkyles.

Par action d'un composé $R_{12}H$ sur un composé de formule (XXI) dans laquelle $R_{34}$ représente un phénoxycarbonyle, on obtient un composé de formule (XXI) dans laquelle R34 représente un groupe $-COR_{12}$.

Les halogénures de benzyle de formule (IV) sont connus ou préparés par des méthodes connues.

A titre d'exemple, on peut citer des publications décrivant les dérivés d'halogénométhylbenzène suivants :

1,2,4- ou 4,2,1- ou 2,4,1-chlorométhyl, méthyl, méthoxybenzène : Bull. Soc. Chim., France, 1937, 4, 1092.
2-chlorométhyl-1,3-diméthoxybenzène: Chem. Listy, 1953, 47, 601-612.
1-bromométhyl-2-méthoxy-4-nitrobenzène : Sci. Sinica (Peking), 1962, 11, 483-498.
1-bromométhyl-2-méthyl-4-nitrobenzène: Pharmazie, 1969, 24 (1), 29-32.
1-bromométhyl-2-méthoxy-4-nitrobenzène: Bull. Soc. Chim., France, 1962, 2255.
1-bromométhyl-4-méthoxy-2-nitrobenzène: Zh. Obshch. Khim., 1963, 33 (8), 2792-2793.
4-bromométhyl-3-méthoxybenzoate de méthyle : demande de brevet européen : EP 179 619.

2-bromométhyl-6-méthoxybenzoate d'éthyle : J. Org. Chem., 1983, 48, 3439-3444.
2-bromométhyl-4,5-diméthoxybenzoate d'éthyle et 2-bromométhyl-3,4-diméthoxybenzoate d'éthyle : J. Org. Chem., 1968, 33, 494.
4-bromométhyl-2-méthoxybenzoate de méthyle : Bull. Soc. Chim. France, 1962, 2255.
1-bromométhyl-4-cyano-2-méthoxybenzène et 4-aminométhyl-1-bromo-méthyl-2-méthoxybenzène : J. Med. Chem., 1990, 33, 2437-2451.

D'une manière générale, les dérivés halogénométhylbenzène peuvent être préparés par action du N-bromosuccinimide sur les dérivés de méthylbenzène correspondants. La réaction est effectuée dans un solvant comme le tétrachlorure de carbone en présence de péroxyde de dibenzoyle. On peut également préparer un dérivé d'halogénométhylbenzène à partir d'un dérivé d'hydroxyméthylbenzène correspondant par action du tribromure de phosphore dans l'éther.

Selon un autre procédé, les dérivés d'halogénométhylbenzène de formule (IV) peuvent être préparés à partir de l'alcool correspondant par action du chlorure de thionyle pour préparer un chlorure de méthylbenzène.

Pour certaines valeurs des substituants $R_1$, $R_2$, $R_4$, $R_6$ et/ou $R_5$ les composés selon l'invention (I) peuvent être préparés à partir d'un précurseur de formule (I') substitué par un groupe $R'_1$, $R'_2$, $R'_4$, $R'_6$ et/ou $R'_5$ appelé groupe précurseur de $R_1$, $R_2$, $R_4$, $R_6$ et/ou $R_5$ en utilisant des méthodes connues de l'homme de l'art.

Dans la description qui va suivre, on décrit la préparation des composés de formule (I) portant des substituants $R_1$, $R_4$ et/ou $R_6$ ; les mêmes méthodes s'appliquent à la préparation des composés dans lesquels les substituants $R_2$ et/ou $R_5$ ont les valeurs indiquées pour $R_1$ et/ou $R_6$.

Les composés de formule (I) dans laquelle $R_4$ représente un groupe $-N(R_9)(CH_2)_pR_{35}$ dans lequel $R_{35}$ représente un pipérid-4-yle non substitué en position 1 s'obtiennent par une réaction de déprotection classique des composés de formule (I) dans laquelle $R_{35}$ représente un pipérid-4-yle substitué en position 1 par un groupe protecteur.

On peut préparer les composés de formule (I) dans laquelle $R_4$ représente un groupe $-N(R_9)CH_2R_{36}$ dans lequel $R_{36}$ représente un carboxy à partir des composés de formule (I) correspondants dans lesquels $R_{36}$ représente un $(C_1\text{-}C_7)$alcoxycarbonyle ou un benzyloxycarbonylc. Puis, par réaction d'un composé de formule (I) dans laquelle $R_4$ représente un groupe $-N(R_9)CH_2COOH$ avec un composé de formule $HNR_{38}R_{39}$ selon les techniques convenables du couplage amidique, on obtient les composés de formule (I) dans laquelle $R_4$ représente un groupe $-N(R_9)CH_2CONR_{38}R_{39}$.

Selon les mêmes procédés décrits ci-dessus, on peut préparer les composés de formule (I) dans laquelle $R_4$ représente un groupe $-N(R_9)(C_2\text{-}C_{10})$alkyl-$R_{36}$ dans lequel $R_{36}$ représente un carboxy ou un groupe $-CONR_{38}R_{39}$.

On peut préparer les composés de formule (I) dans laquelle $R_4$ représente un groupe $-N(R_9)(C_2\text{-}C_{10})$alkyl-$NR_{32}R_{33}$ dans lequel $-NR_{32}R_{33}$ représente une pipérazin-1-yle substituée en position 4 par un $(C_1\text{-}C_7)$alkyle soit directement par le procédé selon l'invention à partir des composés de formule (II) correctement substitués soit par réaction des composés de formule (I) correspondants dans laquelle $-NR_{32}R_{33}$ représente une pipérazin-1-yle non substitué en position 4, avec une aldéhyde ou une cétone, en milieu acide, en présence d'un agent réducteur, tel que le cyanoborohydrure de sodium.

Les composés (I) dans lesquels $R_4$ et/ou $R_6$ représente un $(C_1\text{-}C_7)$alkylsulfonamido ou respectivement un phénylsulfonamido ou un diméthylaminosulfonamido s'obtiennent par action d'un halogénure d'alkylsulfonyle dans lequel l'alkyle est en $C_1\text{-}C_7$ ou respectivement d'un halogénure de benzènesulfonyle ou d'un halogénure de diméthylsulfamoyle sur un composé (I) dans lequel $R_4$ et/ou $R_6$ est un groupe amino.

Les composés (I) dans lesquels $R_4$ représente un groupe lactoylamino ou respectivement mandéloylamino s'obtiennent par action d'un acide lactique ou d'un acide mandélique, sur un composé (I) dans lequel $R_4$ est un groupe amino, selon les techniques convenables de couplage amidique.

Les composés (I) dans lesquels $R_4$ représente un groupe N'-(1-phényléthyl)uréido ou respectivement N'-(1-napht-1-yléthyl)uréido s'obtiennent par action d'un $\alpha$-méthylbenzylisocyanate ou respectivement d'un (napht-1-yl)éthylisocyanate sur un composé (I) dans lequel $R_4$ est un groupe amino.

Les composés (I) dans lesquels $R_4$ et/ou $R_6$ représente un groupe $NR_9R_{11}$, $R_{11}$ étant un formyle ou respectivement un $(C_1\text{-}C_7)$alkylcarbonyle, un $(C_1\text{-}C_7)$cycloalkylcarbonyle, un benzoyle éventuellement substitué, un phénacétyle dans lequel le noyau benzénique est éventuellement substitué, un pyridylcarbonyle, un thiénylcarbonyle, un furylcarbonyle, ou un pipérid-4-ylcarbonyle, s'obtiennent par action de l'acide formique dans l'anhydride acétique ou respectivement par action de l'anhydride approprié ou du chlorure d'acide approprié sur un composé (I) dans lequel $R_4$ et/ou $R_6$ est un groupe $R_9NH$-, en présence d'une amine comme la triéthylamine.

Les composés (I) dans lesquels $R_4$ et/ou $R_6$ représente un groupe $NR_9R_{11}$, $R_{11}$ étant un $(C_1\text{-}C_7)$alcoxycarbonyle ou, respectivement, un phénoxycarbonyle ou un benzyloxycarbonyle, s'obtiennent par action d'un chloroformiate d'alkyle en $C_1\text{-}C_7$ ou, respectivement, de phényle ou de benzyle sur un composé (I) dans lequel $R_4$ et/ou $R_6$ est un groupe $R_9NH$-.

De même, par action d'un chlorure de phénoxythiocarbonyle sur un composé de formule (I) dans laquelle $R_4$

et/ou $R_6$ est un groupe $R_9NH$-, on obtient un composé de formule (I) dans laquelle $R_4$ et/ou $R_6$ est un groupe $-NR_9R_{11}$ dans lequel $R_{11}$ représente un phénoxythiocarbonyle.

Par action de l'ammoniac sur un composé de formule (I) dans laquelle $R_4$ et/ou $R_6$ est un groupe $-NR_9R_{11}$ dans lequel $R_{11}$ représente un phénoxycarbonyle ou un phénoxythiocarbonyle, on prépare un composé de formule (I) dans lequel $R_4$ et/ou $R_6$ est un groupe $-N(R_9)CONH_2$ ou $-N(R_9)CSNH_2$.

On peut également préparer des composés de formule (I) dans lesquels $R_4$ et/ou $R_6$ est un uréido ($-NR_9CONR_{22}R_{23}$) ou, respectivement, un thiouréido ($-NR_9CSNR_{22}R_{23}$) par action d'un composé $NHR_{22}R_{23}$, sur un composé (I) dans lequel $R_4$ et/ou $R_6$ est un groupe $-NR_9R_{11}$ dans lequel $R_{11}$ représente un phénoxycarbonyle ou, respectivement, un phénoxythiocarbonyle.

On peut aussi préparer des composés de formule (I) dans lesquels $R_4$ et/ou $R_6$ est un uréido ($-NR_9CONR_{22}R_{23}$) ou respectivement un thiouréido ($-NR_9CSNR_{22}R_{23}$) par action d'un chlorure de carbamoyle ($ClCONR_{22}R_{23}$) ou, respectivement d'un chlorure de thiocarbamoyle ($ClCSNR_{22}R_{23}$), sur un composé de formule (I) dans lequel $R_4$ et/ou $R_6$ est un groupe $R_9NH$-.

On peut également préparer un composé (I) dans lequel $R_4$ et/ou $R_6$ est un groupe $-NR_9R_{11}$ dans lequel $R_{11}$ représente un ($C_1$-$C_7$)alkylcarbamoyle par action d'un isocyanate d'alkyle en $C_1$-$C_7$ sur un composé (I) dans lequel $R_4$ et/ou $R_6$ est un groupe $R_9NH$-.

On peut également préparer un composé (I) dans lequel $R_4$ et/ou $R_6$ est un groupe $-N(R_9)CONR_{22}R_{23}$ ou $-N(R_9)CSNR_{22}R_{23}$ dans lequel $R_9$ représente un ($C_1$-$C_7$)alkyle, par action d'une base telle que l'hydrure de sodium sur un composé (I) dans lequel $R_4$ et/ou $R_6$ est un groupe $-NHCONR_{22}R_{23}$ ou $-NHCSNR_{22}R_{23}$ puis réaction avec un halogénure de ($C_1$-$C_7$)alkyle.

On peut encore préparer un composé (I) dans lequel $R_4$ et/ou $R_6$ est un thiouréido par action du réactif de Lawesson sur un composé (I) dans lequel $R_4$ et/ou $R_6$ est l'uréido correspondant.

On peut utiliser l'action d'un anhydride, tel que l'anhydride succinique ou l'anhydride glutarique, sur un composé (I) dans lequel $R_4$ et/ou $R_6$ est un amino pour préparer un composé (I) dans lequel $R_4$ et/ou $R_6$ est un groupe $-NHCO(CH_2)_2CO_2H$ ou $-NHCO(CH_2)_3CO_2H$. Le cas échéant on transforme l'acide ainsi obtenu en ester ou en amide.

On peut préparer un composé de formule (I) dans laquelle $R_4$ et/ou $R_6$ représente un groupe $\omega$-$R_{32}R_{33}N$ ($C_1$-$C_4$)alkylCON($R_9$)- par action d'un halogénure d'halogéno($C_1$-$C_4$)alkylcarbonyle, tel que le bromure de bromoacétyle, le chlorure de 3-chloropropionyle, le chlorure de 4-chlorobutyryle ou le chlorure de 5-chlorovaléryle par exemple, sur un composé de formule (I) dans laquelle $R_4$ et/ou $R_6$ représente un groupe $R_9NH$- ; puis par réaction du composé intermédiaire obtenu avec un composé $HNR_{32}R_{33}$, on obtient le composé de formule (I) désigné ci-dessus.

On peut préparer un composé de formule (I) dans lequel $R_4$ est un groupe amino par action d'une solution d'HBr dans l'AcOH sur un composé (I) dans lequel $R_4$ est un groupe $-NR_9CO_2Me$.

Les composés de formule (I) dans lesquels $R_1$ et/ou $R_6$ représente un ($C_1$-$C_7$)alcoxy peuvent être préparés directement par le procédé selon l'invention à partir des composés de formule (II), (III) ou (IV) correctement substitués.

Les composés (I') dans lesquels $R'_1$ et/ou $R_6$ représente un hydroxyle permettent également de préparer des composés (I) dans lesquels $R_1$ et/ou $R_6$ est un ($C_1$-$C_7$)alcoxy par action d'un halogénure d'alkyle en $C_1$-$C_7$ en présence d'une base telle qu'un hydrure métallique ou un carbonate alcalin ou alcalino-terreux comme $K_2CO_3$ ou $Cs_2CO_3$, dans un solvant tel que le THF ou le DMF.

Les composés (I') dans lesquels $R'_1$ et/ou $R_6$ est un hydroxyle peuvent être obtenus par hydrogénation catalytique, par exemple, en présence de palladium sur charbon, d'un composé de formule (I') dans lequel $R'_1$ et/ou $R_6$ est un benzyloxy. Ces composés peuvent également être préparés à partir de composés analogues de formule (I') dans lesquels $R'_1$ et/ou $R_6$ représente un groupe amino en utilisant la méthode décrite dans J. Org. Chem., 1977, 42, 2053.

Un composé de formule (I) dans lequel $R_6$ est un groupe nitro permet d'obtenir par hydrogénation catalytique, par exemple en présence d'oxyde de platine ou de nickel de Raney, ou par réduction chimique, par exemple en présence d'étain ou de fer en milieu acide, un composé (I) dans lequel $R_6$ est un groupe amino ; on peut ensuite préparer d'autres composés dans lesquels le groupe amino est substitué en utilisant des réactions bien connues de l'homme de l'art.

Pour préparer des composés de formule (I) dans laquelle $R_6$ représente un ($C_1$-$C_7$)alkylamino, ou un ($C_1$-$C_{12}$)alkylamino, on fait réagir un composé de formule (I) dans lequel $R_6$ représente un groupe amino avec un aldéhyde ou une cétone, en milieu acide, en présence d'un agent réducteur tel que le cyanoborohydrure de sodium ; par une réaction identique, on prépare les composés (I) dans lesquels $R_6$ représente un dialkylamino dans lequel les alkyles sont semblables ou différents et l'un des alkyles est en $C_1$-$C_7$ et l'autre est en $C_1$-$C_{12}$.

Les composés de formule (I) dans lesquels $R_6$ représente un groupe $\Delta$3-pyrrolin-1-yle se préparent par action, sous atmosphère inerte, du cis-1,4-dichlorobut-2-ène sur les composés de formule (I) dans lesquels $R_6$ est un groupe amino en présence d'une base telle que la triéthylamine. Par hydrogénation on prépare ensuite les composés de formule (I) dans lesquels $R_6$ est un groupe pyrrolidin-1-yle.

La réaction du cis-1,4-dichlorobut-2-ène avec les composés (I) dans lesquels $R_6$ est un groupe amino peut

également s'effectuer à l'air en présence d'une base telle que $Na_2CO_3$ et conduit, dans ces conditions, à la formation d'un mélange d'un composé de formule (I) dans lequel $R_6$ représente un groupe Δ3-pyrrolin-1-yle et d'un composé de formule (I) dans lequel $R_6$ représente un groupe pyrrol-1-yle que l'on peut séparer par chromatographie.

Pour préparer les composés de formule (I) dans lesquels $R_6$ représente un groupe $R_9$NH- substitué par un ($C_3$-$C_7$)cycloalkylméthyle, on fait réagir un composé de formule (I) dans lequel $R_6$ représente un groupe $R_9$NH- avec un cycloalkylcarboxaldéhyde dans lequel le cycloalkyle est en $C_3$-$C_7$, en milieu acide, en présence d'un agent réducteur tel que le cyanoborohydrure de sodium.

Lorsque $R_6$ représente un groupe amino, on peut également effectuer une nitrosation, par exemple en présence d'acide nitreux ou de nitrite de sodium, pour préparer un composé (I') dans lequel $R'_6$ représente un sel de diazonium ; par des réactions connues de l'homme de l'art, on accède alors aux composés (I) selon l'invention dans lesquels $R_6$ est un cyano, un halogéno ou un alkylthio en $C_1$-$C_7$.

Les composés (I) dans lesquels $R_6$ représente un groupe amino substitué par un groupe $CH_2R_{36}$, s'obtiennent par action d'un composé de formule Hal-$CH_2$-COOR dans lequel Hal représente un halogène, par exemple le brome, et R représente un alkyle en $C_1$-$C_7$ ou un benzyle, sur un composé (I) dans lequel $R_6$ est un groupe $R_9$NH-, en présence de chlorure cuivreux ; le cas échéant, on transforme l'ester ainsi obtenu en acide ou en amide.

Les composés (I) dans lesquels $R_6$ est un groupe guanidino non substitué ou substitué une ou deux fois par un ($C_1$-$C_7$)alkyle, un phényle ou un benzyle, peuvent se préparer à partir de composés (I) dans lesquels $R_6$ est un groupe phénoxycarboxamido par action du cyanamide ou d'un de ses dérivés correctement substitué sur l'azote.

Les composés de formule (I) dans lesquels $R_6$ représente un ($C_1$-$C_7$)alcoxycarbonyle peuvent se préparer directement par le procédé selon l'invention. Par des méthodes connues de l'homme de l'art, ils permettent d'obtenir les composés de formule (I) dans lesquels $R_6$ est un groupe carboxy.

Selon un autre mode opératoire, les composés de formule (I) dans lesquels $R_6$ est un benzyloxycarbonyle permettent d'obtenir par hydrogénation catalytique les composés (I) dans lesquels $R_6$ est un carboxyle. Par action d'un halogénure de thionyle, on obtient les composés de formule (I') dans lesquels $R'_6$ est un halogénocarbonyle. A partir de tels composés, on prépare des composés de formule (I) dans lesquels $R_6$ est un carbamoyle substitué par $R_{14}$ et $R_{15}$, par action d'un composé $HNR_{14}R_{15}$.

On peut également utiliser les composés de formule (I) dans lesquels $R_6$ représente un phénoxycarbonyle pour obtenir les composés de formule (I) dans lesquels $R_6$ est un phénylcarbamoyle ou un ($C_1$-$C_7$)alkylcarbamoyle par action d'une aniline ou d'une ($C_1$-$C_7$)alkylamine. Une aniline substituée ou, respectivement, une alkylamine substituée sur l'alkyle permet(tent) d'obtenir des composés de formule (I) dans lesquels $R_6$ est un phénylcarbamoyle substitué sur le phényle ou, respectivement, un alkylcarbamoyle substitué sur l'alkyle.

On peut également utiliser les composés de formule (I) dans laquelle $R_6$ est un carboxy pour obtenir les composés de formule (I) dans laquelle $R_6$ est un groupe $-CONR_{14}R_{15}$ par action d'un composé de formule $HNR_{14}R_{15}$, en présence de BOP et d'une amine telle que la diisopropyléthylamine.

Les composés de formule (I) dans lesquels $R_6$ est un groupe $-COR_{12}$ peuvent également s'obtenir à partir de composés (I) dans lesquels $R_6$ est un phénoxycarbonyle, par action d'un composé $R_{12}H$.

On peut préparer un composé de formule (I) dans laquelle $R_6$ est un thiocarbamoyle par réaction du réactif de Lawesson sur un composé (I) dans lequel $R_6$ est le carbamoyle correspondant.

Les composés de formule (I) dans lesquels $R_6$ est un sulfamoyle substitué par $R_{16}$ et $R_{17}$, s'obtiennent par action d'un composé $HNR_{16}R_{17}$ sur un composé de formule (I') dans lequel $R'_6$ représente un groupe halogénosulfonyle.

On peut effectuer la résolution des énantiomères des composés de formule (I) dans lesquels le carbone en position 3 de l'indol-2-one est asymétrique par des méthodes connues de l'homme de l'art. Par exemple la résolution des composés de formule (I) dans lesquels $R_4$ est un amino peut s'effectuer en utilisant l'acide (S)-(+)-lactique ou l'acide (S)-(+)-mandélique ou l'acide (R)-(-)-mandélique ou le (R)-(+)-(napht-1-yl)éthylisocyanate ou le (R)-(-)-1-(napht-1-yl)éthylisocyanate ou le (S)-(-)-α-méthylbenzylisocyanate ou le (R)-(+)-α-méthylbenzylisocyanate ou l'acide (1S)-(+)-10-camphorsulfonique ou l'acide (1R)-(-)-10-camphorsulfonique.

On peut également effectuer la résolution optique des composés de formule (II). Ainsi la résolution des composés de formule (II) dans laquelle $R'_4$ est un amino peut s'effectuer d'après le procédé décrit dans Bull. Chem. Soc. Jpn., 1992, 65, 2359-2365 et illustré ci-après. Par réaction d'un composé de formule (V) avec le (S)-(+)-2-phénylglycinol ou le (R)-(-)-2-phénylglycinol on obtient un mélange de diastéréoisomères d'un composé de formule (II) dans laquelle $R'_4$ représente $-NH-CH(C_6H_5)-CH_2OH$ et que l'on peut séparer par exemple par chromatographie ou cristallisation. Puis, par oxydation par le tétraacétate de plomb et hydrolyse en milieu acide, d'un des diastéréoisomères précédents, on obtient un composé de formule (II) énantiomériquement pur dans laquelle $R'_4$ est un amino.

Au cours de l'une quelconque des étapes du procédé de préparation des composés de formule (I) ou de leurs composés intermédiaires de formule (II), (III) ou (IV), il peut être nécessaire et/ou souhaitable de protéger les groupes fonctionnels réactifs ou sensibles, tels que les groupes amine, hydroxyle, thiol ou carboxy, présents sur l'une quelconque des molécules concernées. Cette protection peut s'effectuer en utilisant les groupes protecteurs conventionnels,

tels que ceux décrits dans Protective Groups in Organic Chemistry, J.F.W. McOmie, Ed. Plenum Press, 1973 et dans Protective Groups in Organic Synthesis, T.W. Greene et P.G.M. Wutts, Ed. John Wiley et Sons, 1991. L'élimination des groupes protecteurs peut s'effectuer à une étape ultérieure opportune en utilisant les méthodes connues de l'homme de l'art et qui n'affectent pas le reste de la molécule concernée.

L'affinité des composés selon l'invention pour les récepteurs de la vasopressine a été déterminée in vitro en utilisant la méthode décrite dans C. J. Lynch et al., J. Biol. Chem., 1985, 260 (5), 2844-2851. Cette méthode consiste à étudier le déplacement de la vasopressine tritiée fixée aux sites $V_1$ de membranes de foie de rats. Les concentrations inhibitrices de 50 % ($CI_{50}$) de la fixation de la vasopressine tritiée des composés selon l'invention sont faibles, allant jusqu'à $10^{-9}$M.

L'affinité des composés (I) selon l'invention pour les récepteurs $V_2$ a été mesurée sur une préparation membranaire de rein de boeuf selon une méthode adaptée de P. Crause et al., Molecular and Cellular Endocrinology, 1982, 28, 529-541 et de F.L. Stassen et al., J. Pharmacol. Exp. Ther., 1982, 223, 50-54. Les composés selon l'invention inhibent la fixation de l'arginine-vasopressine tritiée aux récepteurs de la préparation membranaire. Les $CI_{50}$ des composés selon l'invention sont faibles, allant jusqu'à $10^{-9}$M.

L'activité antagoniste des récepteurs $V_2$ des composés selon l'invention a été montrée par le test de dosage de l'activité adénylate cyclase effectué selon une méthode adaptée de M. Laburthe et al., Molecular Pharmacol., 1986, 29, 23-27. On utilise une préparation membranaire de rein de boeuf et chaque produit est incubé 10 minutes à 37°C, seul ou en présence d'AVP (arginine vasopressine) à la concentration de $3.10^{-8}$M. L'AMP cyclique (adénosine monophosphate cyclique) produite est mesurée par dosage radioimmunologique. On détermine la concentration inhibant de 50 %($CI_{50}$) la stimulation de l'adénylate cyclase induite par $3.10^{-8}$M d'AVP. Les $CI_{50}$ déterminées sont de l'ordre de $10^{-7}$M, allant jusqu'à $10^{-9}$M.

L'activité agoniste ou antagoniste des récepteurs de la vasopressine des composés selon l'invention, administrés par voie orale, est évaluée chez le rat (Souche OFA, Sprague-Dawley) en surcharge hydrique, traité à la vasopressine. L'activité antagoniste des composés, selon l'invention, a été également évaluée chez le rat normalement hydraté (souche OFA, Sprague-Dawley) selon la technique décrite dans Br. J. Pharmacol., 1992, 105, 787-791. L'effet diurétique a été observée pour certains composés à la dose de 10 mg/kg.

De même, l'affinité des composés (I) selon l'invention pour les récepteurs de l'ocytocine a été déterminée in vitro par déplacement d'un analogue radioiodé de l'ocytocine fixé aux récepteurs d'une préparation membranaire de glandes mammaires de rates en gestation, selon une technique proche de celle décrite par J. Eland et al. dans Eur. J. Pharmacol., 1987, 147, 197-207. Les $CI_{50}$ des composés selon l'invention atteignent $10^{-9}$M.

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement ou la prévention de différentes affections vasopressine-dépendantes ou ocytocine dépendantes, les affections cardiovasculaires, comme l'hypertension, l'hypertension pulmonaire, l'insuffisance cardiaque, l'infarctus du myocarde, ou le vasospasme coronaire, en particulier chez le fumeur, les angines instables et PTCA (percutaneous transluminal coronary angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, les états psychotiques, les troubles de la mémoire par exemple ; les affections du système rénal comme les oedèmes, le vasospasme rénal, la nécrose du cortex rénal, l'hyponatrémie, l'hypokaliémie, le syndrome de Schwartz Bartter ; les affections du système gastrique comme le vasospasme gastrique, l'hépatocirrhose, les ulcères, la pathologie des vomissements, par exemple la nausée y compris la nausée due à une chimiothérapie, le mal des transports, ou encore le syndrome de la sécrétion inappropriée de l'hormone antidiurétique (SIADH), le diabète insipide et l'énurésie. Les composés selon l'invention peuvent également être utilisés dans le traitement des troubles du comportement sexuel ; chez la femme, les composés selon l'invention peuvent être utilisés pour traiter la dysménorrhée ou le travail prématuré. On peut également utiliser les composés selon l'invention dans le traitement des cancers pulmonaires à petites cellules, des encéphalopathies hyponatrémiques, de la maladie de Raynaud, le syndrome pulmonaire, le glaucome, la cataracte et dans les traitements postopératoires, notamment après une chirurgie abdominale.

La présente invention a également pour objet des compositions pharmaceutiques contenant une dose efficace d'un composé selon l'invention ou d'un sel pharmaceutiquement acceptable de celui-ci et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées

comprennent les formes par voie orale telles que les comprimés, les gélules, les poudres, les granules et les solutions ou suspensions orales, les formes d'administration sublinguale, buccale, intratrachéale, intranasale, les aérosols, les implants, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la silice ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en incorporant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le polyéthylèneglycol.

Le principe actif peut être formulé également sous forme de microcapsules ou microsphères, éventuellement avec un ou plusieurs supports ou additifs.

Les compositions de la présente invention peuvent contenir, à côté des produits de formule (I) ci-dessus ou d'un de leurs sels pharmaceutiquement acceptables, d'autres principes actifs qui peuvent être utiles dans le traitement des troubles ou maladies indiqués ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur le système rénine-angiotensine tel qu' un inhibiteur de l'enzyme de conversion, un antagoniste de l'angiotensine II, un inhibiteur de la rénine. On peut également associer un composé selon l'invention, par exemple, avec un vasodilatateur périphérique, un inhibiteur calcique, un béta-bloquant, un alpha-1-bloquant ou un diurétique. De telles compositions seront utiles en particulier dans le traitement de l'hypertension ou de la défaillance cardiaque.

On peut également associer deux composés selon l'invention : un antagoniste spécifique du récepteur $V_1$ à un antagoniste spécifique du récepteur $V_2$ ou bien un antagoniste spécifique du récepteur $V_1$ à un antagoniste spécifique de l'ocytocine.

Ces associations permettront de renforcer les activités thérapeutiques des composés selon l'invention.

L'invention va être décrite maintenant plus en détail par les préparations et exemples illustratifs non limitatifs ci-après.

## PREPARATIONS

Préparations des 1,3-dihydro-indol-2-one (II).

Préparation 1

3-Amino-5-chloro-1,3-dihydro-3-phénylindol-2-one.

A) 5-Chloro-1,3-dihydro-3-phénylindol-2-one.

On prépare ce composé selon Aeberli P. et Houlikan W.J. dans J. Org. Chem., 1968, 33 (4), 1640-1643.

B) 3-Bromo-5-chloro-1,3-dihydro-3-phénylindol-2-one.

On prépare ce composé selon Bolotov V.V. et coll. dans Farm. Zh. (Kiev), 1976, 5, 30-33.

On chauffe à reflux un mélange de 3,0 g du composé obtenu à l'étape précédente dans 80 ml de $CCl_4$ et ajoute lentement une solution de 2,5 g de brome dans 3 ml de $CCl_4$. Après 30 minutes de reflux, on refroidit le mélange réactionnel et évapore sous vide. On utilise le produit attendu tel quel à l'étape suivante.

C) 3-Amino-5-chloro-1,3-dihydro-3-phénylindol-2-one.

On refroidit à 0°C une solution du composé obtenu à l'étape précédente dans 30 ml d'éther et fait passer un courant d'ammoniac gazeux. Après 72 heures d'agitation à TA, on évapore sous vide, reprend le résidu à l'eau, extrait à l'AcOEt, lave à l'eau, sèche sur sulfate de sodium et évapore sous vide. On chromatographie sur silice en éluant par le mélange DCM/AcOEt/hexane (40/40/20 ; v/v/v). On obtient le produit attendu après cristallisation dans le mélange AcOEt/MeOH, m = 1,4 g, F = 230-235°C.

Préparation 2

3-Amino-5-chloro-3-(2-chlorophényl)-1,3-dihydroindol-2-one.

A) N-*p*-chlorophényl-DL-2-chloromandélamide.

On chauffe à 200°C pendant 7 heures un mélange de 38,25 g de *p*-chloroaniline, 55,95 g d'acide DL-2-chloromandélique dans 280 ml de 1,2-dichlorobenzène en éliminant l'eau formée à l'aide d'un appareil de Dean-Stark. On concentre sous vide, en partie, le mélange réactionnel et laisse en cristallisation. On essore le produit cristallisé formé et le lave à l'éther iso. On obtient 44,92 g du produit attendu.

B) 5-Chloro-3-(2-chlorophényl)-1,3-dihydroindol-2-one.

On refroidit à 10°C 172 ml d'acide sulfurique concentré (95 %), ajoute 39 ml d'acide sulfurique fumant (oléum à 30 %) puis par portions 42,92 g du composé obtenu à l'étape précédente en maintenant la température interne en dessous de 40°C. On laisse 24 heures sous agitation à TA, verse le mélange réactionnel sur de l'eau glacée, essore le précipité formé et le lave à l'eau. On dissout le précipité dans du chloroforme, lave la phase organique à l'eau jusqu'à pH = 7, sèche sur sulfate de sodium et évapore sous vide le solvant. On obtient 35 g du produit attendu après cristallisation dans le mélange DCM/THF/éther iso, F = 198-200°C.

C) 3-Bromo-5-chloro-3-(2-chlorophényl)-1,3-dihydroindol-2-one.

On prépare ce composé selon le mode opératoire décrit à l'étape B de la Préparation 1. On peut également préparer ce composé selon le mode opératoire suivant : à une solution de 22,44 g du composé obtenu à l'étape précédente dans 500 ml de chloroforme, on ajoute à TA, lentement, une solution de 11,62 g de brome dans 15 ml de chloroforme. Puis on évapore sous vide, reprend le résidu au DCM et évapore sous vide. On utilise le produit attendu tel quel.

D) 3-Amino-5-chloro-3-(2-chlorophényl)-1,3-dihydroindol-2-one.

On prépare ce composé selon le mode opératoire décrit à l'étape C de la Préparation 1 à partir du composé obtenu à l'étape précédente. On obtient le produit attendu après cristallisation dans le mélange DCM/éther iso, F = 234°C.

Préparation 3.

5-Chloro-3-(2-chlorophényl)-1,3-dihydro-3-(méthylamino)indol-2-one.

A une solution de 4,4 g du composé obtenu à l'étape C de la Préparation 2 dans 30 ml de DCM, on ajoute à TA, 0,460 g de méthylamine gazeuse dissoute dans 4 ml d'éther. Après 30 minutes d'agitation, on extrait au DCM, lave à l'eau, sèche sur sulfate de sodium et évapore sous vide. On chromatographie sur silice en éluant par le mélange AcOEt/hexane (50/50 ; v/v). On obtient le produit attendu après cristallisation dans le mélange DCM/éther iso, m = 0,655 g, F = 230°C.

En opérant de la même façon et en faisant varier l'amine, on obtient les dérivés indol-2-one suivants :

- 5-Chloro-3-(2-chlorophényl)-1,3-dihydro-3-(diméthylamino)indol-2-one, F = 148-151°C.
- 5-Chloro-3-(2-chlorophényl)-3-(diéthylamino)-1,3-dihydroindol-2-one, F = 160-165°C.
- 5-Chloro-3-(2-chlorophényl)-3-(éthylamino)-1,3-dihydroindol-2-one, F = 240-242°C.
- 5-Chloro-3-(2-chlorophényl)-1,3-dihydro-3-(propylamino)indol-2-one, F = 218-220°C.
- 3-(*tert*-Butylamino)-5-chloro-3-(2-chlorophényl)-1,3-dihydroindol-2-one, F = 203-204°C.
- 5-Chloro-3-(2-chlorophényl)-1,3-dihydro-3-(isobutylamino)indol-2-one. F = 185°C.
- 5-Chloro-3-(2-chlorophényl)-1,3-dihydro-3-(pentylamino)indol-2-one. F = 155°C.
- 3-(Benzylamino)-5-chloro-3-(2-chlorophényl)-1,3-dihydroindol-2-one. F = 170-175°C.
- 5-Chloro-3-(2-chlorophényl)-1,3-dihydro-3-[(2-méthoxyéthyl)amino] indol-2-one.

F = 174-176°C.

Préparation 4.

N-[5-chloro-3-(2-chlorophényl)-2,3-dihydro-2-oxoindol-3-yl]glycinate d'éthyle.

A un mélange de 0,500 g du composé obtenu à l'étape C de la Préparation 2, 0,281 g de chlorhydrate de glycinate d'éthyle dans 10 ml de chloroforme, on ajoute lentement 0,435 g de DIPEA. Après 1 heure d'agitation on lave à l'eau, sèche sur sulfate de sodium et évapore sous vide. On chromatographie sur silice en éluant par un mélange DCM/AcOEt (90/10 ; v/v). On obtient le produit attendu après cristallisation dans le mélange DCM/éther iso, m = 0,30 g, F = 175°C.

Préparation 5.

3-(4-*tert*-Butoxycarbonylpipérazin-1-yl)-5-chloro-3-(2-chlorophényl)-1,3-dihydroindol-2-one.

A une solution de 0,525 g de 1-*tert*-butoxycarbonylpipérazine et 0,348 g de DIPEA dans 20 ml de chloroforme on ajoute à TA, une solution de 1 g du composé obtenu à l'étape C de la Préparation 2 dans 5 ml de chloroforme. On laisse 18 heures sous agitation à TA puis lave à l'eau, sèche sur sulfate de sodium et évapore sous vide. On chromatographie sur silice en éluant par le mélange AcOEt/hexane (20/80 ; v/v). On obtient le produit attendu après cristallisation dans le mélange DCM/éther iso, m = 0,54 g, F = 235°C.

Préparation 6.

5-Chloro-1,3-dihydro-3-hydroxy-3-(2-méthoxyphényl)indol-2-one.

On prépare une solution de bromure de 2-méthoxyphénylmagnésium à partir de 1,35 g de magnésium, 10,5 g de 1-bromo-2-méthoxybenzène dans 100 ml d'éther. On ajoute à TA, sous atmosphère d'argon, en 10 minutes, cette solution à un mélange de 4,1 g de 5-chloroisatine dans 70 ml de THF. Après 1 heure d'agitation à TA, on ajoute de l'eau, essore le produit, le redissout dans le THF, sèche sur sulfate de magnésium et évapore sous vide en présence d'éther iso. On chromatographie sur silice en éluant par le mélange DCM/AcOEt (60/40 ; v/v). On obtient le produit attendu après cristallisation dans le mélange THF/éther iso, m = 3,7 g, F = 235-238°C.

Préparation 7.

3-Amino-5-chlore-1,3-dihydro-3-(2-méthoxyphényl)indol-2-one.

A) 5-Chloro-1,3-dihydro-3-(2-méthoxyphényl)-3-(méthylsulfonyloxy)indol-2-one.

On refroidit à -10°C une solution de 0,500 g du composé obtenu à la Préparation 6 dans 7 ml de THF et ajoute 0,350 g de triéthylamine puis 0,400 g de chlorure de méthanesulfonyle. On conserve cette solution pendant 48 heures à une température comprise entre 0 et +20°C et l'utilise telle quelle à l'étape suivante.

B) 3-Amino-5-chloro-1,3-dihydro-3-(2-méthoxyphényl)indol-2-one.

On refroidit à 0°C la solution obtenue à l'étape précédente et introduit par barbottage de l'ammoniac gazeux. On laisse 24 heures sous agitation à TA et évapore sous vide. On reprend le résidu par une solution à 5 % de carbonate de potassium, extrait à l'AcOEt, lave à l'eau, sèche sur sulfate de sodium et évapore sous vide. On chromatographie sur silice en éluant par le mélange DCM/AcOEt (70/30 ; v/v). On obtient le produit attendu après cristallisation dans le mélange DCM/MeOH, m = 0,182 g, F = 231-233°C.

On peut également obtenir ce composé en suivant les deux étapes du procédé décrit ci-après.

A') 3,5-Dichloro-1,3-dihydro-3-(2-méthoxyphényl)indol-2-one.

On refroidit à 0°C un mélange de 2,15 g du composé obtenu à la Préparation 6 dans 75 ml de DCM et ajoute 1,1 ml de pyridine puis 0,75 ml de chlorure de thionyle. Après deux heures d'agitation, on chromatographie directement le mélange réactionnel, sur silice en éluant par du DCM puis par un mélange DCM/AcOEt (80/20 ; v/v). On obtient le produit attendu après cristallisation dans le mélange DCM/AcOEt, m = 1,9 g, F = 220-224°C.

B') 3-Amino-5-chloro-1,3-dihydro-3-(2-méthoxyphényl)indol-2-one.

A une solution de 1,9 g du composé obtenu à l'étape précédente dans 80 ml de THF, on ajoute à TA 10 ml d'une solution d'ammoniac dans le THF (1,16 g d'ammoniac gazeux dans 110 ml de THF). Après 2 heures d'agitation à TA, on rajoute 10 ml de la solution d'ammoniac dans le THF et laisse 30 heures sous agitation. On évapore sous vide le solvant, reprend par une solution à 5 % de carbonate de potassium et extrait à l'AcOEt. On filtre un insoluble qui est le produit attendu et le recristallise dans le mélange THF/MeOH, F = 231-232°C. Pour obtenir un

second lot, on reprend la phase organique d'AcOEt, lave à l'eau, sèche sur sulfate de sodium et évapore sous vide. On chromatographie sur silice en éluant par un mélange DCM/AcOEt (50/50 ; v/v). On obtient au total 0,88 g du produit attendu.

Préparation 8.

3-Azido-5-chloro-3-(2-chlorophényl)-1,3-dihydroindol-2-one.

On chauffe à reflux pendant 1 heure un mélange de 3,00 g du composé obtenu à l'étape C de la Préparation 2, 1,65 g d'azidure de sodium dans 100 ml d'acétonitrile. Après évaporation sous vide, on reprend le résidu à l'eau, extrait à l'AcOEt, lave à l'eau, par une solution saturée de NaCl, sèche sur sulfate de sodium et évapore sous vide. On obtient le produit attendu après cristallisation dans le mélange DCM/éther iso/THF, m = 0,894 g , F =178°C.

Préparations 9 et 10.

3-Amino-5,6-dichloro-1,3-dihydro-3-phénylindol-2-one (préparation 9) et
3-Amino-4,5-dichloro-1,3-dihydro-3-phénylindol-2-one (préparation 10).

A) 5,6-Dichloro-1,3-dihydro-3-phénylindol-2-one et 4,5-dichloro-1,3-dihydro-3-phénylindol-2-one.

On prépare le mélange de ces deux composés selon le mode opératoire décrit à l'étape B de la Préparation 2, à partir du N-3,4-dichlorophényl-D,L-mandélamide obtenu selon le mode opératoire décrit à l'étape A de la Préparation 2 à partir de 3,4-dichloroaniline et d'acide DL-mandélique. On obtient le mélange des deux produits attendus après recristallisation dans l'EtOH.

B) 3-Bromo-5,6-dichloro-1,3-dihydro-3-phénylindol-2-one et 3-bromo-4,5-dichloro-1,3-dihydro-3-phénylindol-2-one.

A une suspension de 4,71 g du mélange des deux composés obtenus à l'étape précédente dans 70 ml de chloroforme, on ajoute à TA, en 5 minutes, une solution de 2,6 g de brome dans 2 ml de chloroforme. On laisse 30 minutes sous agitation et évapore sous vide. On obtient le mélange des deux produits attendus qui est utilisé tel quel à l'étape suivante.

C) 3-Amino-5,6-dichloro-1,3-dihydro-3-phénylindol-2-one (Préparation 9) et
3-Amino-4,5-dichloro-1,3-dihydro-3-phénylindol-2-one (Préparation 10).

On refroidit à 0°C une solution du mélange des deux composés obtenus à l'étape précédente dans 20 ml de THF et ajoute une solution de 0,60 g d'ammoniac gazeux dissous dans 10 ml de THF. Après deux heures d'agitation, on rajoute une solution de 0,30 g d'ammoniac gazeux dissous dans 5 ml de THF et laisse 48 heures sous agitation en laissant remonter la température à TA. On évapore sous vide le milieu réactionnel, extrait à l'AcOEt, lave à l'eau, sèche sur sulfate de sodium et évapore sous vide. On chromatographie sur silice en éluant par le mélange DCM/AcOEt (75/25 ; v/v) et obtient le produit de la Préparation 9 après cristallisation dans le mélange DCM/AcOEt. Puis on élue par le mélange DCM/AcOEt (40/60 ; v/v) et obtient le produit de la Préparation 10 après cristallisation dans le mélange DCM/éther iso,

- composé de la Préparation 9, F = 211-212°C,
- composé de la Préparation 10, F = 185-187°C.

Préparation 11.

5-Ethoxy-3-(2-éthoxycarbonyl-1-éthoxycarbonylhydrazino)-1,3-dihydro-3-phénylindol-2-one.

A) 5-Ethoxy-1,3-dihydro-3-phénylindol-2-one.

On prépare ce composé selon le mode opératoire décrit à l'étape B de la Préparation 66, à partir du N-4-éthoxyphényl-D,L-mandélamide (138°C) obtenu selon le mode opératoire décrit à l'étape A de la Préparation 2 à partir de 4-éthoxyaniline et d'acide DL-mandélique. On obtient le produit attendu, F = 125-130°C.

B) 5-Ethoxy-3-(2-éthoxycarbonyl-1-éthoxycarbonylhydrazino)-1,3-dihydro-3-phénylindol-2-one.

On refroidit à -70°C un mélange de 3,42 g du composé obtenu à l'étape précédente dans 30 ml de THF et ajoute lentement, sous atmosphère d'argon, 20 ml d'une solution 1,5 M de diisopropylamidure de lithium dans le cyclohexane. On laisse agiter 15 minutes en laissant remonter la température à -20°C puis refroidit à -70°C et ajoute en une seule fois 2,38 g d'azodicarboxylate de diéthyle. Après 30 minutes d'agitation, on ajoute de l'eau, laisse remonter la température à TA et évapore sous vide le solvant. On extrait à l'AcOEt, lave à l'eau, par une solution saturée de NaCl, sèche sur sulfate de sodium et évapore sous vide. On chromatographie sur silice en éluant par le mélange DCM/AcOEt (65/35 ; v/v). On obtient le produit attendu après cristallisation dans le mélange

DCM/éther iso, m = 2,7 g, F = 202-204°C.

Préparations 12 et 13.

5-Chloro-3-(2-chlorophényl)-1,3-dihydro-3-[[(1S)-1-(méthoxycarbonyl) éthyl]amino]indol-2-one, isomère A et isomère B.

A un mélange de 2,00 g du composé obtenu à l'étape C de la Préparation 2, 1,12 g de chlorhydrate de (L)-alaninate de méthyle dans 40 ml de chloroforme, on ajoute 2,04 g de DIPEA. Après 18 heures d'agitation on lave à l'eau, sèche sur sulfate de sodium et évapore sous vide. On chromatographie sur silice en éluant par le mélange AcOEt/hexane (40/60 ; v/v). On sépare les deux isomères :

- le moins polaire isomère A : composé de la Préparation 12, m = 0,639 g, F = 214°C, $\alpha_D^{20}$ = +137,2° (c = 0,53 ; chloroforme)
- le plus polaire isomère B : composé de la Préparation 13, m = 0,400 g, F = 165°C, $\alpha_D^{20}$ = -155,2° (c = 0,38 ; chloroforme).

Préparation 14.

5-Chloro-1,3-dihydro-3-hydroxy-3-(2-méthylphényl)indol-2-one.

On prépare une solution de bromure de 2-méthylphénylmagnésium à partir de 2,7 g de magnésium, 19 g de 1-bromo-2-méthylbenzène et 100 ml d'éther. On refroidit au bain de glace, sous atmosphère d'argon, un mélange de 5,04 g de 5-chloroisatine dans 100 ml de THF et ajoute lentement la solution de magnésien préparée ci-dessus. Après 4 heures d'agitation on ajoute de l'eau et évapore sous vide les solvants. On extrait à l'AcOEt, filtre le précipité formé, sèche la phase organique du filtrat sur sulfate de magnésium et évapore sous vide. On cristallise dans le MeOH le résidu obtenu joint au précipité filtré précédemment. On obtient 4,8 g du produit attendu, F = 285-295°C.

Préparation 15.

3-Amino-5-chloro-1,3-dihydro-3-(2-méthylphényl)indol-2-one.

A) 3,5-Dichloro-1,3-dihydro-3-(2-méthylphényl)indol-2-one.

On refroidit à 0°C un mélange de 2 g de composé obtenu à la Préparation 14 dans 72 ml de DCM et ajoute 1,04 ml de pyridine puis 0,76 ml de chlorure de thionyle. Après 15 minutes d'agitation, on chromatographie directement le mélange réactionnel, sur silice en éluant par un gradient du mélange DCM/AcOEt de (95/5 ; v/v) jusqu'à (80/20 ; v/v). On obtient le produit attendu qui est utilisé tel quel à l'étape suivante.

B) 3-Amino-5-chloro-1,3-dihydro-3-(2-méthylphényl)indol-2-one.

On refroidit à 0°C une solution de 1,97 g du composé obtenu à l'étape précédente dans 80 ml de THF et ajoute une solution de 0,230 g d'ammoniac gazeux dissout dans 20 ml de THF. Après 24 heures d'agitation à TA, on ajoute de l'eau, évapore le solvant, extrait à l'AcOEt, lave à l'eau, sèche sur sulfate de sodium et évapore sous vide. On chromatographie sur silice en éluant par le gradient du mélange DCM/AcOEt de (80/20 ; v/v) jusqu'à (60/40 ; v/v). On obtient le produit attendu après cristallisation dans le mélange MeOH/éther iso, m = 1,10 g, F = 218-222°C.

Préparation 16.

3-Amino-3-(2-chlorophényl)-5-éthoxy-1,3-dihydroindol-2-one.

A) 3-(2-chlorophényl)-5-éthoxy-1,3-dihydroindol-2-one.

On prépare ce composé selon le mode opératoire décrit à l'étape B de la Préparation 66, à partir du N-4-éthoxyphényl-DL-2-chloromandélamide (120-130°C) obtenu selon le mode opératoire décrit à l'étape A de la Préparation 2 à partir de 4-éthoxyaniline et d'acide DL-2-chloromandélique. On obtient le produit attendu, F = 176-180°C.

B) 3-Chloro-3-(2-chlorophényl)-5-éthoxy-1,3-dihydroindol-2-one.

A une suspension de 2,5 g du composé obtenu à l'étape précédente dans 100 ml de $CCl_4$ on ajoute 1,16 g de N-chlorosuccinimide et chauffe à reflux pendant 1 heure. Après refroidissement à TA on filtre l'insoluble et évapore sous vide le filtrat. On chromatographie sur silice en éluant par du DCM puis par le mélange DCM/AcOEt (95/5 ; v/v). On obtient le produit attendu après recristallisation dans le mélange DCM/hexane, m = 1,46 g, F = 128-132°C.

On peut également obtenir ce composé en suivant les deux étapes du procédé décrit ci-après.

A') 3-(2-Chlorophényl)-5-éthoxy-1,3-dihydro-3-hydroxyindol-2-one.

On prépare ce composé selon le mode opératoire décrit à la Préparation 6 à partir de 5-éthoxyisatine et de bromure de 2-chlorophénylmagnésium (préparé selon P.G. Gassman dans J.Am.Chem.Soc., 1974, 96, 5512).

B') 3-Chloro-3-(2-chlorophényl)-5-éthoxy-1,3-dihydroindol-2-one.

On prépare ce composé selon le mode opératoire décrit à l'étape A' de la Préparation 7 à partir du composé obtenu à l'étape précédente et du chlorure de thionyle, F = 128-132°C.

C) 3-Azido-3-(2-chlorophényl)-5-éthoxy-1,3-dihydroindol-2-one.

A une solution de 0,560 g du composé obtenu à l'étape B ou B' dans 20 ml d'acétonitrile on ajoute 0,339 g d'azidure de sodium et chauffe à reflux pendant 30 minutes. On évapore sous vide le solvant, reprend le résidu à l'eau, extrait à l'AcOEt, lave par une solution saturée de NaCl, à l'eau, par une solution aqueuse à 5 % d'hydrosulfite de sodium, reextrait au DCM, sèche sur sulfate de sodium et évapore sous vide. On chromatographie sur silice en éluant par du DCM puis par le mélange DCM/AcOEt (95/5 ; v/v). On obtient 0,377 g du produit attendu, spectre infrarouge dans le DCM : 3440 $cm^{-1}$, 2100 $cm^{-1}$, 1745 $cm^{-1}$.

D) 3-Amino-3-(2-chlorophényl)-5-éthoxy-1,3-dihydroindol-2-one.

A une solution de 0,975 g du composé obtenu à l'étape précédente dans 14,8 ml de MeOH on ajoute, sous atmosphère d'argon, à la seringue, 0,83 ml de triéthylamine puis 0,60 ml de propane-1,3-dithiol et chauffe à 60°C pendant une nuit. On évapore sous vide le mélange réactionnel et chromatographie directement le résidu sur silice en éluant par du DCM puis par un mélange DCM/AcOEt (70/30 ; v/v). On obtient 0,786 g du produit attendu dont un échantillon est recristallisé dans le mélange DCM/éther iso, F = 173-175°C.

Préparation 17.

5-Chloro-3-cyclohexyl-1,3-dihydro-3-hydroxyindol-2-one.

On refroidit à +4°C une suspension de 18,15 g de 5-chloroisatine dans 45 ml de THF, ajoute, goutte à goutte, 200 ml d'une solution 2M de chlorure de cyclohexylmagnésium dans l'éther et laisse 3 heures sous agitation à TA. On ajoute 900 ml d'une solution saturée de chlorure d'ammonium et concentre sous vide les solvants. On extrait la phase aqueuse à l'AcOEt, lave la phase organique par une solution saturée de NaCl, sèche sur sulfate de sodium et évapore sous vide le solvant. On obtient 4 g du produit attendu après cristallisation dans le mélange THF/AcOEt.

Spectre de RMN à 200 MHz dans DMSO-$d_6$

0,4 à 2,0 ppm : m : 11H
5,9 ppm : s : 1H
6,8 ppm : d : 1H
7,2 ppm : m : 2H
10,35 ppm : s : 1H

Préparation 18

5-Chloro-3-cyclohexyl-1,3-dihydro-3-(méthylamino)indol-2-one.

A) 3,5-Dichloro-3-cyclohexyl-1,3-dihydroindol-2-one.

On refroidit à +4°C une solution de 1 g du composé obtenu à la Préparation 17 dans 40 ml de DCM et ajoute 0,57 ml de pyridine puis 0,42 ml de chlorure de thionyle. Après 10 minutes d'agitation, on chromatographie directement le mélange réactionnel sur silice en éluant au DCM puis par le mélange DCM/AcOEt (95/5 ; v/v). On obtient 1 g du produit attendu que l'on utilise tel quel à l'étape suivante.

B) 5-Chloro-3-cyclohexyl-1,3-dihydro-3-(méthylamino)indol-2-one.

A une solution de 1 g du composé obtenu à l'étape précédente dans 15 ml de THF, on ajoute à TA 0,88 ml d'une solution à 33 % de méthylamine dans l'EtOH et laisse 24 heures sous agitation. On ajoute de l'eau au mélange réactionnel, concentre sous vide le solvant, extrait la phase aqueuse à l'AcOEt, lave la phase organique par une solution saturée de NaCl, sèche sur sulfate de sodium et évapore sous vide le solvant. On chromatographie le résidu sur silice en éluant au DCM puis par le mélange DCM/AcOEt (80/20 ; v/v). On obtient 0,85 g du produit attendu après cristallisation dans le mélange DCM/éther iso, F = 195-198°C.

Préparation 19

5-Chloro-3-(cyclohexylméthyl)-1,3-dihydro-3-hydroxyindol-2-one.

On prépare une solution de bromure de cyclohexylméthylmagnésium à partir de 5,37 g de magnésium, 30,8 ml de bromométhylcyclohexane dans 15 ml d'éther. On ajoute goutte à goutte à +4°C, sous atmosphère d'argon, cette solution à un mélange de 10 g de 5-chloroisatine dans 25 ml de THF. Après 3 heures d'agitation à TA, on ajoute 500 ml d'une solution saturée de chlorure d'ammonium, extrait à l'AcOEt, lave la phase organique par une solution saturée de NaCl, à l'eau, sèche sur sulfate de sodium et évapore sous vide le solvant. On obtient 12 g du produit attendu après cristallisation dans l'AcOEt et recristallisation dans le mélange THF/AcOEt, F = 252-253°C (déc).

On peut également obtenir ce composé en suivant les trois étapes du procédé décrit ci-après.

A') 5-Chloro-3-(cyclohexylméthylène)-1,3-dihydroindol-2-one.

On chauffe à reflux pendant 1 heure une solution de 10 g de 5-chlore-1,3-dihydroindol-2-one, 6,18 g de cyclohexanecarboxaldéhyde, 4,93 ml de pyrrolidine dans 250 ml de toluène en éliminant l'eau formée à l'aide d'un appareil de Dean-Stark. On concentre le mélange réactionnel à environ 90 ml et laisse en cristallisation. On essore le produit cristallisé formé et le lave au toluène puis à l'éther iso. On obtient 9,7 g du produit attendu après recristallisation dans le toluène, F = 203-204°C.

B') 5-Chloro-3-(cyclohexylméthyl)-1,3-dihydroindol-2-one.

A un mélange de 7,5 g du composé obtenu à l'étape précédente dans 60 ml de MeOH et 60 ml de THF, on ajoute par portions 1,08 g de borohydrure de sodium et laisse 30 minutes sous agitation à TA. On concentre sous vide le solvant, extrait le résidu à l'AcOEt, lave la phase organique par une solution saturée de NaCl, à l'eau, sèche sur sulfate de sodium et évapore sous vide le solvant. On chromatographie le résidu sur silice en éluant au DCM, puis par le mélange DCM/AcOEt (90/10 ; v/v). On obtient 3,76 g du produit attendu après cristallisation dans le mélange DCM/éther iso, F = 152-153°C.

C') 5-Chloro-3-(cyclohexylméthyl)-1,3-dihydro-3-hydroxyindol-2-one.

A une solution de 0,5 g du composé obtenu à l'étape précédente dans 30 ml de THF, on ajoute 0,083 g d'hydrure de sodium à 60 % dans l'huile et laisse 10 minutes sous agitation à TA. Puis on introduit 0,22 ml de diméthyldisulfure et laisse 1 heure sous agitation à TA. On ajoute 200 ml d'eau au mélange réactionnel, essore le précipité formé et le lave à l'eau puis à l'éther iso. On obtient 0,45 g du produit attendu.

Préparation 20

5-Chloro-3-(cyclohexylméthyl)-1,3-dihydro-3-(méthylamino)indol-2-one.

A) 3,5-Dichloro-3-(cyclohexylméthyl)-1,3-dihydroindol-2-one.

On refroidit à +4°C une solution de 1 g du composé obtenu à la Préparation 19 dans 5 ml de DCM et ajoute 0,56 ml de pyridine puis 0,4 ml de chlorure de thionyle. Après 3 heures d'agitation, on chromatographie directement le mélange réactionnel sur silice en éluant au DCM puis par le mélange DCM/AcOEt (95/5 ; v/v). On obtient 1,1 g du produit attendu que l'on utilise tel quel à l'étape suivante.

B) 5-Chloro-3-(cyclohexylméthyl)-1,3-dihydro-3-(méthylamino)indol-2-one.

A une solution de 0,4 g du composé obtenu à l'étape précédente dans 10 ml de DCM, on ajoute à TA 0,33 ml d'une solution 8,03M de méthylamine dans l'EtOH et laisse 3 heures sous agitation à TA. On concentre sous vide, reprend le résidu par une solution à 5 % d'hydrogénocarbonate de sodium, extrait à l'AcOEt, lave par une solution saturée de NaCl, à l'eau, sèche sur sulfate de sodium et évapore sous vide le solvant. On chromatographie le résidu sur silice en éluant au DCM, puis par le mélange DCM/AcOEt (60/40 ; v/v). On obtient 0,225 g du produit attendu après cristallisation dans le mélange DCM/éther iso, F = 168-169°C.

Préparation 21

5-Chloro-3-[(2-cyanoéthyl)amino]-3-(cyclohexylméthyl)-1,3-dihydroindol-2-one.

A une solution de 0,960 g du composé obtenu à l'étape A de la Préparation 20 dans 5 ml de DCM, on ajoute à TA 1,33 ml de triéthylamine puis 0,412 g de fumarate de 3-aminopropionitrile et chauffe à 50°C pendant 18 heures. On concentre sous vide le mélange réactionnel, reprend le résidu par une solution à 5 % de carbonate de sodium, extrait à l'AcOEt, lave par une solution saturée de NaCl, à l'eau, sèche sur sulfate de sodium et évapore sous vide le solvant. On obtient 0,5 g du produit attendu après cristallisation dans l'AcOEt, F = 208-209°C.

Préparation 22

3-Benzyl-5-chloro-1,3-dihydro-3-hydroxyindol-2-one.

On refroidit à +4°C, une suspension de 10 g de 5-chloroisatine dans 150 ml de THF, ajoute, goutte à goutte, 220 ml d'une solution 1M de chlorure de benzylmagnésium dans l'éther et laisse 3 heures sous agitation à TA. On

ajoute 500 ml d'une solution saturée de chlorure d'ammonium et concentre sous vide les solvants. On extrait la phase aqueuse à l'AcOEt, lave la phase organique par une solution saturée de NaCl, à l'eau, sèche sur sulfate de sodium et évapore sous vide le solvant. On obtient 5,6 g du produit attendu après cristallisation dans le mélange THF/AcOEt, F = 204-205°C.

Préparation 23

3-Benzyl-5-chloro-1,3-dihydro-3-(méthylamino)indol-2-one.

A) 3-Benzyl-3,5-dichloro-1,3-dihydroindol-2-one.

On refroidit à +4°C une suspension de 4,63 g du composé obtenu à la Préparation 22 dans 25 ml de THF et ajoute 2,8 ml de pyridine puis 2 ml de chlorure de thionyle. Après 3 heures d'agitation à +4°C, on concentre sous vide le mélange réactionnel. On chromatographie le résidu sur silice en éluant au DCM puis par le mélange DCM/AcOEt (97/3 ; v/v). On obtient 1,2 g du produit attendu que l'on utilise tel quel à l'étape suivante.

B) 3-Benzyl-5-chloro-1,3-dihydro-3-(méthylamino)indol-2-one.

A une solution de 1,2 g du composé obtenu à l'étape précédente dans 3 ml de DCM, on ajoute à TA 1,02 ml d'une solution 8,03M de méthylamine dans l'EtOH et laisse 6 heures sous agitation à TA. On ajoute 50 ml d'eau au mélange réactionnel, extrait à l'AcOEt, lave par une solution saturée de NaCl, à l'eau, sèche la phase organique sur sulfate de sodium et évapore sous vide le solvant. On chromatographie le résidu sur silice en éluant au DCM puis par le mélange DCM/AcOEt (60/40 ; v/v). On obtient 0,8 g du produit attendu après cristallisation dans le mélange DCM/éther iso, F = 196°C.

Préparations 24 et 25

5-Chloro-3-(2-chlorophényl)-1,3-dihydro-3-[[(1S)-2-hydroxy-1-phényléthyl]amino] indol-2-one, isomère A et isomère B.

On laisse 3 jours sous agitation à TA, un mélange de 5 g du composé obtenu à l'étape C de la Préparation 2, 3,83 g de (S)-(+)-2-phénylglycinol dans 100 ml de chloroforme. On verse le mélange réactionnel dans une solution saturée de carbonate de potassium, extrait à l'AcOEt et essore le composé insoluble à l'interphase (isomère B : composé de la Préparation 25). Après décantation du filtrat, on lave 4 fois la phase organique à l'eau, sèche sur sulfate de sodium et évapore sous vide le solvant. On chromatographie le résidu sur silice en éluant par le mélange DCM/AcOEt (80/20 ; v/v). On sépare un isomère :

- le moins polaire, isomère A : composé de la Préparation 24, m = 2,3 g, F = 170-172°C après cristallisation dans le mélange DCM/éther iso,

$$\alpha_D^{25} = +263° \text{ (c = 0,38 ; chloroforme);}$$

En éluant par le mélange DCM/MeOH (50/50 ; v/v) on obtient l'autre isomère :

- le plus polaire, isomère B : composé de la Préparation 25, m = 1,64 g (insoluble et chromatographie), F = 294°C après recristallisation dans le mélange MeOH/HF,

$$\alpha_D^{25} = -31{,}8° \text{ (c = 0,21 ; DMF) ;}$$

Préparations 26 et 27

5-Chloro-3-(2-chlorophényl)-1,3-dihydro-3-[[(1R)-2-hydroxy-1-phényléthyl]amino] indol-2-one, isomère A et isomère B.

On laisse 12 heures sous agitation à TA, un mélange de 5 g du composé obtenu à l'étape C de la Préparation 2, 3,83 g de (R)-(-)-2-phénylglycinol dans 200 ml de chloroforme. On essore le composé insoluble (isomère B: composé de la Préparation 27). On concentre sous vide le filtrat et chromatographie le résidu sur silice en éluant par le gradient du mélange DCM/AcOEt de (80/20 ; v/v) à (70/30 ; v/v). On sépare les deux isomères :

- le moins polaire, isomère A : composé de la Préparation 26, m = 2,43 g, F = 168°C après cristallisation dans le mélange DCM/éther iso,

$$\alpha_D^{25} = -268° \text{ (c = 0,28 ; chloroforme) ;}$$

- le plus polaire, isomère B : composé de la Préparation 27. On met le composé insoluble, obtenu ci-dessus, en suspension dans 500 ml d'AcOEt et lave 6 fois par une solution à 5 % de carbonate de sodium. On essore à nouveau le composé insoluble, le joint avec le composé le plus polaire obtenu après chromatographie et recristallise dans le mélange MeOH/THF, m = 1,49 g, F = 293-298°C,

$$\alpha_D^{25} = +39,4° \text{ (c = 0,2 ; DMF);}$$

Préparation 28

3-Amino-5-chloro-3-(2-chlorophényl)-1,3-dihydroindol-2-one, isomère (+).

On refroidit au bain de glace une solution de 2,12 g du composé obtenu à la Préparation 24 (isomère A) dans 40 ml de DCM et 20 ml de MeOH, ajoute 2,48 g de tétraacétate de plomb et laisse 40 minutes sous agitation à 0°C. On ajoute au mélange réactionnel une solution saturée d'hydrogénocarbonate de sodium, concentre sous vide les solvants organiques, extrait la phase aqueuse à l'AcOEt, lave la phase organique à l'eau, sèche sur sulfate de sodium et évapore sous vide le solvant. On reprend le résidu dans 80 ml d'une solution d'HCl 5N, ajoute 10 ml d'éther et laisse 10 minutes sous agitation à TA. Après décantation, on lave deux fois la phase aqueuse à l'éther, alcanilise la phase aqueuse par ajout d'une solution à 5 % de carbonate de potassium, extrait à l'AcOEt, lave la phase organique à l'eau, par une solution saturée de NaCl, sèche sur sulfate de sodium et évapore sous vide le solvant. On chromatographie le résidu sur silice en éluant par le mélange DCM/AcOEt (70/30 ; v/v). On obtient 1,25 g du produit attendu après cristallisation dans le mélange DCM/éther iso, F = 174-176°C,

$$\alpha_D^{25} = +118° \text{ (c = 0,47 ; chloroforme) ;}$$

Préparation 29

3-Amino-5-chloro-3-(2-chlorophényl)-1,3-dihydroindol-2-one, isomère (-).

On prépare ce composé selon le mode opératoire décrit à la Préparation 28, à partir de 2,3 g du composé obtenu à la Préparation 26 (isomère A), 2,72 g de tétraacétate de plomb dans 40 ml de DCM et 20 ml de MeOH. On chromatographie sur silice en éluant par le mélange DCM/AcOEt (75/25 ; v/v). On obtient 0,76 g du produit attendu après cristallisation dans le mélange DCM/éther iso, F = 175-176°C,

$$\alpha_D^{25} = -121° \text{ (c = 0,26 ; chloroforme) ;}$$

Préparation 30

5-Chloro-3-(2-chlorophényl)-1,3-dihydro-3-(isopentylamino)indol-2-one.

On laisse 1 heure sous agitation à TA un mélange de 1 g du composé obtenu à l'étape C de la Préparation 2, 0,468 g d'isopentylamine dans 20 ml de chloroforme. On lave le mélange réactionnel par une solution à 5 % de carbonate de sodium, puis, après décantation, sèche la phase organique sur sulfate de sodium et évapore sous vide le solvant. On chromatographie le résidu sur silice en éluant par le mélange DCM/AcOEt (95/5 ; v/v). On obtient 0,78 g du produit attendu après cristallisation dans le mélange DCM/éther iso/hexane, F = 152°C.

Préparation 31

5-Chloro-3-(2-chlorophényl)-3-[(1-éthoxycarbonylpipérid-4-yl)amino]-1,3-dihydroindol-2-one.

On laisse 2 heures sous agitation à TA un mélange de 5 g du composé obtenu à l'étape C de la Préparation 2, 2,55 g de 4-aminopipéridine-1-carboxylate d'éthyle, 1,91 g de DIPEA dans 100 ml de chloroforme. On lave le mélange réactionnel à l'eau puis, après décantation, sèche la phase organique sur sulfate de sodium et évapore sous vide le solvant. On chromatographie le résidu sur silice en éluant par le mélange DCM/AcOEt (90/10 ; v/v). On obtient 5 g du produit attendu après cristallisation dans la mélange DCM/éther iso, F = 204°C.

Préparation 32

5-Chloro-3-(2-chlorophényl)-1,3-dihydro-3-[[2-(3-méthoxyphényl)éthyl] amino] indol-2-one.

A une solution de 2 g du composé obtenu à l'étape C de la Préparation 2 dans 40 ml de DCM, on ajoute à TA 1,63 ml de 3-méthoxyphénéthylamine et laisse 1 heure sous agitation. On lave le mélange réactionnel par une solution à 5 % de carbonate de potassium, puis après décantation sèche sur sulfate de sodium et évapore sous vide le solvant. On chromatographie le résidu sur silice en éluant au DCM puis par le mélange DCM/AcOEt (95/5 ; v/v). On obtient 2 g du produit attendu après cristallisation dans le mélange DCM/éther iso, F = 155-160°C.

Préparation 33

5-Chloro-3-(2-chlorophényl)-1,3-dihydro-3-[[2-(pyrid-2-yl)éthyl]amino] indol-2-one.

On laisse 2 heures sous agitation à TA, un mélange de 5 g du composé obtenu à l'étape C de la Préparation 2, 3,56 g de 2-(2-aminoéthyl)pyridine dans 50 ml de chloroforme. On lave le mélange réactionnel à l'eau, puis, après décantation, sèche la phase organique sur sulfate de sodium et évapore sous vide le solvant. On chromatographie le résidu sur alumine en éluant par le mélange DCM/AcOEt (50/50 ; v/v). On obtient 3,3 g du produit attendu après cristallisation dans le mélange DCM/éther iso, F = 178°C.

Préparation 34

5-Chloro-3-(2-chlorophényl)-1,3-dihydro-3-[(2-hydroxyéthyl)amino]indol-2-one.

A une suspension de 6 g du composé obtenu à l'étape C de la Préparation 2 dans 20 ml de chloroforme, on ajoute à TA une solution de 1,03 g de 2-aminoéthanol, 3,45 g de triéthylamine dans 10 ml de chloroforme et laisse 3 heures sous agitation à TA. On concentre sous vide, en partie, le mélange réactionnel et essore le précipité formé. On obtient 4 g du produit attendu après cristallisation dans le mélange THF/AcOEt, F = 198-200°C.

Préparation 35

5-Chloro-3-(2-chlorophényl)-1,3-dihydro-3-[[2-(triméthylsilyloxy)éthyl] amino]indol-2-one.

On chauffe à 60°C pendant 4 heures un mélange de 1,5 g du composé obtenu à la Préparation 34, 0,72 g d'hexaméthyldisilane, 0,03 g de chlorure de zinc dans 15 ml d'acétonitrile. On concentre sous vide le mélange réactionnel, extrait le résidu à l'AcOEt, lave deux fois la phase organique à l'eau, par une solution saturée de NaCl, sèche sur sulfate de sodium et évapore sous vide le solvant. On obtient 1,084 g du produit attendu, après trituration dans l'éther iso puis essorage, et on l'utilise tel quel à l'EXEMPLE 114.

Préparation 36

5-Chloro-3-(2-chlorophényl)-1,3-dihydro-3-[[2-[2-(diméthylamine)éthoxy] éthyl]amino]indol-2-one.

A) 2-[2-(*tert*-Butoxycarbonylamino)éthoxy]éthanol.

A une solution de 10,5 g de 2-(2-aminoéthoxy)éthanol dans 50 ml de 1,4-dioxane on ajoute à TA, par portions, 21,8 g de di-*tert*-butyl dicarbonate et laisse 2 heures sous agitation à TA. On verse le mélange réactionnel dans l'eau, extrait trois fois à l'AcOEt, lave la phase organique deux fois par 20 ml d'eau, sèche sur sulfate de sodium et évapore sous vide le solvant. On obtient 18,5 g du produit attendu sous forme d'huile que l'on utilise tel quel à l'étape suivante.

B) Méthanesulfonate de 2-[2-(*tert*-butoxycarbonylamino)éthoxy]éthyle.

On refroidit à 0°C une solution de 18,5 g du composé obtenu à l'étape précédente, 9,2 g de triéthylamine dans 100 ml de DCM et ajoute goutte à goutte une solution de 10,4 g de chlorure de méthanesulfonyle dans 15 ml de DCM en maintenant la température en dessous de 10°C. Après deux jours d'agitation à TA, on concentre sous vide le mélange réactionnel, reprend le résidu à l'AcOEt, lave la phase organique à l'eau, par une solution saturée de NaCl, sèche sur sulfate de sodium et évapore sous vide le solvant. On obtient 24,3 g du produit attendu sous forme d'huile que l'on utilise tel quel à l'étape suivante.

C) N,N-diméthyl-2-[2-(*tert*-butoxycarbonylamino)éthoxy]éthylamine.

On refroidit à 0°C une solution de 8,0 g du composé obtenu à l'étape précédente dans 100 ml d'éther et ajoute goutte à goutte 7,5 g d'une solution à 33 % de diméthylamine dans l'EtOH dilué dans 10 ml d'éther. On laisse 4 jours sous agitation à TA et concentre sous vide le mélange réactionnel. On reprend le résidu dans une solution d'HCl 1N, lave la phase aqueuse acide à l'éther, alcalinise par ajout de carbonate de potassium, extrait 3 fois à l'éther et 1 fois à l'AcOEt, sèche les phases organiques jointes sur sulfate de sodium et évapore sous vide les

solvants. On obtient 1,15 g du produit attendu sous forme d'huile que l'on utilise tel quel à l'étape suivante.

D) 5-Chloro-3-(2-chlorophényl)-1,3-dihydro-3-[[2-[2-(diméthylamino)éthoxy] éthyl] amino]indol-2-one.

On laisse sous agitation à 0°C, pendant 90 minutes, un mélange de 1,15 g du composé obtenu à l'étape précédente dans 10 ml de TFA et concentre sous vide. On dissout le résidu dans une solution de 2 g de triéthylamine dans 30 ml de chloroforme et ajoute goutte à goutte à TA cette solution à une solution de 1,76 g du composé obtenu à l'étape C de la Préparation 2 dans 30 ml de chloroforme. On laisse 3 heures sous agitation et concentre sous vide le mélange réactionnel. On reprend le résidu dans une solution à 5 % de carbonate de potassium, extrait 3 fois à l'AcOEt, lave la phase organique à l'eau, par une solution saturée de NaCl, sèche sur sulfate de sodium et évapore sous vide le solvant. On chromatographie le résidu sur alumine en éluant par le mélange DCM/MeOH (98/2 ; v/v). On obtient 1,15 g du produit attendu après cristallisation dans le mélange DCM/éther iso, F = 165-168°C.

Préparation 37

3-[[2-[2-(*tert*-Butoxycarbonylamino)éthoxy]éthyl]amino]-5-chloro-3-(2-chlorophényl)-1,3-dihydroindol-2-one.

A) 2-[2-(tert-Butoxycarbonylamino)éthoxy]éthylazide.

On laisse 18 heures sous agitation à TA un mélange de 12,4 g du composé obtenu à l'étape B de la Préparation 36, 2,9 g d'azidure de sodium dans 50 ml de DMSO puis chauffe à 80°C pendant 4 heures. On verse le mélange réactionnel dans l'eau, extrait à l'AcOEt, sèche la phase organique sur sulfate de sodium et évapore sous vide le solvant. On chromatographie le résidu sur silice en éluant par le mélange hexane/AcOEt (70/30 ; v/v). On obtient 7,4 g du produit attendu sous forme d'huile que l'on utilise tel quel à l'étape suivante.

B) 2-[2-(tert-Butoxycarbonylamino)éthoxy]éthylamine.

On laisse 4 heures sous agitation à TA un mélange de 7,4 g du composé obtenu à l'étape précédente, 1,5 g de nickel de Raney ® , 1,5 ml d'hydrazine monohydrate dans 60 ml d'EtOH. On ajoute de nouveau 1,5 ml d'hydrazine monohydrate et laisse 2 heures sous agitation à TA. On filtre le catalyseur, le lave à l'EtOH et concentre sous vide le filtrat. On obtient 6,22 g du produit attendu que l'on utilise tel quel à l'étape suivante.

C) 3-[[2-[2-(*tert*-Butoxycarbonylamino)éthoxy]éthyl]amino]-5-chloro-3-(2-chlorophényl)-1,3-dihydroindol-2-one.

A une suspension de 7,0 g du composé préparé à l'étape C de la Préparation 2 dans 40 ml de chloroforme on ajoute en 2 heures à TA une solution de 6,22 g du composé obtenu à l'étape précédente, 2 g de triéthylamine dans 30 ml de chloroforme et laisse 3 heures sous agitation à TA. On concentre sous vide le mélange réactionnel, reprend le résidu par un solution à 5 % de carbonate de potassium, extrait à l'AcOEt, lave la phase organique à l'eau, par une solution saturée de NaCl, sèche sur sulfate de sodium et évapore sous vide le solvant. On chromatographie le résidu sur silice en éluant par le mélange DCM/AcOEt (50/50 ; v/v). On obtient 7,3 g du produit attendu après cristallisation dans le mélange DCM/éther iso, F = 138-140°C.

Préparation 38

5-Chloro-3-(2-chlorophényl)-3-[[2-(diéthylamino)éthyl]amino]-1,3-dihydroindol-2-one.

A une suspension de 5 g du composé obtenu à l'étape C de la Préparation 2 dans 30 ml de DCM, on ajoute à TA et goutte à goutte une solution de 1,13 ml de N,N-diéthyléthylènediamine, 5,82 ml de triéthylamine dans 20 ml de DCM et laisse 3 heures sous agitation à TA. On concentre sous vide le mélange réactionnel, reprend le résidu par une solution à 5 % de carbonate de sodium, extrait à l'AcOEt, lave la phase organique par une solution saturée de NaCl, à l'eau, sèche sur sulfate de sodium et évapore sous vide le solvant. On chromatographie le résidu sur silice en éluant au DCM puis par le mélange DCM/MeOH (70/30 ; v/v). On obtient 2,21 g du produit attendu.

Spectre de RMN à 200 MHz dans DMSO-$d_6$.

0,8 ppm : t : 6H
2,0 à 2,6 ppm : m : 8H
2,8 ppm : mt : 1H
6,5 à 8,2 ppm : m : 7H
10,75 ppm : s : 1H

Préparation 39

5-Chloro-3-(2-chlorophényl)-3-[N-[2-(diéthylamino)éthyl]-N-méthylamino]-1,3-dihydroindol-2-one.

On laisse 18 heures sous agitation à TA un mélange de 5 g du composé obtenu à l'étape C de la Préparation 2, 3,8 g de N,N-diéthyl-N'-méthyléthylènediamine dans 100 ml de chloroforme. On lave le mélange réactionnel à l'eau,

sèche la phase organique sur sulfate de sodium et évapore sous vide le solvant. On chromatographie le résidu sur alumine en éluant par le mélange DCM/MeOH (97/3 ; v/v). On obtient 3,6 g du produit attendu après cristallisation dans l'éther iso, F = 140°C.

Préparation 40

5-Chloro-3-(2-chlorophényl)-1,3-dihydro-3-[[2-(diisopropylamino)éthyl] amino]-indol-2-one.

On laisse 18 heures sous agitation à TA un mélange de 5 g du composé obtenu à l'étape C de la Préparation 2, 4,3 g de N,N-diisopropyléthylènediamine dans 50 ml de chloroforme. On lave le mélange réactionnel à l'eau, sèche la phase organique sur sulfate de sodium et évapore sous vide le solvant. On reprend le résidu à l'éther iso et après trituration, essore le solide formé. On obtient 4,2 g du produit attendu que l'on utilise tel quel à l'EXEMPLE 159.

Préparation 41

5-Chloro-3-(2-chlorophényl)-1,3-dihydro-3-[N-méthyl-N-[3-(diméthylamino) propyl]amino]indol-2-one.

On laisse une nuit sous agitation à TA un mélange de 3,57 g du composé obtenu à l'étape C de la Préparation 2, 1,16 g de N,N-diméthyl-N'-méthyl-1,3-propanediamine, 1,01 g de triéthylamine dans 40 ml de chloroforme. Après concentration sous vide, on reprend le résidu par une solution à 5 % de carbonate de sodium, extrait à l'AcOEt, lave la phase organique à l'eau, par une solution saturée de NaCl, sèche sur sulfate de sodium et évapore sous vide le solvant. On chromatographie le résidu sur alumine en éluant par le mélange DCM/MeOH (98/2 ; v/v). On obtient 2,36 g du produit attendu que l'on utilise tel quel à l'EXEMPLE 161.

Préparation 42

3-[[4-(*tert*-Butoxycarbonylamino)butyl]amino]-5-chloro-3-(2-chlorophényl)-1,3-dihydroindol-2-one.

A) 3-[(4-aminobutyl)amino]-5-chloro-3-(2-chlorophényl)-1,3-dihydroindol-2-one.

A une solution de 5,6 ml de 1,4-diaminobutane dans 10 ml de chloroforme, on ajoute à TA et goutte à goutte une solution de 5 g du composé obtenu à l'étape C de la Préparation 2 dans 50 ml de chloroforme et 15 ml de THF. On laisse 3 heures sous agitation à TA, concentre en partie le mélange réactionnel et essore le précipité formé. On obtient 4,48 g du produit attendu.

Spectre de RMN à 200 MHz dans DMSO-$d_6$.

1,3 ppm : mt : 4H
2,5 ppm : mt : 4H
4,2 ppm : signal élargi : 4H
6,5 à 8,2 ppm : m : 7H

B) 3-[(4-(*tert*-Butoxycarbonylamino)butyl]amino]-5-chloro-3-(2-chlorophényl)-1,3-dihydroindol-2-one.

A une suspension de 5,13 g du composé obtenu à l'étape précédente dans 150 ml de THF, on ajoute à TA 1,95 ml de triéthylamine puis 3,074 g de di-*tert*-butyldicarbonate et laisse 20 heures sous agitation à TA. On essore un insoluble et concentre sous vide le filtrat. On chromatographie le résidu sur silice en éluant au DCM puis par le mélange DCM/AcOEt (75/25 ; v/v). On obtient 2,8 g du produit attendu après cristallisation dans le mélange DCM/éther iso, F = 175-176°C.

Préparation 43

3-[[5-(*tert*-Butoxycarbonylamino)pentyl]amino]-5-chloro-3-(2-chlorophényl)-1,3-dihydroindol-2-one.

A) Méthanesulfonate de 5-(*tert*-butoxycarbonylamino)pentyle.

On refroidit à 0°C une solution de 28,6 g de 5-(*tert*-butoxycarbonylamino)pentan-1-ol dans 100 ml de pyridine et ajoute à 0°C, en 3 heures, goutte à goutte, 16,2 g de chlorure de méthanesulfonyle. On laisse une nuit sous agitation à TA et concentre sous vide le mélange réactionnel. On reprend le résidu à l'eau, extrait à l'AcOEt, lave la phase organique trois fois à l'eau, trois fois par une solution à 5 % d'hydrogénosulfate de potassium, trois fois à l'eau, par une solution saturée de NaCl, sèche sur sulfate de sodium et évapore sous vide le solvant. On obtient 39 g du produit attendu que l'on utilise tel quel à l'étape suivante.

B) 5-(*tert*-Butoxycarbonylamino)pentylazide.

On chauffe à 80°C pendant 5 heures un mélange de 39 g du composé obtenu à l'étape précédente, 9 g

d'azidure de sodium dans 80 ml de DMSO. On verse le mélange réactionnel dans l'eau, extrait à l'AcOEt, lave la phase organique à l'eau, par une solution saturée de NaCl, sèche sur sulfate de sodium et évapore sous vide le solvant. On chromatographie le résidu sur silice en éluant par le mélange hexane/AcOEt (90/10 ; v/v). On obtient 7,8 g du produit attendu que l'on utilise tel quel à l'étape suivante.

C) 5-(*tert*-Butoxycarbonylamino)pentylamine.

A un mélange de 7,8 g du composé obtenu à l'étape précédente, 1,5 g de nickel de Raney ® dans 60 ml d'EtOH on ajoute 1,5 ml d'hydrazine monohydrate et laisse 1 heure 30 minutes sous agitation à TA. On ajoute de nouveau 1,5 ml d'hydrazine monohydrate et laisse 1 heure 30 minutes sous agitation à TA. On filtre le catalyseur et concentre sous vide le filtrat. On reprend le résidu à l'eau, acidifie à pH = 1 par ajout d'une solution d'HCl 1N, lave la phase aqueuse à l'éther, alcalinise la phase aqueuse par ajout de carbonate de potassium, extrait quatre fois à l'éther, lave la phase organique à l'eau, par une solution saturée de NaCl, sèche sur sulfate de sodium et évapore sous vide le solvant. On obtient 3,0 g du produit attendu que l'on utilise tel quel à l'étape suivante.

D) 3-[[5-(*tert*-Butoxycarbonylamino)pentyl]amino]-5-chloro-3-(2-chlorophényl)-1,3-dihydroindol-2-one.

On laisse 3 heures sous agitation à TA un mélange de 2,65 g du composé obtenu à l'étape C de la Préparation 2, 3 g du composé obtenu à l'étape précédente dans 30 ml de chloroforme. On concentre sous vide le mélange réactionnel, reprend le résidu à l'eau, extrait à l'AcOEt, lave la phase organique par une solution à 5 % de carbonate de potassium, à l'eau, par une solution saturée de NaCl, sèche sur sulfate de sodium et évapore partiellement sous vide le solvant. On essore le produit cristallisé formé. On obtient 2,14 g du produit attendu dont on recristallise un échantillon dans le mélange THF/éther iso, F = 185°C.

Préparation 44

5-Chloro-3-(2-chlorophényl)-1,3-dihydro-3-[[2-(pipérid-1-yl)éthyl]amino] indol-2-one. A une solution de 5 g du composé obtenu à l'étape C de la Préparation 2 dans 120 ml de chloroforme on ajoute à TA 3,23 g de 1-(2-aminoéthyl) pipéridine et laisse 2 heures sous agitation à TA. On lave le mélange réactionnel à l'eau, après décantation, sèche la phase organique sur sulfate de sodium et évapore sous vide le solvant. On obtient 4,8 g du produit attendu après cristallisation dans le mélange éther iso/hexane/pentane ; on l'utilise tel quel à l'EXEMPLE 167.

Préparations 45 et 46

3-[[2-[4-(Benzyloxycarbonyl)pipérazin-1-yl]éthyl]amino]-5-chloro-3-(2-chlorophényl)-1,3-dihydroindol-2-one (préparation 45).
et

3-[4-[2-(Benzyloxycarbonylamino)éthyl]pipérazin-1-yl]-5-chloro-3-(2-chlorophényl)-1,3-dihydroindol-2-one (préparation 46).

A) 5-Chloro-3-(2-chlorophényl)-1,3-dihydro-3-[[2-(pipérazin-1-yl)éthyl]amino]indol-2-one et
3-[4-(2-aminoéthyl)pipérazin-1-yl]-5-chloro-3-(2-chlorophényl)-1,3-dihydroindol-2-one.

A une solution de 4,35 g de 1-(2-aminoéthyl)pipérazine dans 70 ml de chloroforme, on ajoute à TA, en 1 heure, une solution de 3 g du composé obtenu à l'étape C de la Préparation 2 dans 30 ml de chloroforme et 20 ml de THF. Après 1 heure d'agitation à TA, on concentre sous vide le mélange réactionnel. On reprend le résidu par une solution à 5 % de carbonate de potassium, extrait à l'AcOEt, lave la phase organique à l'eau, par une solution saturée de NaCl, sèche sur sulfate de sodium et évapore sous vide le solvant. On obtient 3,6 g du mélange des deux produits sous forme d'huile que l'on utilise tel quel à l'étape suivante.

B) 3-[[2-[4-(Benzyloxycarbonyl)pipérazin-1-yl]éthyl]amino]-5-chloro-3-(2-chlorophényl)-1,3-dihydroindol-2-one (préparation 45). et
3-[4-[2-(Benzyloxycarbonylamino)éthyl]pipérazin-1-yl]-5-chloro-3-(2-chlorophényl)-1,3-dihydroindol-2-one (préparation 46).

On refroidit à 0°C une solution de 3,6 g du mélange des deux composés obtenus à l'étape précédente dans 15 ml de DCM et ajoute 1,1 g de DIPEA puis 1,45 g de chloroformiate de benzyle. On laisse 60 heures sous agitation à TA et concentre sous vide le mélange réactionnel. On extrait le résidu à l'AcOEt, lave la phase organique à l'eau, par une solution saturée de NaCl, sèche sur sulfate de sodium et évapore sous vide le solvant. On chromatographie le résidu sur silice en éluant par le mélange DCM/AcOEt (90/10 ; v/v) et obtient 0,86 g du composé de la Préparation 45. Puis on élue à l'AcOEt et obtient 1,7 g du composé de la Préparation 46.

Spectre de RMN à 200 MHz dans DMSO-$d_6$ du composé de la Préparation 45.

2,0 à 3,6 ppm : m : 13H

5,1 ppm : s : 2H
6,6 à 8,2 ppm : m : 12H
10,8 ppm : s : 1H

Spectre de RMN à 200 MHz dans DMSO-$d_6$ du composé de la Préparation 46.

2,0 à 3,0 ppm : m : 10H
3,15 ppm : qd : 2H
5,05 ppm : s : 2H
6,7 à 8,2 ppm : m : 13H
10,85 ppm : s : 1H

On peut également obtenir le composé de la Préparation 45 en suivant les deux étapes du procédé décrit ci-après.

A') 4-(2-Aminoéthyl)-1-(benzyloxycarbonyl)pipérazine.

A une solution de 10 g de 1-(2-aminoéthyl)pipérazine dans 125 ml de toluène, on ajoute 8,21 g de benzaldéhyde et chauffe à reflux pendant 3 heures en éliminant l'eau formée à l'aide d'un appareil de Dean-Stark. On concentre sous vide le mélange réactionnel, reprend le résidu dans 100 ml de DCM, ajoute 13,45 ml de DIPEA et goutte à goutte 11,03 ml de chloroformiate de benzyle. On laisse une nuit sous agitation à TA et concentre sous vide le mélange réactionnel. On reprend le résidu par une solution saturée d'hydrogénosulfate de potassium et laisse 4 heures sous agitation vigoureuse à TA. On lave la phase aqueuse à l'éther, alcalinise à pH = 9-10 la phase aqueuse par ajout de NaOH concentrée, sature la phase aqueuse par ajout de NaCl, extrait au chloroforme, sèche la phase organique sur sulfate de sodium et évapore sous vide le solvant. On obtient 18,78 g du produit attendu que l'on utilise tel quel à l'étape suivante.

B') 3-[[2-[4-(Benzyloxycarbonyl)pipérazin-1-yl]éthyl]amino]-5-chloro-3-(2-chlorophényl)-1,3-dihydroindol-2-one.

A une solution de 2 g du composé obtenu à l'étape C de la Préparation 2 dans 20 ml de chloroforme et 3 ml de THF, on ajoute à TA et goutte à goutte une solution de 1,77 g du composé obtenu à l'étape précédente, 3,1 ml de triéthylamine dans 10 ml de chloroforme et laisse 3 heures sous agitation à TA. On concentre sous vide, extrait le résidu à l'AcOEt, lave la phase organique par une solution saturée de NaCl, à l'eau, sèche sur sulfate de sodium et évapore sous vide le solvant. On chromatographie le résidu sur silice en éluant au DCM, puis par le mélange DCM/AcOEt (40/60 ; v/v). On obtient 2 g du produit attendu.

Préparation 47

5-Chloro-3-(2-chlorophényl)-1,3-dihydro-3-[[2-(morpholin-4-yl)éthyl]amino]indol-2-one.

On laisse 2 heures sous agitation à TA un mélange de 5 g du composé obtenu à l'étape C de la Préparation 2, 3,5 g de 4-(2-aminoéthyl)morpholine dans 120 ml de chloroforme. On lave le mélange réactionnel à l'eau, après décantation, sèche la phase organique sur sulfate de sodium et évapore sous vide le solvant. On obtient 5,2 g du produit attendu après trituration dans le mélange hexane/pentane puis essorage. On l'utilise tel quel.

Préparation 48

5-Chloro-3-(2-chlorophényl)-1,3-dihydro-3-[[3-(morpholin-4-yl)propyl]amino]indol-2-one.

On refroidit à 0°C une solution de 3,57 g du composé obtenu à l'étape C de la Préparation 2 dans 100 ml de chloroforme et ajoute, goutte à goutte, en 10 minutes, une solution de 1,44 g de 4-(3-aminopropyl)morpholine, 2,02 g de triéthylamine dans 10 ml de chloroforme. On laisse une nuit sous agitation à TA et concentre sous vide. On reprend le résidu à l'eau, alcalinise à pH = 10 par ajout de carbonate de potassium, extrait à l'AcOEt, lave la phase organique à l'eau, par une solution saturée de NaCl, sèche sur sulfate de sodium et évapore sous vide le solvant. On chromatographie le résidu sur silice en éluant par le mélange AcOEt/MeOH (93/7 ; v/v). On obtient 2,8 g du produit attendu sous forme de mousse après cristallisation dans le mélange DCM/hexane puis séchage à 90°C sous vide.

Spectre de RMN à 200 MHz dans DMSO-$d_6$

1,6 ppm : mt : 2H
2,1 à 2,6 ppm : m : 8H
3,35 ppm : t : 1H
3,55 ppm : t : 4H
6,6 à 8,3 ppm : m : 7H

10,8 ppm : s : 1H

Préparation 49

3-[[2-(*tert*-Butoxycarbonylamino)-2-méthylpropyl]amino]-5-chloro-3-(2-chlorophényl)-1,3-dihydroindol-2-one.

A une solution de 2 g du composé obtenu à l'étape C de la Préparation 2 dans 20 ml de chloroforme, on ajoute, goutte à goutte, à TA, une solution de 1,17 ml de 1,2-diamino-2-méthylpropane, 2,32 ml de triéthylamine dans 15 ml de chloroforme et laisse 3 heures sous agitation à TA. On concentre sous vide le mélange réactionnel, dissout le résidu dans 15 ml de DCM, ajoute 1,57 ml de triéthylamine puis 1,45 g de di-*tert*-butyl dicarbonate et laisse 18 heures sous agitation à TA. On concentre sous vide le mélange réactionnel, extrait le résidu à l'AcOEt, lave la phase organique par une solution saturée de NaCl, à l'eau, sèche sur sulfate de sodium et évapore sous vide le solvant. On chromatographie le résidu sur silice en éluant au DCM puis par le mélange DCM/MeOH (85/15 ; v/v). On obtient 1,4 g du produit attendu après cristallisation dans le mélange DCM/éther iso, F = 164°C.

Préparation 50

5-Chloro-3-(2-chlorophényl)-3-[[(diéthylaminocarbonyl)méthyl]amino]-1,3-dihydroindol-2-one.

A) N-[5-Chloro-3-(2-chlorophényl)-2,3-dihydro-2-oxoindol-3-yl]glycinate de *tert*-butyle.

On laisse 18 heures sous agitation à TA un mélange de 6 g du composé obtenu à l'étape C de la Préparation 2, 4 g de glycinate de *tert*-butyle dans 100 ml de chloroforme. On lave le mélange réactionnel à l'eau, après décantation sèche la phase organique sur sulfate de sodium et évapore sous vide le solvant. On chromatographie le résidu sur silice en éluant par le mélange DCM/AcOEt (95/5 ; v/v). On obtient 2,7 g du produit attendu après cristallisation dans le mélange DCM/éther iso, F = 217°C.

B) N-[5-Chloro-3-(2-chlorophényl)-2,3-dihydro-2-oxoindol-3-yl]glycine.

A un mélange de 2,7 g du composé obtenu à l'étape précédente dans 12 ml de DCM on ajoute 12 ml de TFA et laisse 18 heures sous agitation à TA. On concentre sous vide à 35°C le mélange réactionnel, reprend le résidu dans un mélange éther/hexane et essore le précipité formé. On obtient 2,5 g du produit attendu que l'on utilise tel quel à l'étape suivante.

C) 5-Chloro-3-(2-chlorophényl)-3-[[(diéthylaminocarbonyl)méthyl]amino]-1,3-dihydroindol-2-one.

A une solution de 1 g du composé obtenu à l'étape précédente, 0,66 g de diéthylamine dans 10 ml de DMF on ajoute 1,3 g de BOP et laisse 18 heures sous agitation à TA. On ajoute de l'eau au mélange réactionnel, essore le précipité formé et le lave à l'eau. On dissout le précipité dans du DCM, lave la phase organique à l'eau, sèche sur sulfate de sodium et évapore sous vide le solvant. On chromatographie le résidu sur silice en éluant par le mélange DCM/AcOEt (50/50 ; v/v). On obtient 0,95 g du produit attendu après cristallisation dans le mélange DCM/éther iso, F = 223°C.

Préparation 51

5-Chloro-3-(2-chlorophényl)-3-[[2-(diéthylaminocarbonyl)éthyl]amino]-1,3-dihydroindol -2-one.

A) N-[5-Chloro-3-(2-chlorophényl)-2,3-dihydro-2-oxoindol-3-yl]β-alaninate de *tert*-butyle.

A une solution de 6 g du composé obtenu à l'étape C de la Préparation 2 dans 100 ml de chloroforme, on ajoute à TA 3,2 g de chlorhydrate de β-alaninate de *tert*-butyle puis 4,18 g de DIPEA et laisse 2 heures sous agitation à TA. On lave le mélange réactionnel à l'eau, après décantation sèche la phase organique sur sulfate de sodium et évapore sous vide le solvant. On chromatographie le résidu sur silice en éluant par le mélange DCM/AcOEt (85/15 ; v/v). On obtient 5 g du produit attendu après cristallisation dans le mélange DCM/éther iso, F = 188°C.

B) N-[5-Chloro-3-(2-chlorophényl)-2,3-dihydro-2-oxoindol-3-yl]β-alanine.

On mélange à 0°C, 4,7 g du composé obtenu à l'étape précédente et 50 ml de TFA et laisse 5 heures sous agitation en laissant remonter la température à TA. On concentre sous vide à 30°C le mélange réactionnel, reprend le résidu par le mélange éther/hexane et essore le précipité formé. On obtient 1,5 g du produit attendu que l'on utilise tel quel à l'étape suivante.

C) 5-Chloro-3-(2-chlorophényl)-3-[[2-(diéthylaminocarbonyl)éthyl]amino]-1,3-dihydroindol-2-one.

On refroidit à 0°C une solution de 1,5 g du composé obtenu à l'étape précédente, 0,906 g de diéthylamine dans 20 ml de DMF, ajoute 1,4 g de BOP et laisse 18 heures sous agitation en laissant remonter la température à TA. On verse le mélange réactionnel dans l'eau et essore le précipité formé. On dissout le précipité dans l'AcOEt,

lave la phase organique à l'eau, sèche sur sulfate de sodium et évapore sous vide le solvant. On chromatographie le résidu sur silice en éluant par le mélange DCM/AcOEt (50/50 ; v/v). On obtient 0,82 g du produit attendu après cristallisation dans le mélange DCM/éther iso, F = 185°C.

Préparation 52

5-Chloro-3-(2-chlorophényl)-3-[[3-(méthoxycarbonyl)propyl]amino]-1,3-dihydroindol-2-one.

On laisse 1 heure sous agitation à TA un mélange de 2 g du composé obtenu à l'étape C de la Préparation 2, 1,65 g de chlorhydrate de 4-aminobutyrate de méthyle, 2,09 g de DIPEA dans 20 ml de chloroforme. On lave le mélange réactionnel par une solution à 5 % de carbonate de sodium, après décantation sèche la phase organique sur sulfate de sodium et évapore sous vide le solvant. On chromatographie le résidu sur silice en éluant par le mélange AcOEt/hexane (50/50 ; v/v). On obtient 1,7 g du produit attendu après cristallisation dans le mélange DCM/éther iso, F = 142°C.

Préparation 53

5-Chloro-3-(2-chlorophényl)-3-[(cyanométhyl)amino]-1,3-dihydroindol-2-one.

On refroidit à +4°C une solution de 3 g du composé obtenu à l'étape C de la Préparation 2 dans 20 ml de chloroforme et 20 ml de THF, ajoute, goutte à goutte, une solution de 0,92 g de chlorhydrate d'aminoacétonitrile, 4,6 ml de triéthylamine dans 20 ml de chloroforme et laisse 3 heures sous agitation en laissant remonter la température à TA. On ajoute 100 ml d'eau au mélange réactionnel, concentre sous vide les solvants, extrait la phase aqueuse à l'AcOEt, lave la phase organique par une solution saturée de NaCl, à l'eau, sèche sur sulfate de sodium et évapore sous vide le solvant. On chromatographie le résidu sur silice en éluant au DCM puis par le mélange DCM/AcOEt (95/5 ; v/v). On obtient 1,2 g du produit attendu, F = 240-241°C.

Préparations 54 et 55

3-[[(1S)-5-(Benzyloxycarbonylamino)-1-(méthoxycarbonyl)pentyl]amino]-5-chloro-3-(2-chlorophényl)-1,3-dihydroindol-2-one, isomère A et isomère B.

A un mélange de 5,4 g du composé obtenu à l'étape C de la Préparation 2, 5 g de chlorhydrate de l'ester méthylique de la N-ε-(benzyloxycarbonyl)-L-lysine dans 30 ml de THF, on ajoute, à TA et goutte à goutte, une solution de 4,6 g de triéthylamine dans 30 ml de THF et laisse 2 heures sous agitation à TA. On concentre sous vide le mélange réactionnel, reprend le résidu par une solution à 5 % de carbonate de sodium, extrait à l'AcOEt, lave la phase organique à l'eau, sèche sur sulfate de sodium et évapore sous vide le solvant. On chromatographie le résidu sur silice en éluant par le gradient du mélange hexane/AcOEt de (95/5 ; v/v) jusqu'à (50/50 ; v/v). On sépare les deux isomères :

- le moins polaire, isomère A : composé de la Préparation 54 que l'on rechromatographie sur silice en éluant par le mélange DCM/AcOEt (60/40 ; v/v).

$$\alpha_D^{25} = +105° \text{ (c = 0,257 ; chloroforme).}$$

- le plus polaire, isomère B : composé de la Préparation 55 que l'on rechromatographie sur silice en éluant par le mélange DCM/AcOEt (50/50 ; v/v).

$$\alpha_D^{25} = -95{,}7° \text{ (c = 0,279 ; chloroforme).}$$

Préparations 56 et 57

3-[[(1R)-5-(Benzyloxycarbonylamino)-1-(méthoxycarbonyl)pentyl]amino]-5-chloro-3-(2-chlorophényl)-1,3-dihydroindol-2-one, isomère A et isomère B.

On prépare ces deux composés selon le mode opératoire décrit aux Préparations 54 et 55 à partir de 4,1 g du composé obtenu à l'étape C de la Préparation 2, 5,22 g du chlorhydrate de l'ester méthylique de la N-ε-(benzyloxycarbonyl)-D-lysine dans 40 ml de THF et 4,2 g de triéthylamine dans 30 ml de THF. On chromatographie sur silice en éluant par le gradient du mélange pentane/AcOEt de (95/5 ; v/v) jusqu'à (45/55 ; v/v) et sépare un isomère :

- le moins polaire, isomère A : composé de la Préparation 56 ;

$$\alpha_D^{25} = -100{,}7° \text{ (c = 0,275 ; chloroforme).}$$

On rechromatographie le produit restant sur silice H en éluant par le gradient du mélange DCM/AcOEt de (94/6 ; v/v) jusqu'à (75/25 ; v/v) et sépare l'autre isomère :

- le plus polaire, isomère B : composé de la Préparation 57 ;

$$\alpha_D^{25} = +92{,}9° \text{ (c = 0,254 ; chloroforme).}$$

Préparations 58 et 59

3-[[(1S)-5-(Benzyloxycarbonylamino)-1-(hydroxyméthyl)pentyl]amino]-5-chloro-3-(2-chlorophényl)-1,3-dihydroindol-2-one, isomère A et isomère B.

A) N-ε-(benzyloxycarbonyl)-L-lysinol.

A une suspension de 5 g de borohydrure de sodium dans 100 ml d'EtOH, on ajoute en 10 minutes 5 g de chlorhydrate de l'ester méthylique de la N-ε-(benzyloxycarbonyl)-L-lysine et laisse une nuit sous agitation à TA. On ajoute 10 ml d'une solution d'HCl 5N et évapore le solvant sous vide. On reprend le résidu à l'eau, alcalinise la phase aqueuse à pH = 13 par ajout de NaOH concentrée, extrait à l'AcOEt, lave la phase organique deux fois à l'eau, par une solution saturée de NaCl, sèche sur sulfate de sodium et évapore sous vide le solvant. On obtient 3,58 g du produit attendu que l'on utilise tel quel.

B) 3-[[(1S)-5-(Benzyloxycarbonylamino)-1-(hydroxyméthyl)pentyl]amino]-5-chloro-3-(2-chlorophényl)-1,3-dihydroindol-2-one, isomère A et isomère B.

A une solution de 4,90 g du composé obtenu à l'étape C de la Préparation 2 dans 50 ml de THF, on ajoute goutte à goutte une solution de 3,50 g du composé obtenu à l'étape précédente, 4 g de triéthylamine dans 40 ml de THF et laisse 2 heures sous agitation à TA. On ajoute une solution à 5 % de carbonate de potassium, extrait à l'AcOEt, lave la phase organique à l'eau, par une solution saturée de NaCl, sèche sur sulfate de sodium et évapore sous vide le solvant. On chromatographie le résidu sur silice en éluant par le mélange DCM/AcOEt (60/40 ; v/v) et sépare un isomère :

- isomère le moins polaire, isomère A : composé de la Préparation 58 que l'on rechromatographie sur alumine en éluant par le mélange AcOEt/MeOH (97/3 ; v/v) et obtient 2,4 g du produit sous forme d'huile.

$$\alpha_D^{25} = +174{,}3° \text{ (c = 0,25 ; chloroforme).}$$

En éluant dans la 1ère chromatographie par le mélange DCM/AcOEt (20/80 ; v/v) on sépare l'autre isomère :

- isomère le plus polaire, isomère B : composé de la Préparation 59 que l'on reprend dans un mélange hexane/éther iso puis, après essorage du précipité formé, cristallise dans le mélange DCM/éther iso. On obtient 1,3 g, F = 110°C.

$$\alpha_D^{25} = -95{,}2° \text{ (c = 0,25 ; chloroforme).}$$

Préparation 60

3-[[(1S)-5-(Benzyloxycarbonylamino)-1-[(triméthylsilyloxy)méthyl]pentyl] amino]-5-chloro-3-(2-chlorophényl)-1,3-dihydroindol-2-one.

On chauffe à 60°C pendant une nuit un mélange de 2,1 g du composé obtenu à la Préparation 58 (isomère A), 0,63 g d'hexaméthyldisilane, 0,025 g de chlorure de zinc dans 20 ml d'acétonitrile. On concentre sous vide, extrait le résidu à l'AcOEt, lave deux fois rapidement la phase organique à l'eau, par une solution saturée de NaCl, sèche sur sulfate de sodium et évapore sous vide le solvant. On obtient le produit attendu que l'on utilise tel quel à l'EXEMPLE 226.

Préparation 61

5-Chloro-3-(2-chlorophényl)-1,3-dihydro-3-[2-(diméthylamino) acétamido]indol-2-one.

On chauffe à reflux pendant 1 heure un mélange de 1,07 g du composé obtenu à la Préparation 2, 0,32 ml de bromure de bromoacétyle dans 15 ml de benzène. Après refroidissement, on essore le précipité formé. On reprend le précipité dans 10 ml de DCM, ajoute en 1 heure 3 ml d'une solution à 30 % de diméthylamine dans l'EtOH et laisse sous agitation. On concentre sous vide le mélange réactionnel, reprend le résidu par une solution à 5 % de carbonate de sodium, extrait à l'AcOEt, lave la phase organique à l'eau, par une solution saturée de NaCl, sèche sur sulfate de sodium et évapore sous vide le solvant. On chromatographie le résidu sur silice en éluant par le mélange AcOEt/MeOH (90/10 ; v/v). On obtient 0,63 g du produit attendu après cristallisation dans le mélange AcOEt/MeOH, F = 236-238°C.

Préparation 62

5-Chloro-3-(2-chlorophényl)-1,3-dihydro-3-[2-(4-méthylpipérazin-1-yl)acétamido] indol-2-one.

A) 5-Chloro-3-(2-chloroacétamido)-3-(2-chlorophényl)-1,3-dihydroindol-2-one.

On chauffe à reflux un mélange de 8 g du composé obtenu à la Préparation 2 dans 300 ml de benzène, ajoute 3,4 g de chlorure de chloroacétyle et laisse 2 heures sous agitation à reflux. On concentre sous vide le mélange réactionnel et cristallise le résidu dans le mélange DCM/éther iso. On obtient 8 g du produit attendu, F = 230°C.

B) 5-Chloro-3-(2-chlorophényl)-1,3-dihydro-3-[2-(4-méthylpipérazin-1-yl)acétamido] indol-2-one.

On chauffe à 60°C pendant 1 heure un mélange de 1,4 g du composé obtenu à l'étape précédente, 0,455 g de 1-méthylpipérazine, 0,523 g de carbonate de potassium, 0,01 g d'iodure de sodium dans 20 ml de DMF. On concentre sous vide le mélange réactionnel, reprend le résidu à l'eau, extrait à l'AcOEt, sèche la phase organique sur sulfate de sodium et évapore sous vide le solvant. On chromatographie le résidu sur silice en éluant par le mélange DCM/MeOH (96/4 ; v/v). On obtient 1,2 g du produit attendu après cristallisation dans le mélange DCM/éther iso, F = 214°C.

Préparation 63

5-Chloro-3-(2-chlorophényl)-3-[3-(diéthylamino)propionamido]-1,3-dihydroindol-2-one.

A une solution de 1,5 g du composé obtenu à la Préparation 2 dans 5 ml de pyridine, on ajoute 0,43 ml de chlorure d'acryloyle et laisse 12 heures sous agitation à TA. On rajoute 0,43 ml de chlorure d'acryloyle et poursuit l'agitation pendant 12 heures. On concentre sous vide le mélange réactionnel, reprend le résidu à l'eau, extrait à l'AcOEt, lave la phase organique par une solution d'HCl 1N, à l'eau, sèche sur sulfate de sodium et évapore sous vide le solvant. On dissout le résidu dans 5 ml d'EtOH et ajoute à TA en 4 jours 0,5 g de diéthylamine. On concentre sous vide, reprend le résidu par une solution d'HCl 1N, lave la phase aqueuse à l'AcOEt, alcalinise la phase aqueuse à pH = 10 par ajout d'une solution à 5 % de carbonate de potassium, extrait à l'AcOEt, lave la phase organique à l'eau, par une solution saturée de NaCl, sèche sur sulfate de sodium et évapore sous vide le solvant. On chromatographie le résidu sur silice en éluant par le mélange DCM/MeOH/$NH_4OH$ (80/15/5 ; v/v/v). On obtient 0,335 g du produit attendu.

Spectre de RMN à 200 MHz dans DMSO-$d_6$.

0,85 ppm : t : 6H
2,0 à 2,8 ppm : m : 8H
6,7 à 7,7 ppm : m : 7H
9,3 ppm : s : 1H
10,9 ppm : s : 1H

Préparation 64

5-Chloro-3-(2-chlorophényl)-3-[3-(diéthylamino)propionamido]-1,3-dihydroindol-2-one, isomère (+).

On refroidit à 0°C une solution de 1,88 g du composé obtenu à la Préparation 28 isomère (+) dans 8 ml de pyridine, ajoute goutte à goutte 1,225 g de chlorure d'acryloyle et laisse 72 heures sous agitation à TA. On ajoute de l'eau au mélange réactionnel, extrait à l'AcOEt, lave la phase organique à l'eau, par une solution saturée de NaCl, sèche sur sulfate de sodium et évapore sous vide le solvant. On dissout le résidu dans 5 ml d'EtOH, ajoute 2,346 g de diéthylamine et laisse deux jours sous agitation à TA. On concentre sous vide et chromatographie le résidu sur silice en éluant par le mélange DCM/MeOH (75/25 ; v/v). On obtient 0,350 g du produit attendu après cristallisation dans le mélange DCM/éther iso, F = 176-178°C ;

$\alpha_D^{25}$ = +56,3° (c = 0,38 ; chloroforme).

Préparation 65

3-(2-Chlorophényl)-1,3-dihydro-5-méthyl-3-(méthylamino)indol-2-one.

A) N-*p*-méthylphényl-DL-2-chloromandélamide.

On chauffe à reflux pendant 6 heures un mélange de 32,1 g de p-toluidine, 55,95 g d'acide DL-2-chloromandélique dans 250 ml de 1,2-dichlorobenzène en éliminant l'eau formée à l'aide d'un appareil de Dean-Stark. On refroidit le mélange réactionnel, essore le précipité formé et lave à l'éther iso. On obtient 48 g du produit attendu que l'on utilise tel quel.

B) 3-(2-chlorophényl)-1,3-dihydro-5-méthylindol-2-one.

On refroidit à 10°C un mélange de 86 ml d'acide sulfurique concentré et 20 ml d'acide sulfurique fumant (oleum à 30 % ) et ajoute par portions 20 g du composé obtenu à l'étape précédente en maintenant la température interne en dessous de 40°C. On laisse 18 heures sous agitation à TA, verse le mélange réactionnel sur de la glace et essore le précipité formé. On dissout le précipité dans du chloroforme, lave la phase organique à l'eau jusqu'à pH = 7, sèche sur sulfate de sodium et évapore sous vide le solvant. On obtient 12 g du produit attendu après cristallisation dans le mélange THF/DCM/éther iso, F = 208°C.

C) 3-(2-Chlorophényl)-1,3-dihydro-3-hydroxy-5-méthylindol-2-one.

A une solution de 5,6 g du composé obtenu à l'étape précédente dans 150 ml de THF on ajoute à TA 0,952 g d'hydrure de sodium à 60 % dans l'huile et laisse 30 minutes sous agitation. Puis on ajoute 2,46 ml de diméthyldisulfure et laisse 48 heures sous agitation. On ajoute de l'eau au mélange réactionnel, essore le précipité formé et le lave à l'éther puis au pentane. On obtient 5,1 g du produit attendu, F = 295-300°C.

D) 3-Chloro-3-(2-chlorophényl)-1,3-dihydro-5-méthylindol-2-one.

On refroidit à +4°C une suspension de 1,5 g du composé obtenu à l'étape précédente dans 20 ml de DCM, ajoute 0,84 ml de pyridine puis 0,6 ml de chlorure de thionyle et laisse 3 heures sous agitation à TA. On concentre sous vide et chromatographie le résidu sur silice en éluant par le mélange DCM/hexane (80/20 ; v/v) puis au DCM. On obtient 1,06 g du produit attendu que l'on utilise tel quel.

E) 3-(2-Chlorophényl)-1,3-dihydro-5-méthyl-3-(méthylamino)indol-2-one.

A une solution de 1,06 g du composé obtenu à l'étape précédente dans 5 ml de DCM, on ajoute à TA 0,9 ml d'une solution 8,03M de méthylamine dans l'EtOH et laisse 1 heure sous agitation. On concentre sous vide, extrait le résidu à l'AcOEt, lave par une solution saturée de NaCl, à l'eau, sèche sur sulfate de sodium et évapore sous vide le solvant. On chromatographie le résidu sur silice en éluant au DCM puis par le mélange DCM/AcOEt (75/25 ; v/v). On obtient 0,82 g du produit attendu après cristallisation dans le mélange DCM/éther iso, F = 186-187°C.

Préparation 66

3-Amino-3-(2-chlorophényl)-1,3-dihydro-5-méthoxyindol-2-one.

A) N-*p*-méthoxyphényl-DL-2-chloromandélamide;

On chauffe à 225°C pendant 5 heures un mélange de 33,25 g de p-anisidine, 50 g d'acide DL-2-chloromandélique dans 250 ml de 1,2-dichlorobenzène en éliminant l'eau formée à l'aide d'un appareil de Dean-Stark. On concentre le mélange réactionnel à environ 150 ml et laisse une nuit en cristallisation. On essore les cristaux formés et les lave à l'éther. On obtient 43,76 g du produit attendu.

B) 3-(2-chlorophényl)-1,3-dihydro-5-méthoxyindol-2-one.

On chauffe à 60°C pendant 4 heures un mélange de 393 g d'acide polyphosphorique et 30 g du composé obtenu à l'étape précédente. On ajoute 350 g de glace au mélange réactionnel, extrait à l'AcOEt, lave la phase organique par une solution saturée de NaCl jusqu'à pH = 7, sèche sur sulfate de magnésium et évapore sous vide le solvant jusqu'à précipitation du produit. Après essorage et séchage, on obtient 11,29 g du produit attendu que l'on utilise tel quel.

C) 3-Chloro-3-(2-chlorophényl)-1,3-dihydro-5-méthoxyindol-2-one.

A une suspension de 9,29 g du composé obtenu à l'étape précédente dans 100 ml de $CCl_4$ on ajoute 4,9 g de N-chlorosuccinimide et chauffe à reflux pendant 3 heures. Après refroidissement, on filtre un insoluble et évapore sous vide le filtrat. On chromatographie le résidu sur silice en éluant au DCM puis par le mélange DCM/AcOEt (98/2 ; v/v) jusqu'à (96/4 ; v/v). On obtient 6,1 g du produit attendu que l'on utilise tel quel.

D) 3-Azido-3-(2-chlorophényl)-1,3-dihydro-5-méthoxyindol-2-one.

A une solution de 6,1 g du composé obtenu à l'étape précédente dans 100 ml d'acétonitrile on ajoute 3,9 g

d'azidure de sodium et chauffe à reflux pendant 4 heures. On concentre sous vide, reprend le résidu à l'eau, extrait à l'AcOEt, lave la phase organique par une solution saturée de NaCl, par une solution aqueuse à 5 % d'hydrosulfite de sodium, sèche sur sulfate de sodium et évapore sous vide le solvant. On chromatographie le résidu sur silice en éluant au DCM puis par le mélange DCM/AcOEt (99/1 ; v/v). On obtient 2,79 g du produit attendu ; spectre infrarouge dans le DCM : 2125 $cm^{-1}$.

E) 3-Amino-3-(2-chlorophényl)-1,3-dihydro-5-méthoxyindol-2-one.

A une solution de 2,79 g du composé obtenu à l'étape précédente dans 44 ml de MeOH on ajoute, sous atmosphère d'argon, à la seringue, 2,47 ml de triéthylamine puis 1,78 ml de propane-1,3-dithiol et chauffe à 60°C pendant 4 heures. On concentre sous vide le mélange réactionnel et chromatographie le résidu sur silice en éluant au DCM puis par le mélange DCM/AcOEt (70/30 ; v/v). On obtient 1,23 g du produit attendu après cristallisation dans l'AcOEt.

Spectre de RMN à 200 MHz dans DMSO-$d_6$.

2,6 ppm : s : 2H
3,65 ppm : s : 3H
6,2 à 7,0 ppm : m : 3H
7,2 à 8,4 ppm : m : 4H
10,4 ppm : s : 1H

Préparation 67

3-(2-Chlorophényl)-5-éthoxy-3-(éthylamino)-1,3-dihydroindol-2-one.

A une solution de 1 g du composé obtenu à la Préparation 16 dans 20 ml de MeOH, on ajoute à TA 5 ml d'AcOH, puis 0,2 ml d'acétaldéhyde et, par portions, 0,293 g de cyanoborohydrure de sodium. On laisse 30 minutes sous agitation à TA, ajoute 5 gouttes d'HCl concentré puis neutralise le mélange réactionnel par addition d'une solution à 5 % de carbonate de potassium. On évapore sous vide le MeOH, extrait à l'AcOEt, lave la phase organique à l'eau, par une solution saturée de NaCl, sèche sur sulfate de sodium et évapore sous vide le solvant. On chromatographie le résidu sur alumine en éluant au DCM puis par le mélange DCM/AcOEt (95/5 ; v/v). On obtient 0,3 g du produit attendu après cristallisation dans le mélange DCM/éther iso, F = 195-200°C.

Préparation 68

3-(2-Chlorophényl)-5-éthoxy-1,3-dihydro-3-(diméthylamino)indol-2-one.

A une solution de 1 g du composé obtenu à la Préparation 16 dans 20 ml de MeOH, on ajoute à TA 5 ml d'AcOH, puis 0,4 g de paraformaldéhyde et, par portions en 1 heure, 0,532 g de cyanoborohydrure de sodium. On laisse 48 heures sous agitation à TA, ajoute 5 gouttes d'HCl concentré puis neutralise le mélange réactionnel par addition d'une solution à 5 % de carbonate de potassium. On évapore sous vide le MeOH, extrait à l'AcOEt, lave la phase organique à l'eau, par une solution saturée de NaCl, sèche sur sulfate de sodium et évapore sous vide le solvant. On chromatographie le résidu sur silice en éluant au DCM puis par le mélange DCM/AcOEt (90/10 ; v/v). On obtient 1 g du produit attendu après cristallisation dans le mélange DCM/éther iso (produit sous forme de mousse après séchage à 90°C sous vide).

Spectre de RMN à 200 MHz dans DMSO-$d_6$.

1,15 ppm : t : 3H
2,1 ppm : se : 6H
3,75 ppm : qd : 2H
6,1 à 8,1 ppm : m : 7H
10,4 ppm : s : 1H

Préparation 69

3-(2-chlorophényl)-5-fluoro-1,3-dihydro-3-(méthylamino)indol-2-one.

A) N-*p*-fluorophényl-DL-2-chloromandélamide.

On prépare ce composé selon le mode opératoire décrit à l'étape A de la Préparation 2 à partir de 4-fluoroaniline et d'acide DL-2-chloromandélique.

B) 3-(2-chlorophényl)-5-fluoro-1,3-dihydroindol-2-one.

On ajoute, goutte à goutte, 40 ml d'acide sulfurique fumant (oléum à 30 %) dans 160 ml d'acide sulfurique concentré. Puis on ajoute par portions 40 g du composé obtenu à l'étape précédente en maintenant la température interne en dessous de 40°C et laisse 16 heures sous agitation à TA. On verse le mélange réactionnel sur 300 g de glace, extrait à l'AcOEt, lave la phase organique par une solution saturée de NaCl, sèche sur sulfate de sodium et évapore sous vide le solvant. On obtient 42 g du produit attendu que l'on utilise tel quel.

C) 3-Bromo-3-(2-chlorophényl)-5-fluoro-1,3-dihydroindol-2-one.

A une suspension de 4,0 g du composé obtenu à l'étape précédente dans 100 ml de chloroforme on ajoute goutte à goutte une solution de 0,78 ml de brome dans 1 ml de chloroforme et laisse 30 minutes sous agitation à TA. On concentre sous vide, reprend le résidu au DCM et évapore sous vide le solvant. On obtient 5,2 g du produit attendu que l'on utilise tel quel.

D) 3-(2-chlorophényl)-5-fluoro-1,3-dihydro-3-(méthylamino)indol-2-one.

A une suspension de 5,2 g du composé obtenu à l'étape précédente dans 100 ml de DCM, on ajoute goutte à goutte 2,8 ml d'une solution à 33 % de méthylamine dans l'EtOH et laisse 2 heures sous agitation à TA. On concentre sous vide, extrait à l'AcOEt, lave la phase organique par une solution à 5 % de carbonate de sodium, par une solution saturée de NaCl, sèche sur sulfate de sodium et évapore sous vide le solvant. On chromatographie le résidu sur silice en éluant au DCM puis par le mélange DCM/MeOH (98/2 ; v/v). On obtient 2,15 g du produit attendu après cristallisation dans le mélange DCM/éther iso, F = 188-189°C.

Préparations 70 et 71

5-Chloro-3-(2-chlorophényl)-1,3-dihydro-4-méthyl-3-(méthylamino)indol-2-one (préparation 70)
et
5-Chloro-3-(2-chlorophényl)-1,3-dihydro-6-méthyl-3-(méthylamino)indol-2-one (préparation 71)

A) 5-Chloro-3-(2-chlorophényl)-1,3-dihydro-4-méthylindol-2-one et 5-chloro-3-(2-chlorophényl)-1,3-dihydro-6-méthylindol-2-one.

On prépare le mélange de ces deux composés selon le mode opératoire décrit à l'étape B de la Préparation 2 à partir du N-(4-chloro-3-méthylphényl)-DL-2-chloromandélamide obtenu selon le mode opératoire décrit à l'étape A de la Préparation 2 à partir de 4-chloro-3-méthylaniline et d'acide DL-2-chloromandélique.

B) 3-Bromo-5-chloro-3-(2-chlorophényl)-1,3-dihydro-4-méthylindol-2-one et 3-bromo-5-chloro-3-(2-chlorophényl)-1,3-dihydro-6-méthylindol-2-one.

A une suspension de 3,62 g du mélange des deux composés obtenus à l'étape précédente dans 60 ml de DCM, on ajoute goutte à goutte à TA une solution de 0,6 ml de brome dans 60 ml de DCM et laisse 30 minutes sous agitation à TA. On concentre sous vide, reprend le résidu au DCM et évapore sous vide le solvant. On chromatographie le résidu sur silice en éluant par le mélange DCM/AcOEt (97/3 ; v/v). On obtient 5,42 g du mélange des deux produits attendus que l'on utilise tel quel.

C) 5-Chloro-3-(2-chlorophényl)-1,3-dihydro-4-méthyl-3-(méthylamino)indol-2-one (préparation 70)
et
5-Chloro-3-(2-chlorophényl)-1,3-dihydro-6-méthyl-3-(méthylamino)indol-2-one (préparation 71)

A une solution de 2,94 g du mélange des deux composés obtenus à l'étape précédente dans 100 ml d'EtOH on ajoute 2 ml d'une solution 8,03M de méthylamine dans l'EtOH et laisse une nuit sous agitation à TA. On concentre sous vide, reprend le résidu par 100 ml d'une solution à 5 % de carbonate de sodium, extrait quatre fois au DCM, sèche la phase organique sur sulfate de sodium et évapore sous vide le solvant. On chromatographie sur silice en éluant par le mélange DCM/AcOEt (90/10 ; v/v) et obtient le produit de la préparation 70 après cristallisation dans le mélange DCM/THF/éther iso. Puis on élue par le mélange DCM/AcOEt (75/25 ; v/v) et obtient le produit de la préparation 71 après cristallisation dans le mélange DCM/THF/éther iso.

- composé le moins polaire, composé de la Préparation 70

Spectre de RMN à 200 MHz dans DMSO-$d_6$.

1,8 ppm : s : 3H
2,1 ppm : d : 3H
3,1 ppm : qd : 1H
6,7 à 8,3 ppm : m : 6H
10,8 ppm : s : 1H

- composé le plus polaire, composé de la Préparation 71, F = 234°C.

On peut également obtenir le composé de la Préparation 71 en suivant les trois étapes du procédé décrit ci-après.

A') 5-Chloro-3-(2-chlorophényl)-1,3-dihydro-6-méthylindol-2-one.

On prépare ce composé selon le mode opératoire décrit à l'étape A ci-dessus à partir de 35 g de N-(4-chloro-3-méthylphényl)-DL-2-chloromandélamide. Par cristallisation dans l'AcOEt du mélange obtenu à l'étape A ci-dessus, puis recristallisation dans le mélange DCM/THF/AcOEt, on obtient 8,1 g du produit attendu, F = 203-209°C.

B') 3-Bromo-5-chloro-3-(2-chlorophényl)-1,3-dihydro-6-méthylindol-2-one.

On prépare ce composé selon le mode opératoire décrit à l'étape B ci-dessus à partir de 8 g du composé obtenu à l'étape A'. Après concentration sous vide du mélange réactionnel, on reprend le résidu au DCM, essore le précipité formé et obtient 6,0 g du produit attendu. On chromatographie le filtrat sur silice en éluant par le mélange DCM/hexane (30/70 ; v/v) et obtient 1,42 g du produit attendu en deuxième jet après cristallisation dans du DCM, F = 109-111°C.

C') 5-Chloro-3-(2-chlorophényl)-1,3-dihydro-6-méthyl-3-(méthylamino)indol-2-one.

On prépare ce composé selon le mode opératoire décrit à l'étape C ci-dessus à partir du composé obtenu à l'étape B'.

Préparation 72

3-[[2-(1-*tert*-Butoxycarbonylpipérid-4-yl)éthyl]amino]-5,6-dichloro-3-(2-chlorophényl)-1,3-dihydroindol-2-one.

A) N-3,4-dichlorophényl-DL-2-chloromandélamide.

On prépare ce composé selon le mode opératoire décrit à l'étape A de la Préparation 2 à partir de 3,4-dichloroaniline et d'acide DL-2-chloromandélique, F = 160-163°C.

B) 5,6-Dichloro-3-(2-chlorophényl)-1,3-dihydroindol-2-one.

On refroidit à 0°C un mélange de 53 ml d'acide sulfurique concentré et 12 ml d'acide sulfurique fumant (oléum à 30 %) et ajoute par portions 13 g du composé obtenu à l'étape précédente. On laisse 24 heures sous agitation à TA, verse le mélange réactionnel dans l'eau et essore le précipité formé. On dissout le précipité dans de l'AcOEt, lave la phase organique à l'eau jusqu'à pH = 7, sèche sur sulfate de sodium et évapore sous vide en partie le solvant. On essore le produit cristallisé formé et recristallise dans le mélange THF/DCM/AcOEt. On obtient 1,3 g du produit attendu, F = 198-201°C.

C) 3-Bromo-5,6-dichloro-3-(2-chlorophényl)-1,3-dihydroindol-2-one.

A une suspension de 1,95 g du composé obtenu à l'étape précédente dans 30 ml de chloroforme, on ajoute goutte à goutte une solution de 0,32 g de brome dans 1 ml de chloroforme et laisse 30 minutes sous agitation à TA. On concentre sous vide, reprend le résidu au DCM et évapore sous vide le solvant. On extrait le résidu à l'AcOEt, lave la phase organique à l'eau jusqu'à pH = 7, sèche sur sulfate de sodium et évapore sous vide le solvant. On obtient le produit attendu après cristallisation dans le DCM, F = 215-218°C.

D) 4-(2-Aminoéthyl)-1-(tert-butoxycarbonyl)pipéridine.

a) 1-(tert-Butoxycarbonyl)-4-(2-hydroxyéthyl)pipéridine.

A une solution de 51 g de 4-(2-hydroxyéthyl)pipéridine dans 20 ml de *tert*-butanol on ajoute à TA et goutte à goutte une solution de 87 g de di-*tert*-butyldicarbonate dans 100 ml de *tert*-butanol et laisse une nuit sous agitation à TA. On concentre sous vide, extrait le résidu à l'AcOEt, lave la phase organique par une solution tampon pH = 2, sèche sur sulfate de sodium et évapore sous vide le solvant. On obtient 91,5 g du produit attendu que l'on utilise tel quel.

b) Méthanesulfonate de 2-(1-*tert*-butoxycarbonylpipérid-4-yl)éthyle.

On refroidit à 0°C une solution de 86,8 g du composé obtenu à l'étape précédente dans 400 ml de DCM, ajoute une solution de 53 ml de triéthylamine dans 20 ml de DCM puis une solution de 33 ml de chlorure de méthanesulfonyle dans 20 ml de DCM et laisse 5 heures sous agitation. On lave le mélange réactionnel à l'eau, après décantation sèche la phase organique sur sulfate de sodium et évapore sous vide le solvant. On obtient le produit attendu que l'on utilise tel quel.

c) 2-(1-*tert*-Butoxycarbonylpipérid-4-yl)éthylazide;

On chauffe à 50°C pendant 4 heures un mélange de 5 g du composé obtenu à l'étape précédente, 1,2 g d'azidure de sodium dans 15 ml de DMSO. On verse le mélange réactionnel dans l'eau, extrait à l'AcOEt, sèche sur sulfate de sodium et évapore sous vide le solvant. On chromatographie le résidu sur silice en éluant

par le mélange hexane/AcOEt (90/10 ; v/v). On obtient 2,8 g du produit attendu que l'on utilise tel quel à l'étape suivante.

d) 4-(2-Aminoéthyl)-1-(*tert*-butoxycarbonyl)pipéridine.

On laisse 4 heures sous agitation à TA un mélange de 2,8 g du composé obtenu à l'étape précédente, 0,5 g de nickel de Raney® , 0,54 ml d'hydrazine monohydrate dans 20 ml d'EtOH. On rajoute en 4 heures 0,54 ml d'hydrazine monohydrate et laisse 48 heures sous agitation à TA. On filtre le catalyseur et concentre sous vide le filtrat. On obtient 2,3 g du produit attendu que l'on utilise tel quel.

E) 3-[[2-(1-*tert*-Butoxycarbonylpipérid-4-yl)éthyl]amino]-5,6-dichloro-3-(2-chlorophényl)-1,3-dihydroindol-2-one.

A une solution de 1,8 g du composé obtenu à l'étape C dans 50 ml de THF, on ajoute à TA une solution de 1,05 g de 4-(2-aminoéthyl)-1-(*tert*-butoxycarbonyl)pipéridine, 0,46 g de triéthylamine dans 10 ml de chloroforme et laisse 2 heures sous agitation à TA. On concentre sous vide, on reprend le résidu par une solution à 5 % de carbonate de potassium, extrait à l'AcOEt, lave la phase organique à l'eau, par une solution saturée de NaCl, ajoute à la phase organique du THF bouillant, sèche sur sulfate de sodium et évapore en partie les solvants. On essore le produit cristallisé formé et on obtient 1,94 g du produit attendu, F = 248-249°C.

Préparation 73

5-Chloro-3-(2-chlorophényl)-7-fluoro-1,3-dihydro-3-(méthylamino)indol-2-one.

A) N-(4-ehloro-2-fluorophényl)-DL-2-chloromandélamide.

On prépare ce composé selon le mode opératoire décrit à l'étape A de la Préparation 2 à partir de 4-chloro-2-fluoroaniline et d'acide DL-2-chloromandélique.

B) 5-Chloro-3-(2-chlorophényl)-7-fluoro-1,3-dihydroindol-2-one.

On refroidit à +4°C 30 ml d'acide sulfurique concentré, ajoute 7,5 ml d'acide sulfurique fumant (oléum à 30 %) puis, par portions, 7 g du composé obtenu à l'étape précédente. On laisse 48 heures sous agitation à TA et ajoute 300 ml d'eau au mélange réactionnel. On extrait à l'AcOEt, lave la phase organique par une solution saturée de NaCl, à l'eau, sèche sur sulfate de sodium et évapore sous vide le solvant. On obtient 3,4 g du produit atttendu après cristallisation dans l'AcOEt.

C) 3-Bromo-5-chloro-3-(2-chlorophényl)-7-fluoro-1,3-dihydroindol-2-one.

A une suspension de 3,4 g du composé obtenu à l'étape précédente dans 20 ml de chloroforme on ajoute 0,53 ml de brome et laisse 3 heures sous agitation à TA. On concentre sous vide, reprend le résidu au DCM et évapore sous vide le solvant. On obtient 4,2 g du produit attendu que l'on utilise tel quel.

D) 5-Chloro-3-(2-chlorophényl)-7-fluoro-1,3-dihydro-3-(méthylamino)indol-2-one.

A une solution de 4,2 g du composé obtenu à l'étape précédente dans 25 ml de DCM on ajoute à TA et goutte à goutte 2,7 ml d'une solution 8,03M de méthylamine dans l'EtOH diluée dans 5 ml de DCM et laisse 3 heures sous agitation à TA. On concentre sous vide le mélange réactionnel, reprend le résidu par une solution à 5 % de carbonate de sodium, extrait à l'AcOEt, lave la phase organique par une solution saturée de NaCl, à l'eau, sèche sur sulfate de sodium et évapore sous vide le solvant. On chromatographie le résidu sur silice en éluant par le mélange DCM/AcOEt (85/15 ; v/v). On obtient 1,1 g du produit attendu après cristallisation dans le mélange DCM/THF/AcOEt, F = 219°C.

Préparation 74

5-Chloro-3-(2-chlorophényl)-3-[[2-(diéthylamino)-2-méthylpropyl]amino]-1,3-dihydroindol-2-one.

A) 2-(Diéthylamino)-2-méthylpropylamine.

On prépare ce composé selon le mode opératoire décrit dans la demande EP 0429344.

B) 5-Chloro-3-(2-chlorophényl)-3-[[2-(diéthylamino)-2-méthylpropyl]amino]-1,3-dihydroindol-2-one.

On refroidit à 0°C une solution de 2,42 g du composé obtenu à l'étape précédente, 1,41 g de triéthylamine dans 30 ml de chloroforme, ajoute par portions 5 g du composé obtenu à l'étape C de la Préparation 2 et laisse 18 heures sous agitation à TA. On lave le mélange réactionnel à l'eau, après décantation, sèche la phase organique sur sulfate de sodium et évapore sous vide le solvant. On chromatographie le résidu sur silice en éluant à l'AcOEt. On obtient 2,4 g du produit attendu après cristallisation dans l'éther, F = 174°C.

Préparation 75

3-[2-[N-(*tert*-Butoxycarbonylméthyl)-N-méthylamino]acétamido]-5-chloro-3-(2-chlorophényl)-1,3-dihydroindol-2-one.

On chauffe à reflux pendant 18 heures un mélange de 1,5 g du composé obtenu à l'étape A de la Préparation 62, 0,957 g de chlorhydrate de sarcosinate de *tert*-butyle, 1,2 g de DIPEA, 0,01 g d'iodure de sodium dans 20 ml d'acétone. On concentre sous vide, extrait le résidu à l'AcOEt, lave la phase organique à l'eau, sèche sur sulfate de sodium et évapore sous vide le solvant. On chromatographie le résidu sur silice en éluant par le mélange DCM/AcOEt (70/30 ; v/v). On obtient 1,7 g du produit attendu après cristallisation dans le mélange DCM/éther iso, F = 200°C.

Préparation 76

5-Chloro-3-(2-chlorophényl)-1,3-dihydro-3-(diméthylamino)indol-2-one, isomère (+).

A une solution de 1,5 g du composé obtenu à la Préparation 28 isomère (+) dans 30 ml de MeOH et 20 ml d'AcOH on ajoute par portions en 48 heures 0,6 g de paraformaldéhyde et 0,6 g de cyanoborohydrure de sodium. On ajoute 5 gouttes d'HCl concentré, puis alcalinise à pH = 10 par ajout d'une solution à 5 % de carbonate de potassium, évapore sous vide le MeOH, extrait la phase aqueuse à l'AcOEt, lave la phase organique à l'eau, par une solution saturée de NaCl, sèche sur sulfate de sodium et évapore sous vide le solvant. On chromatographie le résidu sur alumine en éluant par le mélange DCM/MeOH (60/40 ; v/v). On obtient 0,62 g du produit attendu après cristallisation dans le mélange DCM/hexane, F = 169-172°C.

$$\alpha_D^{25^\circ} = +325^\circ \text{ (c = 0,26 ; chloroforme).}$$

Spectre de RMN à 200 MHz dans DMSO-$d_6$

2,2 ppm : se : 6H
6,7 à 8,2 ppm : m : 7H
10,85 ppm : s : 1H

Préparation 77

3-[[2-(1-*tert*-Butoxycarbonylpipérid-4-yl)éthyl]amino]-5-chloro-3-(2-chlorophényl)-1,3-dihydro-6-méthylindol-2-one.

A une solution de 2,1 g de 3-bromo-5-chloro-3-(2-chlorophényl)-1,3-dihydro-6-méthylindol-2-one obtenu à l'étape B' de la Préparation 71 dans 20 ml de THF, on ajoute à TA et goutte à goutte une solution de 1,35 g de 4-(2-aminoéthyl)-1-(*tert*-butoxycarbonyl)pipéridine, 1,75 g de triéthylamine dans 20 ml de THF et laisse 1 heure sous agitation à TA. On concentre sous vide le mélange réactionnel, reprend le résidu dans 400 ml d'AcOEt et 50 ml de THF, lave la suspension obtenue deux fois par une solution à 5 % de carbonate de sodium, à l'eau, par une solution saturée de NaCl et après décantation essore le solide en suspension. On dissout le solide dans 400 ml de THF bouillant, sèche sur sulfate de sodium et évapore sous vide en partie le solvant. On essore le produit cristallisé formé et obtient 1,65 g du produit attendu, F = 245-248°C.

Préparation 78

3-[[5-(Benzyloxycarbonyl)pentyl]amino]-5-chloro-3-(2-chlorophényl)-1,3-dihydroindol-2-one.

A) 6-(*tert*-Butoxycarbonylamino)hexanoate de benzyle.

A une solution de 3 g d'acide 6-(*tert*-butoxycarbonylamino)hexanoïque dans 60 ml de DCM on ajoute à TA 1,94 ml de DBU puis 1,54 ml de bromure de benzyle et laisse 24 heures sous agitation à TA. On concentre sous vide, reprend le résidu dans 100 ml d'eau, extrait à l'AcOEt, lave la phase organique par une solution à 5 % de carbonate de potassium, par une solution saturée de NaCl, à l'eau, sèche sur sulfate de sodium et évapore sous vide le solvant. On obtient 2,8 g du produit attendu que l'on utilise tel quel.

B) 3-[[5-(Benzyloxycarbonyl)pentyl]amino]-5-chloro-3-(2-chlorophényl)-1,3-dihydroindol-2-one.

On refroidit à +4°C une solution de 4 g du composé obtenu à l'étape précédente dans 20 ml de DCM, ajoute 40 ml de TFA et laisse 1 heure sous agitation à +4°C. On concentre sous vide, reprend le résidu trois fois par 50 ml de DCM et évapore sous vide le solvant. On reprend l'huile obtenue dans 30 ml de chloroforme, ajoute 4,5 g du composé obtenu à l'étape C de la Préparation 2, puis, goutte à goutte, une solution de 5,24 ml de triéthylamine dans 5 ml de chloroforme et laisse 1 heure sous agitation à TA. On concentre sous vide, extrait le résidu à l'AcOEt, lave la phase organique par une solution saturée de NaCl, à l'eau, sèche sur sulfate de sodium et évapore sous vide le solvant. On chromatographie le résidu sur silice en éluant au DCM puis par le mélange DCM/AcOEt (90/10 ;

v/v). On obtient 3,8 g du produit attendu après cristallisation dans le mélange DCM/hexane, F = 98-99°C.

En procédant selon les modes opératoires décrits dans les Préparations ci-dessus, on prépare les 1,3-dihydroindol-2-one rassemblés dans le TABLEAU I ci-après.

## TABLEAU I

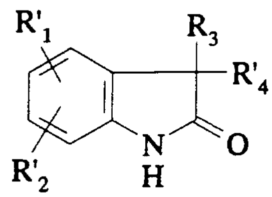

| Préparations | R'1 | R'2 | R3 | R'4 | Solvate F°C ou RMN Solvant de cristallisation |
|---|---|---|---|---|---|
| 79 (a) | 5-Cl | H | 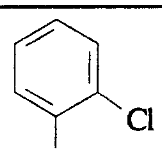 | 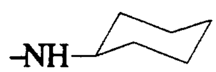 | 200 DCM/éther iso |
| 80 (b) | 5-Cl | H | 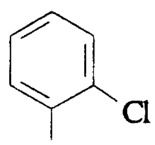 | 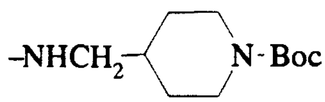 | 238 DCM/éther iso |
| 81 (a) | 5-Cl | H | 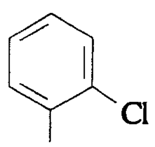 | 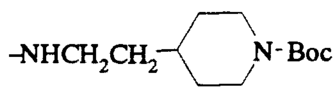 | 218 DCM/éther iso |
| 82 (c) | 5-Cl | H | 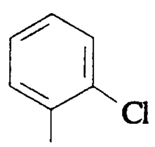 | 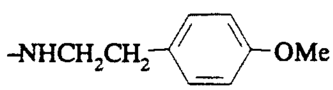 | 165-168 DCM/éther iso |
| 83 (c) | 5-Cl | H | 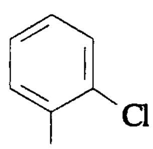 | 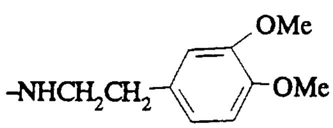 | 189-193 DCM/éther iso |
| 84 (c) | 5-Cl | H | 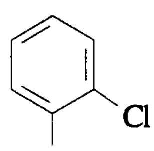 | 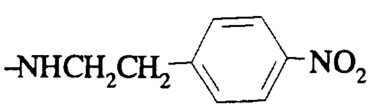 | 0,33 $H_2O$ 195-198 DCM/éther iso |

TABLEAU I (suite)

| Préparations | R'1 | R'2 | R3 | R'4 | Solvate F°C ou RMN Solvant de cristallisation |
|---|---|---|---|---|---|
| 85 (a) | 5-Cl | H | 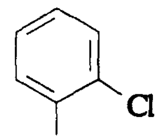 | $-NH(CH_2)_2O(CH_2)_2OH$ | 0,5 $H_2O$ 173-174 DCM/éther iso |
| 86 (d) | 5-Cl | H | 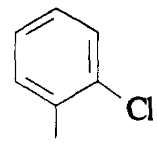 | $-NH(CH_2)_2O(CH_2)_2OSi(Me)_3$ | 140-142 DCM/éther iso |
| 87 (a) | 5-Cl | H | 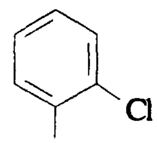 | $-NH(CH_2)_3OMe$ | 192 |
| 88 (a) | 5-Cl | H | 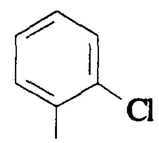 | 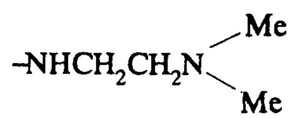 | 159 DCM/éther iso |
| 89 (e) | 5-Cl | H | 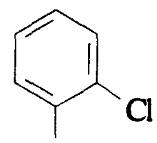 | 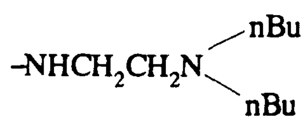 | 110 pentane |
| 90 (a) | 5-Cl | H | 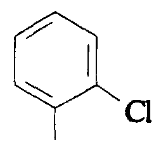 | 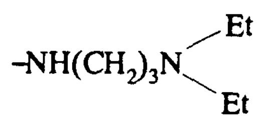 | 148 DCM/éther iso |
| 91 (b) | 5-Cl | H | 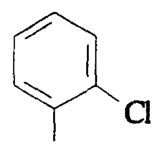 | 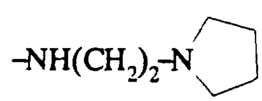 | 190 éther iso |
| 92 (f) | 5-Cl | H | 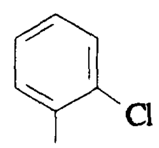 | 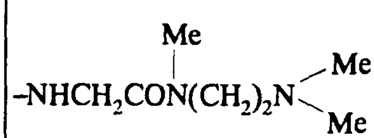 | 185 éther iso/hexane |
| 93 (g) | 5-Cl | H | 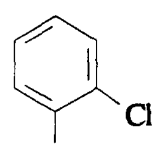 | $-NH(CH_2)_3COOBz$ | utilisé tel quel |

TABLEAU I (suite)

| Préparations | R'1 | R'2 | R3 | R'4 | Solvate F°C ou RMN Solvant de cristallisation |
|---|---|---|---|---|---|
| 94 (o) | 5-Cl | H | 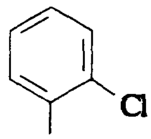 | $-NH(CH_2)_4COOBz$ | 116-117 DCM/éther iso |
| 95 (a) | 5-Cl | H | 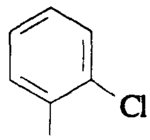 | $-NH(CH_2)_5CN$ | 160-162 DCM/éther iso |
| 96 (b) | 5-Cl | H | 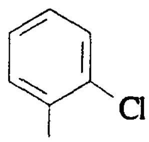 | 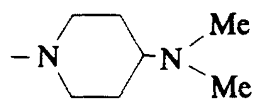 | 251 DCM/éther iso |
| 97 (h) | 5-Cl | H | 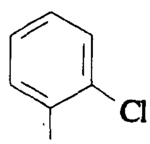 | 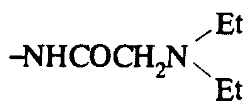 | 194-195 DCM/éther iso |
| 98 (i) | 5-Cl | H | 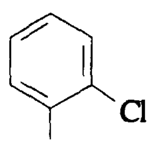 | 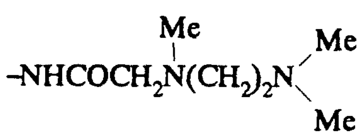 | 120 DCM/éther iso |
| 99 (j) | 5-Et | H | 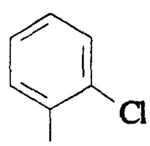 | -NH-Me | 139-140 DCM/éther iso |
| 100 (k) | 5-Cl | 6-Cl | 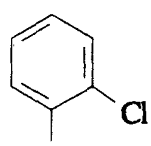 | $-NH(CH_2)_3-OMe$ | 163-164 DCM/éther iso |
| 101 (l) | 5-Cl | 6-Cl | 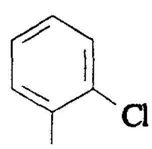 | 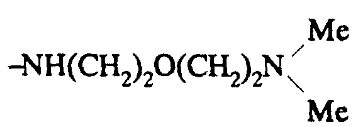 | RMN huile |
| 102 (m) | 5-Cl | 6-Cl | 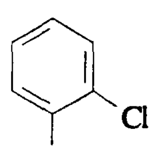 | 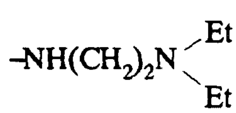 | 130 DCM/éther iso |

TABLEAU I (suite)

| Préparations | R'1 | R'2 | R3 | R'4 | Solvate F°C ou RMN Solvant de cristallisation |
|---|---|---|---|---|---|
| 103 (n) | 5-Cl | 6-Cl | 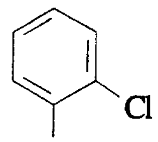 | $-NH(CH_2)_5NH-Boc$ | 205-210 DCM/THF/ éther iso |
| 104 (p) | 5-Cl | 4-Cl | 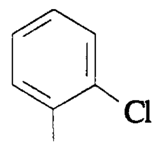 | 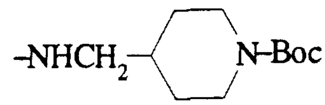 | 238 |
| 105 (q) | 5-Cl | 6-Cl | 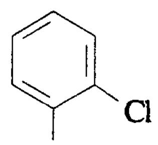 | 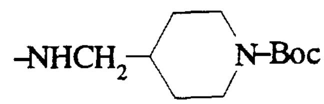 | 173 |
| 106 (r) | 5-Cl | 6-Me | 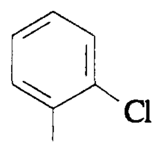 | 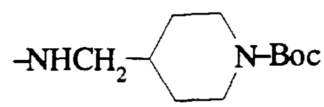 | 184 DCM/éther iso |
| 107 (s) | 5-Cl | H | 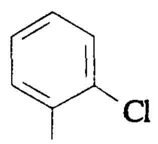 | 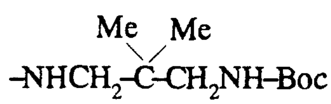 | 195 éther iso |
| 108 (b) | 5-Cl | H | 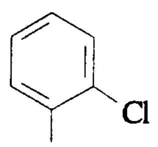 | $-NH(CH_2)_2-S-Et$ | 128 DCM/éther iso |
| 109 (c) | 5-Cl | H | 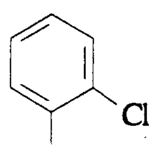 | 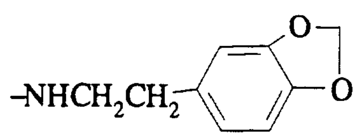 | 168-170 DCM/éther iso |
| 110 (t) | 5-Cl | H | 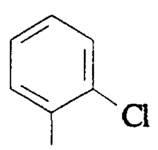 | 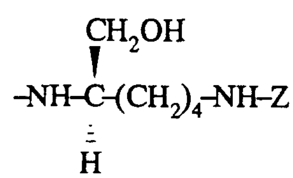 | 96-98 DCM/éther iso |
| 111 (u) | 5-Cl | H | 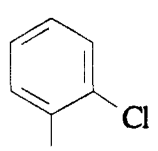 | 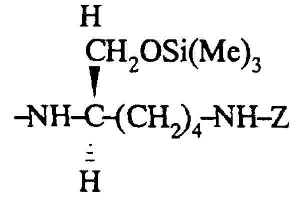 | - |

| | | | | | |
|---|---|---|---|---|---|
| 112 (v) | 5-$CF_3$ | H | 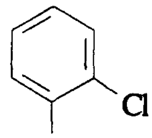 | -NH-Me | 153-155 DCM/éther iso |
| 113 (w) | 5-OEt | H | 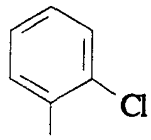 | 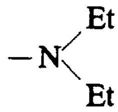 | 170-172 DCM/éther iso |
| 114 (x) | 5-Cl | 4-Me | 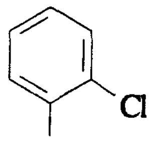 | 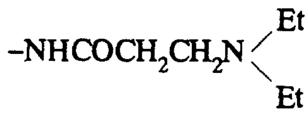 | - |
| 115 (y) | 5-Cl | 6-Me | 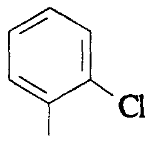 | 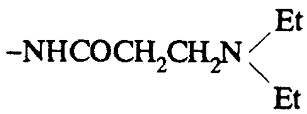 | 200-203 EtOH |

(a) on prépare ce composé selon le mode opératoire décrit à la Préparation 30 en utilisant les amines appropriées.

(b) on prépare ce composé selon le mode opératoire décrit à la Préparation 31 en utilisant les amines appropriées et en présence de DIPEA.

(c) on prépare ce composé selon le mode opératoire décrit à la Préparation 32 en utilisant les amines appropriées.

(d) on prépare ce composé selon le mode opératoire décrit à la Préparation 35 à partir du composé obtenu à la Préparation 85.

(e) on prépare ce composé selon le mode opératoire décrit à la Préparation 39 en utilisant les amines appropriées.

(f) on prépare ce composé selon le mode opératoire décrit à l'étape C de la Préparation 50 à partir du composé obtenu à l'étape B de la Préparation 50 et des amines appropriées.

(g) on prépare ce composé selon le mode opératoire décrit à la Préparation 52.

(h) on prépare ce composé selon le mode opératoire décrit à la Préparation 61 à partir du composé obtenu à la Préparation 2, de bromure de bromoacétyle et de diéthylamine.

(i) on prépare ce composé selon le mode opératoire décrit à l'étape B de la Préparation 62 en utilisant l'amine appropriée.

(j) on prépare ce composé selon les modes opératoires décrits aux étapes A, B, C, D puis E de la Préparation 65.

(k) on prépare ce composé selon le mode opératoire décrit à l'étape E de la Préparation 72 à partir du composé obtenu à l'étape C de la Préparation 72, de (3-méthoxypropyl)amine et en présence de triéthylamine.

(l) on prépare ce composé selon le mode opératoire décrit à l'étape E de la Préparation 36 à partir du composé obtenu à l'étape C de la Préparation 72.

(m) on prépare ce composé selon le mode opératoire décrit à la Préparation 38 à partir du composé obtenu à l'étape C de la Préparation 72.

(n) on prépare ce composé selon le mode opératoire décrit à l'étape D de la Préparation 43 à partir du composé obtenu à l'étape C de la Préparation 72.

(o) on prépare ce composé selon le mode opératoire décrit à l'étape B de la Préparation 78.

(p) on prépare ce composé selon le mode opératoire décrit à la Préparation 80 à partir de 3-bromo-4,5-dichloro-3-(2-chlorophényl)-1,3-dihydroindol-2-one (F = 230°C) obtenu selon le mode opératoire décrit à l'étape C de la Préparation 72 à partir de 4,5-dichloro-3-(2-chlorophényl)-1,3-dihydroindol-2-one obtenu comme sous produit dans l'étape B de la Préparation 72.

(q) on prépare ce composé selon le mode opératoire décrit à la Préparation 80 à partir du composé obtenu à l'étape C de la Préparation 72.

(r) on prépare ce composé selon le mode opératoire décrit à la Préparation 80 à partir du composé obtenu à l'étape B' de la Préparation 71.

(s) on prépare ce composé selon le mode opératoire décrit à la Préparation 49 à partir du composé obtenu à l'étape C de la Préparation 2 et de 1,3-diamino-2,2-diméthylpropane.

(t) isomère le plus polaire ; on prépare ce composé selon le mode opératoire décrit à l'étape B de la Préparation 59 à partir du composé obtenu à l'étape C de la Préparation 2 et de N-ε-(benzyloxycarbonyl)-D-lysinol

$\alpha_D^{25}$ = +99,2° (c = 0,275 ; chloroforme).

(u) on prépare ce composé selon le mode opératoire décrit à la Préparation 60 à partir du composé obtenu la Préparation 110 et on l'utilise tel quel à l'EXEMPLE 269.

(v) on prépare ce composé selon les modes opératoires décrits aux étapes A, B, C puis D de la Préparation 69.

(w) on prépare ce composé selon le mode opératoire décrit à la Préparation 68 à partir du composé obtenu à la Préparation 16 et d'acétaldéhyde.

(x) on prépare ce composé selon le mode opératoire décrit à la Préparation 63 à partir du 3-amino-5-chloro-3-(2-chlorophényl)-1,3-dihydro-4-méthylindol-2-one, lui-même obtenu selon le mode opératoire décrit à l'étape C de la Préparation 70 en utilisant l'ammoniac gaz.

(y) on prépare ce composé selon le mode opératoire décrit à la Préparation 63 à partir du 3-amino-5-chloro-3-(2-chlorophényl)-1,3-dihydro-6-méthylindol-2-one, lui même obtenu selon le mode opératoire décrit à l'étape C de la Préparation 71 en utilisant l'ammoniac gaz.

Spectre de RMN à 200 MHz dans DMSO-$d_6$ du composé de la Préparation 101

2,1 ppm : s : 6H
2,35 ppm : t : 2H
2,5 ppm : mt : 2H
3,0 ppm : t : 1H
3,4 ppm : t : 4H
6,8 à 8,2 ppm : m : 6H
10,95 ppm : s : 1H

Préparations de composés de formule (III) ou (IV).

Préparation 116

Chlorure de 4-(N',N'-diéthyluréido)benzènesulfonyle.

A) N',N'-Diéthyl-N-phénylurée.

On refroidit à 0°C une solution de 10 g d'aniline dans 50 ml de pyridine et ajoute lentement une solution de 14,56 g de chlorure de diéthylcarbamoyle dans 10 ml de pyridine. On laisse 6 heures sous agitation en laissant remonter la température à TA puis verse dans l'eau. On extrait à l'AcOEt, lave à l'eau, par une solution à 5 % d'hydrogénosulfate de potassium, sèche sur sulfate de sodium et évapore sous vide. On chromatographie sur silice en éluant par du DCM puis par un mélange DCM/AcOEt (90/10 ; v/v). On obtient 13 g du produit attendu qui est utilisé tel quel à l'étape suivante.

B) Chlorure de 4-(N',N'-diéthyluréido)benzènesulfonyle.

On refroidit à +5°C, 3,5 ml d'acide chlorosulfonique et ajoute en 15 minutes, 2 g du composé obtenu à l'étape précédente. On chauffe à 60°C, pendant 2 heures, sous agitation vigoureuse, le mélange réactionnel puis redroidit à TA. On ajoute lentement de la glace, puis de l'eau, extrait à l'AcOEt, lave à l'eau jusqu'à pH = 7, sèche sur sulfate de sodium et évapore sous vide. On obtient le produit attendu après recristallisation dans le benzène, m = 1,54 g, F = 148-150°C. Préparation 117

4-Bromométhylbenzoate de *tert*-butyle.

A) 4-Méthylbenzoate de *tert*-butyle.

A 39,04 g de chlorure de *p*-toluoyle on ajoute 37 g de *tert*-butanol puis 26 ml de pyridine et chauffe à reflux pendant 3 heures. Après refroidissement on verse le mélange réactionnel dans l'eau, extrait à l'AcOEt, lave par une solution d'HCl 1N, puis par une solution à 5 % d'hydrogénocarbonate de sodium, sèche sur sulfate de sodium et évapore sous vide. On chromatographie sur silice en éluant à l'hexane puis par un mélange hexane/DCM (80/20 ; v/v). On obtient 38,9 g du produit attendu qui est utilisé tel quel à l'étape suivante.

B) 4-Bromométhylbenzoate de *tert*-butyle.

On chauffe à reflux, pendant 40 minutes, un mélange de 10,4 g du composé obtenu à l'étape précédente, 9,64 g de N-bromosuccinimide, 0,10 g de peroxyde de dibenzoyle dans 250 ml de $CCl_4$. Après refroidissement, on filtre l'insoluble, reprend le filtrat au $CCl_4$, lave par une solution à 5 % d'hydrogénocarbonate de sodium, sèche sur sulfate de sodium et évapore sous vide. On obtient 13 g d'huile limpide qui est utilisée telle quelle par la suite.

Préparation 118

Chlorure de 1-acétylindoline-5-sulfonyle.

A) 1-Acétylindoline.

On refroidit à 0°C une solution de 11,9 g d'indoline dans 100 ml de chloroforme, ajoute 13,9 ml de triéthylamine

puis goutte à goutte une solution de 10,4 ml d'anhydride acétique dans 10 ml de chloroforme, et 0,1 g de 4-diméthylaminopyridine. Après deux heures d'agitation à TA, on ajoute de l'eau au mélange réactionnel, extrait au chloroforme, sèche la phase organique sur sulfate de sodium et évapore sous vide le solvant. On obtient 15,44 g du produit attendu que l'on utilise tel quel.

B) Chlorure de 1-acétylindoline-5-sulfonyle.

On refroidit à +5°C 32 ml d'acide chlorosulfonique et ajoute rapidement, par portions, 15,4 g du composé obtenu à l'étape précédente. On chauffe à 60°C pendant 2 heures le mélange réactionnel puis refroidit à TA. On ajoute lentement de la glace et essore le précipité formé. On obtient 18,07 g du produit attendu après cristallisation dans le mélange DCM/éther iso.

Spectre de RMN à 200 MHz dans DMSO-$d_6$.

2,15 ppm : s : 3H
3,1 ppm : t : 2H
4,1 ppm : t : 2H
7,3 à 8,1 ppm : m : 3H

Préparation 119

Chlorure de 1-(méthoxycarbonyl)indoline-5-sulfonyle.

A) 1-Méthoxycarbonylindoline.

On refroidit à 0°C une solution de 10 g d'indoline dans 10 ml de pyridine, ajoute goutte à goutte une solution de 7,9 g de chloroformiate de méthyle dans 10 ml de DCM et laisse 1 heure sous agitation à 0°C. Puis on ajoute à nouveau une solution de 7,9 g de chloroformiate de méthyle dans 10 ml de DCM et concentre sous vide le mélange réactionnel. On reprend le résidu à l'eau, extrait à l'AcOEt, lave la phase organique par une solution à 5 % d'hydrogénosulfate de potassium, à l'eau, par une solution saturée de NaCl, sèche sur sulfate de sodium et évapore sous vide le solvant. On obtient 8,3 g du produit attendu après trituration dans le mélange DCM/éther iso puis essorage, F = 69-70°C.

B) Chlorure de 1-méthoxycarbonylindoline-5-sulfonyle.

On laisse 90 minutes sous agitation à TA un mélange de 3 g du composé obtenu à l'étape précédente et 7 ml d'acide chlorosulfonique. On ajoute successivement de la glace puis de l'eau au mélange réactionnel, extrait à l'AcOEt, lave la phase organique à l'eau jusqu'à pH = 7, sèche sur sulfate de sodium et évapore sous vide le solvant. On obtient 2,8 g du produit attendu après cristallisation dans le mélange DCM/éther iso, F = 113-114°C.

Préparation 120

Chlorure de 1-(diéthylaminocarbonyl)indoline-5-sulfonyle.

A) 1-(diéthylaminocarbonyl)indoline.

On refroidit à 0°C une solution de 6 g d'indoline dans 10 ml de pyridine, ajoute goutte à goutte 6,4 ml de chlorure de diéthylcarbamoyle et laisse 18 heures sous agitation à TA. On ajoute de l'eau au mélange réactionnel, extrait à l'AcOEt, lave la phase organique à l'eau, par une solution à 5 % d'hydrogénosulfate de potassium, sèche sur sulfate de sodium et évapore sous vide le solvant. On chromatographie le résidu sur silice en éluant au DCM. On obtient 8 g du produit attendu que l'on utilise tel quel.

B) Chlorure de 1-(diéthylaminocarbonyl)indoline-5-sulfonyle.

On refroidit à +5°C 12,3 ml d'acide chlorosulfonique, ajoute rapidement 8 g du composé obtenu à l'étape précédente. On chauffe à 60°C, pendant 2 heures, sous forte agitation, le mélange réactionnel puis refroidit à TA. On ajoute lentement de la glace, puis de l'eau, extrait à l'AcOEt, lave la phase organique à l'eau jusqu'à pH = 7, sèche sur sulfate de sodium et évapore sous vide le solvant. On chromatographie le résidu sur silice en éluant par le mélange DCM/AcOEt (95/5 ; v/v). On obtient 4,54 g du produit attendu.

Spectre de RMN à 200 MHz dans DMSO-$d_6$.

1,15 ppm : t : 6H
2,95 ppm : t : 2H
3,2 ppm : qd : 4H
3,75 ppm : t : 2H

6,7 à 7,5 ppm : m : 3H

EXEMPLE 1

3-Amino-5-chloro-1,3-dihydro-1-(2,4-diméthoxybenzènesulfonyl)-3-phénylindol-2-one.

On refroidit à 0°C, sous atmosphère d'argon, une solution de 0,3 g de 3-amino-5-chloro-1,3-dihydro-3-phénylindol-2-one dans 7 ml de DMF et ajoute 0,037 g d'hydrure de sodium à 80 % dans l'huile. Après 20 minutes d'agitation on ajoute 0,275 g de chlorure de 2,4-diméthoxybenzènesulfonyle et laisse une nuit sous agitation en laissant remonter la température à TA. On verse le mélange réactionnel dans l'eau, extrait à l'AcOEt, lave à l'eau, par une solution saturée de NaCl, sèche sur sulfate de sodium et évapore sous vide. On chromatographie le résidu sur silice en éluant par le mélange DCM/AcOEt (95/5 ; v/v). On obtient le produit attendu après cristallisation dans le mélange DCM/éther iso/hexane, m = 0,31 g, F = 108-110°C.

EXEMPLE 2

3-Acétamido-5-chloro-1,3-dihydro-1-(2,4-diméthoxybenzènesulfonyl)-3-phénylindol-2-one.

A une solution de 0,234 g du composé obtenu à l'EXEMPLE 1 dans 2 ml de DCM, on ajoute 0,051 g de triéthylamine puis 0,04 g de chlorure d'acétyle et laisse deux heures sous agitation à TA. On verse le mélange réactionnel dans l'eau, extrait au DCM, lave à l'eau, sèche sur sulfate de sodium et évapore sous vide. On obtient le produit attendu après cristallisation et recristallisation dans le mélange DCM/éther iso/MeOH, m = 0,146 g, F = 253°C

EXEMPLE 3

3-Amino-5-chloro-3-(2-chlorophényl)-1,3-dihydro-1-(2,4-diméthoxybenzènesulfonyl)indol-2-one.

On prépare ce composé selon le mode opératoire décrit à l'EXEMPLE 1 à partir de 0,315 g de 3-amino-5-chloro-3-(2-chlorophényl)-1,3-dihydroindol-2-one et 0,256 g de chlorure de 2,4-diméthoxybenzènesulfonyle. On obtient le produit attendu après cristallisation dans le mélange DCM/éther iso, m = 0,300 g, F = 179-180°C.

EXEMPLE 4

5-Chloro-3-(éthanecarboxamido)-3-(2-chlorophényl)-1,3-dihydro-1-(2,4-diméthoxybenzènesulfonyl)indol-2-one.

A une solution de 0,33 g du composé obtenu à l'EXEMPLE 3 dans 3 ml de pyridine, on ajoute 0,08 g de chlorure de propionyle et laisse 1 heure sous agitation à TA. On verse le mélange réactionnel dans l'eau, extrait à l'AcOEt, sèche sur sulfate de sodium et évapore sous vide. On chromatographie sur silice en éluant par le mélange DCM/AcOEt (98/2 ; v/v). On obtient le produit attendu après cristallisation dans le mélange DCM/éther iso, m = 0,18 g, F = 188°C.

EXEMPLE 5

5-Chloro-3-(2-chlorophényl)-1,3-dihydro-1-(2,4-diméthoxybenzènesulfonyl) -3-(méthoxycarboxamido)indol-2-one.

A une solution de 0,205 g du composé obtenu à l'EXEMPLE 3 dans 2 ml de DCM, on ajoute, à TA, 0,066 g de pyridine, puis 0,095 g de chloroformiate de méthyle. On laisse 72 heures sous agitation entre 0 et +4°C et verse le mélange réactionnel dans l'eau. On extrait au DCM, lave par une solution à 5 % d'hydrogénosulfate de potassium, à l'eau, sèche sur sulfate de sodium et évapore sous vide. On obtient le produit attendu après cristallisation dans le mélange DCM/éther iso, m = 0,175 g, F = 225-228°C.

EXEMPLE 6

5-Chloro-3-(2-chlorophényl)-1,3-dihydro-1-(2,4-diméthoxybenzènesulfonyl)-3-(méthylsulfonamido)indol-2-one.

On refroidit à 0°C une solution de 0,4 g du composé obtenu à l'EXEMPLE 3 dans 5 ml de pyridine et ajoute 0,1 g de chlorure de méthanesulfonyle. On laisse 18 heures sous agitation en laissant remonter la température à TA et évapore sous vide. On extrait le résidu à l'AcOEt, lave par une solution à 5 % d'hydrogénosulfate de potassium, sèche sur sulfate de sodium et évapore sous vide. On chromatographie sur silice en éluant par le mélange DCM/AcOEt (98/2 ; v/v). On obtient le produit attendu après cristallisation dans le mélange DCM/éther iso, m = 0,22 g, F = 252°C.

EXEMPLE 7

5-Chloro-3-(2-chlorophényl)-1,3-dihydro-1-(2,4-diméthoxybenzènesulfonyl)-3-(phénoxycarboxamido)indol-2-one.

A une solution de 0,530 g du composé obtenu à l'EXEMPLE 3 dans 4 ml de pyridine on ajoute à TA, 0,250 g de chloroformiate de phényle. On laisse 18 heures sous agitation à TA et évapore sous vide. On extrait le résidu au DCM, lave par une solution à 5 % d'hydrogénosulfate de potassium, à l'eau, sèche sur sulfate de sodium et évapore sous vide. On chromatographie sur silice en éluant par le mélange DCM/AcOEt (98/2 ; v/v). On obtient le produit attendu après cristallisation dans le mélange DCM/éther iso, m = 0,47 g, F = 95-100°C.

EXEMPLE 8

5-Chloro-3-(2-chlorophényl)-1,3-dihydro-1-(2,4-diméthoxybenzènesulfonyl)-3-uréidoindol-2-one.

A une solution de 0,400 g du composé obtenu à l'EXEMPLE 7 dans 6 ml d'EtOH, on ajoute à TA, 1 ml d'ammoniaque concentrée et laisse 18 heures sous agitation. On évapore sous vide le mélange réactionnel et reprend le résidu à l'AcOEt, lave à l'eau, sèche sur sulfate de sodium. Après évaporation sous vide, on chromatographie sur silice en éluant par le mélange DCM/AcOEt (50/50 ; v/v). On obtient le produit attendu après cristallisation dans le mélange DCM/éther iso, m = 0,15 g, F = 285°C.

EXEMPLE 9

5-Chloro-3-(2-chlorophényl)-1,3-dihydro-1-(2,4-diméthoxybenzènesulfonyl)-3-(N',N'-diméthyluréido)indol-2-one.

On prépare ce composé selon le mode opératoire décrit à l'EXEMPLE 8, à partir de 0,400 g du composé obtenu à l'EXEMPLE 7 et 0,3 ml d'une solution à 33 % de diméthylamine dans l'EtOH. On obtient le produit attendu après cristallisation dans le mélange DCM/éther iso, m = 0,30 g, F = 246°C.

EXEMPLE 10

5-Chloro-3-(2-chlorophényl)-3-(N',N'-diéthyluréido)-1,3-dihydro-1-(2,4-diméthoxybenzènesulfonyl)indol-2-one.

On chauffe à 60°C pendant 18 heures un mélange de 0,460 g du composé obtenu à l'EXEMPLE 7, 0,082 g de diéthylamine dans 4 ml de chloroforme et 4 ml d'EtOH. On évapore sous vide le mélange réactionnel et chromatographie le résidu sur silice en éluant par le mélange DCM/AcOEt (95/5 ; v/v). On obtient le produit attendu après cristallisation dans le mélange DCM/éther iso, m = 0,20 g, F = 222°C.

EXEMPLE 11

3-Amino-5-chloro-3-(2-chlorophényl)-1,3-dihydro-1-(4-nitrobenzènesulfonyl)-indol-2-one.

On prépare ce composé selon le mode opératoire décrit à l'EXEMPLE 1 à partir de 10 g de 3-amino-5-chloro-3-(2-chlorophényl)-1,3-dihydroindol-2-one et 7,55 g de chlorure de 4-nitrobenzènesulfonyle. On obtient le produit attendu après cristallisation dans le mélange DCM/éther iso, m = 14 g, F = 202°C.

EXEMPLE 12

5-Chloro-3-(2-chlorophényl)-3-(N',N'-diéthyluréido)-1,3-dihydro-1-(4-nitro-benzènesulfonyl)indol-2-one.

A) 5-Chloro-3-(2-chlorophényl)-1,3-dihydro-1-(4-nitrobenzènesulfonyl)-3-(phénoxycarboxamido)indol-2-one.

On prépare ce composé selon le mode opératoire décrit à l'EXEMPLE 7 à partir de 11 g du composé obtenu à l'EXEMPLE 11 et 4,63 g de chloroformiate de phényle. On chromatographie sur silice en éluant par du DCM. On obtient 12,3 g du produit attendu qui est utilisé tel quel à l'étape suivante.

B) 5-Chloro-3-(2-chlorophényl)-3-(N',N'-diéthyluréido)-1,3-dihydro-1-(4-nitrobenzènesulfonyl)indol-2-one.

On prépare ce composé selon le mode opératoire décrit à l'EXEMPLE 10 à partir de 10 g du composé obtenu à l'étape précédente et 1,9 g de diéthylamine. On obtient le produit attendu après cristallisation dans le mélange DCM/éther iso, m = 7,7 g, F = 185°C.

EXEMPLE 13

1-(4-Aminobenzènesulfonyl)-5-chloro-3-(2-chlorophényl)-3-(N',N'-diéthyl-uréido)-1,3-dihydroindol-2-one.

On réduit par l'hydrogène sous 50 bars de pression, à TA, pendant 6 heures, 7,6 g du composé obtenu à l'EXEMPLE 12, en présence de 2 g de nickel de Raney , dans 100 ml d'EtOH et 50 ml de THF. On filtre sur Célite et évapore sous vide le filtrat. On chromatographie le résidu sur silice en éluant par le mélange DCM/AcOEt (95/5 ; v/v). On obtient le produit attendu après cristallisation dans le mélange DCM/éther iso, m = 3,2 g, F = 210°C.

EXEMPLE 14

1-[4-(*tert*-Butanecarboxamido)benzènesulfonyl]-5-chloro-3-(2-chlorophényl)-3-(N',N'-diéthyluréido)-1,3-dihydroindol-2-one.

On refroidit à 0°C une solution de 0,400 g du composé obtenu à l'EXEMPLE 13 dans 3 ml de pyridine et ajoute 0,100 g de chlorure de pivaloyle. On laisse 18 heures sous agitation en laissant remonter la température à TA, puis évapore sous vide. On extrait le résidu à l'AcOEt, lave par une solution à 5 % d'hydrogénosulfate de potassium, sèche sur sulfate de sodium et évapore sous vide. On chromatographie sur silice en éluant par le mélange DCM/AcOEt (95/5; v/v). On obtient le produit attendu après cristallisation dans le mélange DCM/éther iso, m = 0,180 g, F = 228°C.

EXEMPLE 15

5-Chloro-3-(2-chlorophényl)-3-(N',N'-diéthyluréido)-1-[4-(N',N'-diéthyluréido)-benzènesulfonyl]-1,3-dihydroindol-2-one.

A) 5-Chloro-3-(2-chlorophényl)-3-(N',N'-diéthyluréido)-1,3-dihydro-1-[4-(phénoxycarboxamido)benzènesulfonyl]indol-2-one.

On refroidit à 0-5°C une solution de 0,500 g du composé obtenu à l'EXEMPLE 13 dans 10 ml de pyridine et ajoute 0,156 g de chloroformiate de phényle. On laisse 18 heures sous agitation en laissant remonter la température à TA puis évapore sous vide. On extrait le résidu à l'AcOEt, lave par une solution à 5 % d'hydrogénosulfate de potassium, sèche sur sulfate de sodium et évapore sous vide. On chromatographie sur silice en éluant par le mélange AcOEt/hexane (30/70 ; v/v). On obtient 0,53 g du produit attendu qui est utilisé tel quel à l'étape suivante.

B) 5-Chloro-3-(2-chlorophényl)-3-(N',N'-diéthyluréido)-1-[4-(N',N'-diéthyluréido)-benzènesulfonyl]-1,3-dihydroindol-2-one.

On prépare ce composé selon le mode opératoire décrit à l'EXEMPLE 10 à partir de 0,53 g du composé obtenu à l'étape précédente et 0,136 g de diéthylamine. On obtient le produit attendu après cristallisation dans le mélange DCM/éther iso, m = 0,24 g, F = 200°C.

EXEMPLE 16

3-Amino-5-chloro-3-(2-chlorophényl)-1,3-dihydro-1-(2-méthoxy-4-nitrobenzènesulfonyl)indol-2-one.

On prépare ce composé selon le mode opératoire décrit à l'EXEMPLE 1, à partir de 7 g de 3-amino-5-chloro-3-(2-chlorophényl)-1,3-dihydroindol-2-one et 6 g de chlorure de 2-méthoxy-4-nitrobenzènesulfonyle. On obtient le produit attendu après cristallisation dans le mélange DCM/éther iso/THF, m = 9,4 g, F = 229°C.

EXEMPLE 17

3-Amino-1-(4-amino-2-méthoxybenzènesulfonyl)-5-chloro-3-(2-chlorophényl)-1,3-dihydroindol-2-one.

On réduit par l'hydrogène, sous 40 bars de pression, à TA pendant 4 heures, 2,17 g du composé obtenu à l'EXEMPLE 16, en présence de 1 g de nickel de Raney dans 30 ml de MeOH et 30 ml de THF. On filtre sur Célite et évapore sous vide le filtrat. On chromatographie le résidu sur silice en éluant par du DCM puis par le mélange DCM/AcOEt (90/10 ; v/v). On obtient le produit attendu après cristallisation dans le mélange DCM/éther iso, m = 1,3 g, F = 198°C.

EXEMPLE 18 et EXEMPLE 19

5-Chloro-3-(2-chlorophényl)-1,3-dihydro-1-[2-méthoxy-4-(phénoxy-carboxamido)benzènesulfonyl]-3-(phénoxycarboxamido)indol-2-one (EXEMPLE 18) et

3-Amino-5-chloro-3-(2-chlorophényl)-1,3-dihydro-1-[2-méthoxy-4-(phénoxycarboxamido)benzènesulfonyl]indol-2-one (EXEMPLE 19).

On prépare ces composés selon le mode opératoire décrit à l'EXEMPLE 7 à partir de 1,15 g du composé obtenu à l'EXEMPLE 17 et 0,32 ml de chloroformiate de phényle. On chromatographie sur silice en éluant par du DCM puis par un mélange DCM/AcOEt (95/5 ; v/v). On sépare deux composés :

- le moins polaire : composé de l'EXEMPLE 18, m = 0,610 g
- le plus polaire : composé de l'EXEMPLE 19, m = 0,406 g.

Spectre de RMN à 200 MHz dans DMSO-$d_6$ du composé de l'EXEMPLE 18.

3,45 ppm : s : 3H
6,6 à 8,0 ppm : m : 20H
9,3 ppm : s : 1H
10,8 ppm : s : 1H

Spectre de RMN à 200 MHz dans DMSO-$d_6$ du composé de l'EXEMPLE 19.

3,1 ppm : s : 2H
3,6 ppm : s : 3H
6,6 à 8,3 ppm : m : 15H
10,8 ppm : s : 1H

EXEMPLE 20

5-Chloro-3-(2-chlorophényl)-3-(N',N'-diéthyluréido)-1-[4-(N',N'-diéthyluréido)-2-méthoxybenzènesulfonyl]-1,3-dihydroindol-2-one.

On prépare ce composé selon le mode opératoire décrit à l'EXEMPLE 10 à partir de 0,610 g du composé obtenu à l'EXEMPLE 18 et 0,248 g de diéthylamine. On chromatographie sur silice en éluant par un gradient du mélange DCM/AcOEt de (95/5 ; v/v) jusqu'à (80/20 ; v/v). On obtient le produit attendu après cristallisation dans le mélange DCM/éther iso, m = 0,456 g, F = 185-190°C.

EXEMPLE 21

3-Amino-5-chloro-3-(2-chlorophényl)-1-[4-(N',N'-diéthyluréido)-2-méthoxy benzènesulfonyl]-1,3-dihydroindol-2-one.

On chauffe à 60°C pendant 4 heures un mélange de 0,406 g du composé obtenu à l'EXEMPLE 19, 0,100 g de diéthylamine dans 5 ml de chloroforme. On évapore sous vide et chromatographie le résidu sur silice en éluant par du DCM puis par le mélange DCM/AcOEt (90/10 ; v/v). On obtient le produit attendu après cristallisation dans le mélange DCM/éther iso, m = 0,280 g, F = 152-158°C.

EXEMPLE 22

5-Chloro-3-(2-chlorophényl)-1,3-dihydro-3-(méthoxycarboxamido)-1-(2-méthoxy-4-nitrobenzènesulfonyl)indol-2-one.

On refroidit au bain de glace une solution de 2,8 g du composé obtenu à l'EXEMPLE 16 dans 15 ml de pyridine et ajoute une solution de 0,85 ml de chloroformiate de méthyle dans 3 ml de DCM. On laisse 3 jours sous agitation à TA et verse le milieu réactionnel dans l'eau. On extrait à l'AcOEt, lave à l'eau, par une solution d'HCl 1N, à l'eau, sèche sur sulfate de sodium et évapore sous vide. On chromatographie sur silice en éluant par du DCM, puis par un mélange DCM/AcOEt (90/10 ; v/v). On obtient 0,800 g du produit attendu.

Spectre de RMN à 200 MHz dans DMSO-$d_6$

3,4 ppm : s : 3H
3,7 ppm : s : 3H
7,1 à 8,3 ppm : m : 10H
8,75 ppm : s : 1H

EXEMPLE 23

1-(4-Amino-2-méthoxybenzènesulfonyl)-5-chloro-3-(2-chlorophényl)-1,3-dihydro-3-(méthoxycarboxamido) indol-2-one.

On prépare ce composé selon le mode opératoire décrit à l'EXEMPLE 17 à partir de 0,800 g du composé obtenu à l'EXEMPLE 22. Après filtration sur Célite on évapore sous vide le filtrat et obtient 0,742 g du produit attendu.

Spectre de RMN à 200 MHz dans DMSO-$d_6$

3,45 ppm : s : 3H
3,55 ppm : s : 3H
6,0 à 8,0 ppm : m : 10H et 2H
8,65 ppm : s : 1H

EXEMPLE 24

5-Chloro-3-(2-chlorophényl)-1-[4-(N',N'-diéthyluréido)-2-méthoxybenzène-sulfonyl]-1,3-dihydro-3-(méthoxycarboxamido)indol-2-one.

A)5-Chloro-3-(2-chlorophényl)-1,3-dihydro-3-(méthoxycarboxamido)-1-[2-méthoxy-4-(phénoxycarboxamido) benzènesulfonyl]indol-2-one.

On prépare ce composé selon le mode opératoire décrit à l'EXEMPLE 7 à partir de 0,742 g du composé obtenu à l'EXEMPLE 23 et 0,18 ml de chloroformiate de phényle. On chromatographie sur silice en éluant par un gradient du mélange DCM/AcOEt de (95/5 ; v/v) jusqu'à (80/20 ; v/v). On obtient 0,905 g du produit attendu qui est utilisé tel quel à l'étape suivante.

B) 5-Chloro-3-(2-chlorophényl)-1-[4-(N',N'-diéthyluréido)-2-méthoxybenzène-sulfonyl]-1,3-dihydro-3-(méthoxycarboxamido)indol-2-one.

On prépare ce composé selon le mode opératoire décrit à l'EXEMPLE 10 à partir de 0,100 g du composé obtenu à l'étape précédente et 0,022 g de diéthylamine. On obtient le produit attendu après cristallisation à chaud dans le mélange EtOH/DMF, m = 0,050 g, F = 240-245°C.

EXEMPLE 25

3-Acétamido-5-chloro-3-(2-chlorophényl)-1,3-dihydro-1-(2-méthoxy-4-nitrobenzènesulfonyl)indol-2-one.

On refroidit au bain de glace une solution de 2,8 g du composé obtenu à l'EXEMPLE 16 dans 10 ml de pyridine et ajoute 0,51 ml de chlorure d'acétyle. On laisse 3 heures sous agitation à TA et verse le milieu réactionnel dans l'eau. On extrait à l'AcOEt, lave à l'eau, par une solution à 5 % d'hydrogénosulfate de potassium, sèche sur sulfate de sodium et évapore sous vide. On reprend le résidu au DCM et filtre le précipité formé. On obtient 2,8 g du produit attendu dont un échantillon est cristallisé dans le mélange MeOH/éther iso, F = 205-210°C.

EXEMPLE 26

3-Acétamido-1-(4-amino-2-méthoxybenzènesulfonyl)-5-chloro-3-(2-chlorophényl)-1,3-dihydroindol-2-one.

On prépare ce composé selon le mode opératoire décrit à l'EXEMPLE 17 à partir de 2,17 g du composé obtenu à l'EXEMPLE 25. Après filtration sur Célite et évaporation sous vide du filtrat, on reprend le résidu au DCM, filtre le précipité formé, lave à l'éther iso et sèche en étuve à 90°C. On obtient 1,53 g du produit attendu.

Spectre de RMNà 200 MHz dans DMSO-$d_6$

1,8 ppm : s : 3H
3,35 ppm : s : 3H
5,9 à 7,9 ppm : m : 10H et 2H
9,0 ppm : s : 1H

EXEMPLE 27

3-Acétamido-5-chloro-3-(2-chlorophényl)-1-[4-(N',N'-diéthyluréido)-2-méthoxybenzènesulfonyl]-1,3-dihydroindol-2-one.

A) 3-Acétamido-5-chloro-3-(2-chlorophényl)-1,3-dihydro-1-[2-méthoxy-4-(phénoxycarboxamido)benzènesulfonyl]indol-2-one.

On prépare ce composé selon le mode opératoire décrit à l'EXEMPLE 7 à partir de 1,53 g du composé obtenu à l'EXEMPLE 26. Après séchage sur sulfate de sodium et évaporation sous vide, on obtient 1,60 g du produit attendu qui est utilisé tel quel à l'étape suivante.

B) 3-Acétamido-5-chloro-3-(2-chlorophényl)-1-[4-(N',N'-diéthyluréido)-2-méthoxybenzènesulfonyl]-1,3-dihydroindol-2-one.

On prépare ce composé selon le mode opératoire décrit à l'EXEMPLE 10 à partir de 0,300 g du composé obtenu à l'étape précédente et 0,068 g de diéthylamine. On obtient le produit attendu après cristallisation dans le mélange DCM/éther iso, m = 0,160 g, F 180-185°C.

EXEMPLE 28

3-Acétamido-1-[4-(N-*tert*-butylcarbamoyl)-2-méthoxybenzènesulfonyl]-5-chloro-3-(2-chlorophényl)-1,3-dihydroindol-2-one.

A) 4-[3-Amino-5-chloro-3-(2-chlorophényl)-2,3-dihydro-2-oxoindol-1-yl] sulfonyl-3-méthoxybenzoate de benzyle.

On prépare ce composé selon le mode opératoire décrit à l'EXEMPLE 1 à partir de 0,860 g de 3-amino-5-chloro-3-(2-chlorophényl)-1,3-dihydroindol-2-one et 1 g de 4-chlorosulfonyl-3-méthoxybenzoate de benzyle. On chromatographie sur silice en éluant par le mélange DCM/AcOEt (99/1 ; v/v). On obtient 1,38 g du produit attendu qui est utilisé tel quel à l'étape suivante.

B) 4-[3-acétamido-5-chloro-3-(2-chlorophényl)-2,3-dihydro-2-oxoindol-1-yl] sulfonyl-3-méthoxybenzoate de benzyle.

On refroidit à 0°C une solution de 0,600 g du composé obtenu à l'étape précédente dans 4 ml de pyridine et ajoute une solution de 0,102 g de chlorure d'acétyle dans 2 ml de DCM. On laisse 30 minutes sous agitation et verse le mélange réactionnel dans l'eau. On extrait à l'AcOEt, lave par une solution à 5 % d'hydrogénosulfate de potassium, sèche sur sulfate de sodium et évapore sous vide. On chromatographie sur silice en éluant par le mélange DCM/AcOEt (90/10 ; v/v). On obtient le produit attendu après cristallisation dans le mélange DCM/éther iso, m = 0,466 g, F = 177°C.

C) Acide 4-[3-acétamido-5-chloro-3-(2-chlorophényl)-2,3-dihydro-2-oxoindol-1-yl]-sulfonyl-3-méthoxybenzoïque.

On hydrogénolyse à TA, à la pression atmosphérique pendant 30 minutes, un mélange de 0,400 g du composé obtenu à l'étape précédente, 0,040 g de palladium sur charbon à 10 % dans 15 ml d'AcOH. On filtre sur Célite et évapore sous vide le filtrat. On reprend le résidu à l'éther et filtre le précipité formé. On obtient 0,310 g du produit attendu qui est utilisé tel quel à l'étape suivante.

D) 3-Acétamido-1-[4-(N-*tert*-butylcarbamoyl)-2-méthoxybenzènesulfonyl]-5-chloro-3-(2-chlorophényl)-1,3-dihydroindol-2-one.

On refroidit à 0°C une solution de 0,310 g du composé obtenu à l'étape précédente dans 8 ml de DMF et ajoute 0,074 g de DIPEA puis 0,269 g de BOP. Après 15 minutes d'agitation on ajoute 0,055 g de *tert*-butylamine et laisse 6 heures sous agitation en laissant remonter la température à TA. On évapore sous vide, reprend le résidu à l'eau, extrait à l'AcOEt, lave par une solution à 5 % d'hydrogénosulfate de potassium, à l'eau, sèche sur sulfate de sodium et évapore sous vide. On chromatographie sur silice en éluant par le mélange DCM/AcOEt (80/20 ; v/v). On obtient le produit attendu après cristallisation dans le mélange DCM/éther iso, m = 0,15 g, F = 265°C.

EXEMPLE 29

3-Amino-1-(4-(N-*tert*-butylcarbamoyl)-2-méthoxybenzènesulfonyl]-5-chloro-3-(2-chlorophényl)-1,3-dihydroindol-2-one.

A)Acide 4-[3-amino-5-chloro-3-(2-chlorophényl)-2,3-dihydro-2-oxoindol-1-yl]sulfonyl-3-méthoxybenzoïque.

On prépare ce composé selon le mode opératoire décrit à l'étape C de l'EXEMPLE 28 à partir de 0,700 g du composé obtenu à l'étape A de l'EXEMPLE 28. Après filtration sur Célite et évaporation sous vide du filtrat on reprend le résidu à l'éther iso. Après filtration, on obtient 0,530 g du produit attendu qui est utilisé tel quel à l'étape suivante.

B) 3-Amino-1-[4-(N-*tert*-butylcarbamoyl)-2-méthoxybenzènesulfonyl]-5-chloro-3-(2-chlorophényl)-1,3-dihydroindol-2-one.

On refroidit à 0°C un mélange de 0,530 g du composé obtenu à l'étape précédente, 0,134 g de DIPEA, 0,228 g de *tert*-butylamine dans 6 ml de DMF et ajoute progressivement 0,483 g de BOP. On laisse 18 heures sous agitation en laissant remonter la température à TA. On ajoute de l'eau au milieu réactionnel, extrait à l'AcOEt, sèche sur sulfate de sodium et évapore sous vide. On chromatographie sur silice en éluant par le mélange DCM/AcOEt (95/5 ; v/v). On obtient le produit attendu après cristallisation dans l'éther iso, m = 0,150 g, F = 263-265°C.

EXEMPLE 30

5-Chloro-3-(2-chlorophényl)-1-[4-(N',N'-diéthyluréido)benzènesulfonyl]-1,3-dihydro-3-(diméthylamino)indol-2-one.

On prépare ce composé selon le mode opératoire décrit à l'EXEMPLE 1 à partir de 0,500 g de 5-chloro-3-(2-chlorophényl)-1,3-dihydro-3-(diméthylamino)indol-2-one et 0,453 g de chlorure de 4-(N',N'-diéthyluréido)benzènesulfonyle. On chromatographie sur silice en éluant par un gradient du mélange hexane/AcOEt (80/20 ; v/v) jusqu'à (60/40 ; v/v). On obtient le produit attendu après cristallisation dans le mélange DCM/éther iso, m = 0,520 g, F = 146-150°C.

EXEMPLE 31

5-Chloro-3-(2-chlorophényl)-1-[4-(N',N'-diéthyl-N-méthyluréido)benzène-sulfonyl]-1,3-dihydro-3-(diméthylamino)indol-2-one.

On refroidit à 0°C, sous atmosphère d'argon une solution de 0,420 g du composé obtenu à l'EXEMPLE 30 dans 7 ml de DMF et ajoute 0,027 g d'hydrure de sodium à 80 % dans l'huile. Après 30 minutes d'agitation, on ajoute 0,05 ml d'iodure de méthyle et laisse 10 minutes sous agitation. On verse le mélange réactionnel dans l'eau, extrait à l'AcOEt, lave à l'eau, sèche sur sulfate de sodium et évapore sous vide. On chromatographie sur silice en éluant par du DCM, puis par un mélange DCM/AcOEt (95/5 ; v/v). On obtient le produit attendu après cristallisation dans le mélange DCM/éther iso, m = 0,211 g, F = 145-148°C.

EXEMPLE 32

3-Amino-5-chloro-1-[4-(N',N'-diéthyluréido)benzènesulfonyl]-1,3-dihydro-3-(2-méthoxyphényl)indol-2-one.

On prépare ce composé selon le mode opératoire décrit à l'EXEMPLE 1 à partir de 0,266 g de 3-amino-5-chloro-1,3-dihydro-3-(2-méthoxyphényl)indol-2-one et 0,280 g de chlorure de 4-(N',N'-diéthyluréido)benzènesulfonyle. On obtient le produit attendu après cristallisation dans le mélange DCM/éther iso, m = 0,160 g, F = 214-217°C.

EXEMPLE 33

5-Chloro-3-(2-chlorophényl)-1-(4-éthylamino-2-méthoxybenzènesulfonyl)-1,3-dihydro-3-(méthoxycarboxamido)indol-2-one.

A une solution de 0,750 g du composé obtenu à l'EXEMPLE 23 dans 17 ml de MeOH et 5 ml de THF, on ajoute à TA 2 ml d'AcOH, puis 0,15 ml d'acétaldéhyde et en 10 minutes, 0,108 g de cyanoborohydrure de sodium. La réaction étant immédiate, on ajoute 2 gouttes d'HCl concentré puis neutralise le milieu réactionnel par addition d'une solution à 5 % de carbonate de potassium. On évapore sous vide les solvants, extrait à l'AcOEt, lave à l'eau, sèche sur sulfate de sodium et évapore sous vide. On chromatographie sur silice en éluant par du DCM, puis par un mélange DCM/AcOEt (97/3 ; v/v). On obtient le produit attendu après cristallisation dans le mélange DCM/éther iso, m = 0,435 g, F = 192-195°C.

EXEMPLE 34

5-Chloro-3-(2-chlorophényl)-1-(4-diéthylamino-2-méthoxybenzène-sulfonyl)-1,3-dihydro-3-(méthoxycarboxamido)indol-2-one.

A une solution de 0,311 g du composé obtenu à l'EXEMPLE 33 dans 7 ml de MeOH et 2 ml de THF, on ajoute à TA 0,8 ml d'AcOH puis 0,12 ml d'acétaldéhyde. Puis en 4 jours, on ajoute en 3 fois, 0,086 g de cyanoborohydrure de sodium. La réaction terminée, on ajoute 2 gouttes d'HCl concentré puis neutralise le milieu réactionnel par addition d'une solution à 5 % de carbonate de potassium. On évapore sous vide les solvants, extrait à l'AcOEt, lave à l'eau, sèche sur sulfate de sodium et évapore sous vide. On chromatographie sur silice en éluant par du DCM, puis par un mélange DCM/AcOEt (95/5 ; v/v). On obtient le produit attendu après cristallisation dans le mélange DCM/éther iso, m = 0,140 g, F = 210-215°C.

EXEMPLE 35

5-Chloro-3-(2-chlorophényl)-1-[4-(N',N'-diéthyluréido)benzènesulfonyl]-1,3-dihydro-3-[(lS)-1-(méthoxycarbonyl)éthylamino]indol-2-one.

On prépare ce composé selon le mode opératoire décrit à l'EXEMPLE 1 à partir de 0,639 g du composé obtenu à la Préparation 12 (isomère A) et 0,490 g de chlorure de 4-(N',N'-diéthyluréido)benzènesulfonyle. On chromatographie sur silice en éluant par le mélange DCM/AcOEt (95/5 ; v/v). On obtient le produit attendu après cristallisation dans le mélange DCM/éther iso, m = 0,350 g, F = 204°C,

$$\alpha_D^{20} = +114,5° \text{ (c = 0,22 ; chloroforme).}$$

EXEMPLE 36

5-Chloro-3-(2-chlorophényl)-1-[4-(N',N'-diéthyluréido)benzènesulfonyl]-1,3-dihydro-3-[(lS)-1-(méthoxycarbonyl)éthylamino]indol-2-one.

On prépare ce composé selon le mode opératoire décrit à l'EXEMPLE 1 à partir de 0,392 g du composé obtenu à la Préparation 13 (isomère B) et 0,329 g de chlorure de 4-(N',N'-diéthyluréido)benzènesulfonyle. On chromatographie sur silice en éluant par le mélange DCM/AcOEt (95/5 ; v/v). On obtient le produit attendu après cristallisation dans le mélange DCM/éther iso/hexane, m = 0,225 g, F = 204°C,

$$\alpha_D^{20} = -139,4° \text{ (c = 0,19 ; chloroforme).}$$

En procédant selon les modes opératoires décrits dans les exemples ci-dessus, à partir des 1,3-dihydroindol-2-one décrites dans les Préparations , on prépare les composés selon l'invention rassemblés dans le TABLEAU II ci-après.

TABLEAU II

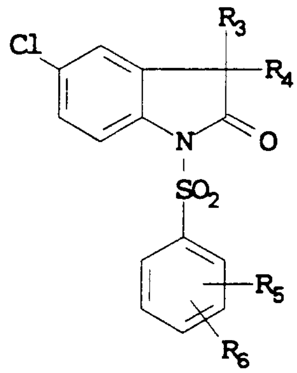

| Exemple | $R_3$ | $R_4$ | $R_5$ | $R_6$ | F°C ou RMN solvant de cristallisation |
|---|---|---|---|---|---|
| 37 (a) | 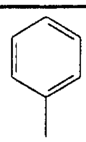 | $-NH_2$ | 3-OMe | 4-OMe | 150 DCM/éther iso |
| 38 (a) | 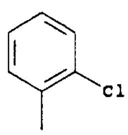 | $-NH_2$ | 3-OMe | 4-OMe | 161 DCM/éther iso |
| 39 (a) | 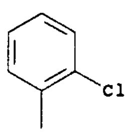 | -NH-Me | 2-OMe | 4-OMe | 205 DCM/éther iso |
| 40 (a) | 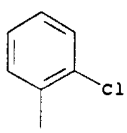 | -NH-Me | 3-OMe | 4-OMe | 182 DCM/éther iso |
| 41 (b) | 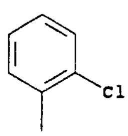 | -NH-Me | 2-OMe | $4-NO_2$ | 145 DCM/éther iso |
| 42 (c) | 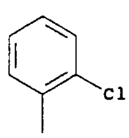 | -NH-Me | 2-OMe | $4-NH_2$ | - |

TABLEAU II (suite)

| Exemple | $R_3$ | $R_4$ | $R_5$ | $R_6$ | F°C ou RMN solvant de cristallisation |
|---|---|---|---|---|---|
| 43 (d) et (m) | 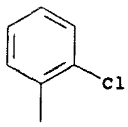 | -NH-Me | 2-OMe | 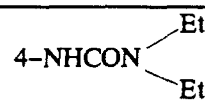 | 208-210 DCM/éther iso |
| 44 (f) | 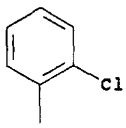 | -NH-Me | H | 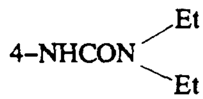 | 232 DCM/éther iso |
| 45 (f) | 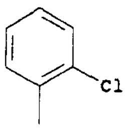 | $-NHCH_2Me$ | H | 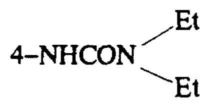 | 224-229 DCM/éther iso |
| 46 (f) | 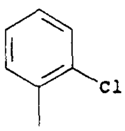 | - $NHCH_2CH_2Me$ | H | 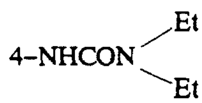 | 160-164 DCM/éther iso |
| 47 (f) | 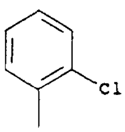 | $-NH(CH_2)_4Me$ | H | 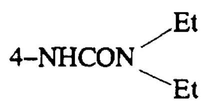 | 219-221 DCM/éther iso |
| 48 (f) | 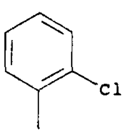 | 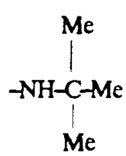 | H | 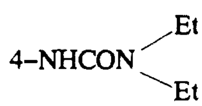 | 220-223 DCM/éther iso |
| 49 (f) | 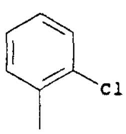 | 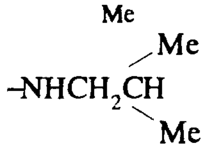 | H | 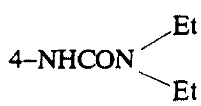 | 177 DCM/éther iso |
| 50 (f) | 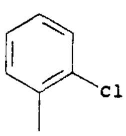 | 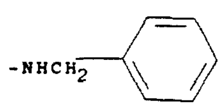 | H | 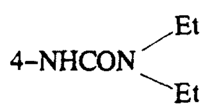 | 246-248 DCM/éther iso |
| 51 (a) | 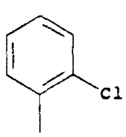 | 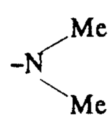 | 2-OMe | 4-OMe | 215-217 DCM/éther iso |

TABLEAU II (suite)

| Exemple | $R_3$ | $R_4$ | $R_5$ | $R_6$ | F°C ou RMN solvant de cristallisation |
|---|---|---|---|---|---|
| 52 (a) | 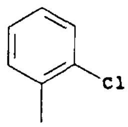 | 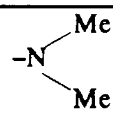 | 3-OMe | 4-OMe | 168-170 DCM/éther iso |
| 53 (g) | 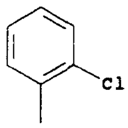 | 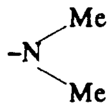 | H | 4-$NO_2$ | 245-247 DCM/éther iso |
| 54 (h) | 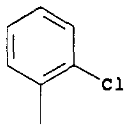 | 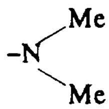 | H | 4-$NH_2$ | RMN |
| 55 (b) | 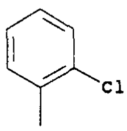 | 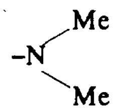 | 2-OMe | 4-$NO_2$ | RMN |
| 56 (c) | 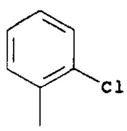 | 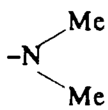 | 2-OMe | 4-$NH_2$ | 155-158 DCM/hexane |
| 57 (d) et (m) | 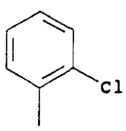 | 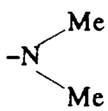 | 2-OMe | 4-NHCON 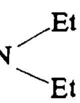 | 205-210 DCM/éther iso |
| 58 (a) | 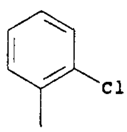 | 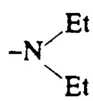 | 2-OMe | 4-OMe | 180-185 DCM/éther iso |
| 59 (g) | 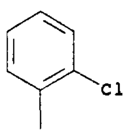 | 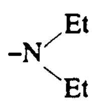 | H | 4-$NO_2$ | 198-205 DCM/éther iso |
| 60 (h) | 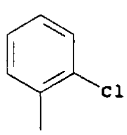 | 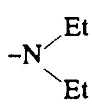 | H | 4-$NH_2$ | 205-208 DCM/éther iso |
| 61 (n) | 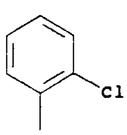 | 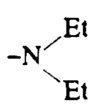 | H | 4-NHCON 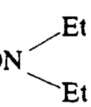 | 176-180 DCM/éther iso |

TABLEAU II (suite)

| Exemple | $R_3$ | $R_4$ | $R_5$ | $R_6$ | F°C ou RMN solvant de cristallisation |
|---|---|---|---|---|---|
| 62 (f) | 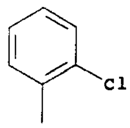 | $-NHCH_2CH_2OMe$ | H | 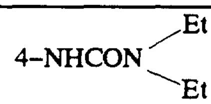 | 149-151 DCM/éther iso |
| 63 (f) | 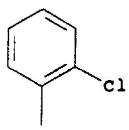 | 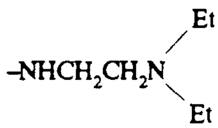 | H | 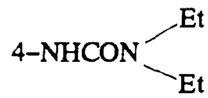 | 165 DCM/éther iso |
| 64 (a) | 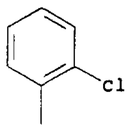 | $-NH-CH_2-CO_2Et$ | 2-OMe | 4-OMe | 178 DCM/éther iso |
| 65 (a) | 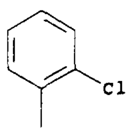 | 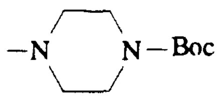 | 2-OMe | 4-OMe | 244 DCM/éther iso |
| 66 (i) | 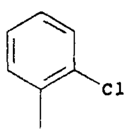 | -NHCOMe | 2-OMe | 4-OMe | 220-223 DCM/éther iso |
| 67 (i) | 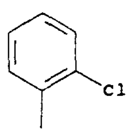 | -NHCOisoBu | 2-OMe | 4-OMe | 255 DCM/éther iso |
| 68 (i) | 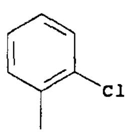 | 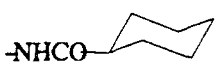 | 2-OMe | 4-OMe | 260 DCM/éther iso |
| 69 (j) | 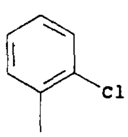 | -NHCOOEt | 2-OMe | 4-OMe | 199 DCM/éther iso |
| 70 (j) | 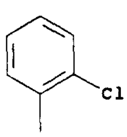 | -NHCOOisoBu | 2-OMe | 4-OMe | 229 DCM/éther iso |
| 71 (k) | 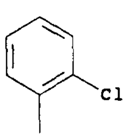 | 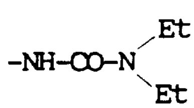 | H | 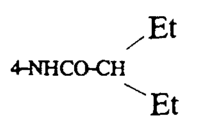 | 188 DCM/éther iso |

TABLEAU II (suite)

| Exemple | $R_3$ | $R_4$ | $R_5$ | $R_6$ | F°C ou RMN solvant de cristallisation |
|---|---|---|---|---|---|
| 72 (e) | 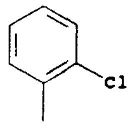 | 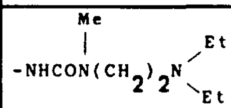 | 2-OMe | 4-OMe | 180 DCM/éther iso |
| 73 (e) | 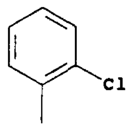 | 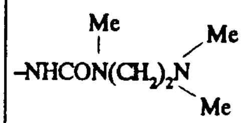 | 2-OMe | 4-OMe | RMN DCM/éther iso |
| 74 (d) et (m) | 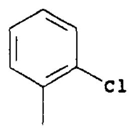 | -NHCOMe | 2-OMe | 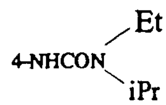 | 190-200 DCM/éther iso |
| 75 (d) et (m) | 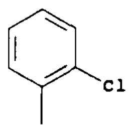 | -NHCOMe | 2-OMe | 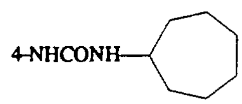 | 190-200 DCM/éther iso |
| 76 (d) et (m) | 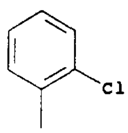 | -NHCOMe | 2-OMe | 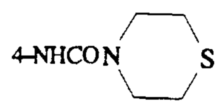 | 255-260 DCM/éther iso |
| 77 (a) | 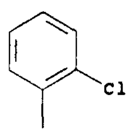 | $-N_3$ | 2-OMe | 4-OMe | 122-125 DCM/éther iso |
| 78 (b) | 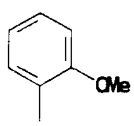 | $-NH_2$ | 2-OMe | $4-NO_2$ | 198-200 DCM/éther iso |
| 79 (c) | 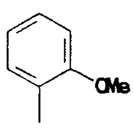 | $-NH_2$ | 2-OMe | $4-NH_2$ | 229-232 THF/éther iso |
| 80 (l) et (m) | 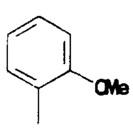 | $-NH_2$ | 2-OMe | 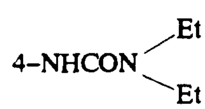 | RMN DCM/éther iso |

TABLEAU II (suitc)

| Exemple | $R_3$ | $R_4$ | $R_5$ | $R_6$ | F°C ou RMN solvant de cristallisation |
|---|---|---|---|---|---|
| 81<br>(l) et (m) | 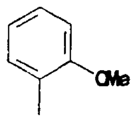 | 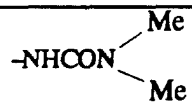 | 2-<br>OMe | 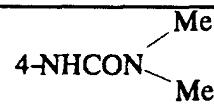 | 204-210<br>DCM/éther iso |
| 82<br>(f) | 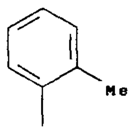 | $-NH_2$ | H | 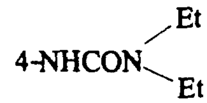 | 170-176<br>DCM/éther iso |

(a) composé préparé selon le mode opératoire décrit à l'EXEMPLE 1

(b) composé préparé selon le mode opératoire décrit à l'EXEMPLE 16

(c) composé préparé selon le mode opératoire décrit à l'EXEMPLE 17

(d) composé préparé selon le mode opératoire décrit à l'étape A de l'EXEMPLE 27

(e) composé préparé selon le mode opératoire décrit à l'EXEMPLE 10 en utilisant les amines appropriées ou les hétérocycles appropriés

(f) composé préparé selon le mode opératoire décrit à l'EXEMPLE 30

(g) composé préparé selon le mode opératoire décrit à l'EXEMPLE 11

(h) composé préparé selon le mode opératoire décrit à l'EXEMPLE 13

(i) composé préparé selon le mode opératoire décrit à l'EXEMPLE 4 en utilisant les chlorures d'acides appropriés

(j) composé préparé selon le mode opératoire décrit à l'EXEMPLE 5 en utilisant les chloroformiates appropriés

(k) composé préparé selon le mode opératoire décrit à l'EXEMPLE 14 en utilisant les chlorures d'acides appropriés

(l) composé préparé selon le mode opératoire décrit aux EXEMPLES 18 et 19.

(m) composé préparé selon le mode opératoire décrit à l'étape B de l'EXEMPLE 27 en utilisant les amines appropriées ou les hétérocycles appropriés

(n) composé préparé selon les modes opératoires décrits aux étapes A puis B de l'EXEMPLE 15.

Spectre de RMN à 200 MHz dans DMSO-$d_6$ du composé de l'EXEMPLE 54.

2,05 ppm : se : 3H
2,3 ppm : se : 3H
6,5 ppm : s : 2H
6,6 à 8,2 ppm : m : 11H

Spectre de RMN à 200 MHz dans DMSO-$d_6$ du composé de l'EXEMPLE 55.

1,8 à 2,4 ppm : mt : 6H
3,8 ppm : s : 3H
6,9 ppm : d : 1H
7,2 à 7,8 ppm : mt : 4H
7,9 ppm : d : 1H
8,1 ppm : mt : 3H
8,4 ppm : d : 1H

Spectre de RMN à 200 MHz dans DMSO-$d_6$ du composé de l'EXEMPLE 73.

1,9 ppm : s : 6H
2,2 ppm : mt : 3H
3,1 ppm : t : 2H
3,4 ppm : s : 3H
3,8 ppm : s : 3H
6,6 à 7,8 ppm : 10H
8,2 à 8,8 ppm : se : 1H

Spectre de RMN à 200 MHz dans DMSO-$d_6$ du composé de l'EXEMPLE 80.

1,1 ppm : t : 6H
2,7 ppm : s : 2H
3,2 à 3,5 ppm : qd + s : 7H
6,9 ppm : mt : 2H
7,1 ppm : t : 1H
7,3 à 7,5 ppm : mt : 3H
7,6 ppm : s : 1H
7,75 à 8,1 ppm : t de d : 3H
8,8 ppm : s : 1H

EXEMPLE 83

5-Chloro-3-(2-chlorophényl)-1,3-dihydro-1-(2,4-diméthoxybenzènesulfonyl) -3-(pipérazin-1-yl)indol-2-one.
On refroidit à 0°C une solution de 0,300 g du composé obtenu à l'EXEMPLE 65 dans 5 ml de DCM et ajoute 5 ml de TFA. On laisse 2 heures sous agitation à 0°C et évapore sous vide. On reprend le résidu par une solution à 5 % de carbonate de potassium, extrait à l'AcOEt, sèche sur sulfate de sodium et évapore sous vide. On chromatographie sur silice en éluant par le mélange DCM/MeOH (90/10 ; v/v). On obtient le produit attendu après cristallisation dans le mélange DCM/éther iso, m = 0,105 g, F = 210°C.

EXEMPLE 84

3-Amino-5,6-dichloro-1-[4-(N',N'-diéthyluréido)benzènesulfonyl]-1,3-dihydro-3-phénylindol-2-one.
On prépare ce composé selon le mode opératoire décrit à l'EXEMPLE 1 à partir de 0,293 g de 3-amino-5,6-dichloro-1,3-dihydro-3-phénylindol-2-one et 0,290 g de chlorure de 4-(N',N'-diéthyluréido)benzènesulfonyle. On chromatographie sur silice en éluant par le mélange DCM/AcOEt (85/15 ; v/v). On obtient le produit attendu après cristallisation dans le mélange DCM/éther iso, m = 0,26 g, F = 119-121°C.

EXEMPLE 85

3-Amino-4,5-dichloro-1-[4-(N',N'-diéthyluréido)benzènesulfonyl]-1,3-dihydro-3-phénylindol-2-one.
On prépare ce composé selon le mode opératoire décrit à l'EXEMPLE 1 à partir de 0,4 g de 3-amino-4,5-dichloro-1,3-dihydro-3-phénylindol-2-one et 0,4 g de chlorure de 4-(N',N'-diéthyluréido)benzènesulfonyle. On chromatographie sur silice en éluant par le mélange DCM/AcOEt (85/15 ; v/v). On obtient le produit attendu après cristallisation dans le mélange DCM/éther iso, m = 0,46 g, F = 161-163°C.

EXEMPLE 86

5-Ethoxy-3-(2-éthoxycarbonyl-1-éthoxycarbonylhydrazino)-1,3-dihydro-1-(2,4-diméthoxybenzènesulfonyl)-3-phénylindol-2-one.

On prépare ce composé selon le mode opératoire décrit à l'EXEMPLE 1 à partir de 0,500 g de 5-éthoxy-3-(2-éthoxycarbonyl-1-éthoxycarbonylhydrazino)-1,3-dihydro-3-phénylindol-2-one et 0,280 g de chlorure de 2,4-diméthoxybenzènesulfonyle. On obtient le produit attendu après cristallisation dans le mélange DCM/éther iso, m = 0,383 g, F = 228-229°C.

EXEMPLE 87

3-Amino-3-(2-chlorophényl)-5-éthoxy-1-[4-(N',N'-diéthyluréido)benzène-sulfonyl]-1,3-dihydroindol-2-one.

On prépare ce composé selon le mode opératoire décrit à l'EXEMPLE 30 à partir de 3-amino-3-(2-chlorophényl)-5-éthoxy-1,3-dihydroindol-2-one et de chlorure de 4-(N',N'-diéthyluréido)benzènesulfonyle.

Spectre de RMN à 200 MHz dans DMSO-$d_6$.

1,0 ppm : t : 6H
1,1 ppm : t : 3H
2,95 ppm : s : 2H
3,3 ppm : qd : 4H
6,2 ppm : d : 1H
6,9 ppm : d de d : 1H
7,1 ppm : d : 1H
7,25 ppm : t : 1H
7,4 ppm : t : 1H
7,6 à 7,9 ppm : mt : 5H
8,1 ppm : d : 1H
8,7 ppm : s : 1H

EXEMPLE 88

3-Amino-3-(2-chlorophényl)-5-éthoxy-1,3-dihydro-1-(2,4-diméthoxybenzènesulfonyl)indol-2-one.

On prépare ce composé selon le mode opératoire décrit à l'EXEMPLE 1 à partir de 3-amino-3-(2-chlorophényl)-5-éthoxy-1,3-dihydroindol-2-one et du chlorure de 2,4-diméthoxybenzènesulfonyle. On obtient le produit attendu après cristallisation dans le mélange DCM/hexane/éther iso, F = 196-198°C.

EXEMPLE 89

4-[3-Amino-5-chloro-3-(2-chlorophényl)-2,3-dihydro-2-oxoindol-1-yl]méthyl-3-méthoxybenzoate de méthyle.

On refroidit à 0°C, sous atmosphère d'argon, une solution de 1,13 g de 3-amino-5-chloro-3-(2-chlorophényl)-1,3-dihydroindol-2-one dans 7 ml de DMF et ajoute 0,120 g d'hydrure de sodium à 80 % dans l'huile. Après 30 minutes d'agitation on ajoute 1 g de 4-bromométhyl-3-méthoxybenzoate de méthyle et laisse 1 heure sous agitation à 0°C. On verse le mélange réactionnel dans l'eau, filtre le précipité formé, le lave à l'eau, le reprend dans l'AcOEt, sèche sur sulfate de sodium et évapore sous vide. On chromatographie sur silice en éluant par le mélange DCM/AcOEt (93/7 ; v/v). On obtient le produit attendu après cristallisation dans le mélange DCM/éther iso, m = 1,88 g, F = 152°C.

EXEMPLE 90

4-[5-Chloro-3-(2-chlorophényl)-2,3-dihydro-3-(méthoxycarboxamido)-2-oxoindol-1-yl]méthyl-3-méthoxybenzoate de méthyle.

On refroidit à 0°C un mélange de 1,67 g du composé obtenu à l'EXEMPLE 89 dans 10 ml de pyridine et ajoute une solution de 0,669 g de chloroformiate de méthyle dans 2 ml de DCM. On laisse 1 heure sous agitation entre 0 et +4°C et verse le mélange réactionnel dans l'eau. On extrait au DCM, lave par une solution à 5 % d'hydrogénosulfate de potassium, sèche sur sulfate de sodium et évapore sous vide. On chromatographie sur silice en éluant par le mélange DCM/AcOEt (95/5 ; v/v). On obtient le produit attendu après cristallisation dans le mélange DCM/éther iso, m = 1,6 g, F = 174°C.

EXEMPLE 91

1-[4-(N-*tert*-Butylcarbamoyl)-2-méthoxybenzyl]-5-chloro-3-(2-chlorophényl)-1,3-dihydro-3-(méthoxycarboxamido)indol-2-one.

A) Acide 4-[5-chloro-3-(2-chlorophényl)-2,3-dihydro-3-(méthoxy-carboxamido)-2-oxoindol-1-yl]méthyl-3-méthoxybenzoïque.

A un mélange de 0,8 g du composé obtenu à l'EXEMPLE 90 dans 9 ml de THF, 9 ml de MeOH et 3 ml d'eau, on ajoute 0,317 g d'hydroxyde de lithium monohydrate et laisse agiter 4 heures à TA. Puis on ajoute de l'eau, acidifie à pH = 1 par ajout d'HCl 1N, extrait au DCM, sèche sur sulfate de sodium et évapore sous vide. On obtient le produit attendu qui est utilisé tel quel à l'étape suivante.

B)1-[4-(N-*tert*-Butylcarbamoyl)-2-méthoxybenzyl]-5-chloro-3-(2-chlorophényl)-1,3-dihydro-3-(méthoxycarboxamido)indol-2-one.

On refroidit à 0°C une solution de 0,450 g du composé obtenu à l'étape précédente dans 10 ml de DCM et ajoute 0,113 g de DIPEA, 0,403 g de BOP et laisse agiter 15 minutes. Puis on ajoute 0,095 g de *tert*-butylamine et laisse 3 heures sous agitation à 0°C. On ajoute de l'eau, extrait au DCM, lave par une solution à 5 % d'hydrogènosulfate de potassium, sèche sur sulfate de sodium et évapore sous vide. On chromatographie sur silice en éluant par le mélange DCM/AcOEt (90/10 ; v/v). On obtient le produit attendu après cristallisation dans le mélange DCM/éther iso, m = 0,260 g, F = 215°C.

EXEMPLE 92

3-Amino-1-[4-(N-*tert*-butylcarbamoyl)-2-méthoxybenzyl]-5-chloro-3-(2-chlorophényl)-1,3-dihydroindol-2-one.

On agite pendant 2 heures à TA un mélange de 0,70 g du composé obtenu à l'EXEMPLE 91 et 35 ml d'une solution à 33 % d'acide bromhydrique dans AcOH. On évapore sous vide à TA, reprend le résidu à l'eau, alcalinise à pH = 10 par ajout de carbonate de sodium, extrait à l'AcOEt, sèche sur sulfate de sodium et évapore sous vide. On chromatographie sur silice en éluant au DCM puis par un mélange DCM/AcOEt (80/20 ; v/v). On obtient le produit attendu après cristallisation dans le mélange DCM/éther iso, m = 0,370 g, F = 234°C.

EXEMPLE 93

4-[5-Chloro-3-(2-chlorophényl)-2,3-dihydro-3-diméthylamino-2-oxoindol-1-yl]-méthylbenzoate de *tert*-butyle.

On prépare ce composé selon le mode opératoire décrit à l'EXEMPLE 89 à partir de 1,5 g de 5-chloro-3-(2-chlorophényl)-1,3-dihydro-3-diméthylaminoindol-2-one et 1,40 g de 4-bromométhylbenzoate de *tert*-butyle. On chromatographie sur silice en éluant par le gradient du mélange AcOEt/hexane de (10/90 ; v/v) jusqu'à (20/80 ; v/v). On obtient le produit attendu, m = 1,5 g.

Spectre de RMN à 200 MHz dans DMSO-$d_6$.

1,5 ppm : s : 9H
2,0 à 2,4 ppm : m : 6H
4,8 à 5,2 ppm : d de d : 2H
6,8 ppm : d : 1H
7,0 ppm : d : 1H
7,2 à 7,6 ppm : mt : 6H
7,8 ppm : d : 2H
8,1 ppm : d : 1H

EXEMPLE 94

1-[4-(N-*tert*-Butylcarbamoyl)benzyl]-5-chloro-3-(2-chlorophényl)-1,3-dihydro-3-diméthylaminoindol-2-one.

A) Acide 4-[5-chloro-3-(2-chlorophényl)-2,3-dihydro-3-diméthylamino-2-oxoindol-1-yl]méthylbenzoïque.

On refroidit à 0°C une solution de 0,615 g du composé obtenu à l'EXEMPLE 93 dans 2 ml de DCM et ajoute 2 ml de TFA. On laisse 30 minutes sous agitation, évapore sous vide puis reprend le résidu au DCM et évapore sous vide à nouveau. On obtient le produit attendu qui est utilisé tel quel à l'étape suivante.

B) 1-[4-(N-*tert*-Butylcarbamoyl)benzyl]-5-chloro-3-(2-chlorophényl)-1,3-dihydro-3-diméthylaminoindol-2-one.

On refroidit à 0°C une solution de 0,546 g du composé obtenu à l'étape précédente dans 5 ml de DCM,

neutralise à pH = 7 par ajout de DIPEA puis introduit 0, 530 g de BOP. On réajuste le pH à 7 par ajout de DIPEA, additionne 0,2 ml de *tert*-butylamine et laisse 16 heures sous agitation en laissant remonter la température à TA. On évapore sous vide, extrait à l'AcOEt, lave par une solution à 5 % de carbonate de sodium, sèche sur sulfate de sodium et évapore sous vide. On chromatographie sur silice en éluant par le gradient du mélange hexane/AcOEt de (80/20 ; v/v) jusqu'à (70/30 ; v/v). On obtient le produit attendu après cristallisation dans le mélange DCM/éther iso, m = 0,280 g, F = 158°C.

EXEMPLE 95

5-Chloro-3-(2-chlorophényl)-1-[4-(N-1-éthylpropylcarbamoyl)benzyl]-1,3-dihydro-3-diméthylaminoindol-2-one.

On prépare ce composé selon le mode opératoire décrit à l'étape B de l'EXEMPLE 94 à partir de 0,623 g du composé obtenu à l'étape A de l'EXEMPLE 94 et 0,24 ml de 1-éthylpropylamine, en présence de 0,605 g de BOP et de DIPEA pour maintenir le pH = 7. On chromatographie sur silice en éluant par le gradient du mélange hexane/AcOEt de (90/10 ; v/v) jusqu'à (70/30 ; v/v). On obtient le produit attendu après cristallisation dans le mélange DCM/hexane, m = 0,490 g, F = 208°C.

EXEMPLE 96

3-Amino-1-[4-(N-tert-butylcarbamoyl)benzyl]-5-chloro-3-(2-chlorophényl)-1,3-dihydroindol-2-one.

A) 4-[3-Amino-5-chloro-3-(2-chlorophényl)-2,3-dihydro-2-oxoindol-1-yl]méthyl-benzoate de *tert*-butyle.

On prépare ce composé selon le mode opératoire décrit à l'EXEMPLE 89 à partir de 1,0 g de 3-amino-5-chloro-3-(2-chlorophényl)-1,3-dihydroindol-2-one et 1,01 g de 4-bromométhylbenzoate de *tert*-butyle. On chromatographie sur silice en éluant par du DCM puis par le mélange DCM/AcOEt (95/5 ; v/v). On obtient 0,98 g du produit attendu qui est utilisé tel quel à l'étape suivante.

B) Acide 4-[3-amino-5-chloro-3-(2-chlorophényl)-2,3-dihydro-2-oxoindol-1-yl]méthyl-benzoïque.

On refroidit à 0°C une solution de 0,98 g du composé obtenu à l'étape précédente, dans 2 ml de DCM et ajoute 4,5 ml de TFA. On laisse 30 minutes sous agitation, évapore sous vide, puis reprend le résidu au DCM et évapore sous vide à nouveau. On obtient le produit attendu qui est utilisé tel quel à l'étape suivante.

C) 3-Amino-1-[4-(N-*tert*-butylcarbamoyl)benzyl]-5-chloro-3-(2-chlorophényl)-1,3-dihydroindol-2-one.

On refroidit à 0°C une solution de 0,867 g du composé obtenu à l'étape précédente dans 8 ml de DCM, neutralise à pH = 7 par ajout de DIPEA puis introduit 1,12 g de BOP. On réajuste le pH à 7 par ajout de DIPEA, additionne 0,64 ml de *tert*-butylamine et laisse 16 heures sous agitation en laissant remonter la température à TA. On évapore sous vide, extrait à l'AcOEt, lave par une solution à 5 % de carbonate de sodium, sèche sur sulfate de sodium et évapore sous vide. On chromatographie sur silice en éluant par le gradient du mélange hexane/AcOEt de (80/20 ; v/v) jusqu'à (70/30 ; v/v). On obtient le produit attendu après cristallisation dans le mélange DCM/MeOH/éther iso, m = 0,610 g, F = 173-178°C.

EXEMPLE 97

3-Acétamido-3-(2-chlorophényl)-5-éthoxy-1-[4-(N',N'-diéthyluréido)benzène sulfonyl]-1,3-dihydroindol-2-one

On prépare ce composé selon le mode opératoire décrit à l'EXEMPLE 2 à partir du composé obtenu à l'EXEMPLE 87 et du chlorure d'acétyle. Après cristallisation dans le mélange DCM/éther iso, on obtient le produit attendu qui cristallise avec 0,25 mole d'éther iso.

Spectre de RMN à 200 MHz dans DMSO-$d_6$

1,0 ppm : t et d : 6H et 12H (éther iso)
1,2 ppm : t : 3H
1,8 ppm : s : 3H
3,3 ppm : qd : 4H
3,55 ppm : sep : 2H (éther iso)
3,9 ppm : qd : 2H
6,7 ppm : d : 1H
6,9 ppm : d de d : 1H
7,2 à 7,4 ppm : mt : 4H
7,6 à 7,9 ppm : mt : 5H

8,7 ppm : s : 1H
9,1 ppm : s : 1H

EXEMPLE 98

3-Amino-3-(2-chlorophényl)-5-éthoxy-1,3-dihydro-1-(2-méthoxy-4-nitro benzènesulfonyl)indol-2-one.
On prépare ce composé selon le mode opératoire décrit à l'exemple 16 à partir de 3-amino-3-(2-chlorophényl)-5-éthoxy-1,3-dihydroindol-2-one et du chlorure de 2-méthoxy-4-nitrobenzènesulfonyle. Après cristallisation dans le mélange DCM/éther iso, on obtient le produit attendu qui cristallise avec 0,25 mole d'éther iso, F = 129-132°C.

EXEMPLE 99

3-Amino-5-chloro-3-(2-chlorophényl)-1-[4-(N',N'-diéthyluréido)benzène-sulfonyl]-1,3-dihydroindol-2-one

On prépare ce composé selon le mode opératoire décrit à l'exemple 30 à partir de 3-amino-5-chloro-3-(2-chlorophényl)-1,3-dihydroindol-2-one et de chlorure de 4-(N',N'-diéthyluréido)benzènesulfonyle, F = 195-196°C.

EXEMPLE 100

3-(2-chlorophényl)-5-éthoxy-1-[4-(N',N'-diéthyluréido)-2-méthoxybenzène-sulfonyl]-1,3-dihydro-3-(méthoxycarboxamido)indol-2-one
On prépare ce composé selon les modes opératoires décrits aux EXEMPLES 22, puis 23, puis 24 à partir du composé obtenu à l'EXEMPLE 98. On obtient le produit attendu après cristallisation dans le chloroforme, F = 263-266°C.

EXEMPLE 101

3-Amino-3-(2-chlorophényl)-5-éthoxy-1-[4-(N',N'-diéthyluréido)-2-méthoxybenzènesulfonyl]-1,3-dihydroindol-2-one, hémihydrate.
On prépare ce composé selon le mode opératoire décrit à l'EXEMPLE 92 à partir du composé obtenu à l'EXEMPLE 100, F = 218-220°C.

EXEMPLE 102

5-Chloro-3-(cyclohexylméthyl)-1,3-dihydro-1-(2-méthoxy-4-nitrobenzènesulfonyl)-3-(méthylamino)indol-2-one.
On prépare ce composé selon le mode opératoire décrit à l'EXEMPLE 16 à partir de 1,49 g de 5-chloro-3-(cyclohexylméthyl)-1,3-dihydro-3-(méthylamino)indol-2-one et 1,8 g de chlorure de 2-méthoxy-4-nitrobenzènesulfonyle. On chromatographie sur silice en éluant au DCM puis par le mélange DCM/AcOEt (95/5 ; v/v). On obtient 1,5 g du produit attendu après cristallisation dans l'AcOEt, F = 207°C.

EXEMPLE 103

5-Chloro-3-(cyclohexylméthyl)-1-[4-(N',N'-diéthyluréido)-2-méthoxybenzène-sulfonyl]-1,3-dihydro-3-(méthylamino)indol-2-one.

A) 5-Chloro-3-(cyclohexylméthyl)-1,3-dihydro-1-(2-méthoxy-4-nitrobenzènesulfonyl)-3-(N-méthyl-méthoxycarboxamido)indol-2-one.

On refroidit à +4°C une solution de 1,62 g du composé obtenu à l'EXEMPLE 102 dans 10 ml de pyridine, ajoute, goutte à goutte, une solution de 0,27 ml de chloroformiate de méthyle dans 5 ml de DCM et laisse 3 heures sous agitation à +4°C. On ajoute 50 ml d'eau au mélange réactionnel, extrait à l'AcOEt, lave la phase organique par une solution saturée de chlorure de sodium, à l'eau, sèche sur sulfate de sodium et évapore sous vide le solvant. On chromatographie le résidu sur silice en éluant au DCM. On obtient 1,14 g du produit attendu après cristallisation dans le mélange DCM/éther iso.

Spectre de RMN à 200 MHz dans DMSO-$d_6$

0,5 à 1,7 ppm : m : 11H
1,9 ppm : mt :2H

3,1 ppm : s : 3H
3,1 ppm : s : 3H
3,3 ppm : s : 3H
3,7 ppm : s : 3H
7,4 à 8,4 ppm : m : 6H

B) 1-(4-Amino-2-méthoxybenzènesulfonyl)-5-chloro-3-(cyclohexylméthyl)-1,3-dihydro-3-(N-méthyl-méthoxycarboxamido)indol-2-one.

On hydrogène pendant 20 minutes, à TA et à pression atmosphérique, un mélange de 1,14 g du composé obtenu à l'étape précédente, 0,5 g de nickel de Raney® et 50 ml de MeOH. On filtre le catalyseur sur Célite® et évapore sous vide le filtrat. On obtient 1,19 g du produit attendu après cristallisation dans le MeOH.

Spectre de RMN à 200 MHz dans DMSO-$d_6$

0,4 à 1,6 ppm : m : 11H
1,8 ppm : mt : 2H
3,15 ppm : s : 3H
3,35 ppm : s : 3H
3,55 ppm : s : 3H
6,1 à 7,9 ppm : m : 8H

C) 5-Chloro-3-(cyclohexylméthyl)-1,3-dihydro-3-(N-méthyl-méthoxycarboxamido)-1-[2-méthoxy-4-(phénoxycarboxamido)benzènesulfonyl]indol-2-one.

On refroidit à +4°C une solution de 1,19 g du composé obtenu à l'étape précédente dans 10 ml de pyridine, ajoute, goutte à goutte, une solution de 0,3 ml de chloroformiate de phényle dans 10 ml de DCM et laisse 3 heures sous agitation à TA. On ajoute 50 ml d'eau au mélange réactionnel, extrait à l'AcOEt, lave la phase organique par une solution saturée de NaCl, à l'eau, sèche sur sulfate de sodium et évapore sous vide le solvant. On chromatographie le résidu sur silice en éluant au DCM puis par le mélange DCM/AcOEt (95/5 ; v/v). On obtient 0,755 g du produit attendu après cristallisation dans le mélange DCM/éther iso.

D) 5-Chloro-3-(cyclohexylméthyl)-1-[4-(N',N'-diéthyluréido)-2-méthoxy-benzène sulfonyl]-1,3-dihydro-3-(N-méthyl-méthoxycarboxamido)indol-2-one.

A une solution de 0,268 g du composé obtenu à l'étape précédente dans 15 ml de chloroforme, on ajoute 0,09 ml de diéthylamine et chauffe à 60°C pendant 3 heures. On concentre sous vide le mélange réactionnel et chromatographie le résidu sur silice en éluant au DCM puis par le mélange DCM/AcOEt (85/15 ; v/v). On obtient 0,18 g du produit attendu après cristallisation dans le mélange DCM/éther iso.

Spectre de RMN à 200 MHz dans DMSO-$d_6$

0,4 à 2,1 ppm : m : 19H
3,15 ppm : s : 3H
3,35 ppm : m : 7H
3,6 ppm : s : 3H
7,2 à 7,9 ppm : m : 6H
8,65 ppm : s : 1H

E) 5-Chloro-3-(cyclohexylméthyl)-1-[4-(N',N'-diéthyluréido)-2-méthoxybenzène-sulfonyl]-1,3-dihydro-3-(méthylamino)indol-2-one.

On prépare ce composé selon le mode opératoire décrit à l'EXEMPLE 92 à partir du composé obtenu à l'étape précédente. On obtient le produit attendu après cristallisation dans le mélange DCM/éther iso, F = 210°C.

EXEMPLE 104

5-Chloro-3-(cyclohexylméthyl)-1-[4-(N',N'-diéthyluréido)-2-méthoxybenzène-sulfonyl]-1,3-dihydro-3-(N-méthyl-phénoxycarboxamido)indol-2-one.

A) 1-(4-Amino-2-méthoxybenzènesulfonyl)-5-chloro-3-(cyclohexylméthyl)-1,3-dihydro-3-(méthylamino)indol-2-one.

On hydrogène à TA et à pression atmosphérique un mélange de 1,2 g du composé obtenu à l'EXEMPLE 102, 3 g de nickel de Raney® dans 30 ml de MeOH et 40 ml de THF. Après 40 minutes, on filtre le catalyseur sur Célite® et évapore sous vide le filtrat. On chromatographie le résidu sur silice en éluant au DCM puis par le mélange DCM/AcOEt (75/25 ; v/v). On obtient 0,6 g du produit attendu que l'on utilise tel quel à l'étape suivante.

B) 5-Chloro-3-(cyclohexylméthyl)-1,3-dihydro-1-[2-méthoxy-4-(phénoxy carboxamido)benzènesulfonyl]-3-(N-méthyl-phénoxycarboxamido)indol-2-one.

On refroidit à +4°C une solution de 0,6 g du composé obtenu à l'étape précédente dans 2 ml de pyridine, ajoute, goutte à goutte, une solution de 0,17 ml de chloroformiate de phényle dans 3 ml de DCM et laisse 4 heures sous agitation en laissant remonter la température à TA. On ajoute 40 ml d'eau au mélange réactionnel, extrait à l'AcOEt, lave la phase organique par une solution saturée de chlorure de sodium, à l'eau, sèche sur sulfate de sodium et évapore sous vide le solvant. On obtient 0,9 g du produit attendu que l'on utilise tel quel à l'étape suivante.

C) 5-Chloro-3-(cyclohexylméthyl)-1-[4-(N',N'-diéthyluréido)-2-méthoxybenzène-sulfonyl]-1,3-dihydro-3-(N-méthyl-phénoxycarboxamido)indol-2-one.

A une solution de 0,9 g du composé obtenu à l'étape précédente dans 5 ml de chloroforme, on ajoute 0,31 ml de diéthylamine et chauffe à 60°C pendant 4 heures. On ajoute 50 ml d'eau au mélange réactionnel, extrait à l'AcOEt, lave la phase organique par une solution saturée de chlorure de sodium, à l'eau, sèche sur sulfate de sodium et évapore sous vide le solvant. On chromatographie le résidu sur silice en éluant au DCM puis par le mélange DCM/AcOEt (85/15 ; v/v). On obtient 0,45 g du produit attendu après cristallisation dans le mélange DCM/éther iso, F = 130°C.

EXEMPLE 105

3-Amino-5-chloro-3-(2-chlorophényl)-1,3-dihydro-1-(4-méthoxy-2-nitrobenzène-sulfonyl)indol-2-one.

On prépare ce composé selon le mode opératoire décrit à l'EXEMPLE 1 à partir de 2 g de 3-amino-5-chloro-3-(2-chlorophényl)-1,3-dihydroindol-2-one et 1,71 g de chlorure de 4-méthoxy-2-nitrobenzènesulfonyle. On chromatographie sur silice en éluant par le mélange DCM/hexane (80/20 ; v/v) puis au DCM. On obtient 2,8 g du produit attendu après cristallisation dans le mélange DCM/éther iso, F = 192-195°C.

EXEMPLE 106

3-Amino-1-(2-amino-4-méthoxybenzènesulfonyl)-5-chloro-3-(2-chlorophényl)-1,3-dihydroindol-2-one.

On hydrogène pendant 1 heure à TA et à pression atmosphérique un mélange de 2,4 g du composé obtenu à l'EXEMPLE 105, 1 g de nickel de Raney® dans 50 ml de MeOH et 50 ml de THF. On filtre le catalyseur sur Célite® et évapore sous vide le filtrat. On chromatographie le résidu sur silice en éluant au DCM puis par le mélange DCM/AcOEt (95/5 ; v/v). On obtient 0,316 g du produit attendu après cristallisation dans le mélange DCM/éther iso, F = 160-166°C.

EXEMPLE 107

3-Amino-5-chloro-3-(2-chlorophényl)-1-[2-(éthylamino)-4-méthoxybenzènesulfonyl]-1,3-dihydroindol-2-one.

A une solution de 0,830 g du composé obtenu à l'EXEMPLE 106 dans 22 ml de MeOH, on ajoute à TA 2,6 ml d'AcOH, puis 0,09 ml d'acétaldéhyde et 0,038 g de cyanoborohydrure de sodium. Après 4 heures d'agitation à TA, on ajoute 2 gouttes d'HCl concentré puis neutralise le mélange réactionnel par ajout d'une solution à 5 % de carbonate de potassium. On concentre sous vide le solvant, extrait le résidu à l'AcOEt, lave la phase organique à l'eau, par une solution saturée de chlorure de sodium, sèche sur sulfate de sodium et évapore sous vide le solvant. On chromatographie le résidu sur silice en éluant par le mélange DCM/hexane (80/20 ; v/v) puis au DCM. On obtient 0,396 g du produit attendu après cristallisation dans le mélange DCM/éther iso, F = 192-196°C.

EXEMPLE 108

3-Amino-5-chloro-3-(2-chlorophényl)-1-[4-(N',N'-diéthyluréido)benzène-sulfonyl]-1,3-dihydroindol-2-one, isomère (+).

On prépare ce composé selon le mode opératoire décrit à l'EXEMPLE 30 à partir de 0,293 g de 3-amino-5-chloro-3-(2-chlorophényl)-1,3-dihydroindol-2-one isomère (+) et 0,290 g de chlorure de 4-(N',N'-diéthyluréido)benzènesulfonyle. On chromatographie sur silice en éluant par le mélange DCM/AcOEt (90/10 ;v/v). On obtient 0,24 g du produit attendu après cristallisation dans le mélange DCM/éther iso,

$$\alpha_D^{25} = +127° \text{ (c = 0,24 ; chloroforme).}$$

EXEMPLE 109

3-Amino-5-chloro-3-(2-chlorophényl)-1-[4-(N',N'-diéthyluréido)benzènesulfonyl]-1,3-dihydroindol-2-one, isomère (-).

On prépare ce composé selon le mode opératoire décrit à l'EXEMPLE 30 à partir de 0,293 g de 3-amino-5-chloro-3-(2-chlorophényl)-1,3-dihydroindol-2-one isomère (-) et 0,290 g de chlorure de 4-(N',N'-diéthyluréido)benzènesulfonyle. On chromatographie deux fois sur silice en éluant par le mélange DCM/AcOEt (90/10 ; v/v). On obtient 0,065 g du produit attendu après cristallisation puis recristallisation dans le mélange DCM/éther iso et séchage à 90°C, sous vide

$$\alpha_D^{25} = -133° \text{ (c = 0,24 ; chloroforme).}$$

EXEMPLE 110

5-Chloro-1-[4-(2-chlorobenzamido)benzènesulfonyl]-3-(2-chlorophényl)-1,3-dihydro-3-(méthylamino)indol-2-one.

A) 5-Chloro-3-(2-chlorophényl)-1,3-dihydro-3-(méthylamino)-1-(4-nitrobenzènesulfonyl)-indol-2-one.

On prépare ce composé selon le mode opératoire décrit à l'EXEMPLE 11 à partir de 7,03 g de 5-chloro-3-(2-chlorophényl)-1,3-dihydro-3-(méthylamino)indol-2-one et 5,73 g de chlorure de 4-nitrobenzènesulfonyle. On chromatographie sur silice en éluant au DCM. On obtient 7,76 g du produit attendu après cristallisation dans l'éther iso, F = 220-221°C.

B) 1-(4-Aminobenzènesulfonyl)-5-chloro-3-(2-chlorophényl)-1,3-dihydro-3-(méthylamino)-indol-2-one.

On hydrogène à TA et à pression atmosphérique un mélange de 7,7 g du composé obtenu à l'étape précédente, 3 g de nickel de Raney® , dans 140 ml d'EtOH et 150 ml de THF. On filtre le catalyseur sur Célite® et concentre sous vide le filtrat. On obtient 6,3 g du produit attendu après cristallisation dans l'éther iso, F = 240°C.

C) 5-Chloro-1-[4-(2-chlorobenzamido)benzènesulfonyl]-3-(2-chlorophényl)-1,3-dihydro-3-(méthylamino)indol-2-one.

On refroidit à +5°C une solution de 0,7 g du composé obtenu à l'étape précédente dans 100 ml de DCM, ajoute 2,5 ml de triéthylamine puis, goutte à goutte, une solution de 0,35 ml de chlorure de 2-chlorobenzoyle dans 4 ml de DCM et laisse 12 heures sous agitation en laissant remonter la température à TA. On concentre sous vide le mélange réactionnel, extrait le résidu à l'AcOEt, lave la phase organique à l'eau, sèche sur sulfate de sodium et évapore sous vide le solvant. On chromatographie le résidu sur silice en éluant au DCM. On obtient 0,31 g du produit attendu après cristallisation dans l'éther iso, F = 221-222°C.

EXEMPLE 111

5-Chloro-3-(2-chlorophényl)-1,3-dihydro-3-(méthylamino)-1-[4-(nicotinoylamino)benzènesulfonyl]indol-2-one.

On refroidit à +5°C une solution de 0,5 g du composé obtenu à l'étape B de l'EXEMPLE 110 dans 5 ml de DCM, ajoute 1,5 ml de triéthylamine puis 0,1 g de 4-diméthylaminopyridine et, goutte à goutte, une solution de 0,64 g de chlorhydrate du chlorure de nicotinoyle dans 15 ml de DCM. On laisse 19 heures sous agitation en laissant remonter la température à TA et concentre sous vide le mélange réactionnel. On reprend le résidu à l'eau, extrait à l'AcOEt, lave la phase organique à l'eau, sèche sur sulfate de sodium et évapore sous vide le solvant. On obtient 0,41 g du produit attendu qui contient 0,25 mole d'éther iso après cristallisation dans l'éther iso, F = 228°C.

EXEMPLE 112

5-Chloro-3-(2-chlorophényl)-1-[4-[N-(1-éthoxycarbonyl-1-méthyléthyl) carbamoyl]-2-méthoxybenzènesulfonyl]-1,3-dihydro-3-(méthylamino)indol-2-one.

A) 4-[[5-Chloro-3-(2-chlorophényl)-2,3-dihydro-3-(méthylamino)-2-oxoindol-1-yl]sulfonyl]-3-méthoxybenzoate de benzyle.

On prépare ce composé selon le mode opératoire décrit à l'étape A de l'EXEMPLE 28 à partir de 6 g de 5-chloro-3-(2-chlorophényl)-1,3-dihydro-3-(méthylamino)indol-2-one et 6,66 g de 4-chlorosulfonyl-3-méthoxybenzoate de benzyle. On obtient 10,3 g du produit attendu après cristallisation dans l'éther iso.

Spectre de RMN à 200 MHz dans DMSO-$d_6$.

1,95 ppm : d : 3H
3,55 ppm : qd : 1H
3,7 ppm : s : 3H
5,3 ppm : s : 2H
6,6 à 8,2 ppm : m : 15H

B) Acide 4-[[5-chloro-3-(2-chlorophényl)-2,3-dihydro-3-méthylamino-2-oxoindol-1-yl]sulfonyl]-3-méthoxybenzoïque.

On hydrogénolyse à TA et à pression atmosphérique un mélange de 10,3 g du composé obtenu à l'étape précédente, 1 g de palladium sur charbon à 10 % et 300 ml d'AcOEt. On filtre le catalyseur sur Célite® et concentre sous vide le filtrat. On obtient 8,05 g du produit attendu après cristallisation dans l'éther iso, F = 248°C.

C) 5-Chloro-3-(2-chlorophényl)-1-[4-[N-(1-éthoxycarbonyl-1-méthyléthyl) carbamoyl]-2-méthoxybenzènesulfonyl]-1,3-dihydro-3-(méthylamino)indol-2-one.

A une solution de 2 g du composé obtenu à l'étape précédente dans 25 ml de DCM, on ajoute successivement à TA 1,02 g de DIPEA, 3,5 g de BOP et 1,91 g de chlorhydrate de 2-amino-2-méthylpropionate d'éthyle. Après 2 heures 30 minutes d'agitation à TA, on concentre sous vide le mélange réactionnel, reprend le résidu à l'eau, extrait à l'AcOEt, sèche la phase organique sur sulfate de sodium et évapore sous vide le solvant. On chromatographie le résidu sur silice en éluant au DCM puis par le mélange DCM/AcOEt (95/5 ; v/v). On obtient 0,708 g du produit attendu après cristallisation dans l'éther iso, F = 141°C.

EXEMPLE 113

5-Chloro-3-(2-chlorophényl)-1-[4-[(2-furoyl)amino]-2-méthoxybenzènesulfonyl]-1,3-dihydro-3-(diméthylamino)indol-2-one.

On refroidit à 0°C une solution de 0,45 g du composé obtenu à l'EXEMPLE 56 dans 5 ml de DCM, ajoute 0,6 ml de triéthylamine puis 0,1 ml de chlorure de 2-furoyle et 0,02 g de 4-diméthylaminopyridine. On laisse 1 heure sous agitation à TA, ajoute 0,1 ml de chlorure de 2-furoyle et laisse 12 heures sous agitation à TA. On concentre sous vide le mélange réactionnel, reprend le résidu à l'eau, extrait à l'AcOEt, lave la phase organique par une solution à 5 % de carbonate de sodium, à l'eau, par une solution saturée de chlorure de sodium, sèche sur sulfate de sodium et évapore sous vide le solvant. On chromatographie le résidu sur silice en éluant au DCM puis par le mélange DCM/AcOEt (95/5 ; v/v). On obtient 0,511 g du produit attendu après cristallisation dans le mélange DCM/éther iso, F = 255°C.

EXEMPLE 114

5-Chloro-3-(2-chlorophényl)-1-[4-(N',N'-diéthyluréido)benzènesulfonyl]-1,3-dihydro-3-[(2-hydroxyéthyl)amino]indol-2-one.

On refroidit à 0°C, sous atmosphère d'argon, une solution de 1,084 g de 5-chloro-3-(2-chlorophényl)-1,3-dihydro-3-[[2-(triméthylsilyloxy)éthyl]amino]indol-2-one dans 20 ml de DMF et ajoute 0,111 g d'hydrure de sodium à 60 % dans l'huile. Après 25 minutes d'agitation on ajoute 0,78 g de chlorure de 4-(N',N'-diéthyluréido)benzènesulfonyle et laisse 3 heures sous agitation à TA. On verse le mélange réactionnel dans l'eau, extrait à l'AcOEt, lave la phase organique à l'eau, par une solution saturée de NaCl, sèche sur sulfate de sodium et évapore sous vide le solvant. On chromatographie le résidu sur silice en éluant par le mélange DCM/AcOEt (70/30 ; v/v). On obtient 0,505 g du produit attendu après cristallisation puis recristallisation dans le mélange DCM/éther iso, F = 192-193°C.

EXEMPLE 115

5-Chloro-3-(2-chlorophényl)-1-[4-(N',N'-diéthyluréido)benzènesulfonyl]-1,3-dihydro-3-[[2-(2-hydroxyéthoxy)éthyl]amino]indol-2-one.

On prépare ce composé selon le mode opératoire décrit à l'EXEMPLE 114 à partir de 1,5 g de 5-chloro-3-(2-chlorophényl)-1,3-dihydro-3-[[2-[2-(triméthylsilyloxy) éthoxy]éthyl]amino]indol-2-one et 1,03 g de chlorure de 4-(N',N'-diéthyluréido)benzènesulfonyle. On chromatographie sur silice en éluant par le mélange DCM/AcOEt (50/50 ; v/v). On obtient 1 g du produit attendu après cristallisation dans le mélange DCM/éther iso, F = 114-118°C.

EXEMPLE 116

5-Chloro-3-(2-chlorophényl)-1,3-dihydro-1-[4-(1,1-diméthylpropyl)benzènesulfonyl]-3-[[2-(morpholin-4-yl)éthyl]amino]indol-2-one.

On refroidit à 0°C, sous atmosphère d'argon, une solution de 1 g de 5-chloro-3-(2-chlorophényl)-1,3-dihydro-3-[[2-(morpholin-4-yl)éthyl]amino]indol-2-one dans 10 ml de DMF et ajoute 0,108 g d'hydrure de sodium à 60 % dans l'huile. Après 30 minutes d'agitation on ajoute 0,607 g de chlorure de 4-(1,1-diméthylpropyl)benzènesulfonyle et laisse 2 heures sous agitation en laissant remonter la température à TA. On verse le mélange réactionnel dans l'eau, extrait à l'AcOEt, lave la phase organique à l'eau, sèche sur sulfate de sodium et évapore sous vide le solvant. On chromatographie le résidu sur silice en éluant par le mélange DCM/AcOEt (80/20 ; v/v). On obtient 0,45 g du produit attendu après cristallisation dans le mélange DCM/éther iso, F = 147°C.

EXEMPLE 117

3-[[(1S)-5-(Benzyloxycarbonylamino)-1-(méthoxycarbonyl)pentyl]amino]-5-chloro-3-(2-chlorophényl)-1-[4-(N',N'-diéthyluréido)benzènesulfonyl]-1,3-dihydroindol-2-one.

On prépare ce composé selon le mode opératoire décrit à l'EXEMPLE 30 à partir de 1,44 g du composé obtenu à la Préparation 54 (isomère A) et 0,738 g de chlorure de 4-(N',N'-diéthyluréido)benzènesulfonyle. On chromatographie sur silice en éluant par le mélange DCM/AcOEt (85/15 ; v/v). On obtient 1,10 g du produit attendu sous forme d'huile épaisse.

$$\alpha_D^{25} = +65,5° \text{ (c = 0,281 ; chloroforme).}$$

EXEMPLE 118

3-[[(1S)-5-(Benzyloxycarbonylamino)-1-(méthoxycarbonyl)pentyl]amino]-5-chloro-3-(2-chlorophényl)-1-[4-(N',N'-diéthyluréido)benzènesulfonyl]-1,3-dihydroindol-2-one.

On prépare ce composé selon le mode opératoire décrit à l'EXEMPLE 30 à partir de 0,8 g du composé obtenu à la Préparation 55 (isomère B) et 0,410 g de chlorure de 4-(N',N'-diéthyluréido)benzènesulfonyle. On chromatographie sur silice en éluant par le mélange DCM/AcOEt (80/20 ; v/v). On obtient 0,85 g du produit attendu sous forme d'huile.

$$\alpha_D^{25} = -66,9° \text{ (c = 0,245 ; chloroforme).}$$

EXEMPLE 119

3-[[(1R)-5-(Benzyloxycarbonylamino)-1-(méthoxycarbonyl)pentyl]amino]-5-chloro-3-(2-chlorophényl)-1-[4-(N',N'-diéthyluréido)benzènesulfonyl]-1,3-dihydroindol-2-one.

On prépare ce composé selon le mode opératoire décrit à l'EXEMPLE 30 à partir de 0,899 g du composé obtenu à la Préparation 56 (isomère A) et 0,445 g de chlorure de 4-(N',N'-diéthyluréido)benzènesulfonyle. On chromatographie sur silice en éluant par le gradient du mélange DCM/AcOEt de (95/5 ; v/v) jusqu'à (85/15 ; v/v). On obtient 0,68 g du produit attendu sous forme de mousse.

$$\alpha_D^{25} = -60,5° \text{ (c = 0,249 ; chloroforme).}$$

EXEMPLE 120

3-[[(lR)-5-(Benzyloxycarbonylamino)-1-(méthoxycarbonyl)pentyl]amino]-5-chloro-3-(2-chlorophényl)-1-[4-(N',N'-diéthyluréido)benzènesulfonyl]-1,3-dihydroindol-2-one.

On prépare ce composé selon le mode opératoire décrit à l'EXEMPLE 30 à partir de 0,88 g du composé obtenu à la Préparation 57 (isomère B) et 0,449 g de chlorure de 4-(N',N'-diéthyluréido)benzènesulfonyle. On chromatographie sur silice en éluant par le mélange DCM/AcOEt (80/20 ; v/v). On obtient 0,65 g du produit attendu sous forme de mousse.

$$\alpha_D^{25} = +92,5° \text{ (c = 0,245 ; chloroforme).}$$

EXEMPLE 121

Trifluoroacétate de 3-[[(1S)-5-amino-1-(méthoxycarbonyl)pentyl]amino]-5-chloro-3-(2-chlorophényl)-1-[4-(N',N'-diéthyluréido)benzènesulfonyl]-1,3-dihydroindol-2-one.

A) 3-[[(1S)-5-amino-1-(méthoxycarbonyl)pentyl]amino]-5-chloro-3-(2-chlorophényl)-1-[4-(N',N'-diéthyluréido)benzènesulfonyl]-1,3-dihydroindol-2-one

On refroidit à 0°C une solution de 1,0 g du composé obtenu à l'EXEMPLE 117 dans 5 ml de DCM, ajoute 15 ml de TFA, puis 0,8 g d'anisole et 3 ml d'acide trifluorométhanesulfonique. On laisse 22 minutes sous agitation à 0°C, puis ajoute de l'éther au mélange réactionnel et essore le précipité formé. On dissout le précipité dans l'AcOEt, lave la phase organique par une solution à 5 % de carbonate de potassium, à l'eau, sèche sur sulfate de sodium et évapore sous vide le solvant. On chromatographie le résidu sur silice en éluant par le mélange DCM/MeOH/triéthylamine (90/10/1 ; v/v/v). On dissout le produit obtenu dans l'AcOEt, lave deux fois la phase organique par une solution à 5 % de carbonate de potassium, par une solution saturée de NaCl, sèche sur sulfate de sodium et évapore sous vide le solvant. On obtient 0,7 g du produit attendu que l'on utilise tel quel.

B) 3-[[(1S)-5-(*tert*-butoxycarbonylamino)-1-(méthoxycarbonyl)pentyl]amino]-5-chloro-3-(2-chlorophényl)-1-[4-(N',N'-diéthyluréido)benzènesulfonyl]-1,3-dihydroindol-2-one

On laisse 3 heures sous agitation à TA un mélange de 0,684 g du composé obtenu à l'étape précédente, 0,26 g de di-tert-butyldicarbonate, 0,1 g de triéthylamine dans 5 ml de THF. On concentre sous vide le mélange réactionnel et chromatographie le résidu sur silice en éluant par le mélange DCM/AcOEt (80/20 ; v/v). On obtient 0,46 g du produit attendu que l'on utilise tel quel.

C) Trifluoroacétate de 3-[[(1S)-5-amino-1-(méthoxycarbonyl)pentyl]amino]-5-chloro-3-(2-chlorophényl)-1-[4-(N',N'-diéthyluréido)benzènesulfonyl]-1,3-dihydroindol-2-one.

On refroidit à 0°C une solution de 0,46 g du composé obtenu à l'étape précédente dans 3 ml de DCM, ajoute 4 ml de TFA et laisse 3 heures sous agitation à 0°C. On concentre sous vide le mélange réactionnel, extrait le résidu à l'AcOEt, lave trois fois la phase organique à l'eau, par une solution saturée de NaCl, sèche sur sulfate de sodium et évapore sous vide le solvant. On reprend le résidu dans l'éther iso et après trituration, essore le solide formé. On obtient 0,35 g du produit attendu sous forme d'un solide contenant 0,33 mole d'éther iso, F = 140°C.

$$\alpha_D^{25} = +69{,}8° \text{ (c = 0,3 ; MeOH).}$$

EXEMPLE 122

5-Chloro-3-(2-chlorophényl)-3-(éthoxycarboxamido)-1,3-dihydro-1-(2-méthoxy-4-nitrobenzènesulfonyl)indol-2-one.

On prépare ce composé selon le mode opératoire décrit à l'EXEMPLE 22 à partir de 4,75 g du composé obtenu à l'EXEMPLE 16 et 1,81 ml de chloroformiate d'éthyle. On chromatographie sur silice en éluant par le mélange DCM/hexane (90/10 ; v/v) puis au DCM. On obtient 4,7 g du produit attendu après cristallisation dans le mélange DCM/éther iso, F = 155-160°C.

EXEMPLE 123

5-Chloro-3-(2-chlorophényl)-3-(éthoxycarboxamido)-1,3-dihydro-1-[2-méthoxy-4-[(morpholin-4-yl)carbonylamino]benzènesulfonyl]indol-2-one.

A) 1-(4-Amino-2-méthoxybenzènesulfonyl)-5-chloro-3-(2-chlorophényl)-3-(éthoxycarboxamido)-1,3-dihydroindol-2-one.

On hydrogène à TA et à pression atmosphérique pendant 1 heure un mélange de 4,45 g du composé obtenu à l'EXEMPLE 122, 1 g de nickel de Raney® dans 30 ml de MeOH et 60 ml de THF. On filtre le catalyseur sur Célite® et évapore sous vide le filtrat. On chromatographie le résidu sur silice en éluant au DCM puis par le mélange DCM/AcOEt (95/5 ; v/v). On obtient 3,62 g du produit attendu que l'on utilise tel quel.

B) 5-Chloro-3-(2-chlorophényl)-3-(éthoxycarboxamido)-1,3-dihydro-1-[2-méthoxy-4-(phénoxycarboxamido)benzènesulfonyl]indol-2-one.

On refroidit à 0-5°C une solution de 3,6 g du composé obtenu à l'étape précédente dans 7 ml de pyridine et ajoute une solution de 0,9 ml de chloroformiate de phényle dans 10 ml de DCM. On laisse 1 heure sous agitation à TA, puis ajoute de l'eau au mélange réactionnel, extrait à l'AcOEt, lave la phase organique à l'eau, par une solution saturée de NaCl, sèche sur sulfate de sodium et évapore sous vide le solvant. On chromatographie le résidu sur silice en éluant au DCM puis par le mélange DCM/AcOEt (95/5 ; v/v). On obtient 3,55 g du produit attendu que l'on utilise tel quel.

C) 5-Chloro-3-(2-chlorophényl)-3-(éthoxycarboxamido)-1,3-dihydro-1-[2-méthoxy-4-[(morpholin-4-yl)carbonylamino]benzènesulfonyl]indol-2-one.

On chauffe à 60°C pendant 4 heures un mélange de 0,6 g du composé obtenu à l'étape précédente, 0,16 ml

de morpholine dans 10 ml de THF. Après refroidissement, on essore le précipité formé et le sèche. On obtient 0,552 g du produit attendu contenant 0,33 mole de THF, F = 255-260°C.

EXEMPLE 124

5-Chloro-3-(2-chlorophényl)-1-[4-(N',N'-diéthyluréido)benzènesulfonyl]-1,3-dihydro-3-[N'-méthyl-N'-(2-diméthylaminoéthyl)uréido]indol-2-one.

A) 5-Chloro-3-(2-chlorophényl)-1-[4-(N',N'-diéthyluréido)benzènesulfonyl]-1,3-dihydro-3-(phénoxycarboxamido) indol-2-one.

A une solution de 0,8 g du composé obtenu à l'EXEMPLE 99 dans 10 ml de pyridine, on ajoute 0,275 g de chloroformiate de phényle et laisse 18 heures sous agitation à TA. On concentre sous vide, extrait le résidu à l'AcOEt, lave la phase organique à l'eau, par une solution à 5 % d'hydrogénosulfate de potassium, sèche sur sulfate de sodium et évapore sous vide le solvant. On chromatographie sur silice en éluant par le mélange DCM/AcOEt (90/10 ; v/v). On obtient 0,63 g du produit attendu que l'on utilise tel quel.

B) 5-Chloro-3-(2-chlorophényl)-1-[4-(N',N'-diéthyluréido)benzènesulfonyl]-1,3-dihydro-3-[N'-méthyl-N'-(2-diméthylaminoéthyl)uréido]indol-2-one.

On chauffe à 60°C pendant 18 heures un mélange de 0,63 g du composé obtenu à l'étape précédente, 0,15 g de N,N,N'-triméthyléthylènediamine dans 10 ml de chloroforme et 10 ml d'EtOH. On concentre sous vide et chromatographie le résidu sur silice en éluant par le mélange DCM/MeOH (90/10 ; v/v). On obtient 0,35 g du produit attendu après cristallisation dans le mélange DCM/éther iso, F = 155°C.

EXEMPLE 125

3-[N'-(Carbamoylméthyl)-N'-méthyluréido]-5-chloro-3-(2-chlorophényl)-1-[4-(N',N'-diéthyluréido)benzènesulfonyl]-1,3-dihydroindol-2-one, hémihydrate.

On chauffe à 60°C pendant 3 heures un mélange de 1 g du composé obtenu à l'étape A de l'EXEMPLE 124, 0,224 g du chlorhydrate de sarcosinamide, 0,234 g de DIPEA dans 20 ml de chloroforme. On concentre sous vide et chromatographie le résidu sur silice en éluant par le mélange DCM/MeOH (97/3 ; v/v). On obtient 0,65 g du produit attendu après cristallisation dans le mélange DCM/éther iso, F = 150°C.

EXEMPLE 126

Trifluoroacétate de 3-[[(1R)-5-amino-1-(méthoxycarbonyl)pentyl]amino]-5-chloro-3-(2-chlorophényl)-1-[4-(N',N'-diéthyluréido)benzènesulfonyl]-1,3-dihydroindol-2-one.

A) 3-[[(IR)-5-amino-1-(méthoxycarbonyl)pentyl]amino]-5-chloro-3-(2-chlorophényl)-1-[4-(N',N'-diéthyluréido)benzènesulfonyl]-1,3-dihydroindol-2-one

On refroidit à 0°C une solution de 0,37 g du composé obtenu à l'EXEMPLE 120 dans 7,5 ml de TFA, puis ajoute 0,2 g d'anisole et 0,8 ml d'acide trifluorométhanesulfonique. On laisse 22 minutes sous agitation à 0°C, puis ajoute 50 ml d'éther au mélange réactionnel et essore le précipité formé. On dissout le précipité dans l'AcOEt, lave la phase organique par une solution à 5 % de carbonate de potassium jusqu'à pH = 9, à l'eau, sèche sur sulfate de sodium et évapore sous vide le solvant. On obtient 0,27 g du produit attendu que l'on utilise tel quel.

B) 3-[[(1R)-5-(tert-butoxycarbonylamino)-1-(méthoxycarbonyl)pentyl]amino]-5-chloro-3-(2-chlorophényl)-1-[4-(N',N'-diéthyluréido)benzènesulfonyl]-1,3-dihydroindol-2-one

On laisse une semaine sous agitation à TA un mélange de 0,254 g du composé obtenu à l'étape précédente, 0,098 g de di-tert-butyldicarbonate, 0,05 ml de triéthylamine dans 4 ml de THF. On concentre sous vide le mélange réactionnel et chromatographie le résidu sur silice en éluant par un gradient du mélange DCM/AcOEt de (90/10 ; v/v) à (80/20 ; v/v). On obtient 0,17 g du produit attendu que l'on utilise tel quel.

C) Trifluoroacétate de 3-[[(1R)-5-amino-1-(méthoxycarbonyl)pentyl]amino]-5-chloro-3-(2-chlorophényl)-1-(4-(N',N'-diéthyluréido)benzènesulfonyl]-1,3-dihydroindol-2-one.

On refroidit à 0°C une solution de 0,17 g du composé obtenu à l'étape précédente dans 0,5 ml de DCM, ajoute 2 ml de TFA et laisse 2 heures sous agitation à 0°C. On concentre sous vide le mélange réactionnel, reprend le résidu au DCM et évapore sous vide le solvant. On reprend le résidu dans le mélange DCM/éther iso et après trituration, essore le solide formé. On obtient 0,07 g du produit attendu sous forme de mousses après séchage à 60°C.

$\alpha_D^{25}$ = +82,2° (c = 0,16 ; chloroforme).

Spectre de RMN à 200 MHz dans DMSO-$d_6$.

0,9 à 1,18 ppm : m : 12H
2,75 ppm : t : 2H
3,0 ppm : qd : 1H
3,35 ppm : qd : 4H
3,6 ppm : s : 3H
3,9 ppm : d : 1H
6,7 à 8,3 ppm : m : 14H
8,8 ppm : s : 1H

En procédant selon les modes opératoires décrits dans les exemples ci-dessus, à partir des 1,3-dihydroindol-2-one décrites dans les Préparations, on prépare les composés selon l'invention rassemblés dans le TABLEAU III ci-après.

TABLEAU III

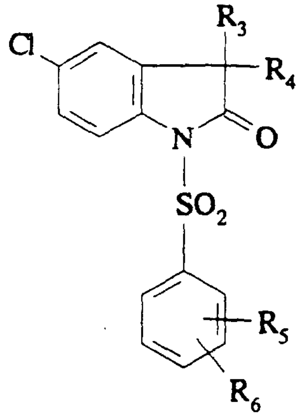

| Exemple | R3 | R4 | R5 | R6 | Sel, solvate ; F°C ou RMN ; solvant de cristallisation |
|---|---|---|---|---|---|
| 127 (a) | 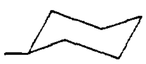 | -NH-Me | H | 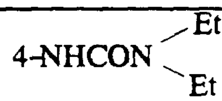 | 225-228 DCM/éther iso |
| 128 (a) | 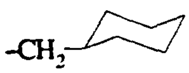 | -NH-Me | H | 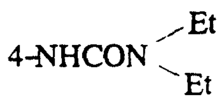 | 169 DCM/éther iso |
| 129 (a) | 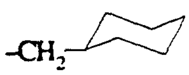 | $-NHCH_2CH_2CN$ | H | 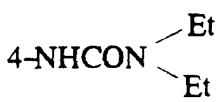 | 180 DCM/éther iso |
| 130 (a) | 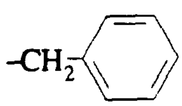 | -NH-Me | H | 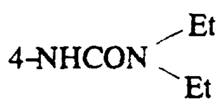 | 0,5 DCM 96 DCM/éther iso |
| 131 (b) | 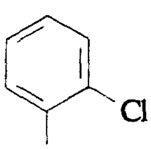 | -NH-Me | H | 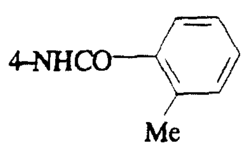 | 223 éther iso |
| 132 (b) | 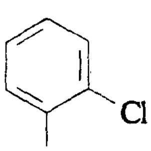 | -NH-Me | H | 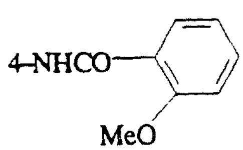 | 0,25 DCM 153-154 éther iso |

TABLEAU III (suite)

| Exemple | R3 | R4 | R5 | R6 | Sel, solvate ; F°C ou RMN ; solvant de cristallisation |
|---|---|---|---|---|---|
| 133<br>(b) | 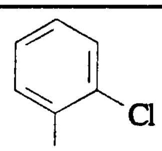 | -NH-Me | H | 4-NHCO 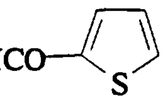 | 251<br>DCM |
| 134<br>(b) | 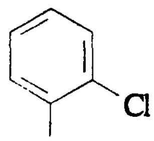 | -NH-Me | H | 4-NHCO 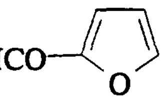 | 239<br>DCM |
| 135<br>(c) | 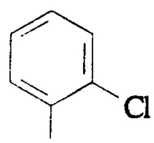 | -NH-Me | 2-OMe | 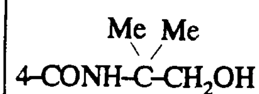 | 0,25 $H_2O$<br>162<br>éther iso |
| 136<br>(c) | 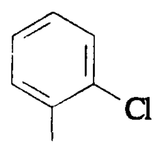 | -NH-Me | 2-OMe | 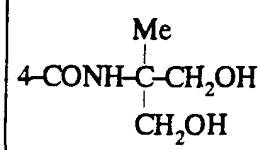 | 150<br>éther iso |
| 137<br>(c) | 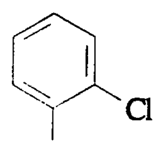 | -NH-Me | 2-OMe | 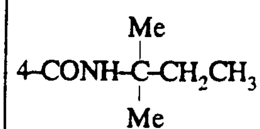 | 220<br>DCM/éther iso |
| 138<br>(a) | 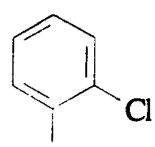 | 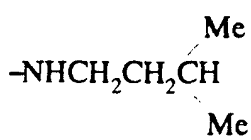 | H | 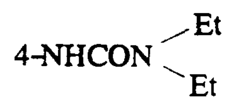 | 224<br>DCM/éther iso |
| 139<br>(a) | 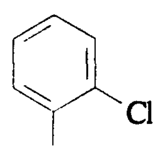 | -NH 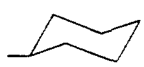 | H | 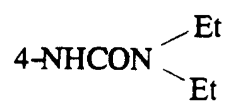 | 212<br>DCM/éther iso |
| 140<br>(a) | 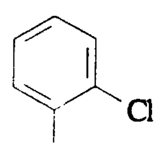 | -NH 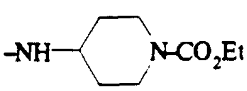 | H | 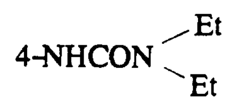 | 207<br>DCM/éther iso |
| 141<br>(a) | 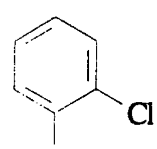 | -NHCH$_2$ 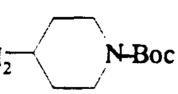 | H | 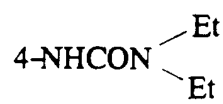 | 232<br>DCM/éther iso |

TABLEAU III (suite)

| Exemple | $R_3$ | $R_4$ | $R_5$ | $R_6$ | Sel, solvate ; F°C ou RMN ; solvant de cristallisation |
|---|---|---|---|---|---|
| 142 (a) | 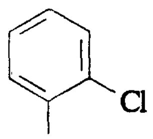 | 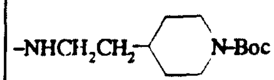 | H | 4-NHCON(Et)Et | 158-160 DCM/éther iso |
| 143 (a) | 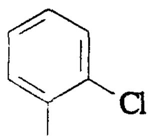 | 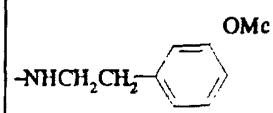 | H | 4-NHCON(Et)Et | 138-142 DCM/éther iso |
| 144 (a) | 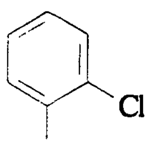 | 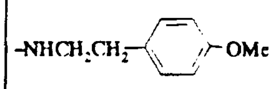 | H | 4-NHCON(Et)Et | 120 DCM/éther iso |
| 145 (d) | 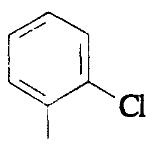 | 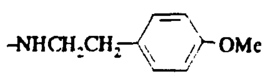 | 2-OMe | 4-$NO_2$ | 75-76 |
| 146 (c), (f) et (g) | 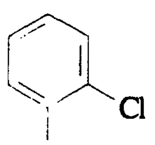 | 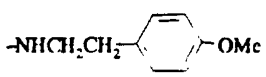 | 2-OMe | 4-NHCON(Et)Et | 190-195 DCM/éther iso |
| 147 (a) | 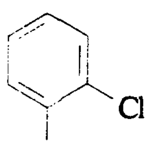 | 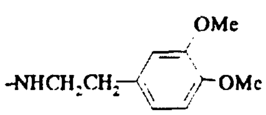 | H | 4-NHCON(Et)Et | 95-100 DCM/éther iso/hexane |
| 148 (a) | 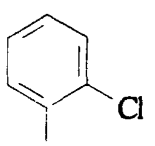 | 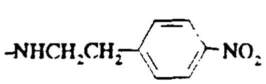 | H | 4-NHCON(Et)Et | 135-138 DCM/éther iso |
| 149 (a) | 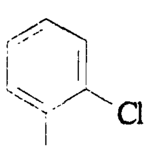 | 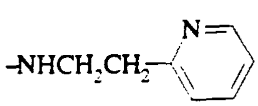 | H | 4-NHCON(Et)Et | 208 DCM/éther iso |
| 150 (a) | 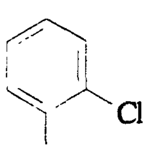 | 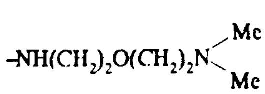 | H | 4-NHCON(Et)Et | 155-158 DCM/éther iso |

TABLEAU III (suite)

| Exemple | $R_3$ | $R_4$ | $R_5$ | $R_6$ | Sel, solvate ; F°C ou RMN ; solvant de cristallisation |
|---|---|---|---|---|---|
| 151 (h) | 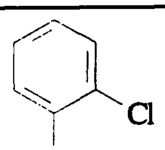 | $-NH(CH_2)_2O(CH_2)_2-NH-Boc$ | 2-OMe | 4-OMe | 111 DCM/éther iso |
| 152 (h) | 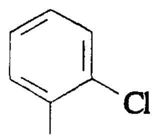 | $-NH(CH_2)_2O(CH_2)_2-NH-Boc$ | 3-OMe | 4-OMe | huile RMN |
| 153 (a) | 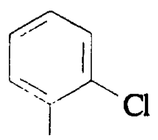 | $-NH(CH_2)_2O(CH_2)_2-NH-Boc$ | H | 4-NHCON$(Et)_2$ | 165 DCM/éther iso |
| 154 (h) | 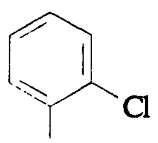 | $-NH(CH_2)_3OMe$ | 3-OMe | 4-OMe | 158-160 DCM/éther iso |
| 155 (a) | 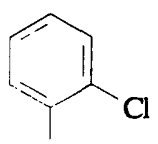 | $-NH(CH_2)_3OMe$ | H | 4-NHCON$(Et)_2$ | 175 DCM/éther iso |
| 156 (a) | 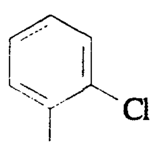 | $-NHCH_2CH_2N(Me)_2$ | H | 4-NHCON$(Et)_2$ | oxalate ; 155 isopropanol |
| 157 (h) | 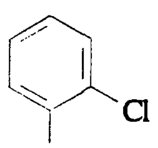 | $-NHCH_2CH_2N(Et)_2$ | 3-OMe | 4-OMe | 127 DCM/éther iso |
| 158 (a) | 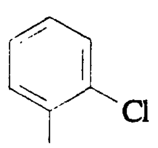 | $-N(Me)CH_2CH_2-N(Et)_2$ | H | 4-NHCON$(Et)_2$ | 123 pentane |
| 159 (a) | 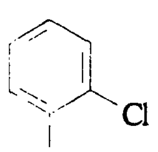 | $-NHCH_2CH_2-N(iPr)_2$ | H | 4-NHCON$(Et)_2$ | 180 DCM/éther iso |

TABLEAU III (suite)

| Exemple | $R_3$ | $R_4$ | $R_5$ | $R_6$ | Sel, solvate ; F°C ou RMN ; solvant de cristallisation |
|---|---|---|---|---|---|
| 160 (a) | 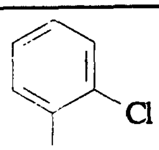 | 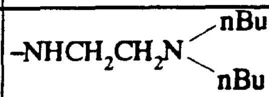 | H | 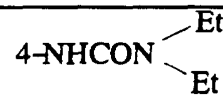 | oxalate 138 éther |
| 161 (a) | 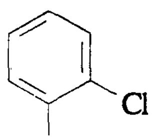 | 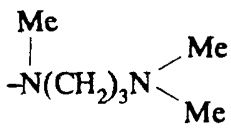 | H | 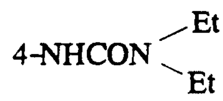 | RMN |
| 162 (a) | 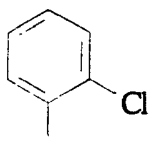 | 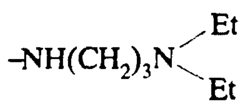 | H | 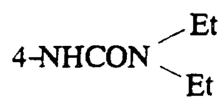 | 159 DCM/éther iso |
| 163 (a) | 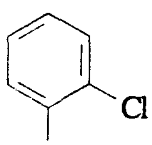 | $-NH(CH_2)_4-NH-$ Boc | H | 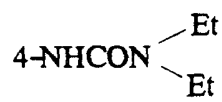 | 178–179 DCM/éther iso |
| 164 (h) | 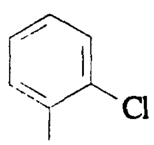 | $-NH(CH_2)_5-NH-$ Boc | 3-OMe | 4–OMe | 0,5 $H_2O$ 155–156 DCM/éther iso |
| 165 (a) | 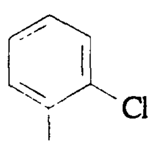 | $-NH(CH_2)_5-NH-$ Boc | H | 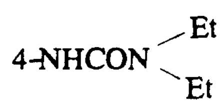 | 175–177 DCM/éther iso |
| 166 (a) | 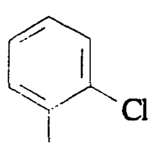 | 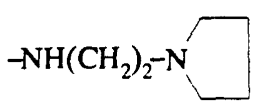 | H | 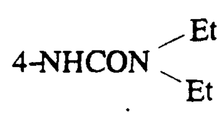 | 206 DCM/éther iso |
| 167 (i) | 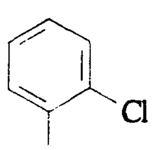 | 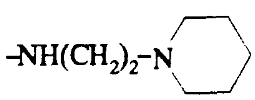 | H | 4–$NO_2$ | 140 éther iso/ hexane |
| 168 (j) | 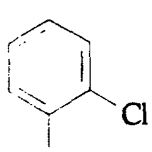 | 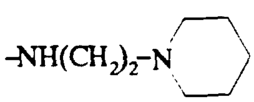 | H | 4–$NH_2$ | 173 DCM/éther iso |

TABLEAU III (suite)

| Exemple | $R_3$ | $R_4$ | $R_5$ | $R_6$ | Sel, solvate ; F°C ou RMN ; solvant de cristallisation |
|---|---|---|---|---|---|
| 169 (k) | 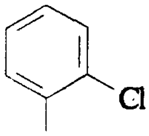 | 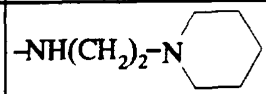 | H | 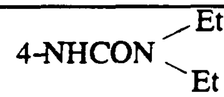 | 204 DCM/éther iso |
| 170 (a) | 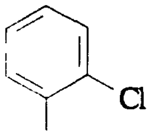 | 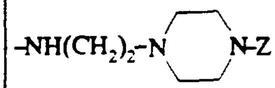 | H | 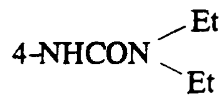 | 174–176 DCM/éther iso |
| 171 (a) | 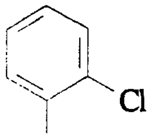 | 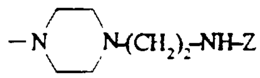 | H | 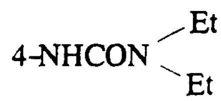 | 133–138 DCM/hexane/ éther iso |
| 172 (a) | 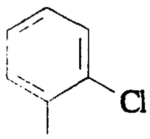 | 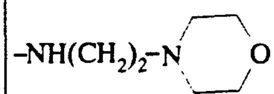 | H | 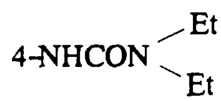 | 180 DCM/éther iso |
| 173 (h) | 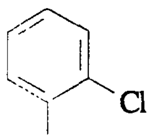 | 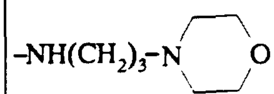 | 3-OMe | 4-OMe | 0,33 éther iso ; 85–88 DCM/éther iso |
| 174 (a) | 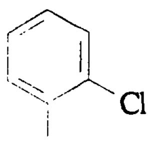 | 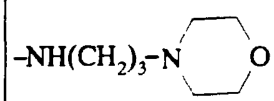 | H | 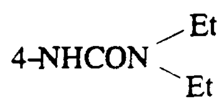 | 184–187 DCM/éther iso |
| 175 (a) | 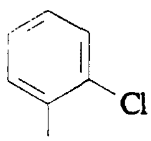 | 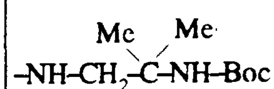 | H | 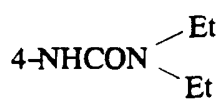 | $0,5H_2O$ 168 DCM/éther iso |
| 176 (a) | 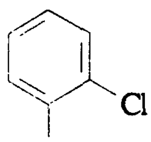 | 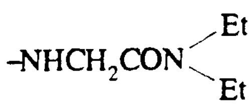 | H | 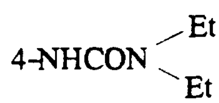 | $0,5H_2O$ 154 DCM/éther iso |
| 177 (a) | 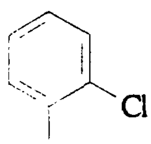 | 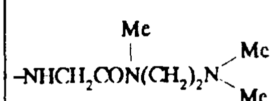 | H | 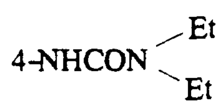 | 130 DCM/éther iso |

TABLEAU III (suite)

| Exemple | $R_3$ | $R_4$ | $R_5$ | $R_6$ | Sel, solvate ; F°C ou RMN ; solvant de cristallisation |
|---|---|---|---|---|---|
| 178 (a) | 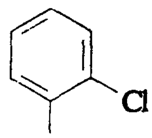 | $-NH(CH_2)_2CON$ Et Et | H | 4-NHCON Et Et | 164 DCM/éther iso |
| 179 (a) | 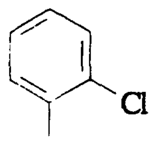 | $-NH(CH_2)_3COOMe$ | H | 4-NHCON Et Et | 151 DCM/éther iso |
| 180 (a) | 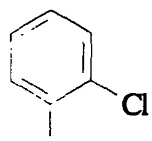 | $-NH(CH_2)_3COOBz$ | H | 4-NHCON Et Et | 149 DCM/éther iso |
| 181 (a) | 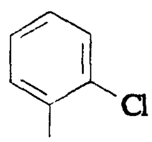 | $-NHCH_2CN$ | H | 4-NHCON Et Et | 201 DCM/éther iso |
| 182 (a) | 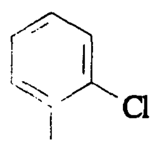 | $-NH(CH_2)_5CN$ | H | 4-NHCON Et Et | 176-180 DCM/éther iso |
| 183 (a) | 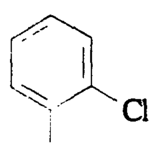 | 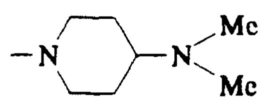 | H | 4-NHCON Et Et | 220 DCM/éther iso |
| 184 (a) | 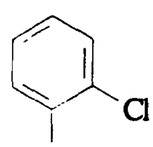 | $-NHCOCH_2N$ Me Me | H | 4-NHCON Et Et | 203-205 DCM/éther iso |
| 185 (a) | 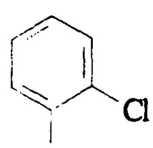 | $-NHCOCH_2N$ Et Et | H | 4-NHCON Et Et | 132-135 DCM/éther iso |
| 186 (a) | 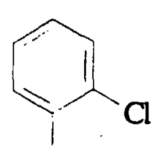 | 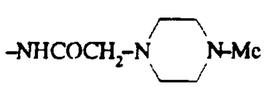 | H | 4-NHCON Et Et | 137 DCM/éther iso |

TABLEAU III (suite)

| Exemple | $R_3$ | $R_4$ | $R_5$ | $R_6$ | Sel, solvate ; F°C ou RMN ; solvant de cristallisation |
|---|---|---|---|---|---|
| 187 (a) | 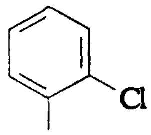 | 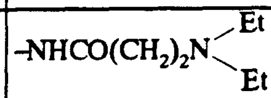 | H | 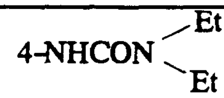 | 174 DCM/éther iso |
| 188 (l) | 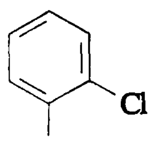 | -NHCOOEt | 2-OMe | 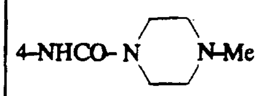 | 254-257 THF |
| 189 (m) | 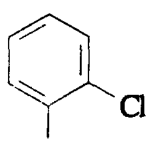 | 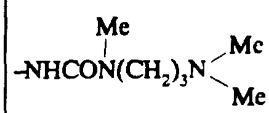 | H | 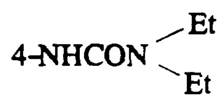 | 195-RMN DCM/éther iso |
| 190 (m) | 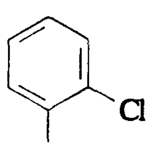 | 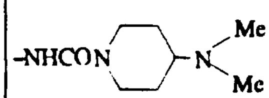 | H | 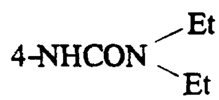 | 173 DCM/éther iso |
| 191 (n) | 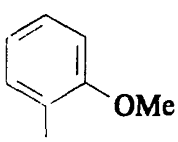 | $-NH_2$ | 2-OMe | 4-CONHtBu | 269 DCM/éther iso |

(a) composé préparé selon le mode opératoire décrit à l'EXEMPLE 30

(b) composé préparé selon le mode opératoire décrit à l'étape C de l'EXEMPLE 110 à partir du composé obtenu à l'étape B de l'EXEMPLE 110 et en utilisant les chlorures d'acides appropriés

(c) composé préparé selon le mode opératoire décrit à l'étape C de l'EXEMPLE 112 à partir du composé obtenu à l'étape B de l'EXEMPLE 112 et en utilisant les amines appropriées

(d) composé préparé selon le mode opératoire décrit à l'EXEMPLE 16

(e) composé préparé selon le mode opératoire décrit à l'EXEMPLE 103 étape B

(f) composé préparé selon le mode opératoire décrit à l'étape A de l'EXEMPLE 27

(g) composé préparé selon le mode opératoire décrit à l'étape B de l'EXEMPLE 27

(h) composé préparé selon le mode opératoire décrit à l'EXEMPLE 1

(i) composé préparé selon le mode opératoire décrit à l'EXEMPLE 11

(j) composé préparé selon le mode opératoire décrit à l'EXEMPLE 110 étape B

(k) composé préparé selon les modes opératoires décrits aux étapes A puis B de l'EXEMPLE 15

(l) composé préparé selon le mode opératoire décrit à l'étape C de l'EXEMPLE 123 en utilisant la 1–méthylpipérazine

(m) composé préparé selon le mode opératoire décrit à l'étape B de l'EXEMPLE 124 en utilisant les amines ou les hétérocycles appropriés

(n) composé préparé selon les modes opératoires décrits aux étapes A, C puis D de l'EXEMPLE 28.

Spectre de RMN à 200 MHz dans DMSO-$d_6$ du composé de l'EXEMPLE 152

1,35 ppm : s : 9H
2,4 ppm : mt : 2H
3,1 ppm : qd : 2H
3,3 à 3,7 ppm : m : 5H
3,75 à 3,95 ppm : 2s : 6H
6,65 à 8,20 ppm : m : 11H

Spectre de RMN à 200 MHz dans DMSO-$d_6$ du composé de l'EXEMPLE 161

1,1 ppm : t : 6H
1,3 à 2,9 ppm : m : 15H
3,3 ppm : qd : 4H
6,6 à 8,1 ppm : m : 11H
8,75 ppm : s : 1H

Spectre de RMN à 200 MHz dans DMSO-$d_6$ du composé de l'EXEMPLE 189

1,0 ppm : t : 6H
1,4 à 1,8 ppm : m : 8H
1,8 à 2,3 ppm : mt : 2H
2,5 ppm : s : 3H
2,8 à 3,7 ppm : m : 6H
7,1 à 7,9 ppm : m : 11H
8,65 ppm : s : 1H
9,1 ppm : se : 1H

EXEMPLE 192

Bromhydrate de 5-chloro-3-(2-chlorophényl)-1-[4-(N',N'-diéthyluréido) benzène-sulfonyl]-1,3-dihydro-3-[(pipérid-4-yl)amino]indol-2-one.

On laisse 18 heures sous agitation à TA un mélange de 1 g du composé obtenu à l'EXEMPLE 140 et 40 ml d'une solution à 33 % d'acide bromhydrique dans l'AcOH. On concentre sous vide, à 30°C, le mélange réactionnel, reprend le résidu à l'éther et essore le précipité formé. On obtient 0,425 g du produit attendu après cristallisation dans le mélange MeOH/éther iso, F = 210°C.

EXEMPLE 193

Trifluoroacétate de 5-chloro-3-(2-chlorophényl)-1-[4-(N',N'-diéthyluréido) benzènesulfonyl]-1,3-dihydro-3-[[(pipérid-4-yl)méthyl]amino]indol-2-one.

On refroidit à 0°C une solution de 0,5 g du composé obtenu à l'EXEMPLE 141 dans 6 ml de DCM, ajoute 6

ml de TFA et laisse deux heures sous agitation à 0°C. On concentre sous vide le mélange réactionnel, reprend le résidu au DCM et évapore sous vide. On reprend le résidu à l'éther et essore le précipité formé. On obtient 0,34 g du produit attendu après cristallisation dans le mélange McOH/éther iso, F = 218°C.

EXEMPLE 194

5-Chloro-3-(2-chlorophényl)-1-[4-(N',N'-diéthyluréido)benzènesulfonyl]-1,3-dihydro-3-[[2-(pipérid-4-yl)éthyl] amino]indol-2-one.

On refroidit à 0°C une solution de 0,63 g du composé obtenu à l'EXEMPLE 142 dans 4 ml de DCM, ajoute 7 ml de TFA et laisse 5 heures 30 minutes sous agitation à 0°C. On concentre sous vide le mélange réactionnel, reprend le résidu par une solution à 5 % de carbonate de sodium, extrait à l'AcOEt, lave la phase organique à l'eau, sèche sur sulfate de magnésium et évapore sous vide le solvant. On chromatographie le résidu sur silice en éluant par le mélange DCM/McOH/triéthylamine (85/15/2 ; v/v/v). On chromatographie à nouveau le produit obtenu sur silice en éluant par le mélange DCM/MeOH/triéthylamine (90/10/0,5 ; v/v/v). On dissout le produit obtenu dans l'AcOEt, lave la phase organique à l'eau, par une solution saturée de chlorure de sodium, sèche sur sulfate de magnésium et évapore sous vide le solvant. On obtient 0,085 g du produit attendu après trituration dans l'hexane puis essorage.

Spectre de RMN à 200 MHz dans DMSO-$d_6$

0,6 à 1,5 ppm : m : 13H
1,8 à 2,95 ppm : m : 6H
3,3 ppm : qd : 4H
6,6 à 8,1 ppm : m : 11H
8,7 ppm : s : 1H

EXEMPLE 195

1,25Trifluoroacétate de 3-[[2-(2-aminoéthoxy)éthyl]amino]-5-chloro-3-(2-chloro phényl)-1,3-dihydro-1-(2,4-diméthoxybenzènesulfonyl)indol-2-one.

On refroidit à 0°C une solution de 0,618 g du composé obtenu à l'EXEMPLE 151 dans 2,6 ml de DCM et ajoute 7,7 ml de TFA. On laisse 2 heures sous agitation à 0°C et concentre sous vide. On obtient 0,506 g du produit attendu après cristallisation dans le mélange DCM/éther iso, F = 145-148°C.

EXEMPLE 196

Trifluoroacétate de 3-[[2-(2-aminoéthoxy)éthyl]amino]-5-chloro-3-(2-chloro phényl)-1,3-dihydro-1-(3,4-diméthoxybenzènesulfonyl)indol-2-one.

On prépare ce composé selon le mode opératoire décrit à l'EXEMPLE 195 à partir de 2 g du composé obtenu à l'EXEMPLE 152 dans 8,3 ml de DCM et 24,9 ml de TFA. On obtient 1,44 g du produit attendu après cristallisation dans le mélange DCM/THF/éther iso, F = 110-114°C.

EXEMPLE 197

Trifluoroacétate de 3-[[2-(2-aminoéthoxy)éthyl]amino]-5-chloro-3-(2-chloro phényl)-1,3-dihydro-1-[4-(N',N'-diéthyluréido)benzènesulfonyl]indol-2-one.

On prépare ce composé selon le mode opératoire décrit à l'EXEMPLE 195 à partir de 2,3 g du composé obtenu à l'EXEMPLE 153 dans 14 ml de DCM et 28 ml de TFA. Après concentration sous vide du mélange réactionnel, on reprend le résidu au DCM et évapore sous vide le solvant. On reprend le résidu à l'éther iso et après trituration, essore le solide formé. On obtient 1,9 g du produit attendu après cristallisation dans le mélange DCM/éther iso, F = 183°C.

EXEMPLE 198

3-[(4-aminobutyl)amino]-5-chloro-3-(2-chlorophényl)-1-[4-(N',N'-diéthyluréido)benzènesulfonyl]-1,3-dihydroindol-2-one.

On refroidit à +4°C une suspension de 0,82 g du composé obtenu à l'EXEMPLE 163 dans 5 ml de DCM et ajoute 10 ml de TFA. On laisse 3 heures sous agitation à +4°C, concentre sous vide le mélange réactionnel, reprend le résidu au DCM et évapore sous vide le solvant. On reprend le résidu par une solution à 5 % de carbonate de sodium, extrait à l'AcOEt, lave par une solution saturée de NaCl, à l'eau, sèche la phase organique sur sulfate de sodium et

évapore sous vide le solvant. On obtient 0,44 g du produit attendu après cristallisation dans le mélange DCM/éther iso, F = 259°C.

EXEMPLE 199

Trifluoroacétate de 3-[(5-aminopentyl)amino]-5-chloro-3-(2-chlorophényl)-1-[4-(N',N'-diéthyluréido)benzène-sulfonyl]-1,3-dihydroindol-2-one.

On refroidit à 0°C une solution de 0,9 g du composé obtenu à l'EXEMPLE 165 dans 5 ml de DCM et ajoute 10 ml de TFA. On laisse 5 heures sous agitation à 0°C et concentre sous vide le mélange réactionnel. On reprend le résidu à l'eau, ajoute de l'AcOEt et du carbonate de potassium solide. On essore le produit insoluble présent à l'interphase. On dissout ce produit insoluble dans un mélange DCM/MeOH, sèche la phase organique sur sulfate de sodium et évapore sous vide les solvants. On obtient 0,155 g du produit attendu après cristallisation puis recristallisation dans le mélange MeOH/éther iso, F = 202-204°C.

Spectre de RMN à 200 MHz dans DMSO-$d_6$

1,15 ppm : t : 6H
1,25 à 1,7 ppm : m : 6H
2,3 ppm : mt : 2H
2,8 ppm : t : 2H
3,3 à 3,5 ppm : m : 5H
6,7 à 8,2 ppm : m : 13H
8,85 ppm : s : 1H

EXEMPLE 200

3-[[5-(acétylamino)pentyl]amino]-5-chloro-3-(2-chlorophényl)-1-[4-(N',N'-diéthyluréido)benzènesulfonyl]-1,3-dihydroindol-2-one.

A une solution de 0,276 g du composé obtenu à l'EXEMPLE 199 sous forme de base libre, 2 ml de pyridine dans 2 ml de DCM, on ajoute à TA une solution de 0,034 g de chlorure d'acétyle dans 0,5 ml de DCM et laisse 1 heure sous agitation. On rajoute 0,034 g de chlorure d'acétyle, laisse 30 minutes sous agitation à TA et concentre sous vide le mélange réactionnel. On reprend le résidu à l'AcOEt, lave la phase organique à l'eau, par une solution saturée de NaCl, sèche sur sulfate de sodium et évapore sous vide le solvant. On chromatographie le résidu sur silice en éluant par le mélange DCM/AcOEt (50/50 ; v/v). On obtient 0,14 g du produit attendu après cristallisation dans le mélange DCM/éther iso, F = 195-198°C.

EXEMPLE 201

5-Chloro-3-(2-chlorophényl)-1-[4-(N',N'-diéthyluréido)benzènesulfonyl]-1,3-dihydro-3-[[2-(pipérazin-1-yl) éthyl]amino]indol-2-one.

A une solution de 1,2 g du composé obtenu à l'EXEMPLE 170, 0,7 g d'anisole dans 24 ml de TFA, on ajoute à TA 3,3 ml d'acide trifluorométhanesulfonique. On laisse 10 minutes précises sous agitation, ajoute 35 ml d'éther et essore le précipité formé. On dissout le précipité dans un mélange AcOEt/eau, ajoute du carbonate de sodium jusqu'à pH = 10, puis après décantation, lave la phase organique à l'eau, sèche sur sulfate de sodium et évapore sous vide le solvant. On chromatographie le résidu sur silice en éluant par le mélange DCM/MeOH/triéthylamine (90/10/1 ; v/v/v). On obtient 0,825 g du produit attendu après trituration dans le mélange DCM/éther iso puis essorage, F = 260°C.

Spectre de RMN à 200 MHz dans DMSO-$d_6$

1,1 ppm : t : 6H
2,0 à 2,8 ppm : m : 12H
3,4 ppm : qd : 4H
6,7 à 8,3 ppm : m : 12H
8,8 ppm : s : 1H

EXEMPLE 202

5-Chloro-3-(2-chlorophényl)-1-[4-(N',N'-diéthyluréido)benzènesulfonyl)-1,3-dihydro-3-[[2-(4-méthylpipérazin-1-yl)éthyl]amino]indol-2-one.

A une solution de 0,16 g du composé obtenu à l'EXEMPLE 201 dans 4 ml de MeOH et 1 ml d'AcOH, on

ajoute, par portions, en 4 heures, 0,03 g de paraformaldéhyde et 0,02 g de cyanoborohydrure de sodium. On laisse une nuit sous agitation à TA, ajoute 5 gouttes d'HCl concentré, puis après 10 minutes, de l'eau et alcalinise le mélange réactionnel par ajout de carbonate de potassium. On concentre sous vide les solvants organiques, extrait au DCM, sèche sur sulfate de sodium et évapore sous vide le solvant. On chromatographie le résidu sur silice en éluant par le mélange DCM/MeOH (88/12 ; v/v). On obtient 0,099 g du produit attendu après cristallisation dans le mélange DCM/ hexane/éther iso.

Spectre de RMN à 200 MHz dans DMSO-$d_6$

1,1 ppm : t : 6H
1,9 à 3,6 ppm : m : 19H
6,6 à 8,2 ppm : m : 11H
8,8 ppm : s : 1H

EXEMPLE 203

3-[4-(2-aminoéthyl)pipérazin-1-yl]-5-chloro-3-(2-chlorophényl)-1-[4-(N',N'-diéthyluréido)benzènesulfonyl]-1,3-dihydroindol-2-one, monohydrate.

A une solution de 0,7 g du composé obtenu à l'EXEMPLE 171, 0,4 g d'anisole dans 15 ml de TFA, on ajoute à TA 1,5 ml d'acide trifluorométhanesulfonique. On laisse 12 minutes sous agitation, ajoute de l'éther et essore le précipité formé. On dissout le précipité dans un mélange AcOEt/eau, ajoute du carbonate de potassium jusqu'à pH = 10, puis après décantation lave la phase organique à l'eau, par une solution saturée de NaCl, sèche sur sulfate de sodium et évapore sous vide le solvant. On chromatographie le résidu sur silice en éluant par le mélange DCM/MeOH/ triéthylamine (90/10/1 ; v/v/v). On dissout le produit obtenu dans l'AcOEt, lave deux fois la phase organique par une solution à 5 % de carbonate de potassium, sèche sur sulfate de sodium et évapore sous vide le solvant. On reprend le résidu dans le mélange DCM/éther iso/hexane, essore un insoluble et concentre sous vide le filtrat. On reprend le résidu dans le mélange DCM/hexane, et après trituration puis essorage, on obtient 0,192 g du produit attendu.

Spectre de RMN à 200 MHz dans DMSO-$d_6$

1,1 ppm : t : 6H
1,8 à 3,0 ppm : m : 12H
3,3 ppm : qd : 4H
6,6 à 8,1 ppm : m : 11H
8,6 ppm : s : 1H

EXEMPLE 204

3-[(3-Carbamoylpropyl)amino]-5-chloro-3-(2-chlorophényl)-1-[4-(N',N'-diéthyluréido)benzènesulfonyl]-1,3-dihydroindol-2-one.

A) Acide 4-[N-[5-chloro-3-(2-chlorophényl)-1-[4-(N',N'-diéthyluréido)benzène sulfonyl]-2,3-dihydro-2-oxoindol-3-yl]amino]butyrique.

On hydrogénolyse à TA et pression atmosphérique un mélange de 0,78 ml du composé obtenu à l'EXEMPLE 180, 0,17 g de palladium sur charbon à 5 % dans 15 ml d'AcOH. Après 30 minutes, on filtre le catalyseur sur Célite® , lave à l'AcOH et concentre sous vide le filtrat. On reprend le résidu à l'éther iso et après trituration essore le précipité formé. On obtient 0,67 g du produit attendu que l'on utilise tel quel.

B) 3-[(3-Carbamoylpropyl)amino]-5-chloro-3-(2-chlorophényl)-1-[4-(N',N'-diéthyluréido)-benzènesulfonyl]-1,3-dihydroindol-2-one.

On refroidit à 0°C une solution de 0,67 g du composé obtenu à l'étape précédente dans 10 ml de DMF, ajoute 0,467 g de BOP et laisse 15 minutes sous agitation. On ajoute ensuite à 0°C, 0,2 ml d'une solution concentrée d'ammoniaque et laisse 18 heures sous agitation en laissant remonter la température à TA. On verse le mélange réactionnel dans l'eau, extrait à l'AcOEt, lave la phase organique à l'eau, sèche sur sulfate de sodium et évapore sous vide le solvant. On chromatographie le résidu sur silice en éluant par le mélange DCM/MeOH (95/5 ; v/v). On obtient 0,32 g du produit attendu après cristallisation dans le mélange DCM/éther iso, F = 192°C.

EXEMPLE 205

5-Chloro-3-(2-chlorophényl)-7-fluoro-1,3-dihydro-1-(2,4-diméthoxy-benzènesulfonyl)-3-(méthylamino)indol-2-one.

On prépare ce composé selon le mode opératoire décrit à l'EXEMPLE 1 à partir de 0,25 g de 5-chloro-3-(2-chlorophényl)-7-fluoro-1,3-dihydro-3-(méthylamino) indol-2-one et 0,165 g de chlorure de 2,4-diméthoxybenzènesulfonyle. On obtient 0,075 g du produit attendu après cristallisation et recristallisation dans le mélange DCM/éther iso, F = 183-185°C.

EXEMPLE 206

3-(2-Chlorophényl)-5-éthoxy-1-[4-(N',N'-diéthyluréido)-2-méthoxy-benzènesulfonyl]-1,3-dihydro-3-(diméthylamino)indol-2-one.

A) 3-(2-Chlorophényl)-5-éthoxy-1,3-dihydro-1-(2-méthoxy-4-nitrobenzènesulfonyl)-3-(diméthylamino)indol-2-one.

On prépare ce composé selon le mode opératoire décrit à l'EXEMPLE 16 à partir de 1g du composé obtenu à la Préparation 68 et 0,762 g de chlorure de 2-méthoxy-4-nitrobenzènesulfonyle. On chromatographie sur silice en éluant au DCM puis par le mélange DCM/AcOEt (90/10 ; v/v). On obtient 0,8 g du produit attendu que l'on utilise tel quel.

B) 1-(4-Amino-2-méthoxybenzènesulfonyl)-3-(2-chlorophényl)-5-éthoxy-1,3-dihydro-3-(diméthylamino)indol-2-one.

On prépare ce composé selon le mode opératoire décrit à l'étape B de l'EXEMPLE 103 à partir de 1,5 g du composé obtenu à l'étape précédente. On chromatographie sur silice en éluant au DCM puis par le mélange DCM/AcOEt (95/5 ; v/v). On obtient 0,756 g du produit attendu que l'on utilise tel quel.

C) 3-(2-Chlorophényl)-5-éthoxy-1,3-dihydro-1-[2-méthoxy-4-(phénoxycarboxamido)benzènesulfonyl]-3-(diméthylamino)indol-2-one.

On prépare ce composé selon le mode opératoire décrit à l'étape A de l'EXEMPLE 27 à partir de 0,756 g du composé obtenu à l'étape précédente et 0,22 ml de chloroformiate de phényle. On chromatographie sur silice en éluant au DCM puis par le mélange DCM/AcOEt (95/5 ; v/v). On obtient 0,785 g du produit attendu que l'on utilise tel quel.

D) 3-(2-Chlorophényl)-5-éthoxy-1-[4-(N',N'-diéthyluréido)-2-méthoxybenzènesulfonyl]-1,3-dihydro-3-(diméthylamino)indol-2-one.

On prépare ce composé selon le mode opératoire décrit à l'étape B de l'EXEMPLE 27 à partir de 0,780 g du composé obtenu à l'étape précédente et 0,25 ml de diéthylamine. On obtient 0,605 g du produit attendu après cristallisation dans le mélange DCM/éther iso, F = 212-214°C.

En procédant selon le mode opératoire décrit à l'EXEMPLE 30, à partir des 1,3-dihydroindol-2-one décrites dans les Préparations et du chlorure de 4-(N',N'-diéthyluréido)benzènesulfonyle, on prépare les composés selon l'invention rassemblés dans le TABLEAU IV ci-après.

TABLEAU IV

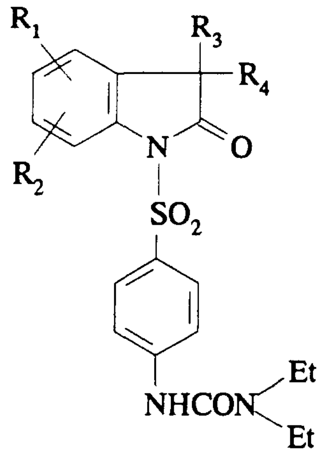

| Exemples | $R_1$ | $R_2$ | $R_3$ | $R_4$ | Sel, solvate ; F°C ou RMN Solvant de cristallisation |
|---|---|---|---|---|---|
| 207 | -Me | H | 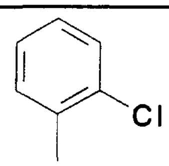 | -NH-Me | 227-228 DCM/éther iso |
| 208 | 5-Et | H | 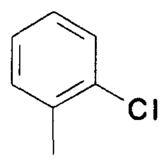 | -NH-Me | 0,5 $H_2O$ 230-231 DCM/éther iso |
| 209 | 5-OMe | H | 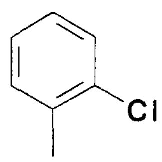 | $-NH_2$ | 201 DCM/éther iso |
| 210 | -OEt | H | 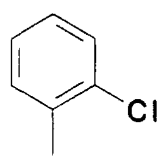 | -NH-Et | 218-220 DCM/éther iso |
| 211 | 5-F | H | 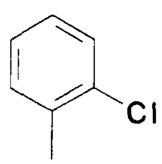 | -NH-Me | 1 $H_2O$ 195 DCM/éther iso |

TABLEAU IV (suite)

| Exemples | $R_1$ | $R_2$ | $R_3$ | $R_4$ | Sel, solvate ; F°C ou RMN Solvant de cristallisation |
|---|---|---|---|---|---|
| 212 | 5-Cl | 4-Me | 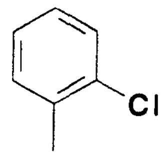 | -NH-Me | 236-239 DCM/éther iso |
| 213 | 5-Cl | 6-Me | 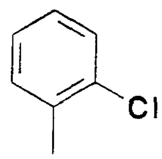 | -NH-Me | 264 DCM/éther iso |
| 214 | 5-Cl | 6-Me | 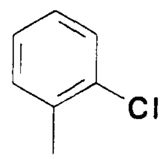 | 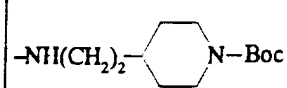 | 0,5 THF 210-211 DCM/éther iso |
| 215 | 5-Cl | 6-Cl | 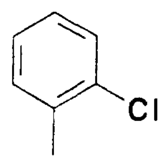 | 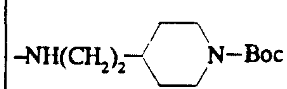 | 0,25 DCM 203-205 DCM/éther iso |
| 216 | 5-Cl | 6-Cl | 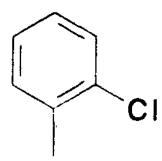 | $-NH(CH_2)_3-OMe$ | 202 DCM/éther iso |
| 217 | 5-Cl | 6-Cl | 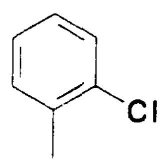 | 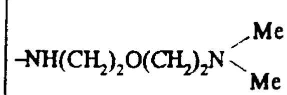 | 119 DCM/éther iso |
| 218 | 5-Cl | 6-Cl | 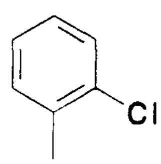 | 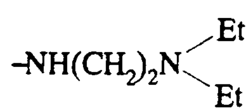 | 155 DCM/éther iso/hexane |
| 219 | 5-Cl | 6-Cl | 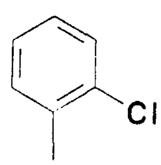 | $-NH(CH_2)_5-NH-$ Boc | 174 DCM/éther iso |

EXEMPLE 220

1,1Trifluoroacétate de 5,6-dichloro-3-(2-chlorophényl)-1-[4-(N',N'-diéthyluréido) benzènesulfonyl]-1,3-dihydro-3-[[2-(pipérid-4-yl)éthyl]amino]indol-2-one.

On laisse 2 heures 15 minutes sous agitation à 0°C un mélange de 1,4 g du composé obtenu à l'EXEMPLE 215, 15 ml de TFA dans 5 ml de DCM et concentre sous vide le mélange réactionnel. On reprend le résidu à l'éther, évapore sous vide le solvant et effectue trois fois cette opération. On dissout le produit obtenu dans l'AcOEt, ajoute de l'éther iso, évapore lentement le solvant, essore le précipité formé et le lave à l'éther iso. On obtient 0,95 g du produit attendu, F = 158-161°C.

EXEMPLE 221

Trifluoroacétate de 3-[(5-aminopentyl)amino]-5,6-dichloro-3-(2-chlorophényl)-1-[4-(N',N'-diéthyluréido)benzènesulfonyl]-1,3-dihydroindol-2-one.

On refroidit à 0°C une solution de 0,8 g du composé obtenu à l'EXEMPLE 219 dans 3 ml de DCM, ajoute 8,8 ml de TFA et laisse 2 heures sous agitation. On concentre sous vide le mélange réactionnel et cristallisse le résidu dans un mélange DCM/éther iso. On obtient 0,467 g du produit attendu, F = 195°C.

EXEMPLE 222

3-[[2-(1-*tert*-butoxycarbonylpipérid-4-yl)éthyl]amino]-5-chloro-3-(2-chlorophényl)-1-[[1-(diéthylaminocarbonyl)indolin-5-yl]sulfonyl]-1,3-dihydroindol-2-one.

On refroidit à +4°C, sous atmosphère d'argon, une solution de 1g de 3-[[2-(1-*tert*-butoxycarbonylpipérid-4-yl) éthyl]amino]-5-chloro-3-(2-chlorophényl)-1,3-dihydroindol-2-one dans 5 ml de DMF et ajoute 0,087 g d'hydrure de sodium à 60 % dans l'huile. Aprés 30 minutes d'agitation à +4°C, on ajoute 0,627 g de chlorure de 1-(diéthylaminocarbonyl)indoline-5-sulfonyle et laisse 3 heures sous agitation à TA. On ajoute 50 ml d'eau au mélange réactionnel, extrait à l'AcOEt, lave la phase organique par une solution saturée de NaCl, à l'eau, sèche sur sulfate de sodium et évapore sous vide le solvant. On chromatographie le résidu sur silice en éluant au DCM puis par le mélange DCM/AcOEt (88/12 ; v/v). On obtient 1,97 g du produit attendu après cristallisation dans le mélange DCM/éther iso, F = 139°C.

EXEMPLE 223

Trifluoroacétate de 5-chloro-3-(2-chlorophényl)-1-[[1-(diéthylaminocarbonyl) indolin-5-yl]sulfonyl]-1,3-dihydro-3-[[2-(pipérid-4-yl)éthyl]amino]indol-2-one, monohydrate.

On refroidit à +4°C une solution de 1,17 g du composé obtenu à l'EXEMPLE 222 dans 7,5 ml de DCM, ajoute 15 ml de TFA et laisse 3 heures 15 minutes sous agitation à +4°C. On concentre sous vide le mélange réactionnel, reprend le résidu au DCM, évapore sous vide le solvant et répète deux fois cette opération. On reprend le résidu à l'AcOEt, lave la phase organique à l'eau, par une solution saturée de NaCl, sèche sur sulfate de sodium et évapore sous vide le solvant. On obtient 0,6 g du produit attendu après trituration dans l'éther iso puis cristallisation dans le mélange DCM/éther iso, F = 133°C.

EXEMPLE 224

1-[(1-Acétylindolin-5-yl)sulfonyl]-5-chloro-3-(2-chlorophényl)-1,3-dihydro-3-[(3-méthoxypropyl)amino]indol-2-one, hémihydrate.

On prépare ce composé selon le mode opératoire décrit à l'EXEMPLE 222 à partir de 1 g de 5-chloro-3-(2-chlorophényl)-1,3-dihydro-3-[(3-méthoxypropyl)-amino]indol-2-one et 0,711 g de chlorure de 1-acétylindoline-5-sulfonyle. On obtient 0,921 g du produit attendu après cristallisation dans le mélange DCM/éther iso, F = 158-162°C.

EXEMPLE 225

5-Chloro-3-(2-chlorophényl)-1,3-dihydro-1-[[1-(méthoxycarbonyl)indolin-5-yl]sulfonyl]-3-[(3-méthoxypropyl) amino]indol-2-one.

On prépare ce composé selon le mode opératoire décrit à l'EXEMPLE 222 à partir de 1,3 g de 5-chloro-3-(2-chorophényl)-1,3-dihydro-3-[(3-méthoxypropyl)-amino]indol-2-one et 0,980 g de chlorure de 1-(méthoxycarbonyl) indoline-5-sulfonyle. On obtient 1,5 g du produit attendu après cristallisation dans le mélange DCM/éther iso/hexane, F = 98°C.

EXEMPLE 226

3-[[(1S)-5-(Benzyloxycarbonylamino)-1-(hydroxyméthyl)pentyl]amino]-5-chloro-3-(2-chlorophényl)-1-[[1-(diéthylaminocarbonyl)indolin-5-yl]sulfonyl]-1,3-dihydroindol-2-one.

A) 3-[[(1S)-5-(Benzyloxycarbonylamino)-1-[(triméthylsilyloxy)méthyl] pentyl]amino]-5-chloro-3-(2-chlorophényl)-1-[[1-(diéthylaminocarbonyl)indolin-5-yl]sulfonyl]-1,3-dihydroindol-2-one.

On prépare ce composé selon le mode opératoire décrit à l'EXEMPLE 222 à partir du composé obtenu à la Préparation 60, 1,1 g de chlorure de 1-(diéthylaminocarbonyl)indoline-5-sulfonyle, 0,162 g d'hydrure de sodium à 60 % dans l'huile et 20 ml de DMF. Après 4 heures d'agitation à TA, on verse le mélange réactionnel dans l'eau, extrait à l'AcOEt, lave la phase organique à l'eau, par une solution saturée de NaCl, sèche sur sulfate de sodium et évapore sous vide le solvant. On obtient le produit attendu sous forme d'huile que l'on utilise tel quel.

B) 3-[[(1S)-5-(Benzyloxycarbonylamino)-1-(hydroxyméthyl)pentyl]amino]-5-chloro-3-(2-chlorophényl)-1-[[1-(diéthylaminocarbonyl)indolin-5-yl]sulfonyl]-1,3-dihydroindol-2-one.

On laisse 30 minutes sous agitation à TA un mélange du composé obtenu à l'étape précédente, 30 ml d'AcOH, 10 ml de THF et 10 ml d'eau. On verse le mélange réactionnel dans l'eau, extrait à l'AcOEt, lave la phase organique à l'eau, par une solution à 5 % d'hydrogénocarbonate de sodium, sèche sur sulfate de sodium et évapore sous vide le solvant. On chromatographie le résidu sur silice en éluant par le mélange DCM/AcOEt (60/40 ; v/v). On obtient 0,23 g du produit attendu sous forme de mousse .

$$\alpha_D^{25} = +119,4^\circ \text{ (c = 0,25 ; chloroforme).}$$

EXEMPLE 227

5-Chloro-3-(2-chlorophényl)-1-[[1-(diéthylaminocarbonyl)indolin-5-yl] sulfonyl]-3-[3-(diéthylamino)propionamido]-1,3-dihydroindol-2-one, isomère (+).

On prépare ce composé selon le mode opératoire décrit à l'EXEMPLE 222 à partir de 0,313 g du composé obtenu à la Préparation 64 isomère (+) et 0,21 g de chlorure de 1-(diéthylaminocarbonyl)indoline-5-sulfonyle. On chromatographie sur silice en éluant par le mélange DCM/MeOH (80/20 ; v/v). On obtient 0,22 g du produit attendu après cristallisation dans le mélange DCM/hexane/éther iso, F = 130-133°C.

$$\alpha_D^{25} = +57,7^\circ \text{ (c = 0,21 ; chloroforme).}$$

En procédant selon le mode opératoire décrit à l'EXEMPLE 222, à partir des 1,3-dihydroindol-2-one décrites dans les Préparations et du chlorure de 1-(diéthylaminocarbonyl)indoline-5-sulfonyle, on prépare les composés selon l'invention rassemblés dans le TABLEAU V ci-après.

# TABLEAU V

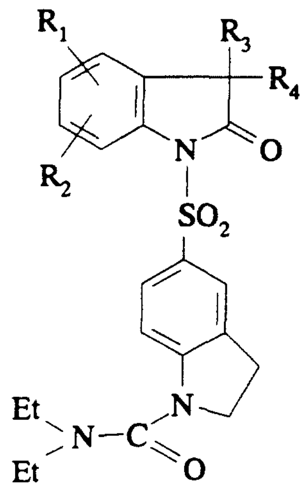

| Exemples | $R_1$ | $R_2$ | $R_3$ | $R_4$ | Solvate ; F°C ou RMN ; solvant de cristallisation |
|---|---|---|---|---|---|
| 228 | 5-Cl | H | 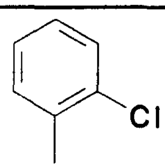 | $-NH(CH_2)_3OMe$ | 148-152 DCM/éther iso |
| 229 | 5-Cl | H | 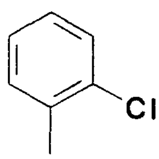 | 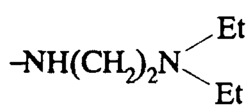 | RMN |
| 230 | 5-Cl | H | 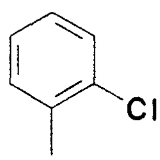 | $-NH(CH_2)_5NH-Boc$ | 0,25 éther iso 148 DCM/éther iso |
| 231 | 5-Cl | H | 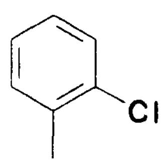 | 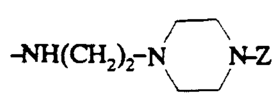 | 130 DCM/éther iso |
| 232 | 5-Cl | H | 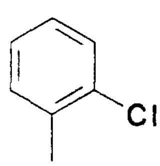 | 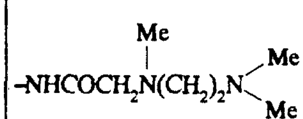 | 175 DCM/éther iso |

TABLEAU V (suite)

| Exemples | $R_1$ | $R_2$ | $R_3$ | $R_4$ | Solvate ; F°C ou RMN ; solvant de cristallisation |
|---|---|---|---|---|---|
| 233 | 5-Cl | H | 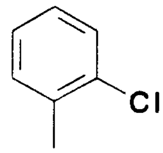 | 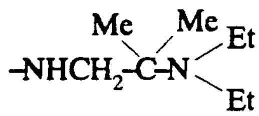 | 145 DCM/éther iso |
| 234 | 5-Cl | H | 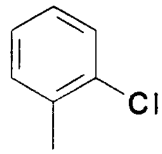 | 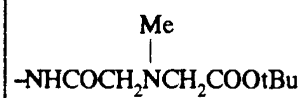 | RMN |
| 235 | 5-Cl | H | 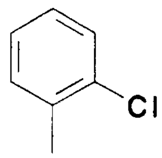 | 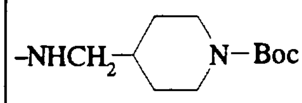 | 187 DCM/éther iso |
| 236 | 5-Cl | H | 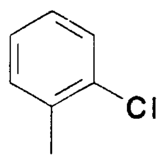 | $-NH(CH_2)_4COOBz$ | 122-123 DCM/éther iso |
| 237 | 5-Cl | H | 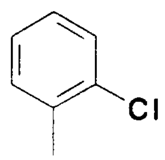 | $-NH(CH_2)_5COOBz$ | 101-102 DCM/éther iso |

Spectre de RMN à 200 MHz dans DMSO-$d_6$ du composé de l'EXEMPLE 229

0,95 ppm : t : 6H
1,2 ppm : t : 6H
2,1 à 2,6 ppm : m : 8H
3,15 ppm : t : 2H
3,3 ppm : qd : 4H
3,9 ppm: t : 2H
6,8 à 8,2 ppm : m : 10H

Spectre de RMN à 200 MHz dans DMSO-$d_6$ du composé de l'EXEMPLE 234

1,05 ppm : t : 6H
1,3 ppm : s : 9H
2,2 ppm : s : 3H
2,95 ppm : t : 2H
3,05 à 3,3 ppm : m : 8H
3,85 ppm : t : 2H
6,8 à 7,8 ppm : m : 10H

EXEMPLE 238

Trifluoroacétate de 3-[(5-aminopentyl)amino]-5-chloro-3-(2-chlorophényl)-1-[[1-(diéthylaminocarbonyl)indolin-5-yl]sulfonyl]-1,3-dihydroindol-2-one.

On refroidit à 0°C une solution de 0,83 g du composé obtenu à l'EXEMPLE 230 dans 5 ml de DCM, ajoute 10 ml de TFA et laisse 5 heures sous agitation à +4°C. On concentre sous vide, à froid, le mélange réactionnel, reprend le résidu au DCM et évapore sous vide le solvant. On reprend le résidu par une solution à 5 % de carbonate de sodium, extrait à l'AcOEt, lave la phase organique par une solution saturée de NaCl, à l'eau, sèche sur sulfate de sodium et évapore sous vide le solvant. On obtient 0,150 g du produit attendu après cristallisation dans l'éther iso, F = 200°C.

EXEMPLE 239

Trifluoroacétate de 3-[2-[N-(carboxyméthyl)-N-méthylamino]acétamido]-5-chloro-3-(2-chlorophényl)-1-[[1-(diéthylaminocarbonyl)indolin-5-yl]sulfonyl]-1,3-dihydroindol-2-one, hémihydrate.

A une solution de 2,5 g du composé obtenu à l'EXEMPLE 234 dans 12 ml de DCM, on ajoute 12 ml de TFA et laisse 3 heures sous agitation à TA. On concentre sous vide à 35°C, reprend le résidu à l'éther, essore le précipité formé et le lave à l'éther. On obtient 2,2 g du composé attendu.

Spectre de RMN à 200 MHz dans DMSO-$d_6$

1,2 ppm : t : 6H
2,8 ppm : s : 3H
3,15 ppm : t : 2H
3,35 ppm : qd : 4H
3,8 à 4,2 ppm : m : 6H
7,0 à 8,0 ppm : m : 10H
10,0 ppm : s : 1H

EXEMPLE 240

Trifluoroacétate de 5-chloro-3-(2-chlorophényl)-1-[[1-(diéthylaminocarbonyl) indolin-5-yl]sulfonyl]-1,3-dihydro-3-[[(pipérid-4-yl)méthyl]amino]indol-2-one, hémihydrate.

On refroidit à 0°C une solution de 1,5 g du composé obtenu à l'EXEMPLE 235 dans 12 ml de DCM, ajoute 12 ml de TFA et laisse 3 heures sous agitation à 0°C. On concentre sous vide à 30°C, reprend le résidu à l'éther iso, essore !e précipité formé et le lave à l'éther iso. On obtient 1,36 g du produit attendu.

Spectre de RMN à 200 MHz dans DMSO-$d_6$

0,8 à 2,4 ppm : m : 13H
2,6 à 3,4 ppm : m : 10H
3,9 ppm : t : 2H
6,6 à 8,8 ppm : m : 12H

EXEMPLE 241

1-[4-(N-*tert*-Butylcarbamoyl)benzyl]-5-chloro-3-(2-chlorophényl)-3-(éthylamino)-1,3-dihydroindol-2-one.

A) 4-[[5-Chloro-3-(2-chlorophényl)-3-(éthylamino)-2,3-dihydro-2-oxoindol-1-yl]méthyl]benzoate de *tert*-butyle.

On prépare ce composé selon le mode opératoire décrit à l'étape A de l'EXEMPLE 96 à partir de 1,19 g de 5-chloro-3-(2-chlorophényl)-3-(éthylamino)-1,3-dihydroindol-2-one et 1,1 g de 4-bromométhylbenzoate de *tert*-butyle. On chromatographie sur silice en éluant par le gradient du mélange hexane/AcOEt de (95/5 ; v/v) à (90/10 ; v/v). On obtient 0,9 g du produit attendu que l'on utilise tel quel.

B) Acide 4-[[5-chloro-3-(2-chlorophényl)-3-(éthylamino)-2,3-dihydro-2-oxoindol-1-yl]méthyl] benzoïque.

On prépare ce composé selon le mode opératoire décrit à l'étape B de l'EXEMPLE 96 à partir de 0,4 g du composé obtenu à l'étape précédente. On obtient le produit attendu que l'on utilise tel quel.

C) 1-[4-(N-*tert*-Butylcarbamoyl)benzyl]-5-chloro-3-(2-chlorophényl)-3-(éthylamino)-1,3-dihydroindol-2-one.

On refroidit à 0°C une solution du composé obtenu à l'étape précédente dans 4 ml de DCM, ajoute 0,14 ml de DIPEA, puis *0,25* ml de *tert*-butylamine et 0,345 g de BOP. On laisse 3 heures sous agitation à TA et concentre sous vide le mélange réactionnel. On reprend le résidu à l'AcOEt, lave la phase organique par une solution à 5 % de carbonate de potassium, à l'eau, par une solution saturée de NaCl, sèche sur sulfate de sodium et évapore

sous vide le solvant. On chromatographie le résidu sur silice en éluant par le gradient du mélange hexane/AcOEt de (90/10 ; v/v) à (50/50 ; v/v). On obtient 0,28 g du produit attendu après cristallisation dans le mélange DCM/éther iso, F = 190-195°C.

EXEMPLE 242

5-Chloro-3-(2-chlorophényl)-3-(éthylamino)-1,3-dihydro-1-[4-[N-(2-hydroxy-1,1-diméthyléthyl)carbamoyl]benzyl]indol-2-one, 0,1 $H_2O$.

On refroidit à 0°C une solution de 0,374 g du composé obtenu à l'étape B de l'EXEMPLE 241 dans 5 ml de DCM, ajoute 0,34 ml de triéthylamine puis 0,16 ml de 2-amino-2-méthylpropan-1-ol et 0,364 g de BOP. On laisse 1 heure sous agitation à TA et concentre sous vide le mélange réactionnel. On reprend le résidu à l'AcOEt, lave la phase organique à l'eau, par une solution à 5 % de carbonate de potassium, par une solution saturée de NaCl, sèche sur sulfate de sodium et évapore sous vide le solvant. On chromatographie le résidu sur silice en éluant au DCM puis par le mélange DCM/AcOEt (70/30 ; v/v). On obtient 0,3 g du produit attendu après cristallisation dans le mélange DCM/éther iso, F = 170-172°C.

Spectre de RMN à 200 MHz dans DMSO-$d_6$

1,1 ppm : t : 3H
1,35 ppm : s : 6H
2,4 ppm : mt : 2H
3,25 ppm : t : 1H
3,75 ppm : d : 2H
4,95 ppm : t : 1H
5,05 ppm : système AB : 2H
6,7 à 8,4 ppm : m : 11H

EXEMPLE 243

5-Chloro-3-(2-chlorophényl)-3-(éthylamino)-1,3-dihydro-1-[4-[N-[1,1-di(hydroxyméthyl) éthyl]carbamoyl]benzyl]indol-2-one.

A un mélange de 0,374 g du composé obtenu à l'étape B de l'EXEMPLE 241, 0,25 g de triéthylamine, 0,364 g de BOP dans 5 ml de DCM, on ajoute à TA une solution de 0,172 g de 2-amino-2-méthylpropan-1,3-diol dans 2 ml de DMF et laisse 3 heures sous agitation à TA. On concentre sous vide le mélange réactionnel, reprend le résidu à l'eau, extrait à l'AcOEt, lave la phase organique deux fois par une solution à 5 % de carbonate de potassium, à l'eau, par une solution saturée de NaCl, sèche sur sulfate de sodium et évapore sous vide le solvant. On chromatographie le résidu sur silice en éluant par le mélange AcOEt/DCM (80/20 ; v/v). On obtient 0,18 g du produit attendu après cristallisation dans le mélange DCM/éther iso, F = 138-141°C.

EXEMPLE 244

1-[4-(N-*tert*-Butylcarbamoyl)benzyl]-5-chloro-3-(2-chlorophényl)-1,3-dihydro-3-(diméthylamino)indol-2-one, isomère (+).

A) 4-[[5-Chloro-3-(2-chlorophényl)-2,3-dihydro-3-(diméthylamino)-2-oxoindol-1-yl]méthyl]benzoate de *tert*-butyle.

On prépare ce composé selon le mode opératoire décrit à l'EXEMPLE 93 à partir de 0,62 g du composé obtenu à la Préparation 76 isomère (+), 0,085 g d'hydrure de sodium à 60 % dans l'huile et 0,555 g de 4-bromométhylbenzoate de *tert*-butyle. On chromatographie sur silice en éluant par le mélange DCM/AcOEt (95/5 ; v/v). On obtient 0,662 g du produit attendu sous forme d'huile que l'on utilise tel quel.

B) Acide 4-[[5-chloro-3-(2-chlorophényl)-2,3-dihydro-3-(diméthylamino)-2-oxoindol-1-yl]méthyl]benzoïque.

On prépare ce composé selon le mode opératoire décrit à l'étape A de l'EXEMPLE 94 à partir de 0,662 g du composé obtenu à l'étape précédente. On obtient le produit attendu sous forme d'huile que l'on utilise tel quel.

C) 1-[4-(N-*tert*-Butylcarbamoyl)benzyl]-5-chloro-3-(2-chlorophényl)-1,3-dihydro-3-(diméthylamino)indol-2-one, isomère (+).

A une solution du composé obtenu à l'étape précédente dans 5 ml de DCM, on ajoute 0,4 g de triéthylamine pour amener à pH = 7 puis 0,14 g de *tert*-butylamine et 0,564 g de BOP. On laisse 48 heures sous agitation à TA en maintenant à pH = 7 par ajout de triéthylamine. On concentre sous vide, reprend le résidu par une solution à 5 % de carbonate de potassium, extrait à l'AcOEt, lave la phase organique à l'eau, par une solution saturée de NaCl, sèche sur sulfate de sodium et évapore sous vide le solvant. On chromatographie le résidu sur silice en

éluant par le mélange DCM/AcOEt (92/8 ; v/v). On obtient 0,225 g du produit attendu après cristallisation dans le mélange DCM/hexane, F = 177-178°C.

$$\alpha_D^{25} = +148,6° \text{ (c = 0,17 ; chloroforme).}$$

EXEMPLE 245

5-Chloro-3-(2-chlorophényl)-1,3-dihydro-3-(diméthylamino)-1-(4-nitrobenzyl)indol-2-one.

On prépare ce composé selon le mode opératoire décrit à l'EXEMPLE 89 à partir de 1 g de 5-chloro-3-(2-chlorophényl)-1,3-dihydro-3-(diméthylamino)indol-2-one, 0,132 g d'hydrure de sodium à 60 % dans l'huile, 5 ml de DMF et 0,741 g de bromure de 4-nitrobenzyle. On chromatographie sur silice en éluant par le mélange DCM/hexane (80/20 ; v/v) puis au DCM. On obtient 1,32 g de produit attendu après cristallisation dans le mélange DCM/éther iso, F = 189-193°C.

EXEMPLE 246

5-Chloro-3-(2-chlorophényl)-1-[4-(N',N'-diéthyluréido)benzyl]-1,3-dihydro-3-(diméthylamino)indol-2-one.

A) 1-(4-aminobenzyl)-5-chloro-3-(2-chlorophényl)-1,3-dihydro-3-(diméthylamino)indol-2-one.
On hydrogène pendant 2 heures à TA et pression atmosphérique un mélange de 1,88 g du composé obtenu à l'EXEMPLE 245, 0,9 g de nickel de Raney® dans 40 ml de MeOH et 40 ml de THF. On filtre le catalyseur sur Célite® et évapore sous vide le filtrat. On chromatographie le résidu sur silice en éluant au DCM puis par le mélange DCM/AcOEt (95/5 ; v/v). On obtient 1,58 g du produit attendu que l'on utilise tel quel.

B) 5-Chloro-3-(2-chlorophényl)-1,3-dihydro-3-(diméthylamino)-1-[4-(phénoxycarboxamido)benzyl]indol-2-one.
On refroidit à 0°C une solution de 1 g du composé obtenu à l'étape précédente dans 5 ml de pyridine, ajoute 0,31 ml de chloroformiate de phényle et laisse 2 heures sous agitation à TA. On ajoute de l'eau au mélange réactionnel, extrait à l'AcOEt, lave la phase organique à l'eau, par une solution saturée de NaCl, sèche sur sulfate de sodium et évapore sous vide le solvant. On chromatographie le résidu sur silice en éluant au DCM puis par le mélange DCM/AcOEt (95/5 ; v/v). On obtient 0,98 g du produit attendu que l'on utilise tel quel.

C) 5-Chloro-3-(2-chlorophényl)-1-[4-(N',N'-diéthyluréido)benzyl]-1,3-dihydro-3-(diméthylamino)indol-2-one.
On chauffe à 60°C pendant 4 heures un mélange de 0,45 g du composé obtenu à l'étape précédente, 0,17 ml de diéthylamine dans 5 ml de chloroforme. On concentre sous vide le mélange réactionnel et chromatographie le résidu sur silice en éluant au DCM puis par le mélange DCM/AcOEt (95/5 ; v/v). On obtient 0,37 g du produit attendu après cristallisation dans le mélange DCM/éther iso, F = 193-198°C.

EXEMPLE 247

3-[[5-(tert-Butoxycarbonylamino)pentyl]amino]-5-chloro-3-(2-chloro-phényl)-1,3-dihydro-1-[2-méthoxy-4-(N, N-diméthylcarbamoyl)benzyl]indol-2-one, hémihydrate.

A) 4-[[3-[[5-(*tert*-Butoxycarbonylamino)pentyl]amino]-5-chloro-3-(2-chlorophényl)-2,3-dihydro-2-oxoindol-1-yl]méthyl]-3-méthoxybenzoate de méthyle.
On prépare ce composé selon le mode opératoire décrit à l'EXEMPLE 89 à partir de 2 g de 3-[[5-(*tert*-butoxycarbonylamino)pentyl]amino]-5-chloro-3-(2-chlorophényl)-1,3-dihydroindol-2-one et 1,19 g de 4-bromométhyl-3-méthoxy-benzoate de méthyle. On obtient 2,06 g du produit attendu.
Spectre de RMN à 200 MHz dans DMSO-$d_6$

1,10 à 1,65 ppm : m : 15H
2,35 ppm : mt : 2H
3,2 ppm : t : 1H
3,9 ppm : s : 3H
4,0 ppm : s : 3H
5,0 ppm : système AB : 2H
6,8 ppm : t : 1H
6,85 à 8,3 ppm : m : 10H

B) Acide 4-[[3-[[5-(tert-butoxycarbonylamino)pentyl]amino]-5-chloro-3-(2-chlorophényl)-2,3-dihydro-2-oxoindol-1-yl]méthyl]-3-méthoxybenzoïque.

A une solution de 1,95 g du composé obtenu à l'étape précédente dans 9,4 ml de 1,4-dioxane, on ajoute à TA une solution de 0,375 g d'hydroxyde de lithium monohydrate dans 3 ml d'eau et laisse 4 jours sous agitation à TA. On ajoute de l'eau au mélange réactionnel, neutralise à pH = 7 par ajout d'HCl 1N, évapore sous vide le 1,4-dioxane et acidifie la phase aqueuse à pH = 1 par ajout d'HCl 1N. On extrait à l'AcOEt, lave la phase organique à l'eau, par une solution saturée de NaCl, sèche sur sulfate de sodium et évapore sous vide le solvant. On obtient 2 g du produit attendu que l'on utilise tel quel.

C) 3-[[5-(*tert*-Butoxycarbonylamino)pentyl]amino]-5-chloro-3-(2-chloro-phényl)-1,3-dihydro-1-[2-méthoxy-4-(N,N-diméthylcarbamoyl)benzyl]indol-2-one, hémihydrate.

On refroidit à 0°C une solution de 1 g du composé obtenu à l'étape précédente dans 10 ml de DCM, ajoute 0,3 ml de DIPEA puis 0,13 ml de diméthylamine et 0,69 g de BOP. On laisse 12 heures sous agitation à TA et concentre sous vide. On extrait le résidu à l'AcOEt, lave la phase organique à l'eau, par une solution saturée de NaCl, sèche sur sulfate de sodium et évapore sous vide le solvant. On chromatographie le résidu sur silice en éluant au DCM puis par le mélange DCM/AcOEt (80/20 ; v/v). On obtient 0,54 g du produit attendu après cristallisation dans le mélange DCM/éther iso, F = 75-80°C.

EXEMPLE 248

Ditrifluoroacétate de 3-[(5-aminopentyl)amino]-5-chloro-3-(2-chlorophényl)-1,3-dihydro-1-[2-méthoxy-4-(N,N-diméthylcarbamoyl)benzyl]indol-2-one, monohydrate.

On refroidit à 0°C une solution de 0,1 g du composé obtenu à l'EXEMPLE 247 dans 1 ml de DCM, ajoute 1,3 ml de TFA et laisse 2 heures sous agitation à 0°C. On concentre sous vide le mélange réactionnel, reprend le résidu au DCM et évapore sous vide le solvant. On obtient 0,07 g du produit attendu après cristallisation et recristallisation dans le mélange DCM/éther iso, F = 178-180°C.

EXEMPLE 249

1,5Trifluoroacétate de 3-[(5-aminopentyl)amino]-1-[4-(N-*tert*-butylcarbamoyl)-2-méthoxybenzyl]-5-chloro-3-(2-chlorophényl)-1,3-dihydroindol-2-one, 1,5 hydrate.

A) 3-[[5-(*tert*-Butoxycarbonylamino)pentyl]amino]-1-[4-(N-*tert*-butylcarbamoyl)-2-méthoxybenzyl]-5-chloro-3-(2-chlorophényl)-1,3-dihydroindol-2-one.

On prépare ce composé selon le mode opératoire décrit à l'étape C de l'EXEMPLE 247 à partir de 1,2 g du composé obtenu à l'étape B de l'EXEMPLE 247, 0,33 ml de DIPEA, 0,6 ml de *tert*-butylamine et 0,827 g de BOP. On chromatographie sur silice en éluant au DCM puis par le mélange DCM/AcOEt (90/10 ; v/v). On obtient 0,685 g du produit attendu que l'on utilise tel quel.

B) 1,5-Trifluoroacétate de 3-[(5-aminopentyl)amino]-1-[4-(N-*tert*-butylcarbamoyl)-2-méthoxybenzyl]-5-chloro-3-(2-chlorophényl)-1,3-dihydroindol-2-one, 1,5 hydrate.

On refroidit à 0°C une solution de 0,52 g du composé obtenu à l'étape précédente dans 2 ml de DCM, ajoute 6,3 ml de TFA et laisse 2 heures sous agitation à 0°C. On concentre sous vide, reprend le résidu au DCM et évapore sous vide le solvant. On obtient 0,3 g du produit attendu après cristallisation et recristallisation dans le mélange DCM/éther iso, F = 138-140°C.

EXEMPLE 250

1-[4-(N-*tert*-Butylcarbamoyl)-2-méthoxybenzyl]-5-chloro-3-(2-chlorophényl)-1,3-dihydro-3-propionamidoindol-2-one.

A) 4-[[5-Chloro-3-(2-chlorophényl)-2,3-dihydro-2-oxo-3-propionamidoindol-1-yl]méthyl]-3-méthoxybenzoate de méthyle.

A une solution de 1,323 g du composé obtenu à l'EXEMPLE 89 dans 5 ml de pyridine, on ajoute à TA 0,52 g de chlorure de propionyle et laisse 24 heures sous agitation. On concentre sous vide, extrait le résidu à l'AcOEt, lave la phase organique à l'eau, par une solution saturée de NaCl, sèche sur sulfate de sodium et évapore sous vide le solvant. On chromatographie le résidu sur silice en éluant par le mélange DCM/AcOEt (90/10 ; v/v). On obtient 1,3 g du produit attendu.

B) Acide 4-[[5-Chloro-3-(2-chlorophényl)-2,3-dihydro-2-oxo-3-propionamidoindol-1-yl]méthyl]-3-méthoxybenzoïque.

A un mélange de 1,3 g du composé obtenu à l'étape précédente dans 5 ml de 1,4-dioxane, 2 ml de MeOH et

2 ml d'eau, on ajoute à TA 0,24 g d'hydroxyde de lithium monohydrate et laisse 24 heures sous agitation. On acidifie le milieu réactionnel à pH = 1 par ajout d'HCl concentré et évapore sous vide les solvants. On reprend le résidu à l'eau, extrait à l'AcOEt, lave la phase organique à l'eau, par une solution saturée de NaCl, sèche sur sulfate de sodium et évapore sous vide le solvant. On obtient 1,342 g du produit attendu que l'on utilise tel quel.

C) 1-[4-(N-*tert*-Butylcarbamoyl)-2-méthoxybenzyl]-5-chloro-3-(2-chlorophényl)-1,3-dihydro-3-propionamidoindol-2-one.

A une solution de 0,432 g du composé obtenu à l'étape précédente dans 7 ml de DCM, on ajoute à TA 0,22 g de DIPEA puis 0,382 g de BOP et 0,185 g de *tert*-butylamine et laisse 1 heure sous agitation à TA. On concentre sous vide, extrait le résidu à l'AcOEt, lave trois fois la phase organique par une solution à 5 % de carbonate de potassium, à l'eau, par une solution saturée de NaCl, sèche sur sulfate de sodium et évapore sous vide le solvant. On chromatographie le résidu sur silice en éluant par le mélange DCM/AcOEt (65/35 ; v/v). On obtient 0,303 g du produit attendu après cristallisation dans le mélange DCM/éther iso, F = 249°C.

En procédant selon les modes opératoires décrits dans les EXEMPLES ci-dessus, à partir des 1,3-dihydroindol-2-one décrites dans les Préparations, on prépare les composés selon l'invention rassemblés dans le TABLEAU VI ci-après.

TABLEAU VI

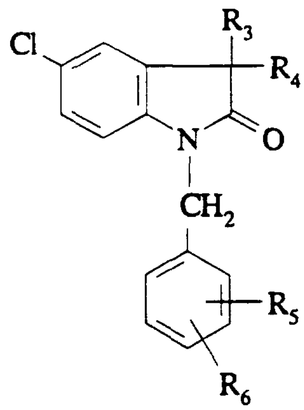

| Exemples | $R_3$ | $R_4$ | $R_5$ | $R_6$ | Solvate ; F°C ou RMN ; solvant de cristallisation |
|---|---|---|---|---|---|
| 251 (a) | 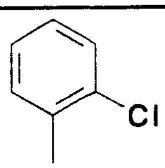 | 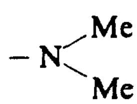 | H | 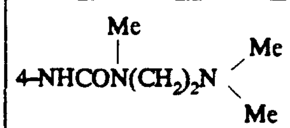 | 115–120 DCM/éther iso |
| 252 (b) | 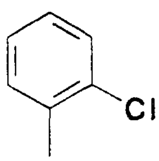 | -NHCOEt | 2-OMe | 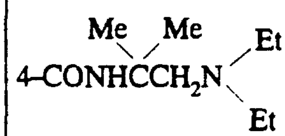 | 177–178 DCM/éther iso |
| 253 (c) | 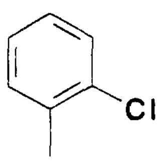 | $-NH_2$ | H | $4-NO_2$ | 172 DCM/éther iso |
| 254 (d) | 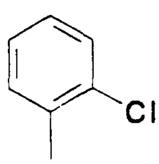 | -NHCOOMe | H | $4-NO_2$ | 203 DCM/éther iso |
| 255 (e) | 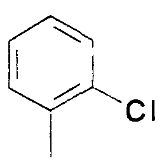 | -NHCOOMe | H | $4-NH_2$ | 225 DCM/éther iso |

TABLEAU VI (suite)

| Exemples | $R_3$ | $R_4$ | $R_5$ | $R_6$ | Solvate ; F°C ou RMN ; solvant de cristallisation |
|---|---|---|---|---|---|
| 256 (f) | 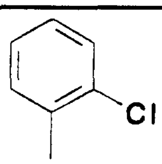 | -NHCOOMe | H | 4-NHCON(Et)Et | 217 DCM/éther iso |
| 257 (g) | 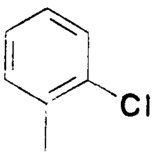 | $-NH_2$ | H | 4-NHCON(Et)Et | 150 DCM/éther iso |
| 258 (h) | 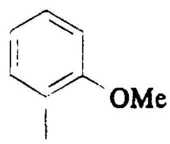 | $-NH_2$ | 2-OMe | $4-CO_2Me$ | RMN |
| 259 (d) | 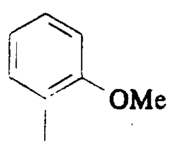 | - NHCOOMe | 2-OMe | $4-CO_2Me$ | utilisé tel quel |
| 260 (i) | 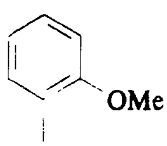 | -NHCOOMe | 2-OMe | 4-CONH-tBu | 203 DCM/éther iso |
| 261 (j) | 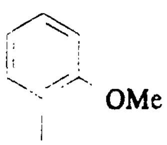 | $-NH_2$ | 2-OMe | 4-CONH-tBu | 0.25 $H_2O$ 210 DCM/éther iso |

(a) composé préparé selon le mode opératoire décrit à l'étape C de l'EXEMPLE 246 en utilisant les amines appropriées.

(b) composé préparé selon le mode opératoire décrit à l'étape C de l'EXEMPLE 250 en utilisant le dichlorhydrate de 2-amino-1-(diéthylamino)-2-méthylpropane (synthétisé selon J. Am. Chem. Soc., 1946, 68, 12-14).

(c) composé préparé selon le mode opératoire décrit à l'EXEMPLE 245

(d) composé préparé selon le mode opératoire décrit à l'EXEMPLE 90.

(e) composé préparé selon le mode opératoire décrit à l'étape A de l'EXEMPLE 246 à partir du composé obtenu à l'EXEMPLE 254.

(f) composé préparé selon les modes opératoires décrits à l'étape B puis à l'étape C de l'EXEMPLE 246.

(g) composé préparé selon le mode opératoire décrit à l'EXEMPLE 92 à partir du composé obtenu à l'EXEMPLE 256.

(h) composé préparé selon le mode opératoire décrit à l'EXEMPLE 89.

(i) composé préparé selon les modes opératoires décrits à l'étape B puis à l'étape C de l'EXEMPLE 250 à partir du composé obtenu à l'EXEMPLE 259.

(j) composé préparé selon le mode opératoire décrit à l'EXEMPLE 92 à partir du composé obtenu à l'EXEMPLE 260.

Spectre de RMN à 200 MHz dans DMSO-$d_6$ du composé de l'EXEMPLE 258

2,65 ppm : s : 2H
3,3 ppm : s : 3H
3,8 ppm : s : 3H
3,95 ppm : s : 3H
4,95 ppm : système AB : 2H
6,7 à 8,1 ppm : m : 10H

EXEMPLE 262

3-Amino-1-[4-(N-*tert*-butylcarbamoyl)benzyl]-3-(2-chlorophényl)-5-éthoxy-1,3-dihydroindol-2-one, hémihydrate.

A) 4-[[3-Amino-3-(2-chlorophényl)-5-éthoxy-2,3-dihydro-2-oxoindol-1-yl]méthyl]benzoate de *tert*-butyle.

On prépare ce composé selon le mode opératoire décrit à l'EXEMPLE 93 à partir de 1 g de 3-amino-3-(2-chlorophényl)-5-éthoxy-1,3-dihydroindol-2-one et de 0,984 g de 4-bromométhylbenzoate de *tert*-butyle. On chromatographie sur silice en éluant au DCM puis par le mélange DCM/AcOEt (70/30 ; v/v). On obtient 1,053 g du produit attendu.

Spectre de RMN à 200 MHz dans DMSO-$d_6$

1,2 ppm : t : 3H
1,55 ppm : s : 9H
2,8 ppm : s : 2H
3,85 ppm : qd : 2 H
5,0 ppm : système AB : 2H
6,3 à 8,4 ppm : m : 11H

B) Acide 4-[[3-amino-3-(2-chlorophényl)-5-éthoxy-2,3-dihydro-2-oxoindol-1-yl]méthyl]benzoïque.

On prépare ce composé selon le mode opératoire décrit à l'étape A de l'EXEMPLE 94 à partir de 1 g du composé obtenu à l'étape précédente. On obtient 0,9 g du produit attendu que l'on utilise tel quel.

C) 3-Amino-1-[4-(N-*tert*-butylcarbamoyl)benzyl]-3-(2-chlorophényl)-5-éthoxy-1,3-dihydroindol-2-one, hémihydrate.

On prépare ce composé selon le mode opératoire décrit à l'étape C de l'EXEMPLE 241 à partir de 0,4 g du composé obtenu à l'étape précédente, 0,16 ml de DIPEA, 0,29 ml de *tert*-butylamine et 0,405 g de BOP. On chromatographie sur silice en éluant au DCM puis par le mélange DCM/AcOEt (70/30 ; v/v). On obtient 0,161 g du produit attendu après cristallisation dans le mélange DCM/éther iso, F = 208-210°C.

EXEMPLE 263

3-Amino-3-(2-chlorophényl)-5-éthoxy-1-[4-[N-[2-(diéthylamino)-1,1-diméthyléthyl]carbamoyl]benzyl]-1,3-dihydroindol-2-one.

On refroidit à 0°C une solution de 0,896 g du composé obtenu à l'étape B de l'EXEMPLE 262 dans 10 ml de DCM, ajoute 0,36 ml de DIPEA puis 0,445 g de dichlorhydrate de 2-amino-1-(diéthylamino)-2-méthylpropane puis 1,14 ml de triéthylamine et 0,906 g de BOP. On laisse 1 heure sous agitation à TA et concentre sous vide. On reprend le résidu à l'AcOEt, lave la phase organique par une solution à 5 % de carbonate de potassium, à l'eau, par une solution saturée de NaCl, sèche sur sulfate de sodium et évapore sous vide le solvant. On chromatographie le résidu sur silice en éluant à l'AcOEt puis par le mélange AcOEt/MeOH (80/20 ; v/v). On obtient 0,12 g du produit attendu après cristallisation dans le mélange DCM/hexane, F = 59°C.

En procédant selon les modes opératoires décrits dans les EXEMPLES précédents, on prépare les composés selon l'invention rassemblés dans le TABLEAU VII ci-après.

TABLEAU VII

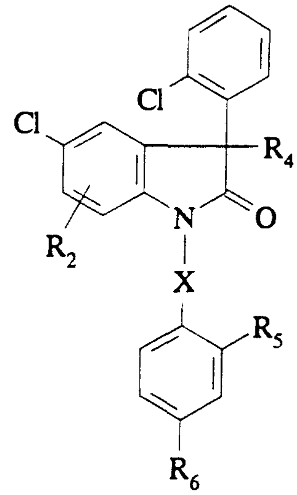

| Exemples | $R_2$ | $R_4$ | X | $R_5$ | $R_6$ | Sel, Solvate ; F°C ou RMN ; solvant de cristallisation |
|---|---|---|---|---|---|---|
| 264 (a) | H | –NH–Me | $SO_2$ | OMe | Me Me Me<br>$-CONHCCH_2N$<br>Me | RMN |
| 265 (b) | H | 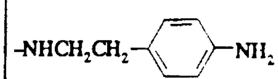 | $SO_2$ | H | Et<br>-NHCON<br>Et | 169–171<br>DCM/éther iso |
| 266 (c) | H | 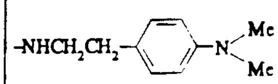 | $SO_2$ | H | Et<br>-NHCON<br>Et | 132–134 |
| 267 (d) | H | 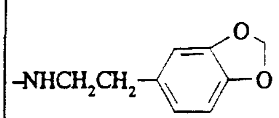 | $SO_2$ | H | Et<br>-NHCON<br>Et | 153–154<br>DCM/éther iso |
| 268 (e) | H | Me Me<br>$-NHCH_2-C-NH_2$ | $SO_2$ | H | Et<br>-NHCON<br>Et | 1TFA, $2H_2O$<br>163–164<br>MeOH/éther iso |
| 269 (f) | H | 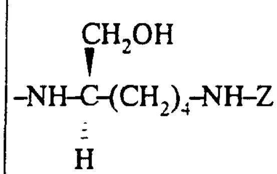 | $SO_2$ | H | Et<br>-NHCON<br>Et | - |

| | | | | | | |
|---|---|---|---|---|---|---|
| 270 (g) | H | 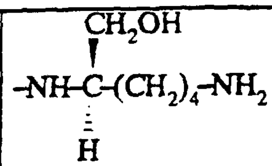 | $SO_2$ | H | 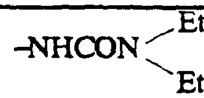 | - |
| 271 (d) | 4-Cl | 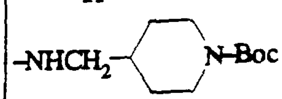 | $SO_2$ | H | 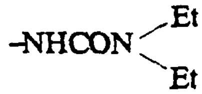 | 234 |
| 272 (h) | 4-Cl | 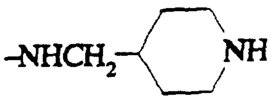 | $SO_2$ | H | 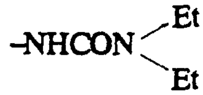 | 1TFA 175 |
| 273 (d) | 6-Cl | 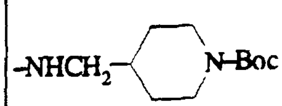 | $SO_2$ | H | 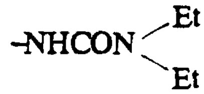 | 220 |
| 274 (i) | 6-Cl | 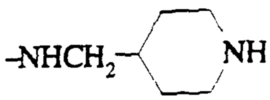 | $SO_2$ | H | 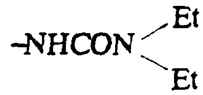 | 1TFA 190 |
| 275 (d) | 6-Me | 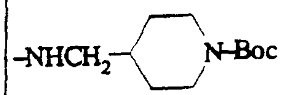 | $SO_2$ | H | 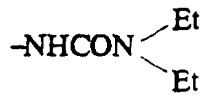 | 264 DCM/éther iso |
| 276 (j) | 6-Me | 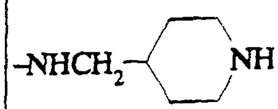 | $SO_2$ | H | 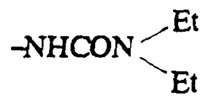 | 1TFA 180 |
| 277 (k) | 6-Me | 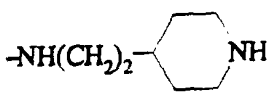 | $SO_2$ | H | 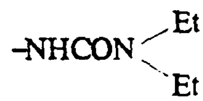 | RMN |
| 278 (l) | H | $-NH_2$ | $CH_2$ | H | -COOtBu | - |
| 279 (m) | H | $-NH_2$ | $CH_2$ | H | -CONHtBu | 0,5 $H_2O$ |
| 280 (d) | H | $-NH(CH_2)_2S-Et$ | $SO_2$ | H | 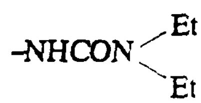 | 197 DCM/éther iso |

(a) composé préparé selon le mode opératoire décrit à l'étape C de l'EXEMPLE 112 à partir du composé obtenu à l'étape B de l'EXEMPLE 112 et en utilisant les amines appropriées.

(b) composé préparé par hydrogénation à TA et pression atmosphérique en présence de nickel de Raney ® du composé obtenu à l'EXEMPLE 148.

(c) composé préparé par réaction du composé obtenu à l'EXEMPLE 265 avec du paraformaldéhyde et en présence de cyanoborohydrure de sodium selon le procédé décrit à l'EXEMPLE 34.

(d) composé préparé selon le mode opératoire décrit à l'EXEMPLE 30.

(e) composé préparé selon le mode opératoire décrit à l'EXEMPLE 198 à partir du composé obtenu à l'EXEMPLE 175.

(f) composé préparé selon le mode opératoire décrit à l'EXEMPLE 30 à partir du composé obtenu à la Préparation 111.

(g) composé préparé selon les modes opératoires décrits aux étapes A, B puis C de l'EXEMPLE 121 à partir du composé obtenu à l'EXEMPLE 269.

(h) composé préparé selon le mode opératoire décrit à l'EXEMPLE 193 à partir du composé obtenu à l'EXEMPLE 271.

(i) composé préparé selon le mode opératoire décrit à l'EXEMPLE 193 à partir du composé obtenu à l'EXEMPLE 273.

(j) composé préparé selon le mode opératoire décrit à l'EXEMPLE 193 à partir du composé obtenu à l'EXEMPLE 275.

(k) composé préparé selon le mode opératoire décrit à l'EXEMPLE 220 à partir du composé obtenu à l'EXEMPLE 214.

(l) isomère (+) ; composé préparé selon le mode opératoire décrit à l'étape A de l'EXEMPLE 96 à partir du composé obtenu à la Préparation 28, isomère (+)

$\alpha_D^{25}$ = +148,8° (c = 0,38 ; chloroforme).

(m) isomère (+) ; composé préparé selon les modes opératoires décrits aux étapes B puis C de l'EXEMPLE 96 à partir du composé obtenu à l'EXEMPLE 278

$\alpha_D^{25}$ = +91,7° (c = 0,218 ; chloroforme).

Spectre de RMN à 200 MHz dans DMSO-$d_6$ du composé de l'EXEMPLE 264

1,3 ppm : s : 6H
1,95 ppm : d : 3H
2,1 à 2,8 ppm : m : 6H
3,5 ppm : qd : 1H
3,61 ppm : s : 3H
6,6 à 8,2 ppm : m : 11H

Spectre de RMN à 200 MHz dans DMSO-$d_6$ du composé de l'EXEMPLE 277

0,8 à 1,8 ppm : m : 13H
2,2 à 2,5 ppm : m : 5H
2,6 à 3,3 ppm : m : 4H
3,4 ppm : qd : 4H
6,8 à 8,1 ppm : m : 10H

En procédant selon les modes opératoires décrits dans les EXEMPLES précédents, on prépare les composés selon l'invention rassemblés dans le TABLEAU VIII ci-après.

TABLEAU VIII

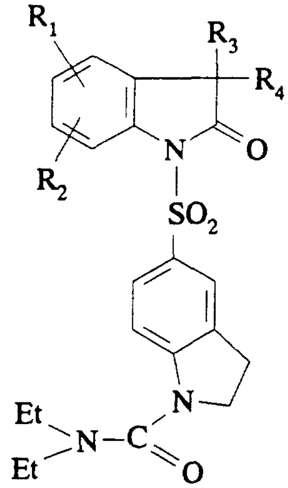

| Exemples | $R_1$ | $R_2$ | $R_3$ | $R_4$ | Sel, Solvate F°C ou RMN Solvant de cristallisation |
|---|---|---|---|---|---|
| 281 (a) | 5-Cl | H | 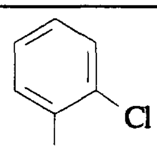 | 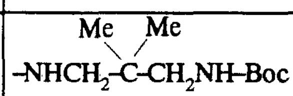 | 190 DCM/éther iso |
| 282 (b) | 5-Cl | H | 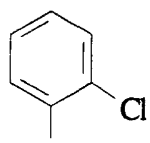 | 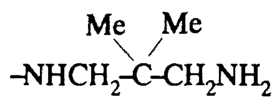 | 1TFA 232 éther |
| 283 (c) | 5-Cl | H | 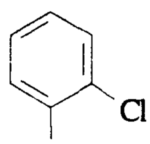 | $-NH(CH_2)_5COOH$ | $1H_2O$, 0,25 éther iso 126-127 DCM/éther iso |
| 284 (d) | 5-Cl | H | 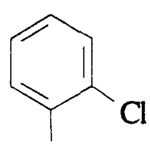 | $-NH(CH_2)_5CONH_2$ | - |
| 285 (a) | 5-Cl | 6-Cl | 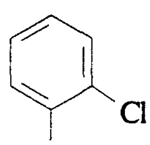 | 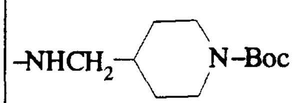 | - |
| 286 (e) | 5-Cl | 6-Cl | 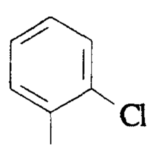 | 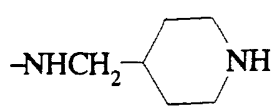 | 1TFA - |

(a) composé préparé selon le mode opératoire décrit à l'EXEMPLE 222.

(b) composé préparé selon le mode opératoire décrit à l'EXEMPLE 193 à partir du composé obtenu à l'EXEMPLE 281.

(c) composé préparé par hydrogénation à TA et pression atmosphérique, en présence de palladium sur charbon à 5 % dans l'AcOH, du composé de l'EXEMPLE 237.

(d) composé préparé par réaction du composé obtenu à l'EXEMPLE 283 avec de l'ammoniaque concentrée en présence de BOP et DIPEA.

(e) composé préparé selon le mode opératoire décrit à l'EXEMPLE 193 à partir du composé obtenu à l'EXEMPLE 285.

EXEMPLE 287

3-(2-Chlorophényl)-1-[4-(N',N'-diéthyluréido)benzènesulfonyl]-1,3-dihydro-3-(méthylamino)-5-(trifluorométhyl)indol-2-one.

On prépare ce composé selon le mode opératoire décrit à l'EXEMPLE 30 à partir du composé obtenu à la Préparation 112, F = 233-236°C.

EXEMPLE 288

5-chloro-3-(2-chlorophényl)-3-[3-(diéthylamino)propionamido]1-[4-(N',N'-diéthyluréido)benzènesulfonyl]-1,3-dihydro-4-méthylindol-2-one

On prépare ce composé selon le mode opératoire décrit à l'EXEMPLE 30 à partir du composé obtenu à la Préparation 114, F = 208-210°C.

EXEMPLE 289

5-chloro-3-(2-chlorophényl)-3-[3-(diéthylamino)propionamido]-1-[4-(N',N'-diéthyluréido)benzènesulfonyl]-1,3-dihydro-6-méthylindol-2-one

On prépare ce composé selon le mode opératoire décrit à l'EXEMPLE 30 à partir du composé obtenu à la Préparation 115.

Spectre de RMN à 200 MHz dans DMSO-$d_6$

0,65 à 1,15 ppm : m : 12H
2 à 2,7 ppm : m : 8H
3,1 à 3,5 ppm : m : 4H
7 à 8 ppm : m : 10H
8,65 ppm : s : 1H
9,55 ppm : s : 1H.

## Revendications

1. Un composé de formule :

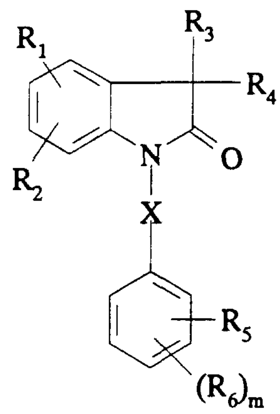

(I)

dans laquelle :

- $R_1$ et $R_2$ représentent chacun indépendamment un hydrogène ; un halogène ; un $(C_1\text{-}C_7)$alkyle ; un $(C_1\text{-}C_7)$ alcoxy ; un trifluorométhyle ;
- $R_3$ représente un $(C_1\text{-}C_7)$alkyle ; un $(C_3\text{-}C_7)$cycloalkyle ; un cyclohexyle substitué par un ou deux $(C_1\text{-}C_4)$ alkyles ; un cyclohexylméthyle ; un phényle non substitué ou substitué une ou plusieurs fois par un halogène, un $(C_1\text{-}C_7)$alkyle, un hydroxy, un $(C_1\text{-}C_7)$alcoxy, un benzyloxy, un acétoxy ; un benzylc non substitué ou substitué une ou plusieurs fois par un halogène, un $(C_1\text{-}C_7)$alkyle, un hydroxy, un $(C_1\text{-}C_7)$alcoxy, un benzyloxy, un acétoxy ;
- $R_4$ représente un azido ; un groupe 2,2-diméthylhydrazino ; un $(C_1\text{-}C_7)$alkylsulfonamido ; un phénylsulfonamido dans lequel le phényle est non substitué ou substitué une ou plusieurs fois par un halogène, un $(C_1\text{-}C_7)$ alkyle, un hydroxy, un $(C_1\text{-}C_7)$alcoxy, un benzyloxy, un acétoxy ; un diméthylaminosulfonamido ; un groupe $NR_7R_8$ ; un groupe $NR_9R_{10}$ ; un groupe $NR_9R_{11}$ ; un radical hétérocyclique $R_{12}$ ; un pipérazin-1-yle substitué en position 4 par un groupe $(C_2\text{-}C_{10})$alkylène substitué par un groupe amino libre ou portant un groupe protecteur ; un groupe lactoylamino ; un groupe mandéloylamino ; un groupe N'-(1-phényléthyl)uréido; un groupe N'-(1-napht-1-yléthyl)uréido ;
- $R_5$ représente l'hydrogène ou l'une des valeurs désignées pour $R_6$ ;
- $R_6$ représente un halogène ; un $(C_1\text{-}C_7)$alkyle; un trifluorométhyle ; un cyano ; un aminométhyle dans lequel l'amino est libre ou substitué par un ou deux $(C_1\text{-}C_7)$alkyles ; un nitro ; un groupe $NR_9R_{11}$ ; un radical hétérocyclique choisi parmi le pyrrol-1-yle, le Δ3-pyrrolin-1-yle, la pyrrolidin-1-yle, la morpholin-4-yle ; un groupe $OR_{13}$ ; un groupe $SR_{13}$ ; un guanidino non substitué ou substitué en 3 par un ou deux $(C_1\text{-}C_7)$alkyles, un phényle, un benzyle ; un formyle ; un $(C_1$ $C_7)$alkylcarbonyle ; un carbamoyle substitué par $R_{14}$ et $R_{15}$ ; un thiocarbamoyle libre ou substitué par un ou deux $(C_1\text{-}C_7)$alkyles ; un sulfamoyle substitué par $R_{16}$ et $R_{17}$ ; un carboxy ; un $(C_1\text{-}C_7)$alcoxycarbonyle ; un phénoxycarbonyle ; un benzyloxycarbonyle ; un $(C_1\text{-}C_7)$ alkylsulfonamido ; un phénylsulfonamido dans lequel le phényle est non substitué ou substitué une ou plusieurs fois par un halogène, un $(C_1\text{-}C_7)$alkyle, un hydroxy, un $(C_1\text{-}C_7)$alcoxy, un benzyloxy, un acétoxy ; un diméthylaminosulfonamido ; un 2-(4-méthylphényl)benzamido ;
- ou $R_5$ et $R_6$ ensemble avec le phényle auquel ils sont liés constituent un groupe:

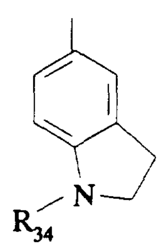

à la condition que X soit $-SO_2-$ ;

- $R_7$ représente un $(C_1\text{-}C_7)$alcoxycarbonyle ;
- $R_8$ représente un $(C_1\text{-}C_7)$alcoxycarbonylamino ; un N-méthyl-N-$(C_1\text{-}C_7)$alcoxy-carbonylamino ;
- $R_9$ représente un hydrogène ; un $(C_1\text{-}C_7)$alkyle ;
- $R_{10}$ représente un groupe $CR_{18}R_{19}R_{20}$ ; un groupe $(CH_2)_pR_{35}$ ; un $(C_2\text{-}C_{10})$alkylène substitué par $R_{21}$ ; un

groupe $CH_2CN$ ; un groupe $C(CH_3)(CH_2OH)_2$ ou $C(CH_2PH)_3$ ; un radical carbocyclique non aromatique en $C_3$-$C_{15}$ ; un phényle non substitué ou substitué une ou plusieurs fois par un halogène, un ($C_1$-$C_7$)alkyle, un hydroxy, un ($C_1$-$C_7$)alcoxy, un benzyloxy, un acétoxy, un nitro, un amino libre ou substitué par un ou deux ($C_1$-$C_7$)alkyles ; un benzyle dans lequel le phényle est non substitué ou substitué une ou plusieurs fois par un halogène, un ($C_1$-$C_7$)alkyle, un hydroxy, un ($C_1$-$C_7$)alcoxy, un benzyloxy, un acétoxy ; un nitro, un amino libre ou substitué par un ou deux ($C_1$-$C_7$)alkyles ; un phénéthyle dans lequel le phényle est non substitué ou substitué une ou plusieurs fois par un halogène, un ($C_1$-$C_7$)alkyle, un hydroxy, un ($C_1$-$C_7$)alcoxy, un benzyloxy, un acétoxy, un nitro, un amino libre ou substitué par un ou deux ($C_1$-$C_7$)alkyles ; un phénéthyle dans lequel le phényle est substitué par un 3,4-méthylènedioxy ou un 3,4-éthylènedioxy ;

- $R_{11}$ représente un hydrogène ; un ($C_1$-$C_{12}$)alkyle ; un ($C_3$-$C_7$)cycloalkylméthyle ; un groupe $OR_{13}$ ; un formyle ; un ($C_1$-$C_7$)alkyl-carbonyle ; un ($C_1$-$C_7$)alkylthiocarbonyle ; un ($C_3$-$C_7$)cycloalkylcarbonyle; un ($C_3$-$C_7$) cycloalkylthiocarbonyle ; un benzoyle non substitué ou substitué une ou plusieurs fois par un halogène, un ($C_1$-$C_7$)alkyle, un hydroxy, un ($C_1$-$C_7$)alcoxy, un benzyloxy, un acétoxy ; un phénylacétyle dans lequel le noyau benzénique est non substitué ou substitué une ou plusieurs fois par un halogène, un ($C_1$-$C_7$)alkyle, un hydroxy, un ($C_1$-$C_7$)alcoxy, un benzyloxy, un acétoxy ; un pyridylcarbonyle ; un thiénylcarbonyle ; un furylcarbonyle ; un pipérid-4-ylcarbonyle non substitué ou substitué en 1 par un ($C_1$-$C_7$)alkyle ou par un groupe protecteur ; un ($C_1$-$C_7$)alcoxycarbonyle ; un phénoxycarbonyle ; un phénoxythiocarbonyle ; un benzyloxycarbonyle ; un carbamoyle substitué par $R_{22}$ et $R_{23}$ ; un thiocarbamoyle substitué par $R_{22}$ et $R_{23}$ ; un groupe $CH_2R_{36}$ ; un ω-$R_{24}$ ($C_1$-$C_6$)alkylcarbonyle; un ω-$R_{32}R_{33}N$($C_1$-$C_4$)alkylcarbonyle ;
- $R_{12}$ représente un morpholin-4-yle ; un thiomorpholin-4-yle ; un azétidin-1-yle non substitué ou substitué en 2 par un carboxy ou substitué en 3 par un amino libre ou portant un groupe protecteur ; une perhydroazépin-1-yle ; une pipérazin-1-yle non substituée ou substituée en 4 par $R_{25}$ ; un pipérid-1-yle non substitué ou substitué par $R_{26}$ ; une pyrrolidin-1-yle non substituée ou substituée par $R_{27}$ ; une thiazolidin-3-yle non substituée ou substituée par $R_{27}$ ;
- $R_{13}$ représente un hydrogène ; un ($C_1$-$C_7$)alkyle ; un benzyle ; un allyle ; un tétrahydropyran-2-yle ;
- $R_{14}$ et $R_{15}$ représentent chacun indépendamment l'hydrogène ; un ($C_1$-$C_7$)alkyle ; $R_{15}$ peut de plus représenter un ($C_1$-$C_7$)alkylène substitué par $R_{24}$, un cyano, un trifluorométhyle, un amino libre ou substitué par un ou deux ($C_1$-$C_7$)alkyles ; un ($C_3$-$C_7$)cycloalkyle ; un phényle non substitué ou substitué une ou plusieurs fois par un halogène, un ($C_1$-$C_7$)alkyle, un hydroxy, un ($C_1$-$C_7$)alcoxy, un benzyloxy ; un groupe $R_{28}$ ;
- ou $R_{14}$ et $R_{15}$ ensemble avec l'atome d'azote auquel ils sont liés constituent un radical hétérocyclique $R_{12}$ ;
- $R_{16}$ et $R_{17}$ représentent chacun indépendamment un hydrogène, un ($C_1$-$C_7$)alkyle ; $R_{17}$ peut de plus représenter un ($C_3$-$C_7$)cycloalkyle ou un groupe $R_{28}$ ;
- ou $R_{16}$ et $R_{17}$ ensemble avec l'atome d'azote auquel ils sont liés constituent un radical hétérocyclique $R_{12}$ ;
- $R_{18}$ représente un ($C_1$-$C_7$)alkyle ; un phényle ; un benzyle ; un cyclohexylméthyle ; un phénéthyle ; un imidazol-4-ylméthyle libre ou portant un groupe protecteur ; un indol-3-ylméthyle libre ou portant un groupe protecteur ; un hydroxyméthyle libre ou portant un groupe protecteur ; un 2-hydroxyéthyle libre ou portant un groupe protecteur ; un 1-hydroxyéthyle libre ou portant un groupe protecteur ; un 4-hydroxybenzyle libre ou portant un groupe protecteur ; un mercaptométhyle libre ou portant un groupe protecteur ; un 2-mercaptoéthyle libre ou portant un groupe protecteur ; un 2-méthylthioéthyle ; un 2-méthylsulfinyléthyle ; un 2-méthylsulfonyléthyle ; un 4-aminobutyle libre ou portant un groupe protecteur ; un 3-aminopropyle libre ou portant un groupe protecteur ; un carboxyméthyle libre ou portant un groupe protecteur ; un 2-carboxyéthyle libre ou portant un groupe protecteur ; un carbamoylméthyle ; un 2-carbamoyléthyle ; un 3-guanidinopropyle libre ou portant un groupe protecteur ; un radical carbocyclique non aromatique en $C_3$-$C_{15}$ ;
- $R_{19}$ représente l'hydrogène ; $R_{19}$ peut de plus représenter un ($C_1$-$C_7$)alkyle lorsque $R_{18}$ représente un ($C_1$-$C_7$) alkyle ;
- ou $R_{18}$ et $R_{19}$ ensemble avec l'atome de carbone auquel ils sont liés constituent un radical carbocyclique non aromatique en $C_3$-$C_{15}$ ;
- $R_{20}$ représente $R_{24}$ ; un groupe -$CH_2OR_{13}$ ; un aminométhyle dans lequel l'amino est libre ou substitué par un ou deux ($C_1$-$C_7$)alkyles ou par un groupe protecteur ;
- $R_{21}$ représente $R_{36}$ ; un groupe $OR_{37}$ ; un groupe $NR_{32}R_{33}$ ; un cyano ; un groupe -S($C_1$-$C_7$)alkyle ; un groupe -SO($C_1$-$C_7$)alkyle ; un groupe -$SO_2$($C_1$-$C_7$)alkyle ;
- $R_{22}$ et $R_{23}$ représentent chacun indépendamment un hydrogène ; un ($C_1$-$C_7$)alkyle ; et $R_{23}$ peut de plus représenter un ($C_3$-$C_7$)cycloalkyle ; un groupe $CR_{18}R_{19}R_{20}$ ; un groupe $CH_2R_{24}$ ; un groupe $C(CH_3)(CH_2OH)_2$ ou $C(CH_2OH)_3$ ; un ($C_2$-$C_6$)alkylène substitué par $R_{29}$ ;
- ou $R_{22}$ et $R_{23}$ ensemble avec l'atome d'azote auquel ils sont liés constituent un radical hétérocyclique $R_{12}$ ou une cis-2,6-diméthylpipérid-1-yle ;
- $R_{24}$ représente un carboxy ; un ($C_1$-$C_7$)alcoxycarbonyle ; un benzyloxycarbonyle ; un carbamoyle libre ou substitué par un ou deux ($C_1$-$C_7$)alkyles ;

- $R_{25}$ représente un ($C_1$-$C_7$)alkyle ; un phényle ; un benzyle ; un formyle ; un ($C_1$-$C_7$)alkylcarbonyle ; un ($C_1$-$C_7$) alcoxycarbonyle ; un benzyloxycarbonyle ;
- $R_{26}$ représente $R_{24}$ ; un amino libre ou substitué par un ou deux ($C_1$-$C_7$)alkyles ou par un groupe protecteur ; un groupe $OR_{13}$ ; un groupe $CH_2OR_{13}$;
- $R_{27}$ représente $R_{24}$, un groupe $CH_2R_{24}$, un groupe $CH_2OR_{13}$, un aminométhyle dans lequel l'amino est libre ou substitué par un ou deux ($C_1$-$C_7$)alkyles ou par un groupe protecteur ;
- $R_{28}$ représente un groupe $CH(CH_2OH)_2$ ou $CH(CH_3)CH_2OH$ ou $C(CH_3)(CH_2OH)_2$ ou $C(CH_3)_2CH_2OH$ ou $C(CH_2OH)_3$ ou $CH_2CH_2OH$ ;
- $R_{29}$ représente un groupe $R_{24}$, un groupe $OR_{13}$, un groupe $NR_{30}R_{31}$ ;
- $R_{30}$ et $R_{31}$ représentent chacun indépendamment un hydrogène, un ($C_1$-$C_7$)alkyle;
- ou $R_{30}$ et $R_{31}$ ensemble avec l'atome d'azote auquel ils sont liés constituent un radical hététocyclique $R_{12}$ ;
- $R_{32}$ et $R_{33}$ représentent chacun indépendamment un hydrogène ; un ($C_1$-$C_7$)alkyle ; et $R_{33}$ peut de plus représenter un acétyle ; un phényle ; un benzyle ; un ($C_1$-$C_7$)alcoxycarbonyle ; un benzyloxycarbonyle ; un ($C_1$-$C_6$)alkylène substitué par $R_{24}$ ; un ($C_2$-$C_6$)alkylène substitué par un hydroxy ou un ($C_1$-$C_7$)alcoxy ; un ($C_2$-$C_6$)alkylène substitué par un amino libre ou substitué par un ou deux ($C_1$-$C_7$)alkyles ou par un groupe protecteur ;
- ou $R_{32}$ et $R_{33}$ ensemble avec l'atome d'azote auquel ils sont liés constituent un radical hétérocyclique $R_{12}$ ;
- $R_{34}$ représente un formyle ; un ($C_1$-$C_7$)alkylcarbonyle ; un ($C_1$-$C_7$)alcoxycarbonyle ; un phénoxycarbonyle ; un carbamoyle substitué par deux ($C_1$-$C_7$)alkyles ; un groupe $COR_{12}$ ;
- $R_{35}$ représente un pipérid-4-yle non substitué ou substitué en position 1 par un ($C_1$-$C_7$)alcoxycarbonyle ou par un ($C_1$-$C_7$)alkyle ; un pyrid-2-yle ;
- $R_{36}$ représente un carboxy ; un ($C_1$-$C_7$)alcoxycarbonyle ; un benzyloxycarbonyle ; un carbamoyle libre ou substitué par $R_{38}$ et $R_{39}$ ;
- $R_{37}$ représente $R_{13}$ ; un ($C_3$-$C_7$)cycloalkyle ; un ($C_1$-$C_6$)alkylène substitué par $R_{24}$ ; un ($C_2$-$C_6$)alkylène substitué par un hydroxy ou un ($C_1$-$C_7$)alcoxy ; un ($C_2$-$C_6$)alkylène substitué par un amino libre ou substitué par un ou deux ($C_1$-$C_7$)alkyles ou par un groupe protecteur;
- $R_{38}$ et $R_{39}$ représentent chacun indépendamment un hydrogène ; un ($C_1$-$C_7$)alkyle ; $R_{39}$ peut de plus représenter un ($C_1$-$C_6$)alkylène substitué par $R_{24}$ ; un ($C_2$-$C_6$)alkylène substitué par un hydroxy ou un ($C_1$-$C_7$) alcoxy ; un ($C_2$-$C_6$)alkylène substitué par un amino libre ou substitué par un ou deux ($C_1$-$C_7$)alkyles ou par un groupe protecteur .
- X représente $SO_2$ ; $CH_2$ ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi : halogène ; ($C_1$-$C_7$) alkyle ; ($C_1$-$C_7$)alcoxy ;
- p représente un nombre entier qui peut varier de 0 à 3 ; ainsi que ses sels.

**2.** Un composé selon la revendication 1 de formule :

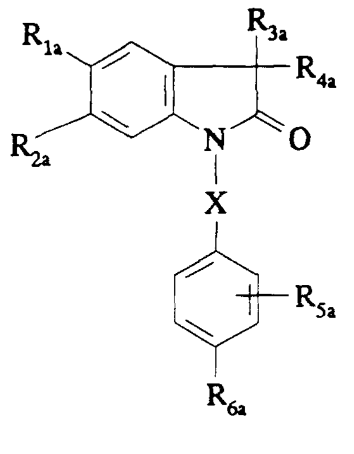

(Ia)

dans laquelle :

- $R_{1a}$ représente un éthoxy ou un chlore ;
- $R_{2a}$ représente l'hydrogène, un chlore ou un groupe méthyle ;

- $R_{3a}$ représente un chlorophényle, un méthoxyphényle ou un cyclohexyle ;
- $R_{4a}$ représente un amino ; un $(C_1\text{-}C_{12})$alkylamino ; un radical pipérazin-1-yle substitué en position 4 par un groupe $(C_2\text{-}C_{10})$alkylène substitué par un amino ; un groupe $N(R_9)(CH_2)_pR_{35}$ ; un groupe $N(R_9)(C_2\text{-}C_{10})$alkyl-$R_{21}$ ; un groupe $N(R_9)CO(C_1\text{-}C_4)$alkyl-$NR_{32}R_{33}$ ;
- $R_{5a}$ représente l'hydrogène ou un 2-méthoxy ;
- $R_{6a}$ représente un benzamido dans lequel le phényle est non substitué ou substitué par un méthoxy ; un groupe $-CONR_{14}R_{15}$ ; un groupe N',N'-di$(C_1\text{-}C_7)$alkyluréido ;
- ou $R_{5a}$ et $R_{6a}$ ensemble avec le phényle auquel ils sont liés constituent un groupe:

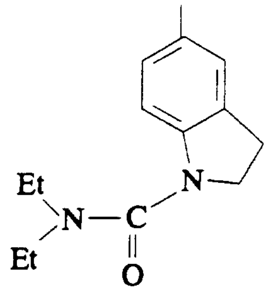

à la condition que X soit $SO_2$ ;

- les substituants X, $R_9$, $R_{14}$, $R_{15}$, $R_{21}$, $R_{32}$, $R_{33}$, $R_{35}$ étant tels que définis pour le composé de formule (I) dans la revendication 1 ; ainsi que ses sels.

**3.** Procédé pour la préparation d'un composé selon l'une quelconque des revendications 1 ou 2 ou d'un de ses sels, **caractérisé en ce que** :

1) on fait agir sur un composé de formule :

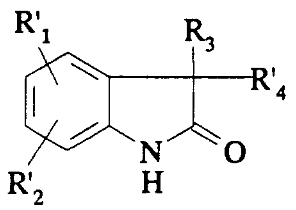

(II)

dans laquelle R'$_1$, R'$_2$ et R'$_4$ représentent, respectivement, soit $R_1$, $R_2$ et $R_4$ tels que définis pour (I) dans la revendication 1, soit des groupes précurseurs de $R_1$, $R_2$ et $R_4$, et $R_3$ est tel que défini pour (I) dans la revendication 1, un halogénure de formule :

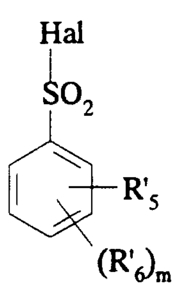

(III)

ou

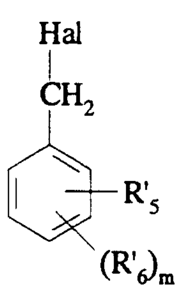

(IV)

dans laquelle Hal représente un atome d'halogène, et R'$_5$ et R'$_6$ représentent, respectivement, soit $R_5$ et $R_6$ tels que définis pour (I) dans la revendication 1, soit des groupes précurseurs de $R_5$ et $R_6$ ; et,

2) soit, lorsque $R'_1 = R_1$, $R'_2 = R_2$, $R'_4 = R_4$, $R'_5 = R_5$ et $R'_6 = R_6$, on isole le composé de formule (I) ainsi obtenu ;
3) soit, lorsque l'un quelconque des groupes $R'_1$, $R'_2$, $R'_4$, $R'_5$ et/ou $R'_6$ représente respectivement un groupe précurseur de $R_1$, $R_2$, $R_4$, $R_5$ et/ou $R_6$, on soumet le composé obtenu à l'étape 1) à un traitement ultérieur pour préparer le composé de formule (I) par transformation de l'un quelconque des groupes $R'_1$, $R'_2$, $R'_4$, $R'_5$ et/ou $R'_6$ en, respectivement, $R_1$, $R_2$, $R_4$, $R_5$ et/ou $R_6$ ; et
4) éventuellement, on transforme le composé obtenu à l'étape 2) ou à l'étape 3) en l'un de ses sels.

**4.** Composition pharmaceutique contenant à titre de principe actif, un composé selon l'une quelconque des revendications 1 ou 2, ou un de ses sels pharmaceutiquement acceptables.

**5.** Composition pharmaceutique contenant un composé selon l'une quelconque des revendications 1 ou 2, ou un de ses sels pharmaceutiquement acceptables en association avec un autre principe actif.

**6.** Composition pharmaceutique contenant deux composés selon l'une quelconque des revendications 1 ou 2, l'un étant un antagoniste spécifique du récepteur $V_1$ et l'autre étant un antagoniste spécifique du récepteur $V_2$.

**7.** Composition pharmaceutique contenant deux composés selon l'une quelconque des revendications 1 ou 2, l'un étant un antagoniste spécifique du récepteur $V_1$ et l'autre étant un antagoniste spécifique de l'ocytocine.

**Patentansprüche**

**1.** Verbindung der Formel:

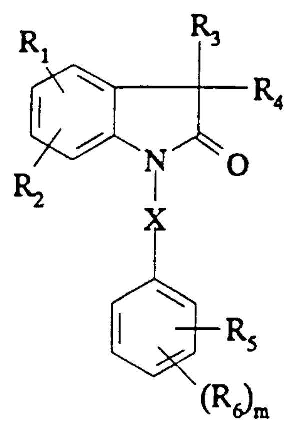

(I)

worin:

- $R_1$ und $R_2$ jeweils unabhängig voneinander Wasserstoff; Halogen; $(C_1-C_7)$-Alkyl; $(C_1-C_7)$-Alkoxy, Trifluormethyl darstellen;
- $R_3$ $(C_1-C_7)$-Alkyl; $(C_3-C_7)$-Cycloalkyl; durch ein oder zwei $(C_1-C_7)$-Alkyle substituiertes Cyclohexyl; Cyclohexylmethyl; gegebenenfalls ein- oder mehrmals durch ein Halogen, $(C_1-C_7)$-Alkyl, Hydroxy, $(C_1-C_7)$-Alkoxy, Benzyloxy, Acetoxy substituiertes Phenyl; gegebenenfalls ein- oder mehrmals durch ein Halogen, $(C_1-C_7)$ -Alkyl, Hydroxy, $(C_1-C_7)$-Alkoxy, Benzyloxy, Acetoxy substituiertes Benzyl darstellt;
- $R_4$ Azido; 2,2-Dimethylhydrazino; $(C_1-C_7)$-Alkylsulfonamido; Phenylsulfonamido, in dem das Phenyl gegebenenfalls ein- oder mehrmals durch ein Halogen, $(C_1-C_7)$-Alkyl, Hydroxy, $(C_1-C_7)$-Alkoxy, Benzyloxy, Acetoxy substituiert ist; Dimethylaminosulfonamido; eine Gruppe $NR_7R_8$; eine Gruppe $NR_9R_{10}$; eine Gruppe $NR_9R_{11}$; einen heterocyclischen Rest $R_{12}$; in Position 4 durch durch eine freie Aminogruppe substituiertes oder eine Schutzgruppe tragendes $(C_2-C_{10})$-Alkylen substituiertes Piperazin-1-yl; Lactylamino; Mandelylamino; N'-(1-Phenylethyl)ureido; N'-(1-Naphth-1-ylethyl)ureido darstellt;
- $R_5$ Wasserstoff oder einen der für $R_6$ bezeichneten Werte darstellt;
- $R_6$ ein Halogen; $(C_1-C_7)$-Alkyl; Trifluormethyl; Cyano; Aminomethyl, in dem das Amino frei oder durch ein oder zwei $(C_1-C_7)$-Alkyle substituiert ist; Nitro; eine Gruppe $NR_9R_{11}$; einen heterocyclischen Rest ausgewählt aus Pyrrol-1-yl, Δ3-Pyrrolin-1-yl, Pyrrolidin-1-yl, Morpholin-4-yl; eine Gruppe $OR_{13}$; eine Gruppe $SR_{13}$; gegebe-

nenfalls in 3 durch ein oder zwei ($C_1$-$C_7$)-Alkyle, Phenyl, Benzyl substituiertes Guanidino; Formyl; ($C_1$-$C_7$)-Alkylcarbonyl; durch $R_{14}$ und $R_{15}$ substituiertes Carbamoyl; freies oder durch ein oder zwei ($C_1$-$C_7$)-Alkyle substituiertes Thiocarbamoyl; durch $R_{16}$ und $R_{17}$ substituiertes Sulfamoyl; Carboxy; ($C_1$-$C_7$)-Alkoxycarbonyl; Phenoxycarbonyl; Benzyloxycarbonyl; ($C_1$-$C_7$)-Alkylsulfonamido; Phenylsulfonamido, in dem das Phenyl gegebenenfalls ein- oder mehrmals durch ein Halogen, ($C_1$-$C_7$)-Alkyl, Hydroxy, ($C_1$-$C_7$)-Alkoxy, Benzyloxy, Acetoxy substituiert ist; Dimethylaminosulfonamido; 2-(4-Methylphenyl)benzamido darstellt;

- oder $R_5$ und $R_6$ zusammen mit dem Phenyl, an das sie gebunden sind, eine Gruppe:

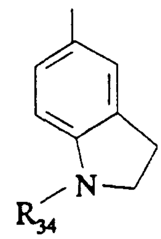

darstellen, mit der Maßgabe, das X -$SO_2$- ist;

- $R_7$ ($C_1$-$C_7$)-Alkoxycarbonyl darstellt;
- $R_8$ ($C_1$-$C_7$)-Alkoxycarbonylamino; N-Methyl-N-($C_1$-$C_7$)-alkoxycarbonylamino darstellt;
- $R_9$ Wasserstoff; ($C_1$-$C_7$)-Alkyl darstellt;
- $R_{10}$ eine Gruppe $CR_{18}R_{19}R_{20}$; eine Gruppe $(CH_2)_pR_{35}$; durch $R_{21}$ substituiertes ($C_2$-$C_{10}$)-Alkylen; eine Gruppe $CH_2CN$; eine Gruppe $C(CH_3)(CH_2OH)_2$ oder $C(CH_2OH)_3$; einen nicht aromatischen $C_3$-$C_{15}$-carbocyclischen Rest; gegebenenfalls ein- oder mehrmals durch ein Halogen, ($C_1$-$C_7$)-Alkyl, Hydroxy, ($C_1$-$C_7$)-Alkoxy, Benzyloxy, Acetoxy, Nitro, freies oder durch ein oder zwei ($C_1$-$C_7$)-Alkyle substituiertes Amino substituiertes Phenyl; Benzyl, in dem das Phenyl gegebenenfalls ein- oder mehrmals durch ein Halogen, ($C_1$-$C_7$)-Alkyl, Hydroxy, ($C_1$-$C_7$)-Alkoxy, Benzyloxy, Acetoxy substituiert ist; Nitro; freies oder durch ein oder zwei ($C_1$-$C_7$)-Alkyle substituiertes Amino; Phenethyl, in dem das Phenyl gegebenenfalls ein- oder mehrmals durch ein Halogen, ($C_1$-$C_7$)-Alkyl, Hydroxy, ($C_1$-$C_7$)-Alkoxy, Benzyloxy, Acetoxy, Nitro, freies oder durch ein oder zwei ($C_1$-$C_7$)-Alkyle substituiertes Amino substituiert ist; Phenethyl, in dem das Phenyl durch 3,4-Methylendioxy oder 3,4-Ethylendioxy substituiert ist, darstellt;
- $R_{11}$ Wasserstoff; ($C_1$-$C_{12}$)-Alkyl; ($C_3$-$C_7$)-Cycloalkylmethyl; eine Gruppe $OR_{13}$; Formyl; ($C_1$-$C_7$)-Alkylcarbonyl; ($C_1$-$C_7$)-Alkylthiocarbonyl; ($C_3$-$C_7$)-Cycloalkylcarbonyl; ($C_3$-$C_7$)-Cyclo-alkylthiocarbonyl; gegebenenfalls ein- oder mehrmals durch ein Halogen, ($C_1$-$C_7$)-Alkyl, Hydroxy, ($C_1$-$C_7$)-Alkoxy, Benzyloxy, Acetoxy substituiertes Benzoyl; Phenylacetyl, in dem der Benzolkern gegebenenfalls ein- oder mehrmals durch ein Halogen, ($C_1$-$C_7$)-Alkyl, Hydroxy, ($C_1$-$C_7$)-Alkoxy, Benzyloxy, Acetoxy substituiert ist; Pyridylcarbonyl; Thienylcarbonyl; Furylcarbonyl; gegebenenfalls in 1 durch ($C_1$-$C_7$)-Alkyl oder eine Schutzgruppe substituiertes Piperid-4-ylcarbonyl; ($C_1$-$C_7$)-Alkoxycarbonyl; Phenoxycarbonyl; Phenoxythiocarbonyl; Benzyloxycarbonyl; durch $R_{22}$ und $R_{23}$ substituiertes Carbamoyl; durch $R_{22}$ und $R_{23}$ substituiertes Thiocarbamoyl; eine Gruppe $CH_2R_{36}$; ω-$R_{24}$-($C_1$-$C_6$)-Alkylcarbonyl; ω-$R_{32}R_{33}$N-($C_1$-$C_4$)-Alkylcarbonyl darstellt;
- $R_{12}$ Morpholin-4-yl; Thiomorpholin-4-yl; gegebenenfalls in 2 durch ein Carboxy substituiertes oder in 3 durch ein freies oder eine Schutzgruppe tragendes Amino substituiertes Azetidin-1-yl; Perhydroazepin-1-yl; gegebenenfalls in 4 durch $R_{25}$ substituiertes Piperazin-1-yl; gegebenenfalls durch $R_{26}$ substituiertes Piperid-1-yl; gegebenenfalls durch $R_{27}$ substituiertes Pyrrolidin-1-yl; gegebenenfalls durch $R_{27}$ substituiertes Thiazolidin-3-yl darstellt;
- $R_{13}$ Wasserstoff; ($C_1$-$C_7$)-Alkyl; Benzyl, Allyl; Tetrahydropyran-2-yl darstellt;
- $R_{14}$ und $R_{15}$ jeweils unabhängig voneinander Wasserstoff; ($C_1$-$C_7$)-Alkyl darstellen; $R_{15}$ weiters durch $R_{24}$, Cyano, Trifluormethyl, freies oder durch ein oder zwei ($C_1$-$C_7$)-Alkyle substituiertes Amino substituiertes ($C_1$-$C_7$)-Alkylen; ($C_3$-$C_7$)-Cycloalkyl; gegebenenfalls ein- oder mehrmals durch ein Halogen, ($C_1$-$C_7$)-Alkyl, Hydroxy, ($C_1$-$C_7$)-Alkoxy, Benzyloxy substituiertes Phenyl; eine Gruppe $R_{28}$ darstellen kann;
- oder $R_{14}$ und $R_{15}$ zusammen mit dem Stickstoffatom, an das sie gebunden sind, einen heterocyclischen Rest $R_{12}$ darstellen;
- $R_{16}$ und $R_{17}$ jeweils unabhängig voneinander Wasserstoff; ($C_1$-$C_7$)-Alkyl darstellen; $R_{17}$ weiters ($C_3$-$C_7$)-Cycloalkyl oder eine Gruppe $R_{28}$ darstellen kann;
- oder $R_{16}$ und $R_{17}$ zusammen mit dem Stickstoffatom, an das sie gebunden sind, einen heterocyclischen Rest $R_{12}$ darstellen;
- $R_{18}$ ($C_1$-$C_7$)-Alkyl; Phenyl; Benzyl; Cyclohexylmethyl; Phenethyl; freies oder eine Schutzgruppe tragendes

Imidazol-4-ylmethyl; freies oder eine Schutzgruppe tragendes Indol-3-ylmethyl; freies oder eine Schutzgruppe tragendes Hydroxymethyl; freies oder eine Schutzgruppe tragendes 2-Hydroxyethyl; freies oder eine Schutzgruppe tragendes 1-Hydroxyethyl; freies oder eine Schutzgruppe tragendes 4-Hydroxybenzyl; freies oder eine Schutzgruppe tragendes Mercaptomethyl; freies oder eine Schutzgruppe tragendes 2-Mercaptoethyl; 2-Methylthioethyl; 2-Methylsulfinylethyl; 2-Methylsulfonylethyl; freies oder eine Schutzgruppe tragendes 4-Aminobutyl; freies oder eine Schutzgruppe tragendes 3-Aminopropyl; freies oder eine Schutzgruppe tragendes Carboxymethyl; freies oder eine Schutzgruppe tragendes 2-Carboxyethyl; Carbamoylmethyl; 2-Carbamoylethyl; freies oder eine Schutzgruppe tragendes 3-Guanidinopropyl; einen nicht aromatischen ($C_3$-$C_{15}$)-carbocyclischen Rest darstellt;

- $R_{19}$ Wasserstoff darstellt; $R_{19}$ weiters ($C_1$-$C_7$)-Alkyl darstellen kann, wenn $R_{18}$ ($C_1$-$C_7$)-Alkyl darstellt;
- oder $R_{18}$ und $R_{19}$ zusammen mit dem Kohlenstoffatom, an das sie gebunden sind, einen nicht aromatischen ($C_3$-$C_{15}$)-carbocyclischen Rest darstellen;
- $R_{20}$ für $R_{24}$; eine Gruppe $-CH_2OR_{13}$; Aminomethyl, in dem das Amino frei oder durch ein oder zwei ($C_1$-$C_7$)-Alkyle oder durch eine Schutzgruppe substituiert ist, steht;
- $R_{21}$ für $R_{36}$; eine Gruppe $OR_{37}$; eine Gruppe $NR_{32}R_{33}$; Cyano, eine Gruppe -S($C_1$-$C_7$)-Alkyl; eine Gruppe -SO($C_1$-$C_7$)-Alkyl; eine Gruppe $-SO_2$($C_1$-$C_7$)-Alkyl steht;
- $R_{22}$ und $R_{23}$ jeweils unabhängig voneinander Wasserstoff; ($C_1$-$C_7$)-Alkyl darstellen; und $R_{23}$ weiters ($C_3$-$C_7$)-Cycloalkyl; eine Gruppe $CR_{18}R_{19}R_{20}$; eine Gruppe $CH_2R_{24}$; eine Gruppe $C(CH_3)(CH_2OH)_2$ oder $C(CH_2OH)_3$; durch $R_{29}$ substituiertes ($C_2$-$C_6$)-Alkylen darstellen kann;
- oder $R_{22}$ und $R_{23}$ zusammen mit dem Stickstoffatom, an das sie gebunden sind, einen heterocyclischen Rest $R_{12}$ oder cis-2,6-Dimethylpiperid-1-yl darstellen;
- $R_{24}$ Carboxy; ($C_1$-$C_7$)-Alkoxycarbonyl; Benzyloxycarbonyl; freies oder durch ein oder zwei ($C_1$-$C_7$)-Alkyle substituiertes Carbamoyl darstellt;
- $R_{25}$ ($C_1$-$C_7$)-Alkyl; Phenyl; Benzyl; Formyl; ($C_1$-$C_7$)-Alkylcarbonyl; ($C_1$-$C_7$)-Alkoxycarbonyl; Benzyloxycarbonyl darstellt;
- $R_{26}$ für $R_{24}$; freies oder durch ein oder zwei ($C_1$-$C_7$)-Alkyle oder durch eine Schutzgruppe substituiertes Amino; eine Gruppe $OR_{13}$; eine Gruppe $CH_2OR_{13}$ steht;
- $R_{27}$ für $R_{24}$; eine Gruppe $CH_2R_{24}$; eine Gruppe $CH_2OR_{13}$; Aminomethyl, in dem das Amino frei oder durch ein oder zwei ($C_1$-$C_7$)-Alkyle oder durch eine Schutzgruppe substituiert ist, steht;
- $R_{28}$ eine Gruppe $CH(CH_2OH)_2$ oder $CH(CH_3)CH_2OH$ oder $C(CH_3)(CH_2OH)_2$ oder $C(CH_3)_2CH_2OH$ oder $C(CH_2OH)_3$ oder $CH_2CH_2OH$ darstellt;
- $R_{29}$ eine Gruppe $R_{24}$; eine Gruppe $OR_{13}$; eine Gruppe $NR_{30}R_{31}$ darstellt;
- $R_{30}$ und $R_{31}$ jeweils unabhängig voneinander Wasserstoff; ($C_1$-$C_7$)-Alkyl darstellen;
- oder $R_{30}$ und $R_{31}$ zusammen mit dem Stickstoffatom, an das sie gebunden sind, einen heterocyclischen Rest $R_{12}$ darstellen;
- $R_{32}$ und $R_{33}$ jeweils unabhängig voneinander Wasserstoff; ($C_1$-$C_7$)-Alkyl darstellen; und $R_{33}$ weiters Acetyl; Phenyl; Benzyl; ($C_1$-$C_7$)-Alkoxycarbonyl; Benzyloxycarbonyl; durch $R_{24}$ substituiertes ($C_1$-$C_6$)-Alkylen; durch Hydroxy oder ($C_1$-$C_7$)-Alkoxy substituiertes ($C_2$-$C_6$)-Alkylen; durch ein freies oder durch ein oder zwei ($C_1$-$C_7$)-Alkyle oder eine Schutzgruppe substituiertes Amino substituiertes ($C_2$-$C_6$)-Alkylen darstellen kann;
- oder $R_{32}$ und $R_{33}$ zusammen mit dem Stickstoffatom, an das sie gebunden sind, einen heterocyclischen Rest $R_{12}$ darstellen;
- $R_{34}$ Formyl; ($C_1$-$C_7$)-Alkylcarbonyl; ($C_1$-$C_7$)-Alkoxycarbonyl; Phenoxycarbonyl; durch zwei ($C_1$-$C_7$)-Alkyle substituiertes Carbamoyl; eine Gruppe $COR_{12}$ darstellt;
- $R_{35}$ gegebenenfalls in Position 1 durch ($C_1$-$C_7$)-Alkoxycarbonyl oder ($C_1$-$C_7$)-Alkyl substituiertes Piperid-4-yl; Pyrid-2-yl darstellt;
- $R_{36}$ Carboxy; ($C_1$-$C_7$)-Alkoxycarbonyl; Benzyloxycarbonyl; freies oder durch $R_{38}$ und $R_{39}$ substituiertes Carbamoyl darstellt;
- $R_{37}$ für $R_{13}$; ($C_3$-$C_7$)-Cycloalkyl; durch $R_{24}$ substituiertes ($C_1$-$C_6$)-Alkylen; durch Hydroxy oder ($C_1$-$C_7$)-Alkoxy substituiertes ($C_2$-$C_6$)-Alkylen; durch ein freies oder durch ein oder zwei ($C_1$-$C_7$)-Alkyle oder eine Schutzgruppe substituiertes Amino substituiertes ($C_2$-$C_6$)-Alkylen steht;
- $R_{38}$ und $R_{39}$ jeweils unabhängig voneinander Wasserstoff; ($C_1$-$C_7$)-Alkyl darstellen; $R_{39}$ weiters durch $R_{24}$ substituiertes ($C_1$-$C_6$)-Alkylen; durch Hydroxy oder ($C_1$-$C_7$)-Alkoxy substituiertes ($C_2$-$C_6$)-Alkylen; durch ein freies oder durch ein oder zwei ($C_1$-$C_7$)-Alkyle oder eine Schutzgruppe substituiertes Amino substituiertes ($C_2$-$C_6$)-Alkylen darstellen kann;
- X $SO_2$; $CH_2$ darstellt;
- m für 1 steht oder, wenn $R_6$ Halogen, ($C_1$-$C_7$)-Alkyl oder ($C_1$-$C_7$)-Alkoxy ist, m auch 2, 3 oder 4 sein kann, oder aber $(R_6)_m$ m Substituenten mit verschiedenen Werten ausgewählt aus Halogen; ($C_1$-$C_7$)-Alkyl; ($C_1$-$C_7$)-Alkoxy darstellen kann;

- p eine ganze Zahl von 0 bis 3 darstellt; sowie die Salze derselben.

**2.** Verbindung nach Anspruch 1 der Formel:

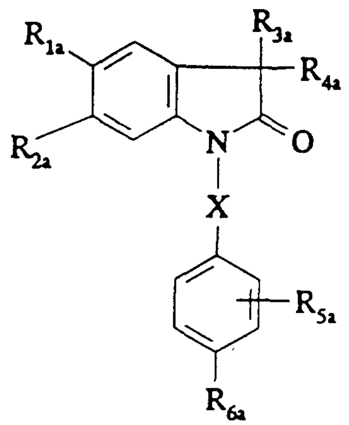

(Ia)

worin:

- $R_{1a}$ Ethoxy oder Chlor darstellt;
- $R_{2a}$ Wasserstoff, Chlor oder eine Methylgruppe darstellt;
- $R_{3a}$ Chlorphenyl, Methoxyphenyl oder Cyclohexyl darstellt;
- $R_{4a}$ Amino; ($C_1$-$C_{12}$)-Alkylamino; einen in Position 4 durch durch ein Amino substituiertes ($C_2$-$C_{10}$)-Alkylen substituierten Piperazin-1-yl-Rest; eine Gruppe $N(R_9)(CH_2)_pR_{35}$; eine Gruppe $N(R_9)(C_2$-$C_{10})$Alkyl-$R_{21}$; eine Gruppe $N(R_9)CO(C_1$-$C_4)$-Alkyl-$NR_{32}R_{33}$ darstellt;
- $R_{5a}$ Wasserstoff oder 2-Methoxy darstellt;
- $R_{6a}$ Benzamido, in dem das Phenyl gegebenenfalls durch Methoxy substituiert ist; eine Gruppe
- $CONR_{14}R_{15}$; eine Gruppe N',N'-Di($C_1$-$C_7$)-alkylureido darstellt;
- oder $R_{5a}$ und $R_{6a}$ zusammen mit dem Phenyl, an das sie gebunden sind, eine Gruppe:

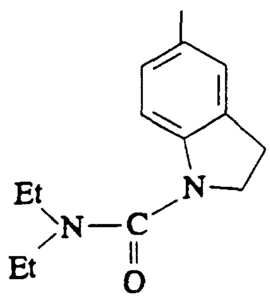

darstellen, mit der Maßgabe, dass X $SO_2$ ist;
- wobei die Substituenten X, $R_9$, $R_{14}$, $R_{15}$, $R_{21}$, $R_{32}$, $R_{33}$, $R_{35}$ wie für die Verbindung der Formel (I) in Anspruch 1 definiert sind; sowie die Salze derselben.

**3.** Verfahren zur Herstellung einer Verbindung nach einem der Ansprüche 1 oder 2 oder eines der Salze derselben, **dadurch gekennzeichnet, dass**:

1) eine Verbindung der Formel:

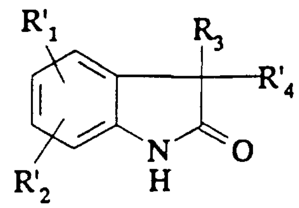

(II)

worin $R'_1$, $R'_2$ und $R'_4$ entweder $R_1$, $R_2$ bzw. $R_4$ wie für (I) in Anspruch 1 definiert oder Vorläufergruppen von $R_1$, $R_2$ bzw. $R_4$ darstellen und $R_3$ wie für (I) in Anspruch 1 definiert ist, mit einem Halogenid der Formel:

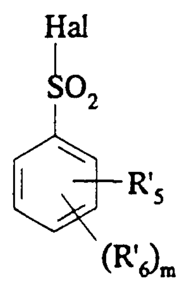

(III)

oder

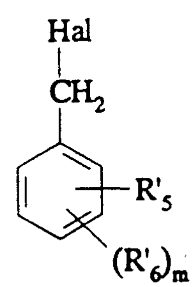

(IV)

worin Hal ein Halogenatom darstellt, und $R'_5$ und $R'_6$ entweder $R_5$ bzw. $R_6$ wie für (I) in Anspruch 1 definiert oder Vorläufergruppen von $R_5$ bzw. $R_6$ darstellen; zur Umsetzung gebracht wird; und
2) entweder, wenn $R'_1 = R_1$, $R'_2 = R_2$, $R'_4 = R_4$, $R'_5 = R_5$ und $R'_6 = R_6$, die so erhaltene Verbindung der Formel (I) isoliert wird;
3) oder, wenn eine der Gruppen $R'_1$, $R'_2$, $R'_4$, $R'_5$ und/oder $R'_6$ eine Vorläufergruppe von $R_1$, $R_2$, $R_4$, $R_5$ bzw. $R_6$ darstellt, die in Schritt 1) erhaltene Verbindung 1) einer Nachbehandlung zur Herstellung der Verbindung der Formel (I) durch Überführen einer der Gruppen $R'_1$, $R'_2$, $R'_4$, $R'_5$ und/oder $R'_6$ in $R_1$, $R_2$, $R_4$, $R_5$ bzw. $R_6$ unterzogen wird; und
4) gegebenenfalls die in Schritt 2) oder in Schritt 3) erhaltene Verbindung in eines ihrer Salze übergeführt wird.

**4.** Pharmazeutische Zusammensetzung, die als Wirkstoff eine Verbindung nach einem der Ansprüche 1 oder 2 oder ein pharmazeutisch annehmbares Salz derselben enthält.

**5.** Pharmazeutische Zusammensetzung, die eine Verbindung nach einem der Ansprüche 1 oder 2 oder ein pharmazeutisch annehmbares Salz derselben in Kombination mit einem anderen Wirkstoff enthält.

**6.** Pharmazeutische Zusammensetzung, die zwei Verbindungen nach einem der Ansprüche 1 oder 2 enthält, wobei

die eine ein spezifischer Antagonist des $V_1$-Rezeptors und die andere ein spezifischer Antagonist des $V_2$-Rezeptors ist.

**7.** Pharmazeutische Zusammensetzung, die zwei Verbindungen nach einem der Ansprüche 1 oder 2 enthält, wobei die eine ein spezifischer Antagonist des $V_1$-Rezeptors und die andere ein spezifischer Antagonist von Oxytocin ist.

**Claims**

**1.** A compound of the formula

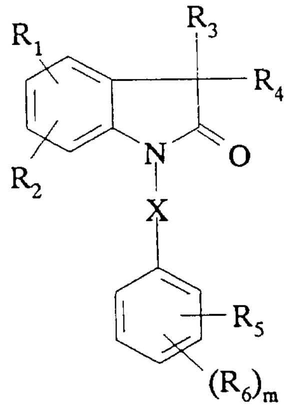

(I)

in which:

- $R_1$ and $R_2$ are each independently a hydrogen; a halogen; a $(C_1-C_7)$alkyl; a $(C_1-C_7)$alkoxy; or a trifluoromethyl;
- $R_3$ is a $(C_1-C_7)$alkyl; a $(C_3-C_7)$cycloalkyl; a cyclohexyl substituted by one or two $(C_1-C_4)$alkyls; a cyclohexylmethyl; a phenyl which is unsubstituted or monosubstituted or polysubstituted by a halogen, a $(C_1-C_7)$-alkyl, a hydroxyl, a $(C_1-C_7)$alkoxy, a benzyloxy or an acetoxy; or a benzyl which is unsubstituted or monosubstituted or polysubstituted by a halogen, a $(C_1-C_7)$-alkyl, a hydroxyl, a $(C_1-C_7)$alkoxy, a benzyloxy or an acetoxy;
- $R_4$ is an azido; a 2,2-dimethylhydrazino group; a $(C_1-C_7)$alkylsulfonamido; a phenylsulfonamido in which the phenyl is unsubstituted or monosubstituted or polysubstituted by a halogen, a $(C_1-C_7)$alkyl, a hydroxyl, a $(C_1-C_7)$alkoxy, a benzyloxy or an acetoxy; a dimethylaminosulfonamido; a group $NR_7R_8$; a group $NR_9R_{10}$; a group $NR_9R_{11}$; a heterocyclic radical $R_{12}$; a piperazin-1-yl substituted in the 4-position by a $(C_2-C_{10})$alkylene group substituted by an amino group which is free or carries a protective group; a lactoylamino group; a mandeloylamino group; an N'-(1-phenylethyl)ureido group; or an N'-(1-naphth-1-ylethyl)ureido group;
- $R_5$ is hydrogen or has one of the meanings given for $R_6$;
- $R_6$ is a halogen; a $(C_1-C_7)$alkyl; a trifluoromethyl; a cyano; an aminomethyl in which the amino is free or substituted by one or two $(C_1-C_7)$alkyls; a nitro; a group $NR_9R_{11}$; a heterocyclic radical selected from pyrrol-1-yl, Δ3-pyrrolin-1-yl, pyrrolidin-1-yl and morpholin-4-yl; a group $OR_{13}$; a group $SR_{13}$; a guanidino which is unsubstituted or substituted in the 3-position by one or two $(C_1-C_7)$alkyls, a phenyl or a benzyl; a formyl; a $(C_1-C_7)$alkylcarbonyl; a carbamoyl substituted by $R_{14}$ and $R_{15}$; a thiocarbamoyl which is free or substituted by one or two $(C_1-C_7)$alkyls; a sulfamoyl substituted by $R_{16}$ and $R_{17}$; a carboxyl; a $(C_1-C_7)$alkoxycarbonyl; a phenoxycarbonyl; a benzyloxycarbonyl; a $(C_1-C_7)$alkylsulfonamido; a phenylsulfonamido in which the phenyl is unsubstituted or monosubstituted or polysubstituted by a halogen, a $(C_1-C_7)$alkyl, a hydroxyl, a $(C_1-C_7)$alkoxy, a benzyloxy or an acetoxy; a dimethylaminosulfonamido; or a 2-(4-methylphenyl)benzamido;
- or $R_5$ and $R_6$, together with the phenyl to which they are bonded, form a group

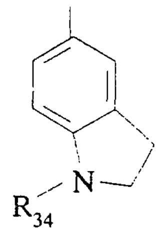

with the proviso that X is $-SO_2-$;

- $R_7$ is a $(C_1-C_7)$alkoxycarbonyl ;
- $R_8$ is a $(C_1-C_7)$alkoxycarbonylamino; or an N-methyl-N-$(C_1-C_7)$alkoxycarbonylamino;
- $R_9$ is a hydrogen; or a $(C_1-C_7)$alkyl;
- $R_{10}$ is a group $CR_{18}R_{19}R_{20}$; a group $(CH_2)_pR_{35}$; a $(C_2-C_{10})$alkylene substituted by $R_{21}$; a group $CH_2CN$; a group $C(CH_3)(CH_2OH)_2$ or $C(CH_2OH)_3$; a non-aromatic $C_3-C_{15}$ carbocyclic radical; a phenyl which is unsubstituted or monosubstituted or polysubstituted by a halogen, a $(C_1-C_7)$alkyl, a hydroxyl, a $(C_1-C_7)$alkoxy, a benzyloxy, an acetoxy, a nitro or an amino which is free or substituted by one or two $(C_1-C_7)$alkyls; a benzyl in which the phenyl is unsubstituted or monosubstituted or polysubstituted by a halogen, a $(C_1-C_7)$alkyl, a hydroxyl, a $(C_1-C_7)$alkoxy, a benzyloxy, an acetoxy, a nitro or an amino which is free or substituted by one or two $(C_1-C_7)$alkyls; a phenethyl in which the phenyl is unsubstituted or monosubstituted or polysubstituted by a halogen, a $(C_1-C_7)$alkyl, a hydroxyl, a $(C_1-C_7)$alkoxy, a benzyloxy, an acetoxy, a nitro or an amino which is free or substituted by one or two $(C_1-C_7)$alkyls; or a phenethyl in which the phenyl is substituted by a 3,4-methylenedioxy or a 3,4-ethylenedioxy;
- $R_{11}$ is a hydrogen; a $(C_1-C_{12})$alkyl; a $(C_3-C_7)$cycloalkylmethyl; a group $OR_{13}$; a formyl; a $(C_1-C_7)$alkylcarbonyl; a $(C_1-C_7)$alkylthiocarbonyl; a $(C_3-C_7)$cycloalkylcarbonyl; a $(C_3-C_7)$cycloalkylthiocarbonyl; a benzoyl which is unsubstituted or monosubstituted or polysubstituted by a halogen, a $(C_1-C_7)$alkyl, a hydroxyl, a $(C_1-C_7)$alkoxy, a benzyloxy or an acetoxy; a phenylacetyl in which the benzene ring is unsubstituted or monosubstituted or polysubstituted by a halogen, a $(C_1-C_7)$alkyl, a hydroxyl, a $(C_1-C_7)$alkoxy, a benzyloxy or an acetoxy; a pyridylcarbonyl; a thienylcarbonyl; a furylcarbonyl; a piperid-4-ylcarbonyl which is unsubstituted or substituted in the 1-position by a $(C_1-C_7)$-alkyl or by a protective group; a $(C_1-C_7)$alkoxycarbonyl; a phenoxycarbonyl; a phenoxythiocarbonyl; a benzyloxycarbonyl; a carbamoyl substituted by $R_{22}$ and $R_{23}$; a thiocarbamoyl substituted by $R_{22}$ and $R_{23}$; a group $CH_2R_{36}$; an ω-$R_{24}(C_1-C_6)$alkylcarbonyl; or an w-$R_{32}R_{33}N(C_1-C_4)$alkylcarbonyl ;
- $R_{12}$ is a morpholin-4-yl; a thiomorpholin-4-yl; an azetidin-1-yl which is unsubstituted or substituted in the 2-position by a carboxyl or substituted in the 3-position by an amino which is free or carries a protective group; a perhydroazepin-1-yl; a piperazin-1-yl which is unsubstituted or substituted in the 4-position by $R_{25}$; a piperid-1-yl which is unsubstituted or substituted by $R_{26}$; a pyrrolidin-1-yl which is unsubstituted or substituted by $R_{27}$; or a thiazolidin-3-yl which is unsubstituted or substituted by $R_{27}$;
- $R_{13}$ is a hydrogen; a $(C_1-C_7)$alkyl; a benzyl; an allyl; or a tetrahydropyran-2-yl;
- $R_{14}$ and $R_{15}$ are each independently hydrogen; or a $(C_1-C_7)$alkyl; $R_{15}$ can also be a $(C_1-C_7)$alkylene substituted by $R_{24}$, a cyano, a trifluoromethyl or an amino which is free or substituted by one or two $(C_1-C_7)$-alkyls; a $(C_3-C_7)$cycloalkyl; a phenyl which is unsubstituted or monosubstituted or polysubstituted by a halogen, a $(C_1-C_7)$alkyl, a hydroxyl, a $(C_1-C_7)$alkoxy or a benzyloxy; or a group $R_{28}$ ;
- or $R_{14}$ and $R_{15}$, together with the nitrogen atom to which they are bonded, form a heterocyclic radical $R_{12}$;
- $R_{16}$ and $R_{17}$ are each independently a hydrogen; or a $(C_1-C_7)$alkyl; $R_{17}$ can also be a $(C_3-C_7)$cycloalkyl; or a group $R_{28}$;
- or $R_{16}$ and $R_{17}$, together with the nitrogen atom to which they are bonded, form a heterocyclic radical $R_{12}$;
- $R_{18}$ is a $(C_1-C_7)$alkyl; a phenyl; a benzyl; a cyclohexylmethyl; a phenethyl; an imidazol-4-ylmethyl which is free or carries a protective group; an indol-3-yl-methyl which is free or carries a protective group; a hydroxymethyl which is free or carries a protective group; a 2-hydroxyethyl which is free or carries a protective group; a 1-hydroxyethyl which is free or carries a protective group; a 4-hydroxybenzyl which is free or carries a protective group; a mercaptomethyl which is free or carries a protective group; a 2-mercaptoethyl which is free or carries a protective group; a 2-methylthioethyl; a 2-methylsulfinylethyl; a 2-methylsulfonylethyl; a 4-aminobutyl which is free or carries a protective group; a 3-aminopropyl which is free or carries a protective group; a carboxymethyl which is free or carries a protective group; a 2-carboxyethyl which is free or carries a protective group; a carbamoylmethyl; a 2-carbamoylethyl; a 3-guanidinopropyl which is free or carries a protective group; or a non-aromatic $C_3-C_{15}$ carbocyclic radical;
- $R_{19}$ is hydrogen; $R_{19}$ can also be a $(C_1-C_7)$alkyl if $R_{18}$ is a $(C_1-C_7)$alkyl;

- or $R_{1a}$ and $R_{19}$, together with the carbon atom to which they are bonded, form a non-aromatic $C_3$-$C_{15}$ carbocyclic radical;
- $R_{20}$ is $R_{24}$; a group $CH_2OR_{13}$; or an aminomethyl in which the amino is free or substituted by one or two ($C_1$-$C_7$) alkyls or by a protective group;
- $R_{21}$ is $R_{36}$; a group $OR_{37}$; a group $NR_{32}R_{33}$; a cyano; a group S($C_1$-$C_7$)alkyl; a group SO($C_1$-$C_7$)alkyl; or a group $SO_2$($C_1$-$C_7$)alkyl;
- $R_{22}$ and $R_{23}$ are each independently a hydrogen; or a ($C_1$-$C_7$)alkyl; $R_{23}$ can also be a ($C_3$-$C_7$)cycloalkyl; a group $CR_{18}R_{19}R_{20}$; a group $CH_2R_{24}$; a group $C(CH_3)$-$(CH_2OH)_2$ or $C(CH_2OH)_3$; or a ($C_2$-$C_6$)alkylene substituted by $R_{29}$;
- or $R_{22}$ and $R_{23}$, together with the nitrogen atom to which they are bonded, form a heterocyclic radical $R_{12}$; or a cis-2,6-dimethylpiperid-1-yl;
- $R_{24}$ is a carboxyl; a ($C_1$-$C_7$)alkoxycarbonyl; a benzyloxycarbonyl; or a carbamoyl which is free or substituted by one or two ($C_1$-$C_7$)alkyls;
- $R_{25}$ is a ($C_1$-$C_7$)alkyl; a phenyl; a benzyl; a formyl; a ($C_1$-$C_7$)alkylcarbonyl; a ($C_1$-$C_7$)alkoxycarbonyl; or a benzyloxycarbonyl;
- $R_{26}$ is $R_{24}$; an amino which is free or substituted by one or two ($C_1$-$C_7$)alkyls or by a protective group; a group $OR_{13}$; or a group $CH_2OR_{13}$;
- $R_{27}$ is $R_{24}$; a group $CH_2R_{24}$; a group $CH_2OR_{13}$; or an aminomethyl in which the amino is free or substituted by one or two ($C_1$-$C_7$)alkyls or by a protective group;
- $R_{28}$ is a group $CH(CH_2OH)_2$, $CH(CH_3)CH_2OH$, $C(CH_3)$-$(CH_2OH)_2$, $C(CH_3)_2CH_2OH$, $C(CH_2OH)_3$ or $CH_2CH_2OH$;
- $R_{29}$ is a group $R_{24}$; a group $OR_{13}$; or a group $NR_{30}R_{31}$;
- $R_{30}$ and $R_{31}$ are each independently a hydrogen; or a ($C_1$-$C_7$)alkyl;
- or $R_{30}$ and $R_{31}$, together with the nitrogen atom to which they are bonded, form a heterocyclic radical $R_{12}$;
- $R_{32}$ and $R_{33}$ are each independently a hydrogen; or a ($C_1$-$C_7$)alkyl; $R_{33}$ can also be an acetyl; a phenyl; a benzyl; a ($C_1$-$C_7$)alkoxycarbonyl; a benzyloxycarbonyl; a ($C_1$-$C_6$)alkylene substituted by $R_{24}$; a ($C_2$-$C_6$)alkylene substituted by a hydroxyl or a ($C_1$-$C_7$)alkoxy; or a ($C_2$-$C_6$)alkylene substituted by an amino which is free or substituted by one or two ($C_1$-$C_7$)alkyls or by a protective group;
- or $R_{32}$ and $R_{33}$, together with the nitrogen atom to which they are bonded, form a heterocyclic radical $R_{12}$;
- $R_{34}$ is a formyl; a ($C_1$-$C_7$)alkylcarbonyl; a ($C_1$-$C_7$)-alkoxycarbonyl; a phenoxycarbonyl; a carbamoyl substituted by two ($C_1$-$C_7$)alkyls; or a group $COR_{12}$;
- $R_{35}$ is a piperid-4-yl which is unsubstituted or substituted in the 1-position by a ($C_1$-$C_7$)alkoxycarbonyl or by a ($C_1$-$C_7$)alkyl; or a pyrid-2-yl;
- $R_{36}$ is a carboxyl; a ($C_1$-$C_7$)alkoxycarbonyl; a benzyloxycarbonyl; or a carbamoyl which is free or substituted by $R_{38}$ and $R_{39}$;
- $R_{37}$ is $R_{13}$; a ($C_3$-$C_7$)cycloalkyl; a ($C_1$-$C_6$)alkylene substituted by $R_{24}$; a ($C_2$-$C_6$)alkylene substituted by a hydroxyl or a ($C_1$-$C_7$)alkoxy; or a ($C_2$-$C_6$)alkylene substituted by an amino which is free or substituted by one or two ($C_1$-$C_7$)alkyls or by a protective group;
- $R_{38}$ and $R_{39}$ are each independently a hydrogen; or a ($C_1$-$C_7$)alkyl; $R_{39}$ can also be a ($C_1$-$C_6$)alkylene substituted by $R_{24}$; a ($C_2$-$C_6$)alkylene substituted by a hydroxyl or a ($C_1$-$C_7$)alkoxy; or a ($C_2$-$C_6$)alkylene substituted by an amino which is free or substituted by one or two ($C_1$-$C_7$)alkyls or by a protective group;
- X is $SO_2$; or $CH_2$;
- m is 1 or, if $R_6$ is a halogen, a ($C_1$-$C_7$)alkyl or a ($C_1$-$C_7$)alkoxy, m can also be 2, 3 or 4, or else $(R_6)_m$ can be m substituents having different meanings selected from halogen; ($C_1$-$C_7$)alkyl; and ($C_1$-$C_7$)alkoxy; and
- p is an integer which can vary from 0 to 3; and its salts.

**2.** A compound according to claim 1 of the formula

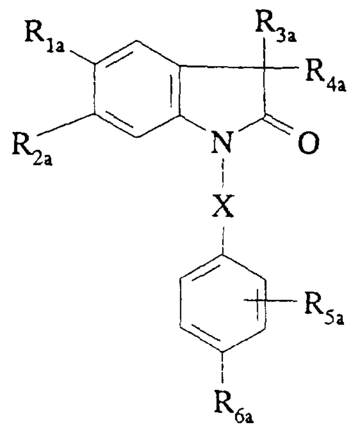

(Ia)

in which:

- $R_{1a}$ is an ethoxy or a chlorine;
- $R_{2a}$ is hydrogen, a chlorine or a methyl group;
- $R_{3a}$ is a chlorophenyl, a methoxyphenyl or a cyclohexyl;
- $R_{4a}$ is an amino; a $(C_1-C_{12})$alkylamino; a piperazin-1-yl radical substituted in the 4-position by a $(C_2-C_{10})$-alkylene group substituted by an amino; a group $N(R_9)-(CH_2)_pR_{35}$; a group $N(R_9)(C_2-C_{10})$alkyl-$R_{21}$; or a group $N(R_9)CO(C_1-C_4)$alkyl-$NR_{32}R_{33}$;
- $R_{5a}$ is hydrogen or a 2-methoxy;
- $R_{6a}$ is a benzamido in which the phenyl is unsubstituted or substituted by a methoxy; a group $CONR_{14}R_{15}$; or an N',N'-di$(C_1-C_7)$alkylureido group;
- or $R_{5a}$ and $R_{6a}$, together with the phenyl to which they are bonded, form a group

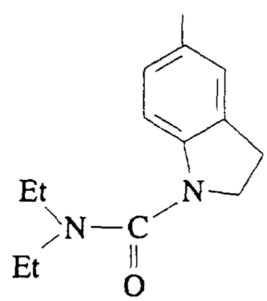

with the proviso that X is $SO_2$; and
- the substituents X, $R_9$, $R_{14}$, $R_{15}$, $R_{21}$, $R_{32}$, $R_{33}$ and $R_{35}$ are as defined for the compound of formula (I) in claim 1; and its salts.

**3.** A method of preparing a compound according to either one of claims 1 or 2, or one of its salts, which comprises:

1) reacting a halide of the formula

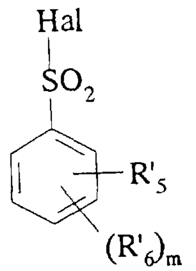

(III)

or

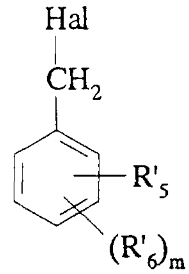

(IV)

in which Hal is a halogen atom and $R'_5$ and $R'_6$ are respectively either $R_5$ and $R_6$ as defined for (I) in claim 1, or precursor groups of $R_5$ and $R_6$, with a compound of the formula

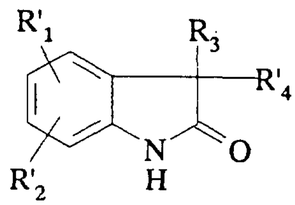

(II)

in which $R'_1$, $R'_2$ and $R'_4$ are respectively either $R_1$, $R_2$ and $R_4$ as defined for (I) in claim 1, or precursor groups of $R_1$, $R_2$ and $R_4$, and $R_3$ is as defined for (I) in claim 1; and
2) either, if $R'_1 = R_1$, $R'_2 = R_2$, $R'_4 = R_4$, $R'_5 = R_5$ and $R'_6 = R_6$, isolating the resulting compound of formula (I);
3) or, if any one of the groups $R'_1$, $R'_2$, $R'_4$, $R'_5$ and/or $R'_6$ is respectively a precursor group of $R_1$, $R_2$, $R_4$, $R_5$ and/or $R_6$, subjecting the compound obtained in step 1) to a subsequent treatment in order to prepare the compound of formula (I) by converting any one of the groups $R'_1$, $R'_2$, $R'_4$, $R'_5$ and/or $R'_6$ to $R_1$, $R_2$, $R_4$, $R_5$ and/or $R_6$ respectively; and
4) if appropriate, converting the compound obtained in step 2) or step 3) to one of its salts.

**4.** A pharmaceutical composition in which a compound according to either one of claims 1 or 2, or one of its pharmaceutically acceptable salts, is present as the active principle.

**5.** A pharmaceutical composition in which a compound according to either one of claims 1 or 2, or one of its pharmaceutically acceptable salts, is present in association with another active principle.

**6.** A pharmaceutical composition in which two compounds according to either one of claims 1 or 2 are present, one being a specific $V_1$ receptor antagonist and the other being a specific $V_2$ receptor antagonist.

**7.** A pharmaceutical composition in which two compounds according to either one of claims 1 or 2 are present, one being a specific $V_1$ receptor antagonist and the other being a specific ocytocin antagonist.